# EUROPEAN PATENT SPECIFICATION

(11) **EP 1 926 737 B1**
(45) Date of publication and mention of the grant of the patent: **15.03.2017**
(21) Application number: 06793204.6
(22) Date of filing: 05.09.2006
(51) Int. Cl.: C07D 495/04, A61K 31/435, A61P 35/00

(54) **4-AMINO-THIENO[3,2-C]PYRIDINE-7-CARBOXYLIC ACID DERIVATIVES**
4-AMINO-THIENO [3,2-C]PYRIDIN-7-CARBONSÄURE-DERIVATE
DÉRIVÉS D'ACIDE 4-AMINO-THIÉNO[3,2-C]PYRIDINE-7-CARBOXYLIQUE

(30) Priority: 15.09.2005 US 717271; 21.07.2006 US 832460
(43) Date of publication of application: 04.06.2008
(73) Proprietor: F. Hoffmann-La Roche AG, 4070 Basel (CH)
(72) Inventor: BARTKOVITZ, David Joseph, Nutley, New Jersey 07110 (US); CHEN, Yi, Nutley, New Jersey 07110 (US); CHU, Xin-Jie, Livingston, New Jersey 07039 (US); LUK, Kin-Chun, North Caldwell, New Jersey 07006 (US); ROSSMAN, Pamela Loreen, Nutley, New Jersey 07110 (US); SO, Sung-Sau, Nutley, New Jersey 07110 (US)
(74) Representative: Klein, Thomas
(86) International application number: PCT/EP2006/065986
(87) International publication number: WO 2007/031428

(56) References cited:
- WO-A-2005/056562
- US-A1- 2005 256 154

## Description

The ras protein in its normal, unmutated form is a key element of the signal transduction cascade directed by growth factor receptors in almost all tissues. See J. Avruch et al., TIBS (19), 279-283 (1994). Biochemically, ras is a guanine nucleotide binding protein, and cycling between a GTP-bound activated and a GDP-bound resting form is strictly controlled by ras endogenous GTPase activity and other regulatory proteins. In the ras mutants in cancer cells, the endogenous GTPase activity is activated and, therefore, the protein delivers constitutive growth signals to downstream effectors such as the enzyme raf kinase. This leads to the cancerous growth of the cells which carry these mutants, Magnuson et al., Cancer Biology, 5, 247-253 (1994). It has been shown that inhibiting the effect of active ras by inhibiting the raf kinase signaling pathway by administration of antisense RNA or by small molecule Raf kinase inhibitors leads to the reversion of transformed cells to the normal growth phenotype, Sridhar et al., Mol Cancer Ther 2005, 4(4), April 2005 and Bollag et al., Current Opinion in Investigational Drugs, 4, vol 12, 2003**.**

WO 2005/056562 relates to specific substituted 3-amino-thieno [2,3-b] pyridine-2-carboxylic acid amide compounds which are stated to be useful as inhibitors of the kinase activity of the IκB kinase (IKK) complex. The compounds are said to be useful in the treatment of IKK-mediated diseases including autoimmune diseases, inflammatory diseases and cancer.

The present invention provides 4-amino-thieno [3,2-c] pyridine-7-carboxylic acid derivatives which are small molecule inhibitors of Raf kinase. In addition to inhibiting Raf, these compounds may also inhibit some other important kinases including ABL, BRAF, BRAF(V600E) mutant, EPHA, EPHB, FGFR1, FGFR2, FGFR3, FLT3, FLT4, KIT, PDGFRA, PDGFRB, and VEFGR-2. These compounds can be potent and selective anticancer agents, especially agents for the treatment of colorectal, breast, lung, prostate, pancreatic, gastric, bladder, ovarian, melanoma, neuroblastoma, cervical, kidney or renal cancers, leukemias or lymphomas.

The present invention is defined in the appended claims. Herein disclosed is a compound of the formula (I-A) 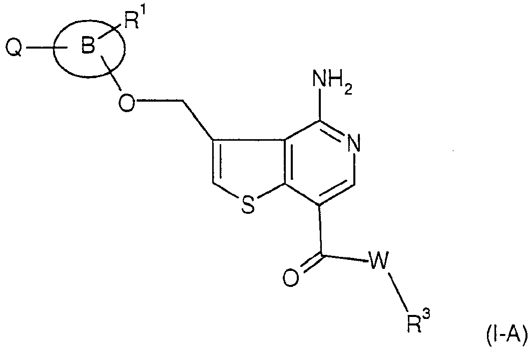 or pharmaceutically acceptable salts thereof
wherein
R¹ is selected from the group consisting of hydrogen, lower alkyl, halogen, cyano, trifluoromethyl, lower alkoxy, -C(O)-NH-lower alkyl, -C(O)-NH-lower alkoxy, oxo and NO₂;
R³ is selected from the group consisting of hydrogen, lower alkyl, lower alkyl substituted by -OR⁴ and -NR⁴R⁵;
R⁴ and R⁵ are selected from hydrogen, lower alkyl, lower alkyl substituted with OH or lower alkoxy; or
   R⁴ and R⁵ together with the nitrogen atom to which they are attached form morpholinyl;
Q is hydrogen or a group 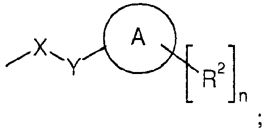
R² may be the same or different lower alkenyl, lower alkynyl, methyl sulfonyl, sulfonamide, hydrogen, lower alkyl, halogen, cyano, trifluoromethyl, lower alkoxy, NO₂, sulfonyl urea, amide, ester, carbamoyl, carbamate, urea,
   -NR⁴R⁵; and lower alkyl substituted with -OR⁴ or -NR⁴R⁵;
the group -X-Y- is selected from -OCH₂-, -CH₂O-, -NHCO-, -CONH-, -O-, -OCH₂CH₂-, -CH₂OCH₂, -CH₂CH₂O-, -CF=CH-,-CH=CF-, -NH-, -NHCH₂-, -CH₂NH-, -SCH₂-, -NHSO₂, -SO₂NH-, -CH₂S-, -SOCH₂-, -CH₂SO-, -SO₂CH₂-, -CH₂SO₂-, -S-, -CH=CH- and lower alkylene;
W is -O- or -NH-;
n is 1 or 2;
Ring A is selected from the group consisting of aryl, heteroaryl cycloalkyl and heterocycle; and
Ring B is selected from the group consisting of aryl, heteroaryl.

As a preferred embodiment there are provided 4-amino-thieno [3,2-c] pyridine-7-carboxylic acid derivatives of the formula (I) 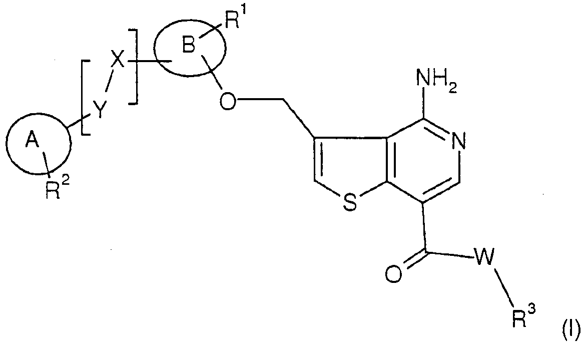 or pharmaceutically acceptable salts thereof,
wherein
R¹ is selected from the group consisting of hydrogen, lower alkyl, halogen, cyano, trifluoromethyl, lower alkoxy and NO₂;
R² is selected from the group consisting of lower alkenyl, lower alkynyl, methyl sulfonyl, sulfonamide, hydrogen, lower alkyl, halogen, cyano, trifluoromethyl, lower alkoxy, NO₂, sulfonyl urea, amide, ester, carbamoyl, carbamate, urea, lower alkyl substituted with OR⁴, and NR⁴R⁵;
R³ is selected from the group consisting of hydrogen, lower alkyl, lower alkyl substituted by OR⁴ and NR⁴R⁵;
R⁴ and R⁵ are selected from hydrogen, lower alkyl, or lower alkyl substituted with OH or lower alkoxy;
|X-Y| are selected from the group consisting of -OCH₂-, -CH₂O-, -NHCO-, -CONH-, -O-, -OCH₂CH₂-, -CH₂OCH₂, -CH₂CH₂O-, -CF=CH-, -CH=CF-, -NH-, -NHCH₂-, -CH₂NH-, -SCH₂-, -CH₂S-, -SOCH₂-, -CH₂SO-, -SO₂CH₂-, -CH₂SO₂-, -S-, -CH=CH-and lower alkylene;
W is -O- or -NH-;
Ring A is selected from the group consisting of aryl, heteroaryl cycloalkyl and heterocycle; and
Ring B is selected from the group consisting of aryl, heteroaryl.

Especially preferred are compounds of formula (I-A) or (I) wherein -X-Y- is selected from the group consisting of -OCH₂-, -CH₂O-, -NHCO- and -CONH-.

Also preferred are compounds of formula (I-A) or (I) wherein R¹ is selected from the group consisting of -CH₃, -Cl and -F.
the ring B is pyrimidinyl, 2-oxo-pyrimidinyl or phenyl; and
the remaining substituents have the meanings given above.

Still another preferred embodiment is the compound of formula (I-A) or (I), wherein both rings A and B are phenyl.

Also preferred are compounds of formula (I-A) or (I) where Ring B is phenyl or pyridinyl.

More preferred are compounds of formula (I-A) or (I) where Ring B is 2,5-disubstituted phenyl.

Also more preferred are compounds of formula (I-A) or (I) where Ring B is 3-hydroxy-2,5-disubstituted pyridinyl.

Especially preferred are compounds
4-Amino-3-[3-(4-chloro-3-trifluoromethyl-phenylcarbamoyl)-phenoxymethyl]thieno[3,2-c]pyridine-7-carboxylic acid ethyl ester;
4-Amino-3-(3-benzyloxy-phenoxymethyl)-thieno[3,2-c]pyridine-7-carboxylic acid ethyl ester;
4-Amino-3-(3-benzyloxy-phenoxymethyl)-thieno[3,2-c]pyridine-7-carboxylic acid (2-hydroxy-ethyl)-amide;
4-Amino-3-[3-(4-chloro-benzyloxy)-phenoxymethyl]-thieno[3,2-c]pyridine-7-carboxylic acid ethyl ester;
4-Amino-3-[3-(4-chloro-benzyloxy)-phenoxymethyl]-thieno[3,2-c]pyridine-7-carboxylic acid (2-hydroxy-ethyl)-amide;
4-Amino-3-[3-(3-chloro-benzyloxy)-phenoxymethyl]-thieno[3,2-c]pyridine-7-carboxylic acid ethyl ester;
4-Amino-3-[3-(3-chloro-benzyloxy)-phenoxymethyl]-thieno[3,2-c]pyridine-7-carboxylic acid (2-hydroxy-ethyl)-amide;
4-Amino-3-[3-(3-trifluoromethyl-benzyloxy)-phenoxymethyl]-thieno[3,2-c]pyridine-7-carboxylic acid ethyl ester;
4-Amino-3-[3-(3-trifluoromethyl-benzyloxy)-phenoxymethyl]-thieno[3,2-c]pyridine-7-carboxylic acid (2-hydroxy-ethyl)-amide;
4-Amino-3-(3-benzylsulfanyl-phenoxymethyl)-thieno[3,2-c]pyridine-7-carboxylic acid ethyl ester;
4-Amino-3-(3-benzylsulfanyl-phenoxymethyl]-thieno[3,2-c]pyridine-7-carboxylic acid (2-hydroxy-ethyl)-amide;
4-Amino-3-(3-phenylmethanesulfonyl-phenoxymethyl)-thieno[3,2-c]pyridine-7-carboxylic acid ethyl ester;
4-Amino-3-(3 phenylmethanesulfonyl-phenoxymethyl]-thieno[3,2-c]pyridine-7-carboxylic acid (2-hydroxy-ethyl)-amide;
4-Amino-3-[3-(3-methoxy-benzyloxy)-phenoxymethyl]-thieno[3,2-c]pyridine-7-carboxylic acid ethyl ester;
4-Amino-3-[3-(3-methoxy-benzyloxy)-phenoxymethyl]-thieno[3,2-c]pyridine-7-carboxylic acid (2-hydroxy-ethyl)-amide;
4-Amino-3-[3-(4-methoxy-benzyloxy)-phenoxymethyl]-thieno[3,2-c]pyridine-7-carboxylic acid ethyl ester;
4-Amino-3-[3-(4-methoxy-benzyloxy)-phenoxymethyl]-thieno[3,2-c]pyridine-7-carboxylic acid (2-hydroxy-ethyl)-amide;
4-Amino-3-[3-(4-methoxy-3-trifluoromethyl-benzyloxy)-phenoxymethyl]-thieno[3,2-c]pyridine-7-carboxylic acid ethyl ester;
4-Amino-3-[3-(4-methoxy-3-trifluoromethyl-benzyloxy)-phenoxymethyl]-thieno[3,2-c]pyridine-7-carboxylic acid (2-hydroxy-ethyl)-amide;
4-Amino-3-(3-phenoxy-phenoxymethyl)-thieno[3,2-c]pyridine-7-carboxylic acid ethyl ester;
4-Amino-3-(3-phenoxy-phenoxymethyl]-thieno[3,2-c]pyridine-7-carboxylic acid (2-hydroxy-ethyl)-amide;
4-Amino-3-(3-phenylamino-phenoxymethyl)-thieno[3,2-c]pyridine-7-carboxylic acid ethyl ester;
4-Amino-3-(3-phenylamino-phenoxymethyl]-thieno[3,2-c]pyridine-7-carboxylic acid (2-hydroxy-ethyl)-amide;
4-Amino-3-(3-benzylamino-phenoxymethyl)-thieno[3,2-c]pyridine-7-carboxylic acid ethyl este;r
4-Amino-3-(3-benzylamino-phenoxymethyl]-thieno[3,2-c]pyridine-7-carboxylic acid (2-hydroxy-ethyl)-amide;
4-Amino-3-[3-(4-cyano-benzyloxy)-phenoxymethyl]-thieno[3,2-c]pyridine-7-carboxylic acid ethyl ester;
4-Amino-3-[3-(4-cyano-benzyloxy)-phenoxymethyl]-thieno[3,2-c]pyridine-7-carboxylic acid;
4-Amino-3-[3-(4-cyano-benzyloxy)-phenoxymethyl]-thieno[3,2-c]pyridine-7-carboxylic acid (2-hydroxy-ethyl)-amide;
4-Amino-3-[3-(3-cyano-benzyloxy)-phenoxymethyl]-thieno[3,2-c]pyridine-7-carboxylic acid ethyl ester;
4-Amino-3-[3-(3-cyano-benzyloxy)-phenoxymethyl]-thieno[3,2-c]pyridine-7-carboxylic acid;
4-Amino-3-[3-(3-cyano-benzyloxy)-phenoxymethyl]-thieno[3,2-c]pyridine-7-carboxylic acid (2-hydroxy-ethyl)-amide trifluoroacetic acid salt;
3-(3-benzyloxy-phenoxymethyl)-4-(2-hydroxy-etnylamino)-thieno[3,2-c]pyridine-7-carboxylic acid (2-hydroxy-ethyl)-amide;
4-Amino-3-(3-methoxy-phenoxymethyl)-thieno[3,2-c]pyridine-7-carboxylic acid ethyl ester;
4-Amino-3-(3-methoxy-phenoxymethyl)-thieno[3,2-c]pyridine-7-carboxylic acid (2-hydroxy-ethyl)-amide;
4-Amino-3-(3-benzoylamino-phenoxymethyl)-thieno[3,2-c]pyridine-7-carboxylic acid ethyl ester;
4-Amino-3-(3-benzoylamino-phenoxymethyl)-thieno[3,2-c]pyridine-7-carboxylic acid (2-hydroxy-ethyl)-amide;
4-Amino-3-(3-benzoylamino-phenoxymethyl)-thieno[3,2-c]pyridine-7-carboxylic acid (2-hydroxy-ethyl)-amide hydrochloride;
4-Amino-3-(3-benzoylamino-phenoxymethyl)-thieno[3,2-c]pyridine-7-carboxylic acid (2-hydroxy-ethyl)-amide toluene-4-sulfonic acid salt;
4-Amino-3-[3-(4-chloro-phenylcarbamoyl)-phenoxymethyl]-thieno[3,2-c]pyridine-7-carboxylic acid ethyl ester;
4-Amino-3-[3-(4-chloro-phenylcarbamoyl)-phenoxymethyl]-thieno[3,2-c]pyridine-7-carboxylic acid (2-hydroxy-ethyl)-amide;
4-Amino-3-[3-(4-fluoro-phenylcarbamoyl)-phenoxymethyl]-thieno[3,2-c]pyridine-7-carboxylic acid ethyl ester;
4-Amino-3-[3-(4-fluoro-phenylcarbamoyl)-phenoxymethyl]-thieno[3,2-c]pyridine-7-carboxylic acid (2-hydroxy-ethyl)-amide;
4-Amino-3-[3-(4-chloro-benzyloxy)-phenoxymethyl]-thieno[3,2-c]pyridine-7-carboxylic acid trifluoro-acetic acid salt;
4-Amino-3-[3-(3-chloro-phenylcarbamoyl)-phenoxymethyl]-thieno[3,2-c]pyridine-7-carboxylic acid ethyl ester;
4-Amino-3-[3-(3-chloro-phenylcarbamoyl)-phenoxymethyl]-thieno[3,2-c]pyridine-7-carboxylic acid (2-hydroxy-ethyl)-amide;
4-Amino-3-[3-(4-chloro-phenylcarbamoyl)-phenoxymethyl]-thieno[3,2-c]pyridine-7-carboxylic acid (3-hydroxy-propyl)-amide;
4-Amino-3-[3-(4-chloro-phenylcarbamoyl)-phenoxymethyl]-thieno[3,2-c]pyridine-7-carboxylic acid propylamide;
4-Amino-3-[3-(4-chloro-phenylcarbamoyl)-phenoxymethyl]-thieno[3,2-c]pyridine-7-carboxylic acid ethylamide;
4-Amino-3-{3-[(4-chloro-phenyl)-methyl-carbamoyl]-phenoxymethyl}-thieno[3,2-c]pyridine-7-carboxylic acid ethyl ester;
4-Amino-3-[3-(1-methyl-piperidin-4-ylcarbamoyl)-phenoxymethyl]-thieno[3,2-c]pyridine-7-carboxylic acid ethyl ester;
4-Amino-3-[3-(5-methyl-pyridin-2-ylcarbamoyl)-phenoxymethyl]-thieno[3,2-c]pyridine-7-carboxylic acid ethyl ester;
4-Amino-3-[3-(1-methanesulfonyl-piperidin-4-ylcarbamoyl)-phenoxymethyl]-thieno[3,2-c]pyridine-7-carboxylic acid ethyl ester;
4-Amino-3-{3-[(4-chloro-phenyl)-methyl-carbamoyl]-phenoxymethyl}-thieno[3,2-c]pyridine-7-carboxylic acid (2-hydroxy-ethyl)-amide;
4-Amino-3-[5-(3-carbamoyl-phenylcarbamoyl)-2-methyl-phenoxymethyl]-thieno[3,2-c]pyridine-7-carboxylic acid ethyl ester;
4-Amino-3-(2-methyl-5-phenylcarbamoyl-phenoxymethyl)-thieno[3,2-c]pyridine-7-carboxylic acid ethyl ester;
4-Amino-3-[3-(4-chloro-benzyloxy)-phenoxymethyl]-thieno[3,2-c]pyridine-7-carboxylic acid (3-hydroxy-propyl)-amide trifluoro-acetic acid salt;
4-Amino-3-[3-(4-chloro-phenoxymethyl)-phenoxymethyl]-thieno[3,2-c]pyridine-7-carboxylic acid ethyl ester;
4-Amino-3-{3-[2-(4-chloro-phenyl)-vinyl]-phenoxymethyl}-thieno[3,2-c]pyridine-7-carboxylic acid ethyl ester;
4-Amino-3-{3-[2-(4-chloro-phenyl)-vinyl]-phenoxymethyl}-thieno[3,2-c]pyridine-7-carboxylic acid (2-hydroxy-ethyl)-amide;
4-Amino-3-[3-(4-chloro-phenoxymethyl)-phenoxymethyl]-thieno[3,2-c]pyridine-7-carboxylic acid (2-hydroxy-ethyl)-amide;
4-Amino-3-[3-(4-chloro-phenylcarbamoyl)-phenoxymethyl]-thieno[3,2-c]pyridine-7-carboxylic acid (2-hydroxy-ethyl)-methyl-amide;
4-Amino-3-[3-(4-chloro-phenylcarbamoyl)-phenoxymethyl]-thieno[3,2-c]pyridine-7-carboxylic acid (2-methoxy-ethyl)-amide;
4-Amino-3-[3-(4-chloro-phenylcarbamoyl)-phenoxymethyl]-thieno[3,2-c]pyridine-7-carboxylic acid methylamide;
3-[4-Amino-7-(morpholine-4-carbonyl)-thieno[3,2-c]pyridin-3-ylmethoxy]-N-(4-chlorophenyl)-benzamide;
4-Amino-3-(3-cyclohexylcarbamoyl-phenoxymethyl)-thieno[3,2-c]pyridine-7-carboxylic acid ethyl ester;
4-Amino-3-[3-(tetrahydro-pyran-4-ylcarbamoyl)-phenoxymethyl]-thieno[3,2-c]pyridine-7-carboxylic acid ethyl ester;
4-Amino-3-[3-(1-methanesulfonyl-piperidin-4-ylcarbamoyl)-phenoxymethyl]-thieno[3,2-c]pyridine-7-carboxylic acid (2-hydroxy-ethyl)-amide;
4-Amino-3-[3-(1-methyl-piperidin-4-ylcarbamoyl)-phenoxymethyl]-thieno[3,2-c]pyridine-7-carboxylic acid (2-hydroxy-ethyl)-amide;
4-Amino-3-(3-phenylaminomethyl-phenoxymethyl)-thieno[3,2-c]pyridine-7-carboxylic acid (2-hydroxy-ethyl)-amide trifluoro-acetic acid salt;
4-Amino-3-{3-[2-(4-chloro-phenyl)-ethoxy]-phenoxymethyl}-thieno[3,2-c]pyridine-7-carboxylic acid ethyl ester;
4-Amino-3-{3-[2-(4-chloro-phenyl)-ethoxy]-phenoxymethyl}-thieno[3,2-c]pyridine-7-carboxylic acid (2-hydroxy-ethyl)-amide;
4-Amino-3-[3-(4-chloro-benzoylamino)-phenoxymethyl]-thieno[3,2-c]pyridine-7-carboxylic acid ethyl ester;
4-Amino-3-[3-(4-chloro-benzoylamino)-phenoxymethyl]-thieno[3,2-c]pyridine-7-carboxylic acid (2-hydroxy-ethyl)-amide;
4-Amino-3-[3-(4-chloro-benzoylamino)-phenoxymethyl]-thieno[3,2-c]pyridine-7-carboxylic acid (3-hydroxy-propyl)-amide;
4-Amino-3-[3-(4-chloro-benzoylamino)-phenoxymethyl]-thieno[3,2-c]pyridine-7-carboxylic acid (3-hydroxy-propyl)-amide hydrochloride;
4-Amino-3-[3-(4-chloro-benzoylamino)-phenoxymethyl]-thieno[3,2-c]pyridine-7-carboxylic acid (3-hydroxy-propyl)-amide toluene-4-sulfonic acid salt;
4-Amino-3-(2-oxo-1-phenethyl-1,2-dihydro-pyrimidin-4-yloxymethyl)-thieno[3,2-c]pyridine-7-carboxylic acid ethyl ester;
4-Amino-3-(2,6-dioxo-3-phenethyl-3,6-dihydro-2H-pyrimidin-1-ylmethyl)-thieno[3,2-c]pyridine-7-carboxylic acid ethyl ester;
4-Amino-3-(2,6-dioxo-3-phenethyl-3,6-dihydro-2H-pyrimidin-1-ylmethyl)-thieno[3,2-c]pyridine-7-carboxylic acid (2-hydroxy-ethyl)-amide;
4-Amino-3-[2-methoxy-5-(3-methoxy-phenylcarbamoyl)-phenoxymethyl]-thieno[3,2-c]pyridine-7-carboxylic acid ethyl este;
4-Amino-3-[5-(3,5-dimethoxy-phenylcarbamoyl)-2-methoxy-phenoxymethyl]-thieno[3,2-c]pyridine-7-carboxylic acid ethyl ester;
4-Amino-3-[5-(4-chloro-3-methyl-phenylcarbamoyl)-2-methoxy-phenoxymethyl]-thieno[3,2-c]pyridine-7-carboxylic acid ethyl ester;
4-Amino-3-(5-benzylcarbamoyl-2-nitro-phenoxymethyl)-thieno[3,2-c]pyridine-7-carboxylic acid ethyl ester;
4-Amino-3-(2-methyl-3-phenylcarbamoyl-phenoxymethyl)-thieno[3,2-c]pyridine-7-carboxylic acid ethyl ester;
4-Amino-3-[3-(3,5-dimethoxy-phenylcarbamoyl)-2-methyl-phenoxymethyl]-thieno[3,2-c]pyridine-7-carboxylic acid ethyl ester;
4-Amino-3-[2-nitro-5-(3-trifluoromethoxy-phenylcarbamoyl)-phenoxymethyl]-thieno[3,2-c]pyridine-7-carboxylic acid ethyl ester;
4-Amino-3-[2-methyl-3-(3-trifluoromethoxy-phenylcarbamoyl)-phenoxymethyl]-thieno[3,2-c]pyridine-7-carboxylic acid ethyl ester;
4-Amino-3-[3-(3-methanesulfonyl-phenylcarbamoyl)-2-methyl-phenoxymethyl]-thieno[3,2-c]pyridine-7-carboxylic acid ethyl ester;
4-Amino-3-[3-(3-chloro-phenylcarbamoyl)-2-methyl-phenoxymethyl]-thieno[3,2-c]pyridine-7-carboxylic acid ethyl ester;
4-Amino-3-[2-methyl-3-(5-methyl-1H-pyrazol-3-ylcarbamoyl)-phenoxymethyl]-thieno[3,2-c]pyridine-7-carboxylic acid ethyl ester;
4-Amino-3-[3-(3,5-difluoro-benzyloxy)-5-methyl-phenoxymethyl]-thieno[3,2-c]pyridine-7-carboxylic acid ethyl ester;
4-Amino-3-[3-(3,4-difluoro-benzyloxy)-5-methyl-phenoxymethyl]-thieno[3,2-c]pyridine-7-carboxylic acid ethyl ester;
4-Amino-3-[3-(5-chloro-thiophen-2-ylmethoxy)-5-methyl-phenoxymethyl]-thieno[3,2-c]pyridine-7-carboxylic acid ethyl ester;
4-Amino-3-[3-(3-methyl-benzyloxy)-phenoxymethyl]-thieno[3,2-c]pyridine-7-carboxylic acid ethyl ester;
4-Amino-3-[3-(5-chloro-thiophen-2-ylmethoxy)-phenoxymethyl]-thieno[3,2-c]pyridine-7-carboxylic acid ethyl ester;
4-Amino-3-[3-(2,5-difluoro-benzyloxy)-phenoxymethyl]-thieno[3,2-c]pyridine-7-carboxylic acid ethyl ester;
4-Amino-3-[3-(4-fluoro-benzyloxy)-5-methyl-phenoxymethyl]-thieno[3,2-c]pyridine-7-carboxylic acid ethyl ester;
4-Amino-3-[3-(2,4-difluoro-benzyloxy)-5-methyl-phenoxymethyl]-thieno[3,2-c]pyridine-7-carboxylic acid ethyl ester;
4-Amino-3-[3-(2,5-difluoro-benzyloxy)-5-methyl-phenoxymethyl]-thieno[3,2-c]pyridine-7-carboxylic acid ethyl ester;
4-Amino-3-[3-methyl-5-(4-methyl-benzyloxy)-phenoxymethyl]-thieno[3,2-c]pyridine-7-carboxylic acid ethyl ester;
4-Amino-3-[3-methyl-5-(2-methyl-benzyloxy)-phenoxymethyl]-thieno[3,2-c]pyridine-7-carboxylic acid ethyl ester;
4-Amino-3-[3-(2-fluoro-benzyloxy)-5-methyl-phenoxymethyl]-thieno[3,2-c]pyridine-7-carboxylic acid ethyl ester;
4-Amino-3-[3-(2-methoxy-benzyloxy)-phenoxymethyl]-thieno[3,2-c]pyridine-7-carboxylic acid ethyl ester;
4-Amino-3-{3-[3-(2-morpholin-4-yl-ethoxy)-benzoylamino]-phenoxymethyl}-thieno[3,2-c]pyridine-7-carboxylic acid (2-hydroxy-ethyl)-amide;
4-Amino-3-(3-methylcarbamoyl-phenoxymethyl)-thieno[3,2-c]pyridine-7-carboxylic acid ethyl ester;
4-Amino-3-(3-cyclopropylcarbamoyl-phenoxymethyl)-thieno[3,2-c]pyridine-7-carboxylic acid ethyl ester;
4-Amino-3-(3-cyclopentylcarbamoyl-phenoxymethyl)-thieno[3,2-c]pyridine-7-carboxylic acid ethyl ester;
4-Amino-3-(3-cyclohexylcarbamoyl-phenoxymethyl)-thieno[3,2-c]pyridine-7-carboxylic acid (2-hydroxy-ethyl)-amide;
4-Amino-3-[3-(tetrahydro-pyran-4-ylcarbamoyl)-phenoxymethyl]-thieno[3,2-c]pyridine-7-carboxylic acid (2-hydroxy-ethyl)-amide;
4-Amino-3-(3-methylcarbamoyl-phenoxymethyl)-thieno[3,2-c]pyridine-7-carboxylic acid (2-hydroxy-ethyl)-amide;
4-Amino-3-(3-cyclopropylcarbamoyl-phenoxymethyl)-thieno[3,2-c]pyridine-7-carboxylic acid (2-hydroxy-ethyl)-amide;
4-Amino-3-(3-cyclopentylcarbamoyl-phenoxymethyl)-thieno[3,2-c]pyridine-7-carboxylic acid (2-hydroxy-ethyl)-amide;
4-Amino-3-(5-methylcarbamoyl-pyridin-3-yloxymethyl)-thieno[3,2-c]pyridine-7-carboxylic acid ethyl ester;
4-Amino-3-(5-methylcarbamoyl-pyridin-3-yloxymethyl)-thieno[3,2-c]pyridine-7-carboxylic acid (2-hydroxy-ethyl)-amide,
4-Amino-3-[3-(2-fluoro-benzyloxy)-5-methyl-phenoxymethyl]-thieno[3,2-c]pyridine-7-carboxylic acid (2-hydroxy-ethyl)-amide,
4-Amino-3-[3-(3,5-difluoro-benzyloxy)-5-methyl-phenoxymethyl]-thieno[3,2-c]pyridine-7-carboxylic acid (2-hydroxy-ethyl)-amide,
4-Amino-3-[3-methyl-5-(2-methyl-benzyloxy)-phenoxymethyl]-thieno[3,2-c]pyridine-7-carboxylic acid (2-hydroxy-ethyl)-amide,
4-Amino-3-[3-(2,5-difluoro-benzyloxy)-phenoxymethyl]-thieno[3,2-c]pyridine-7-carboxylic acid (2-hydroxy-ethyl)-amide,
4-Amino-3-[3-(3-fluoro-benzyloxy)-5-methyl-phenoxymethyl]-thieno[3,2-c]pyridine-7-carboxylic acid (2-hydroxy-ethyl)-amide,
4-Amino-3-[3-(2,5-difluoro-benzyloxy)-5-methyl-phenoxymethyl]-thieno[3,2-c]pyridine-7-carboxylic acid (2-hydroxy-ethyl)-amide,
4-Amino-3-[3-methyl-5-(4-methyl-benzyloxy)-phenoxymethyl]-thieno[3,2-c]pyridine-7-carboxylic acid (2-hydroxy-ethyl)-amide,
4-Amino-3-[3-(3,4-difluoro-benzyloxy)-5-methyl-phenoxymethyl]-thieno[3,2-c]pyridine-7-carboxylic acid (2-hydroxy-ethyl)-amide,
4-Amino-3-[3-(2,3-difluoro-benzyloxy)-phenoxymethyl]-thieno[3,2-c]pyridine-7-carboxylic acid (2-hydroxy-ethyl)-amide,4-Amino-3-[3-(4-chloro-benzoylamino)-phenoxymethyl]-thieno[3,2-c]pyridine-7-carboxylic acid,
4-Amino-3-[3-(pyrimidin-5-ylmethoxy)-phenoxymethyl]-thieno[3,2-c]pyridine-7-carboxylic acid ethyl ester,
4-Amino-3-[3-(pyrimidin-5-ylmethoxy)-phenoxymethyl]-thieno[3,2-c]pyridine-7-carboxylic acid (2-hydroxy-ethyl)-amide,
4-Amino-3-[3-(4-chloro-benzenesulfonylamino)-phenoxymethyl]-thieno[3,2-c]pyridine-7-carboxylic acid ethyl ester,
4-Amino-3-[3-(4-chloro-benzenesulfonylamino)-phenoxymethyl]-thieno[3,2-c]pyridine-7-carboxylic acid (2-hydroxy-ethyl)-amide,
4-Amino-3-[2-chloro-5-(4-chloro-benzyloxy)-phenoxymethyl]-thieno[3,2-c]pyridine-7-carboxylic acid ethyl ester,
4-Amino-3-[2-chloro-5-(4-chloro-benzyloxy)-phenoxymethyl]-thieno[3,2-c]pyridine-7-carboxylic acid (2-hydroxy-ethyl)-amide,
4-Amino-3-{3-[(pyridine-2-carbonyl)-amino]-phenoxymethyl}-thieno[3,2-c]pyridine-7-carboxylic acid ethyl ester,
4-Amino-3-{3-[(pyridine-2-carbonyl)-amino]-phenoxymethyl}-thieno[3,2-c]pyridine-7-carboxylic acid (2-hydroxy-ethyl)-amide,
4-Amino-3-{3-[(6-methyl-pyridine-3-carbonyl)-amino]-phenoxymethyl}-thieno[3,2-c]pyridine-7-carboxylic acid ethyl ester,
4-Amino-3-{3-[(6-methyl-pyridine-3-carbonyl)-amino]-phenoxymethyl}-thieno[3,2c]pyridine-7-carboxylic acid (2-hydroxy-ethyl)-amide,
4-Amino-3-{3-[(pyridine-4-carbonyl)-amino]-phenoxymethyl}-thieno[3,2-c]pyridine-7-carboxylic acid ethyl ester,
4-Amino-3-{3-[(pyridine-4-carbonyl)-amino]-phenoxymethyl}-thieno[3,2-c]pyridine-7-carboxylic acid (2-hydroxy-ethyl)-amide,
4-Amino-3-[5-(3-chloro-4-fluoro-benzoylamino)-2-methyl-phenoxymethyl]-thieno[3,2-c]pyridine-7-carboxylic acid ethyl ester,
4-Amino-3-[5-(3-chloro-4-benzoylamino)-2-methyl-phenoxymethyl]-thieno[3,2-c]pyridine-7-carboxylic acid ethyl ester,
4-Amino-3-[5-(3-chloro-benzoylamino)-2-methyl-phenoxymethyl]-thieno[3,2-c]pyridine-7-carboxylic acid (2-hydroxy-ethyl)-amide,
4-Amino-3-{5-[3-chloro-4-(2-hydroxy-ethylamino)-benzoylamino)-2-methyl-phenoxymethyl]-thieno[3,2-c]pyridine-7-carboxylic acid (2-hydroxy-ethyl)-amide,
4-Amino-3-{5-[3-chloro-4-(2-hydroxy-ethylamino)-benzoylamino)-2-methyl-phenoxymethyl]-thieno[3,2-c]pyridine-7-carboxylic acid (2-hydroxy-ethyl)-amide toluene-4-sulfonic acid salt,
4-Amino-3-{5-[3-chloro-4-(2-hydroxy-ethylamino)-benzoylamino]-2-methyl-phenoxymethyl}-thieno[3,2-c]pyridine-7-carboxylic acid ethyl ester; and
4-Amino-3-{5-[3-chloro-4-(2-hydroxy-ethylamino)-benzoylamino]-2-methyl-phenoxymethyl}-thieno[3,2-c]pyridine-7-carboxylic acid amide.

As used herein, the following terms shall have the following definitions.

"Aryl" means a monocyclic or bicyclic, aromatic carbocyclic hydrocarbon radical, preferably a 6-10 membered aromatic ring system. Preferred aryl groups include, but are not limited to, phenyl, naphthyl and tolyl.

"Heteroaryl" means an aromatic heterocyclic ring system containing up to two rings. Preferred heteroaryl groups include, but are not limited to, thienyl, furyl, indolyl, pyrrolyl, pyridinyl, pyrazinyl, oxazolyl, thiaxolyl, quinolinyl, pyrimidinyl, imidazolyl, pyrazolyl, benzofuran and tetrazolyl.

As used herein, in particular in R², the term
(a) "amide" means a substituent of the formula 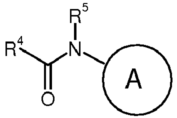
(b) "ester" means a substituent of the formula 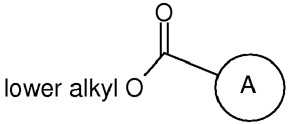
(c) "carbamoyl" means a substituent of the formula 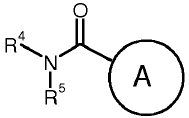
(d) "carbamate" means a substituent of the formula 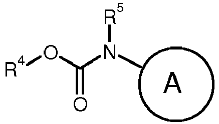
(e) "urea" means a substituent of the formula 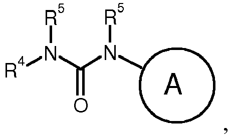 and
R⁴ and R⁵ are selected from hydrogen; lower alkyl; lower alkyl substituted with OH or lower alkoxy.

"Effective amount" means an amount that is effective to prevent, alleviate or ameliorate symptoms of disease or prolong the survival of the subject being treated.

"Halogen" means fluorine, chlorine, bromine or iodine, preferably fluorine or chlorine.

"Hetero atom" means an atom selected from N, O and S.

"Lower alkyl" alone or in conjunction with another term, e.g. lower alkyl-heterocycle, denotes a straight-chain or branched, saturated aliphatic hydrocarbon having 1 to 6, preferably 1 to 4, carbon atoms. Typical lower alkyl groups include methyl, ethyl, propyl, isopropyl, butyl, t-butyl, 2-butyl, pentyl, hexyl and the like.

The term "alkoxy" means a group -O-alkyl, preferably a group -O-lower alkyl, wherein "lower alkyl" has the meaning given above.

The term "alkenyl", alone or in combination with other groups, stands for a straight-chain or branched hydrocarbon residue comprising an olefinic bond and up to 20, preferably up to 16 carbon atoms, more preferably up to 10 carbon atoms. Lower-alkenyl groups as described below also are preferred alkenyl groups. The term "lower-alkenyl" refers to a straight-chain or branched hydrocarbon residue comprising an olefinic (double) bond and up to 7, preferably up to 4 carbon atoms, such as e.g. 2-propenyl. An alkenyl or lower-alkenyl group may have a substitution pattern as described earlier in connection with the term "alkyl".

The term "alkynyl", alone or in combination with other groups, stands for a straight-chain or branched hydrocarbon residue comprising a triple bond and up to 20, preferably up to 16 carbon atoms. The term "lower-alkynyl" refers to a straight-chain or branched hydrocarbon residue comprising a triple bond and up to 7, preferably up R⁴ and R⁵ are selected from hydrogen; lower alkyl; lower alkyl substituted with OH or lower alkoxy.

"Effective amount" means an amount that is effective to prevent, alleviate or ameliorate symptoms of disease or prolong the survival of the subject being treated.

"Halogen" means fluorine, chlorine, bromine or iodine, preferably fluorine or chlorine.

"Heteroatom" means an atom selected from N, O and S.

"Lower alkyl" alone or in conjunction with another term, e.g. lower alkyl-heterocycle, denotes a straight-chain or branched, saturated aliphatic hydrocarbon having 1 to 6, preferably 1 to 4, carbon atoms. Typical lower alkyl groups include methyl, ethyl, propyl, isopropyl, butyl, t-butyl, 2-butyl, pentyl, hexyl and the like.

The term "alkoxy" means a group -O-alkyl, preferably a group -O-lower alkyl, wherein "lower alkyl" has the meaning given above.

The term "alkenyl", alone or in combination with other groups, stands for a straight-chain or branched hydrocarbon residue comprising an olefinic bond and up to 20, preferably up to 16 carbon atoms, more preferably up to 10 carbon atoms. Lower alkenyl groups as described below also are preferred alkenyl groups. The term "lower alkenyl" refers to a straight-chain or branched hydrocarbon residue comprising an olefinic (double) bond and up to 7, preferably up to 4 carbon atoms, such as e.g. 2-propenyl. An alkenyl or lower alkenyl group may have a substitution pattern as described earlier in connection with the term "alkyl".

The term "alkynyl", alone or in combination with other groups, stands for a straight-chain or branched hydrocarbon residue comprising a triple bond and up to 20, preferably up to 16 carbon atoms. The term "lower alkynyl" refers to a straight-chain or branched hydrocarbon residue comprising a triple bond and up to 7, preferably up to 4 carbon atoms, such as e.g. 2-propinyl. An alkynyl or loweralkynyl group may have a substitution pattern as described earlier in connection with the term "alkyl".

The term "cycloalkyl" denotes a saturated mono- or bicyclic hydrocarbon with 3 to 10 carbon atoms. Preferred are monocyclic hydrocarbons with 3 to 6 carbon atoms, such as cyclopropyl, cyclopentyl and cyclohexyl and the like.

The term "heterocycle" means a "cycloalkyl" as defined above, wherein one, two or three carbon atoms, preferably one or two carbon atoms, are replaced by a heteroatom selected from N, S or O. Especially preferred are 5- and 6-membered heterocycles. Examples of such heterocycles are piperidine, tetrahydropyran, piperazine, morpholine, pyrrolidine, pyrazolidine, tetrahydrothiophene, imidazolidine and the like.

The term "pharmaceutically acceptable salt" refers to conventional acid-addition salts or base-addition salts that retain the biological effectiveness and properties of the compounds of formula (I-A) and are formed from suitable non-toxic organic or inorganic acids or organic or inorganic bases. Sample acid-addition salts include those derived from inorganic acids such as hydrochloric acid, hydrobromic acid, hydroiodic acid, sulfuric acid, sulfamic acid, phosphoric acid and nitric acid, and those derived from organic acids such as p-toluene sulfonic acid, salicylic acid, methanesulfonic acid, oxalic acid, succinic acid, citric acid, malic acid, lactic acid, fumaric acid, and the like. Sample base-addition salts include those derived from ammonium, potassium, sodium, lithium, magnesium, calcium and quaternary ammonium hydroxides, such as for example, tetramethylammonium hydroxide. The chemical modification of a pharmaceutical compound (i.e. drug) into a salt is a technique well known to pharmaceutical chemists to obtain improved physical and chemical stability, hygroscopicity, flowability and solubility of compounds. *See, e.g.,* H. Ansel et. al., Pharmaceutical Dosage Forms and Drug Delivery Systems (6th Ed. 1995) at pp. 196 and 1456-1457.

"Pharmaceutically acceptable", such as pharmaceutically acceptable carrier, excipient, etc., means pharmacologically acceptable and substantially non-toxic to the subject to which the particular compound is administered.

"Substituted" as used herein, for example in substituted alkyl or substituted heteroaryl, means that the substitution can occur at one or more positions and, unless otherwise indicated, that the substituents at each substitution site are independently selected from the specified options.

"Therapeutically effective amount" means an amount of at least one compound of Formula (I-A), or a pharmaceutically acceptable salt or ester thereof, that significantly inhibits proliferation and/or prevents differentiation of a human tumor cell, including human tumor cell lines.

Medicaments containing a compound of the present invention or a pharmaceutically acceptable salt thereof and a therapeutically inert carrier are an object of the present invention, as is a process for their production, which comprises bringing one or more compounds of the present invention and/or pharmaceutically acceptable salts and, if desired, one or more other therapeutically valuable substances into a galenical administration form together with one or more therapeutically inert excipients/carriers.

In accordance with the invention the compounds of the present invention as well as their pharmaceutically acceptable salts are useful in the control or prevention of illnesses, particularly proliferative disorders such as cancer, more particularly colorectal, breast, lung, prostate, pancreatic, gastric, bladder, ovarian, melanoma, neuroblastoma, cervical, kidney or renal cancers, leukemias or lymphomas. Based on their Raf kinase inhibition and their antiproliferative activity, said compounds are useful for the treatment of diseases such as cancer in humans or animals and for the production of corresponding medicaments. The dosage depends on various factors such as manner of administration, species, age and/or individual state of health.

### Intermediate 1

### 4-Methyl-2-thiophenecarboxaldehyde

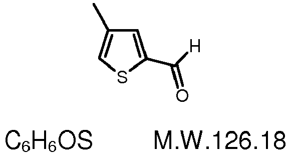

A solution of 3-methylthiophene (58.90 g, 0.60 mol) (Fluka) in anhydrous ether (600 mL) was stirred and cooled in an ice - water bath. This solution was treated dropwise over 15 minutes with n-butyllithium in pentane (2 M, 450 mL, 0.90 mol) (Aldrich). After stirring for 2 hours at room temperature the mixture was cooled in an ice - water bath and treated dropwise over 5 minutes with *N,N*-dimethylformamide (48.24 g, 0.66 mol) (Fisher) followed by stirring at room temperature over night. The mixture was diluted with ether (600 mL) and washed with water and brine. After drying (sodium sulfate) ether was evaporated on a rotary evaporator without vacuum to give 114 g of red liquid. This liquid was purified by chromatography over a pad of silica gel 60 (1 Kg, 70-230 mesh) eluting with 40% dichloromethane - hexanes. Evaporation without vacuum gave 4-methyl-2-thiophenecarboxaldehyde as a light red oil. (Yielded 56.62 g, 74.7%). This product contained small amounts of hexanes.

### Intermediate 2

### 3-(4-Methyl-thiophen-2-yl)-acrylic acid

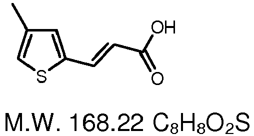

A solution of 4-methyl-2-thiophenecarboxaldehyde (56.62 g, 0.448 mol) (from Intermediate 1 *supra),* malonic acid (186.77 g, 1.79 mol) (Aldrich) and piperidine (1.90 g, 0.022 mol) (Fluka) in pyridine (550 mL) was heated at reflux with stirring over night. Reaction mixture was evaporated to dryness. The resulting residue was dissolve in dichloromethane and washed successively with 3 N hydrochloric acid, water and brine. The organic layer was dried and evaporated to give 3-(4-methyl-thiophen-2-yl)-acrylic acid as a tan solid. (Yield 49.52 g, 65.7%).

### Intermediate 3

### 3-(4-Methyl-thiophen-2-yl)-acryloyl azide

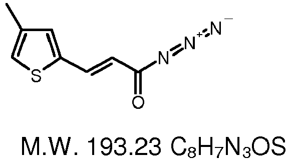

To a solution of 3-(4-methyl-thiophen-2-yl)-acrylic acid (49.52 g, 0.294 mol) (from Intermediate 2 *supra)* and triethylamine (44.68 g, 0.441 mol) (Aldrich) in acetone (2000 mL) with stirring and cooling in an ice - water bath was added ethyl chloroformate (35.14 g, 0.323 mol) (Aldrich). After stirring at room temperature for 20 minutes, sodium azide (28.70 g, 0.441 mol) (Aldrich) was added and stirring continued for another 20 minutes at room temperature. Acetone was then evaporated off at reduced pressure and residue was diluted with water. This was extracted with dichloromethane. The organic extract was washed with brine, dried and concentrated to give 3-(4-methyl-thiophen-2-yl)-acryloyl azide as a brown solid. (Yield 48.51 g, 85.4%).

### Intermediate 4

### 3-Methyl-5H-thieno[3,2-c]pyridin-4-one

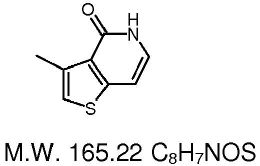

3-(4-Methyl-thiophen-2-yl)-acryloyl azide (1.54 g; 7.95 mmol) (from Intermediate 3 *supra)* was dissolved in meta-xylenes (16 mL). The solution was heated in an oil bath at 105 - 115 °C for 30 minutes until nitrogen evolution ceased. At this point a few crystals of iodine were added to the reaction and the oil bath temperature was increased to 145 - 150 °C. The reaction was heated at reflux for 6 hours. Upon cooling, solid precipitated out of solution. Filtration and drying yielded 3-methyl-5H-thieno[3.2-c]pyridine-4-one. (Yield 1.05 g; 80.1 %).
HRMS(EI+) *m*/*z* Calcd for C₈H₇NOS [(M⁺)]: 165.0248. Found: 165.0250.

### Intermediate 5

### 7-lodo-3-methyl-5H-thieno[3,2-c]pyridin-4-one

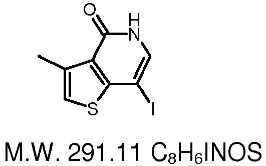

A solution of 3-methyl-5H-thieno[3,2-c]pyridin-4-one (24.27 g, 0.146 mol) (from Intermediate 4 *supra)* and *N*-iodosuccinimide (34.70 g, 0.154 mol) (Avocado) in dimethylformamide (1000 mL) was stirred at room temperature over night. Reaction mixture was concentrated under reduced pressure and residue was stirred with ether (1000 mL) for 0.5 hour. Suspension was filtered, washed with ether and sucked dry to give 7-iodo-3-methyl-5H-thieno[3,2-c]pyridin-4-one as a brown solid. (Yield 41.88 g, 97.9%).

### Intermediate 6

### 3-Methyl-4-oxo-4,5-dihydro-thieno[3,2-c]pyridine-7-carboxylic acid ethyl ester

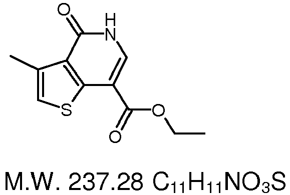

A stirred suspension of 7-iodo-3-methyl-5H-thieno[3,2-c]pyridin-4-one (1.14 g, 3.92 mmol) (from Intermediate 5 *supra),* triethylamine (2.5 mL, 17.94 mmol) (Aldrich) and bis(triphenylphosphine)palladium(II) chloride (0.14 g, 0.2 mmol) (Aldrich) in ethanol (50 mL) was degassed with argon and then saturated with carbon monoxide. The mixture was stirred with heating in a 75 °C oil bath over night under a blanket of carbon monoxide. After cooling, reaction mixture was concentrated under reduced pressure to remove a portion of ethanol (about 20%). The solid formed was collected by filtration, washed with ethanol - diethyl ether (1:1) and then diethyl ether and finally dried under vacuum to give 3-methyl-4-oxo-4,5-dihydro-thieno[3,2-c]pyridine-7-carboxylic acid ethyl ester. (Yield 0.78 g, 84.0%).
HRMS(EI+) *m*/*z* Calcd for C₁₁H₁₁NO₃S [(M⁺)]: 237.0460. Found: 237.0451.

### Intermediate 7

### 4-Chloro-3-methyl-thieno[3,2-c]pyridine-7-carboxylic acid ethyl ester

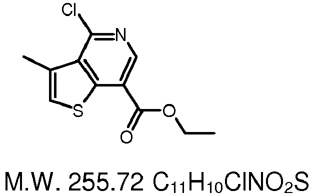

A mixture of 3-methyl-4-oxo-4,5-dihydro-thieno[3,2-c]pyridine-7-carboxylic acid ethyl ester (2.43 g, 10.24 mmol) (from Intermediate 6 *supra)* and *N*,*N-*diisopropylethylamine (2.4 mL, 13.87 mmol) (Fluka) was stirred with cooling in an ice - water bath. This mixture was slowly treated with phosphorous oxychloride (7.8 mL, 83.68 mmol) (Fluka) and then allowed to warm to room temperature. *N*,*N-*Dimethylformamide (1.0 mL, 12.86 mmol) was then added and the mixture stirred with heating at 70 °C for 30 minutes. A second portion of *N*,*N*-dimethylformamide (0.5 mL, 6.43 mmol) was added and the mixture was heated at 70 °C for another 30 minutes. After cooling, ice was added to the solution and the mixture was extracted with ethyl acetate. Organic extract was washed with water, saturated aqueous sodium bicarbonate solution, water and brine. The aqueous phases were back washed with ethyl acetate. The ethyl acetate solutions were combined, dried (sodium sulfate) and concentrated under reduced pressure. This residue was purified by flash chromatography over silica gel (Biotage 65M, 5 : 95 ethyl acetate - hexanes) to give 4-chloro-3-methyl-thieno[3,2-c]pyridine-7-carboxylic acid ethyl ester. (Yield 1.57 g, 60.0%).
HRMS(EI+) *m*/*z* Calcd for C₁₁H₁₀ClNO₂S [(M⁺)]: 255.0121. Found: 255.0119.

### Intermediate 8

### 3-Bromomethyl-4-chloro-thieno[3,2-c]pyridine-7-carboxylic acid ethyl ester

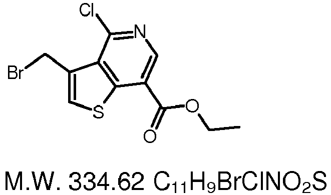

To a solution of 4-chloro-3-methyl-thieno[3,2-c]pyridine-7-carboxylic acid ethyl ester (14.65 g, 57.29 mmol) (from Intermediate 7 *supra)* in carbon tetrachloride (250 mL) was added N-bromosuccinimide (12.24 g, 68.75 mmol) and 2,2'-azobisisobutyronitrile (0.94 g, 5.73 mmol). The reaction mixture was heated at 80 °C for 24 hours. The mixture was then cooled and concentrated under reduced pressure. The residue was recrystallized from ethyl acetate - hexanes to give 3-bromomethyl-4-chloro-thieno[3,2-c]pyridine-7-carboxylic acid ethyl ester as white crystals. (Yield 11.73 g). The mother liquid was concentrated and purified by flash chromatography (diethyl ether - hexanes, 1:4, V/V) to give second crop of 3-bromomethyl-4-chloro-thieno[3,2-c]pyridine-7-carboxylic acid ethyl ester. (Yield 4.39 g, 84% combined).

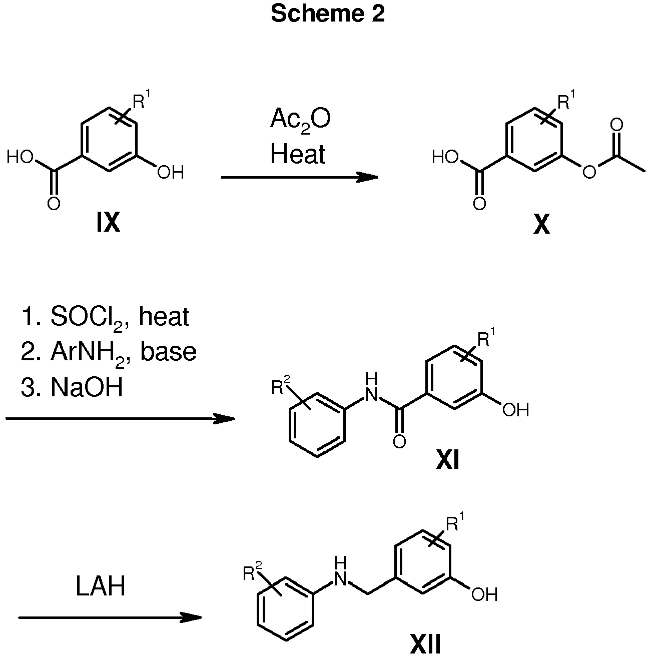

Conversion of X to XI can also be achieved via other coupling methods well know in the art.

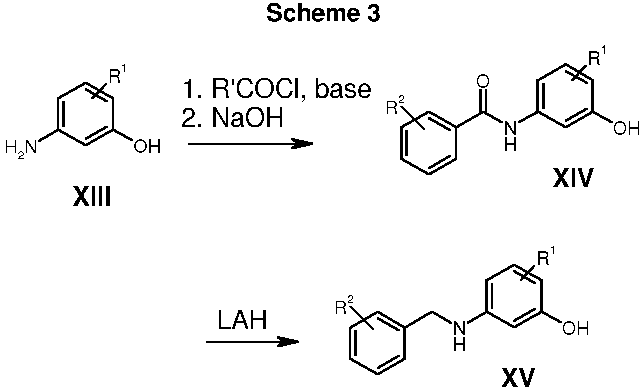

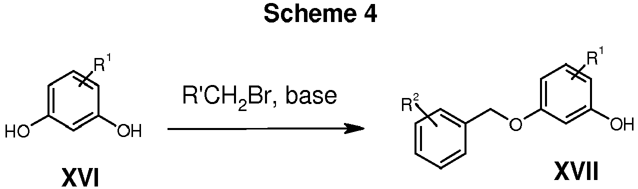

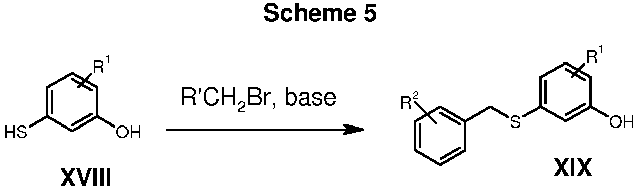

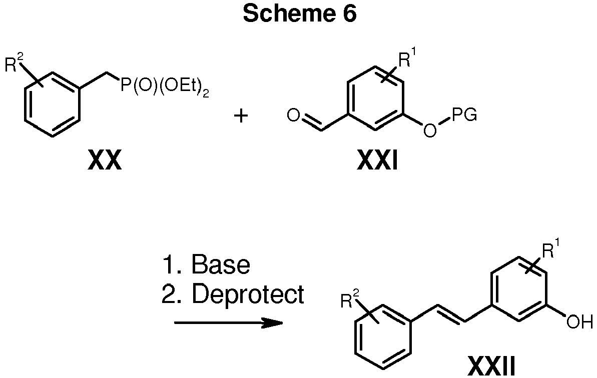

PG is an appropriate protecting group, such as for example *boc*, a *mom ether*, a *sam ether*, etc.

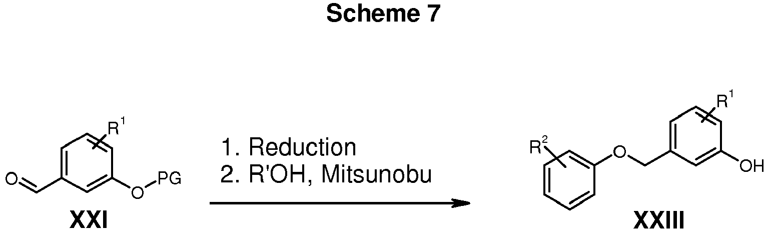

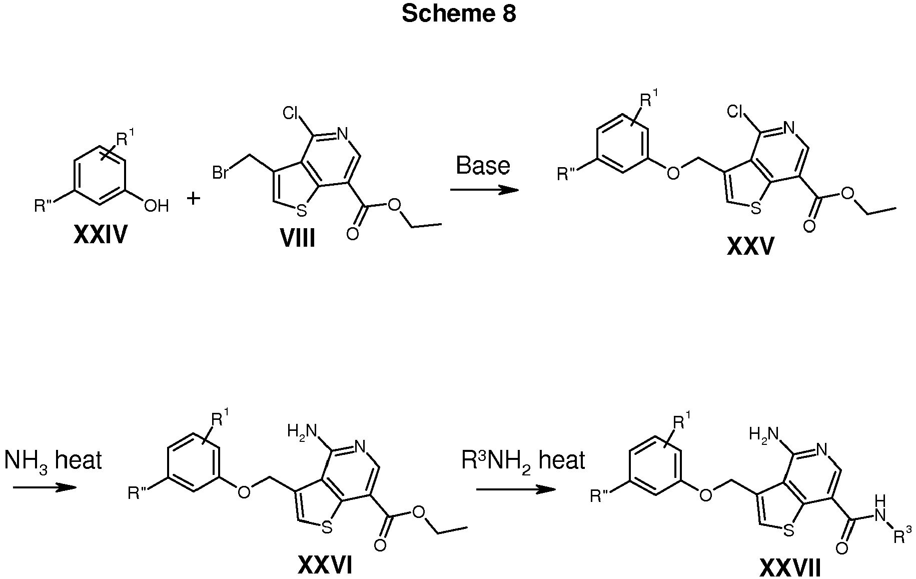

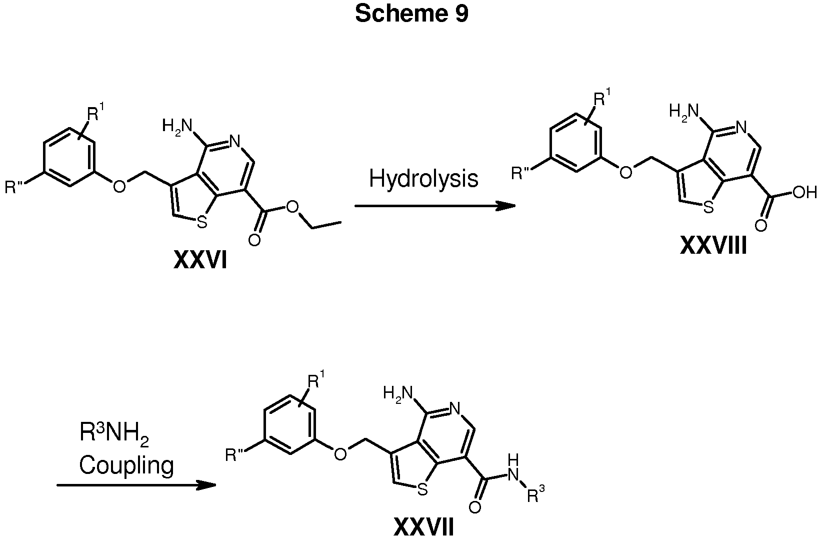

R" as used in schemes 8 and 9 is with n being 1 or 2.

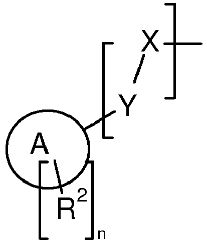

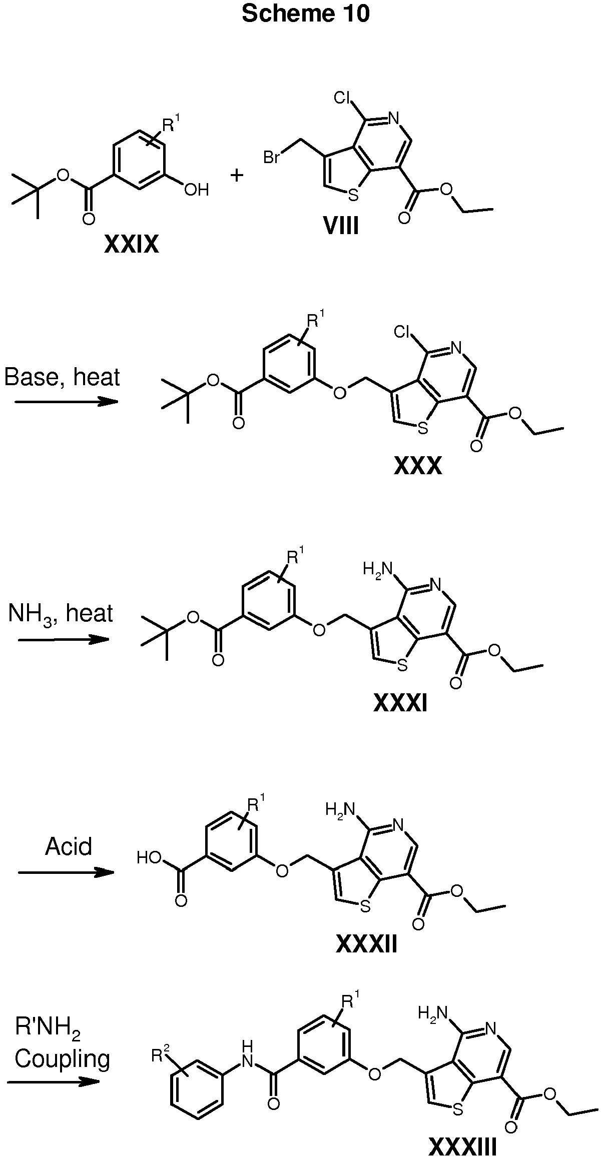

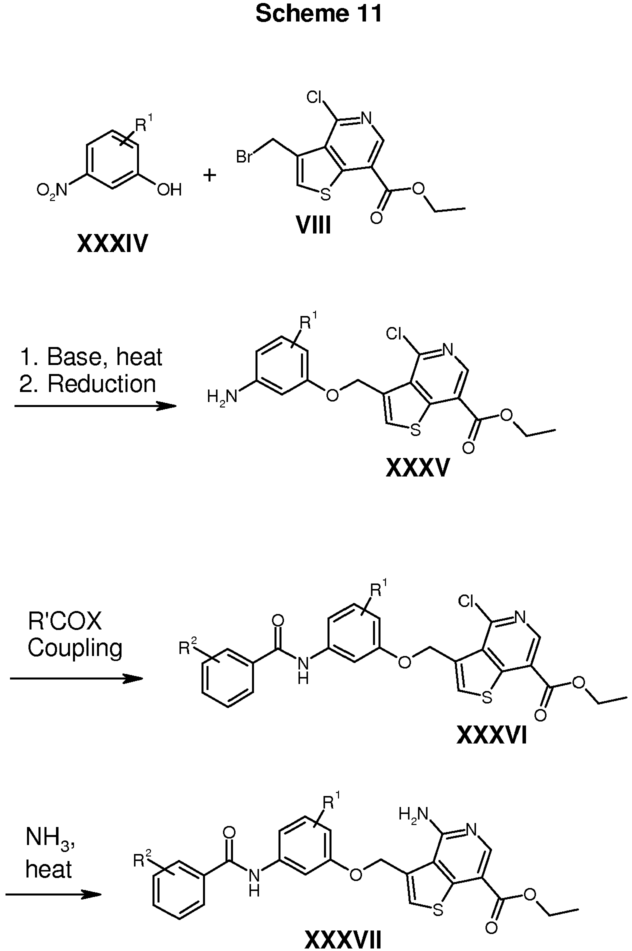

### General Procedure 1

### Synthesis of XXX

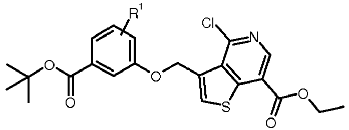

Compound XXIX (18 mmol) (Scheme 10) was dissolved in N,N'-dimethylformamide (10 vol). To this was added potassium carbonate (1.2 equivalents), potassium iodide (1.0 equivalent) and 3-bromomethyl-4-chloro-thieno[3,2-c]pyridine-7-carboxylic acid ethyl ester (1.0 equivalent) (from Intermediate 8 *supra).* The reaction was stirred at 70 °C for 16 hours. Water (100 mL) was added and the crude reaction mixture extracted with ethyl acetate (4 x 50 mL). The combined organic layers were dried (MgSO₄), filtered and the solvent removed *in vacuuo.* The crude solid was suspended in ethyl acetate (50 mL). The resulting suspension was filtered, and the solid washed with a further aliquot of ethyl acetate (20 mL) to give crude product XXX.

### General Procedure 2

### Synthesis of XXXI

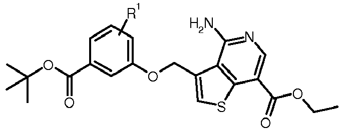

Compound XXX (8 mmol) (from General Procedure 1 *supra)* was dissolved in 2-propanol saturated with ammonia (10 vol). The solution was heat at 160 °C in a microwave reactor for 20 minutes. The reaction solvent was removed *in vacuuo.* Hot methanol was added and the resulting suspension filtered. The solid (mainly unreacted starting material) was re-treated under microwave conditions (as above); dioxane (∼5 mL) was added to aid solubility in some cases as required. The desired product was contained in the filtrate, which was evaporated to dryness and analysed. This process was repeated until >90% conversion of starting material to desired product XXXI was achieved.

### General Procedure 3

### Synthesis of XXXII

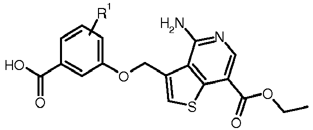

Compound XXX1 (6 mmol) (from General Procedure 2 *supra)* was dissolved in 20% trifluoroacetic acid in dichloromethane (10 vol). The reaction mixture was stirred at room temperature for 1.5 hours. The reaction mixture was evaporated to dryness to give the product XXXII.

### General Procedure 4

### Synthesis of XXXIII

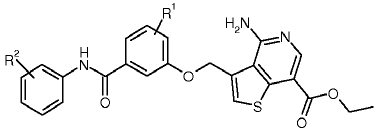

Compound XXXII (160 µmol) (from General Procedure 3 *supra)* was dissolved in dichloroethane (3 mL) and DMF (1 drop). Thionyl chloride (3 equivalents) was added and the reaction mixture agitated at room temperature until acid chloride formation was complete (monitored by LCMS; ∼16 hours). Triethylamine (6 equivalents) was added to the crude reaction mixture followed by aniline (3 equivalents). The reaction mixture was agitated at 40 °C for 16 hours during which time a precipitate formed. The reaction mixture was filtered and the filtrate washed with 1 N HCl (2 mL). The organic layer was passed through a filter of MgSO₄ and then combined with the precipitate from the reaction mixture. The solvent was removed under reduced pressure and the resulting solid was washed with THF. The collected solid was suspended in a mixture of 2-propanol / chloroform (1:1) and loaded onto a preparative TLC plate (run in 20% m ethanol - dichloromethane). The desired material was removed from the silica by washing with methanol and concentrating to give product XXXIII.

### General Procedure 5

### Synthesis of XVII

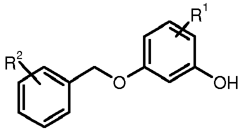

To substituted resorcinol (6.4 mmol) (Scheme 4) dissolved in N,N-dimethylformamide (5 vol.) was added cesium carbonate (1.2 equivalents) and benzyl halide (1 equivalent) (Scheme 4). The reaction was stirred overnight at room temperature. The crude reaction mixture was dissolved in dichloromethane (20 mL) and washed with 1 N HCl (2 x 20 mL). The combined aqueous layers were extracted with dichloromethane (2 x 20 mL) and organic layers combined and washed with brine (20 mL), then dried (MgSO₄). The desired products were purified by chromatography, eluting with heptane then 20% EtOAc / heptane. Yields were typically 30 - 50%.

### General Procedure 6

### Synthesis of XXV

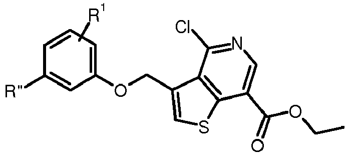

To alkylated resorcinol XVII (from General Procedure 5 *supra)* or other substituted phenol XXIV (Scheme 8) dissolved in N,N-dimethylformamide (5 vol.) was added potassium carbonate (1.2 equivalents), potassium iodide (1 equivalent) and 3-bromomethyl-4-chloro-thieno[3,2-c]pyridine-7-carboxylic acid ethyl ester (1.0 equivalent) (from Intermediate 8 *supra).* The reaction was stirred overnight at 75 °C. The crude reaction mixture was dissolved in dichloromethane (20 mL) and washed with 1 N HCl (20 mL). The organic layer was collected and the aqueous layer was extracted with dichloromethane (20 mL). The combined organic layers were dried (MgSO₄). The desired products were purified by chromatography, eluting with heptane then 10% EtOAc / heptane, then recrystallisation from acetonitrile. Yields were typically 25 - 30%.

### General Procedure 7

### Synthesis of XXVI

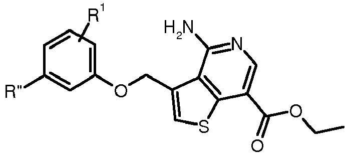

Compound XXV (from General Procedure 6 *supra)* was dissolved in 2-propanol saturated with ammonia (10 vol.). The solution was heated at 140 °C in a microwave reactor for 3 x 30 minutes. Any precipitate formed was filtered and washed with 2-propanol. In cases where no precipitate was formed, the solvent was removed under reduced pressure and the resulting solid was washed with 2-propanol. No further purification was necessary. Yields were typically 60 - 75%.

### General Procedure 8

### Synthesis of XXVII

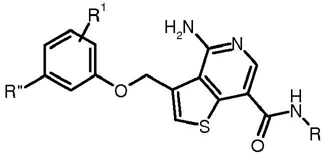

To an oven dried vessel was added compound XXVI (from General Procedure 7 *supra)* dissolved in anhydrous dioxane (25 vol) under nitrogen. Trimethylaluminium (2.0 M in heptane; 4 equivalents) was added dropwise under nitrogen at room temperature, and then the reaction mixture stirred for 10 minutes. Ethanolamine (4 equivalents) was added dropwise and the reaction mixture stirred at room temperature for a further 10 minutes before heating to 95 °C and stirring overnight. Re-treatment using 2 equivalents of reagents may be necessary. Rochelle's salt (1 vol. of a saturated solution) was added to the cooled reaction mixture and stirred for one hour. The mixture was diluted with dichloromethane (50 mL) and water (50 mL) was added. The organic layer was removed and the aqueous layer extracted with 1:1 CHCl₃:2-propanol (3 x 20 mL). The combined organic washings were dried (Na₂SO₄), filtered and the solvent removed under reduced pressure. Preparative HPLC was used to purify the final compounds.

### Intermediate 9

### N-(4-Chloro-3-trifluoromethyl-phenyl)-3-hydroxy-benzamide

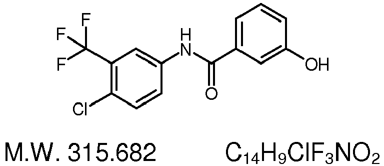

A suspension of 3-hydroxybenzoic acid (16.2 g, 117.3 mmol) (Aldrich) in acetic anhydride (20 mL) (Aldrich) was heated at reflux for 5 hours. On heating a clear solution was formed. After cooling to room temperature, mixture was poured into ice - water (400 mL) and stirred. A white crystalline material was formed. This was collected by filtration, washed with water and dried in vacuum oven to give 3-acetoxybenzoic acid as white crystals in two crops. (Yield 18.97 g, 89.8%).

A suspension of 3-acetoxybenzoic acid (1.80 g, 10 mmol) in a mixture of N,N-dimethyl-formamide (3 drops) and thionyl chloride (3 mL, 40 mmol) (Aldrich) was heated in an 90 °C bath for 2 hours. After cooling, mixture was diluted with toluene (20 mL) and concentrated under reduced pressure to remove excess thionyl chloride. The resulting oil was dissolved in dichloromethane (10 mL) and added dropwise to a solution of 4-chloro-3-(trifluoromethyl)aniline (1.96 g, 10 mmol) (Aldrich), 4-dimethylamino-pyridine (0.1 g, 0.08 mmol) (Fluka) and N,N-diisopropylethylamine (1.6 g, 12.4 mmol) (Aldrich) in dichloromethane (10 mL) with cooling in an ice - water bath and magnetic stirring. When addition was complete, cooling bath was removed and mixture stirred at room temperature for 2 hours. Reaction mixture was then partitioned between water (50 mL) and dichloromethane (2 X 50 mL). Organic layers were combined, and concentrated to dryness. Residue was dissolved in THF (10 mL), methanol (10 mL) and 1 N aqueous sodium hydroxide (10 mL) and stirred at room temperature for 16 hours. The yellow solution was diluted with water (100 mL) and acetic acid (5 mL) and extracted with ethyl acetate (2 X 100 mL). Ethyl acetate layers were washed with saturated brine (100 mL), combined, dried (MgSO₄), filtered, and concentrated. Residue was recrystallized from ethyl acetate - hexanes to give *N*-(4-chloro-3-trifluoromethyl-phenyl)-3-hydroxy-benzamide as colorless plates. (Yield 2.54 g, 80.4%).

### Intermediate 10

### 4-Chloro-3-[3-(4-chloro-3-trifluoromethyl-phenylcarbamoyl)-phenoxymethyl]-thieno[3,2-c]pyridine-7-carboxylic acid ethyl ester

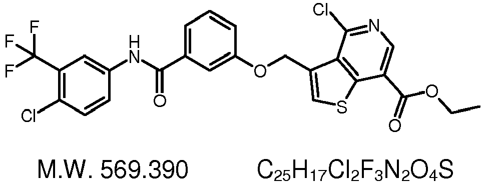

A suspension of *N*-(4-chloro-3-trifluoromethyl-phenyl)-3-hydroxy-benzamide (0.33 g, 1.05 mmol) (from Intermediate 9 *supra)* and potassium carbonate (0.16 g, 1.15 mmol) in N,N-dimethylformamide was heated at 65 °C for 2 hours with magnetic stirring. 3-Bromomethyl-4-chloro-thieno[3,2-c]pyridine-7-carboxylic acid ethyl ester (0.33 g, 1 mmol) (from Intermediate 8 *supra)* was added and mixture heated for another 5 hours at the same temperature. After cooling, mixture was partitioned between ethyl acetate (2 X 50 mL) and water (2 X 50 mL). Organic layers were washed with brine (50 mL), combined, dried (MgSO₄), filtered and concentrated. Residue was recrystallized from ethyl acetate - dichloromethane - hexanes to give 4-chloro-3-[3-(4-chloro-3-trifluoromethyl-phenylcarbamoyl)-phenoxymethyl]-thieno[3,2-c]pyridine-7-carboxylic acid ethyl ester as a white crystalline material. (Yield 0.16 g, 28.5%).

### Examples

The following examples are provided to aid the understanding of the present invention, the true scope of which is set forth in the appended claims.

### Example 1

### 4-Amino-3-[3-(4-chloro-3-trifluoromethyl-phenylcarbamoyl)-phenoxymethyl]-thieno[3,2-c]pyridine-7-carboxylic acid ethyl ester

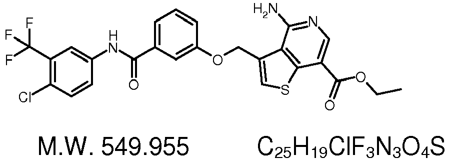

Ammonia gas was bubbled into a solution of 4-chloro-3-[4-(4-chloro-3-trifluoromethyl-phenyl-carbamoyl)-phenoxymethyl]-thieno[3,2-c]pyridine-7-carboxylic acid ethyl ester (0.11 g, 0.19 mmol) (from Intermediate 10 *supra)* in dioxane (20 mL) for 20 minutes. The mixture was heated in a sealed pressure vessel at 165 °C for 1.5 days. After cooling, the reaction mixture was concentrated. The residue was washed with hot methanol and the solid was filtered. The solution was concentrated. The residue was purified by flash chromatography eluting with (40 - 100%) ethyl acetate in hexanes to give 4-amino-3-[3-(4-chloro-3-trifluoromethyl-phenylcarbamoyl)-phenoxymethyl]-thieno[3,2-c]pyridine-7-carboxylic acid ethyl ester. (Yield 0.05 g, 50%).
HRMS (ES⁺) *m*/*z* Calcd for C₂₅H₁₉ClF₃N₃O₄S + H [(M+H)⁺]: 550.0810. Found: 550.0811.

### Intermediate 11

### 3-(3-Benzyloxy-phenoxymethyl)-4-chloro-thieno[3,2-c]pyridine-7-carboxylic acid ethyl ester

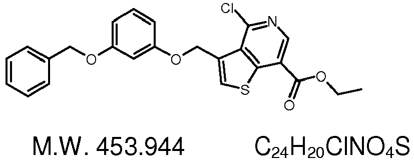

A suspension of potassium carbonate (0.16g, 1.17 mmol), 3-(benzyloxy)phenol (0.21 g, 1.07 mmol) (TCI-US) and a catalytic amount of 18-crown-6 (Aldrich) in *N,N-*dimethylformamide (15 mL) was heated at 65 °C for 2 hours. 3-Bromomethyl-4-chloro-thieno[3,2-c]pyridine-7-carboxylic acid ethyl ester (0.34 g, 1.02 mmol) (from Intermediate 8 *supra)* was then added. Heating was continued for 18 hours. The reaction mixture was partitioned between ethyl acetate and water. The aqueous phase was extracted with ethyl acetate (2X). The combined organic phase was washed with water and brine, dried (magnesium sulfate), filtered and concentrated. The residue was purified by flash chromatography eluting with 10 - 20% ethyl acetate in hexanes to give 3-(3-benzyloxy -phenoxymethyl)-4-chloro-thieno[3,2-c]pyridine-7-carboxylic acid ethyl ester. (Yield 0.14 g, 30%).
HRMS (ES⁺) *m*/*z* Calcd for C₂₄H₂₀ClNO₄S + H [(M+H)⁺]: 454.0875. Found: 454.0876.

### Example 2

### 4-Amino-3-(3-benzyloxy-phenoxymethyl)-thieno[3,2-c]pyridine-7-carboxylic acid ethyl ester

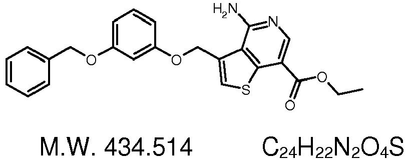

Ammonia gas was bubbled into a solution of 3-(3-benzyloxy-phenoxymethyl)-4-chloro-thieno[3,2-c]pyridine-7-carboxylic acid ethyl ester (0.14 g, 0.31 mmol) (from Intermediate 11 *supra)* in dioxane (20 mL) for 20 minutes. The mixture was heated in a sealed pressure vessel at 160 °C for 1 day. The reaction mixture was concentrated. The residue was diluted with dichloromethane and washed with water and brine, dried (magnesium sulfate), filtered and concentrated. The residue was purified by flash chromatography eluting with 5% ethyl acetate in dichloromethane to give 4-amino-3-(3-benzyloxy-phenoxymethyl)-thieno [3,2-c]pyridine-7-carboxylic acid ethyl ester. (Yield 80 mg, 62%).
HRMS (ES⁺) *m*/*z* Calcd for C₂₄H₂₂N₂O₄S + H [(M+H)⁺]: 435.1373. Found: 435.1373.

### Example 3

### 4-Amino-3-(3-benzyloxy-phenoxymethyl)-thieno[3,2-c]pyridine-7-carboxylic acid (2-hydroxy-ethyl)-amide

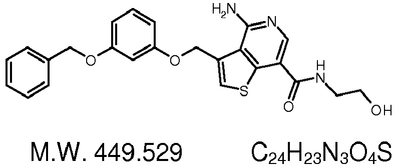

A solution of 4-amino-3-(3-benzyloxy-phenoxymethyl)-thieno [3,2-c]pyridine-7-carboxylic acid ethyl ester (0.07 g, 0.16 mmol) (from Example 2 *supra)* in dimethylsulfoxide (0.5 mL) was treated with ethanolamine (2.0 mL, 33.24 mmol) (Aldrich) and heated at 130 °C for 2 hours in a microwave reactor. The precipitate formed was filtered and washed with hot ethyl acetate and dried to give 4-amino-3-(3-benzyloxy-phenoxymethyl)-thieno[3,2-c]pyridine-7-carboxylic acid (2-hydroxy-ethyl)-amide as a white solid. (Yield 56 mg, 80%).
HRMS (ES⁺) *m*/*z* Calcd for C₂₄H₂₃N₃O₄S + H [(M+H)⁺]: 450.1482. Found: 450.1483.

### Intermediate 12

### 3-(4-Chloro-benzyloxy)-phenol

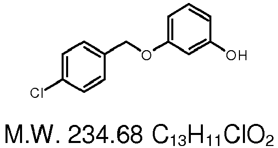

A suspension of potassium carbonate (2.02 g, 14.6 mmol) and resorcinol (16.08 g, 0.146 mol) (Aldrich) in acetonitrile (60 mL) was heated at reflux for 1 hour. A solution of 4-chlorobenzyl bromide (3.0 g, 14.6 mmol) (Aldrich) in acetonitrile (60 mL) was added. The reaction mixture was heated at reflux for 2.5 days. The solution was concentrated. The residue was partitioned between dichloromethane and water. The aqueous phase was extracted with dichloromethane (1X). The combined organic phase was washed with brine, dried (magnesium sulfate), filtered and concentrated. The residue was purified by flash chromatography eluting with 20% ethyl acetate in hexanes to give 3-(4-chloro-benzyloxy)-phenol. (Yield 2.62 g, 76%).

### Intermediate 13

### 4-Chloro-3-[3-(4-chloro-benzyloxy)-phenoxymethyl]-thieno[3,2-c]pyridine-7-carboxylic acid ethyl ester

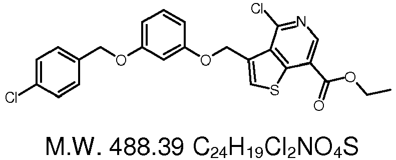

A suspension of potassium carbonate (0.16 g, 1.17 mmol), 3-(4-chloro-benzyloxy)-phenol (0.25 g, 1.07 mmol) (from Intermediate 12 *supra)* and a catalytic amount of 18-crown-6 (Aldrich) in *N,N*-dimethylformamide (15 mL) was heated at 65 °C for 2 hours. 3-Bromomethyl-4-chloro-thieno[3,2-c]pyridine-7-carboxylic acid ethyl ester (0.34 g, 1.02 mmol) (from Intermediate 8 *supra)* was added. Heating was continued for 10 hours. The reaction mixture was partitioned between ethyl acetate and water. The aqueous phase was extracted with ethyl acetate (2X). The combined organic phase was washed with water and brine, dried (magnesium sulfate), filtered and concentrated. The residue was purified by flash chromatography eluting with 10 - 20% ethyl acetate in hexanes to give 4-chloro-3-[3-(4-chloro-benzyloxy)-phenoxymethyl]-thieno[3,2-c]pyridine-7-carboxylic acid ethyl ester. (Yield 0.14 g, 28%).

### Example 4

### 4-Amino-3-[3-(4-chloro-benzyloxy)-phenoxymethyl]-thieno[3,2-c]pyridine-7-carboxylic acid ethyl ester

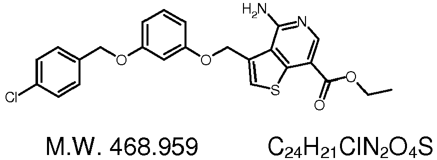

Ammonia gas was bubbled into a solution of 4-chloro-3-[3-(4-chloro-benzyloxy)-phenoxy-methyl]-thieno[3,2-c]pyridine-7-carboxylic acid ethyl ester (0.14 g, 0.29 mmol) (from Intermediate 13 *supra)* in dioxane (20 mL) for 20 minutes. The mixture was heated in a sealed pressure vessel at 160 °C for 1 day. The reaction mixture was concentrated. The residue was washed with hot methanol, filtered and dried to give 4-amino-3-[3-(4-chloro-benzyloxy)-phenoxymethyl]-thieno[3,2-c]pyridine-7-carboxylic acid ethyl ester as a white solid. (Yield 0.13 g, 100%).
HRMS (ES⁺) *m*/*z* Calcd for C₂₄H₂₁ClN₂O₄S + H [(M+H)⁺]: 469.0984. Found: 469.0983.

### Example 5

### 4-Amino-3-[3-(4-chloro-benzyloxy)-phenoxymethyl]-thieno[3,2-c]pyridine-7-carboxylic acid (2-hydroxy-ethyl)-amide

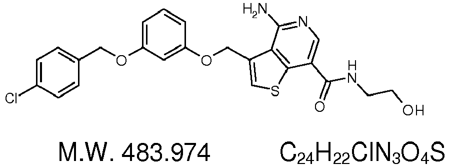

A solution of 4-amino-3-[3-(4-chloro-benzyloxy)-phenoxymethyl]-thieno[3,2-c]pyridine-7-carboxylic acid ethyl ester (0.05 g, 0.11 mmol) (from Example 4 *supra)* in dimethylsulfoxide (0.5 mL) was treated with ethanolamine (1.5 mL) (Aldrich) and heated at 130 °C for 2 hours in a microwave reactor. The precipitate formed was filtered and washed with hot ethyl acetate and dried to give 4-amino-3-[3-(4-chloro-benzyloxy)-phenoxymethyl]-thieno[3,2-c]pyridine-7-carboxylic acid (2-hydroxy-ethyl)-amide as a white solid. (Yield 40 mg, 77%).
HRMS (ES⁺) *m*/*z* Calcd for C₂₄H₂₂ClN₃O₄S + H [(M+H)⁺]: 484.1093. Found: 484.1096.

### Intermediate 14

### 3-(3-Chloro-benzyloxy)-phenol

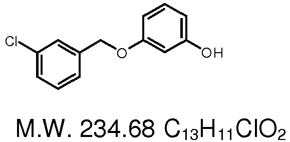

A suspension of potassium carbonate (2.02 g, 14.6 mmol) and resorcinol (16.08 g, 0.146 mol) (Aldrich) in acetonitrile (60 mL) was heated at reflux for 1 hour. A solution of 3-chlorobenzyl bromide (3.0 g, 14.6 mmol) (Aldrich) in acetonitrile (60 mL) was added. Heating at reflux was continued for 2.5 days. After cooling, the reaction mixture was concentrated under reduced pressure. The residue was partitioned between dichloromethane and water. The aqueous phase was extracted with dichloromethane (1 X). The combined organic phase was washed with brine, dried (magnesium sulfate), filtered and concentrated. The residue was purified by flash chromatography eluting with 20% ethyl acetate in hexanes to give 3-(3-chloro-benzyloxy)-phenol. (Yield 2.80 g, 82%).

### Intermediate 15

### 4-Chloro-3-[3-(3-chloro-benzyloxy)-phenoxymethyl]-thieno[3,2-c]pyridine-7-carboxylic acid ethyl ester

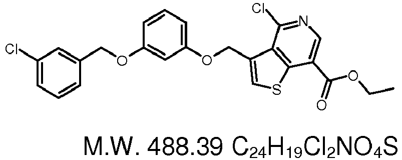

A suspension of potassium carbonate (0.16 g, 1.17 mmol), 3-(3-chloro-benzyloxy)-phenol (0.25g, 1.07 mmol) (from Intermediate 14 *supra)* and a catalytic amount of 18-crown-6 in *N,N*-dimethylformamide (15 mL) was heated at 65 °C for 2 hours. 3-Bromomethyl-4-chloro-thieno[3,2-c]pyridine-7-carboxylic acid ethyl ester (0.34 g, 1.02 mmol) (from Intermediate 8 *supra)* was added. Heating was continued for 10 hours. The reaction mixture was partitioned between ethyl acetate and water. The aqueous phase was extracted with ethyl acetate (2X). The combined organic phase was washed with water and brine, dried (magnesium sulfate), filtered and concentrated. The residue was purified by flash chromatography eluting with 10 - 20% ethyl acetate in hexanes to give 4-chloro-3-[3-(3-chloro-benzyloxy)-phenoxymethyl]-thieno[3,2-c]pyridine-7-carboxylic acid ethyl ester. (Yield 0.13 g, 26%).

### Example 6

### 4-Amino-3-[3-(3-chloro-benzyloxy)-phenoxymethyl]-thieno[3,2-c]pyridine-7-carboxylic acid ethyl ester

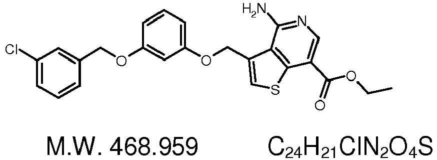

Ammonia gas was bubbled into a solution of 4-chloro-3-[3-(3-chloro-benzyloxy)-phenoxy-methyl]-thieno[3,2-c]pyridine-7-carboxylic acid ethyl ester (0.13 g, 0.27 mmol) (from Intermediate 15 *supra)* in dioxane (20 mL) for 20 minutes. The mixture was heated in a sealed pressure vessel at 160 °C for 1 day. The reaction mixture was concentrated. The residue was recrystallized from methanol to give 4-amino-3-[3-(3-chloro-benzyloxy)-phenoxy-methyl]-thieno[3,2-c]pyridine-7-carboxylic acid ethyl ester. (Yield 0.11 g, 92%).
HRMS (ES⁺) *m*/*z* Calcd for C₂₄H₂₁ClN₂O₄S + H [(M+H)⁺]: 469.0984. Found: 469.0983.

### Example 7

### 4-Amino-3-[3-(3-chloro-benzyloxy)-phenoxymethyl]-thieno[3,2-c]pyridine-7-carboxylic acid (2-hydroxy-ethyl)-amide

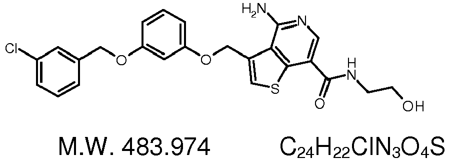

A solution of 4-amino-3-[3-(3-chloro-benzyloxy)-phenoxymethyl]-thieno[3,2-c]pyridine-7-carboxylic acid ethyl ester (0.05 g, 0.11 mmol) (from Example 6 *supra)* in dimethylsulfoxide (0.5 mL) was treated with ethanolamine (1.5 mL) (Aldrich) and heated at 130 °C for 2 hours in a microwave reactor. The precipitate formed was filtered and washed with hot ethyl acetate and dried to give 4-amino-3-[3-(4-chloro-benzyloxy)-phenoxymethyl]-thieno[3,2-c]pyridine-7-carboxylic acid (2-hydroxy-ethyl)-amide as a white solid. (Yield 25 mg, 48%).
HRMS (ES⁺) *m*/*z* Calcd for C₂₄H₂₂ClN₃O₄S + H [(M+H)⁺]: 484.1093. Found: 484.1095.

### Intermediate 16

### 3-(3-Trifluoromethyl-benzyloxy)-phenol

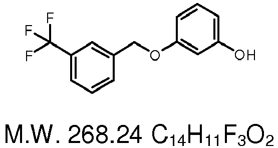

A suspension of potassium carbonate (1.73 g, 12.6 mmol) and resorcinol (13.87 g, 0.126 mol) (Aldrich) in acetonitrile (60 mL) was heated at reflux froor 1 hour. A solution of 1-bromomethyl-3-trifluoromethyl-benzene (2.3 g, 12.6 mmol) (Aldrich) in acetonitrile (60 mL) was added. The reaction mixture was heated at reflux for 2.5 days. The solution was concentrated. The residue was partitioned between dichloromethane and water. The aqueous phase was extracted with dichloromethane (1 X). The combined organic phase was washed with brine, dried (magnesium sulfate), filtered and concentrated. The residue was purified by flash chromatography eluting with 20% ethyl acetate in hexanes to 3-(3-trifluoromethyl-benzyloxy)-phenol. (Yield 2.29 g, 68%).

### Intermediate 17

### 4-Chloro-3-[3-(3-trifluoromethyl-benzyloxy)-phenoxymethyl]-thieno[3,2-c]pyridine-7-carboxylic acid ethyl ester

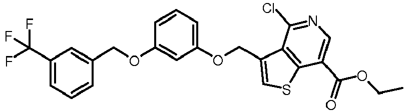

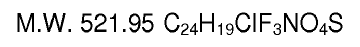

A suspension of potassium carbonate (0.16 g, 1.17 mmol), 3-(3-trifluoromethyl-benzyloxy)-phenol (0.29 g, 1.07 mmol) (from Intermediate 16 *supra)* and a catalytic amount of 18-crown-6 (Aldrich) in *N,N*-dimethylformamide (15 mL) was heated at 65 °C for 2 hours. 3-Bromomethyl-4-chloro-thieno[3,2-c]pyridine-7-carboxylic acid ethyl ester (0.34 g, 1.02 mmol) (from Intermediate 8 *supra)* was added. Heating was continued for 10 hours. The reaction mixture was partitioned between ethyl acetate and water. The aqueous phase was extracted with ethyl acetate (2X). The combined organic phase was washed with water and brine, dried (magnesium sulfate), filtered and concentrated. The residue was purified by flash chromatography eluting with 10 - 20% ethyl acetate in hexanes to give 4-chloro-3-[3-(3-trifluoromethyl-benzyloxy)-phenoxymethyl]-thieno[3,2-c]pyri-dine-7-carboxylic acid ethyl ester. (Yield 0.13 g, 25%).

### Example 8

### 4-Amino-3-[3-(3-trifluoromethyl-benzyloxy)-phenoxymethyl]-thieno[3,2-c]pyridine-7-carboxylic acid ethyl ester

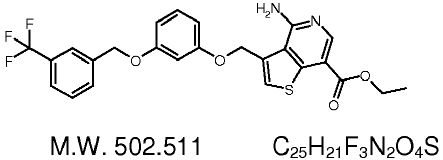

Ammonia gas was bubbled into a solution of 4-chloro-3-[3-(3-trifluoromethyl-benzyloxy)-phenoxy-methyl]-thieno[3,2-c]pyridine-7-carboxylic acid ethyl ester (0.13 g, 0.25 mmol)(from Intermediate 17 *supra)* in dioxane (20 mL) for 20 minutes. The mixture was heated in a sealed pressure vessel at 160 °C for 1 day. The reaction mixture was concentrated. The residue was washed with hot methanol to give 4-amino-3-[3-(3-trifluoromethyl-benzyloxy)-phenoxymethyl]-thieno[3,2-c]pyridine-7-carboxylic acid ethyl ester as a white powder. (Yield 0.08 g, 64%).
HRMS (ES⁺) *m*/*z* Calcd for C₂₅H₂₁F₃N₂O₄S + H [(M+H)⁺]: 503.1247. Found: 503.1246.

### Example 9

### 4-Amino-3-[3-(3-trifluoromethyl-benzyloxy)-phenoxymethyl]-thieno[3,2-c]pyridine-7-carboxylic acid (2-hydroxy-ethyl)-amide

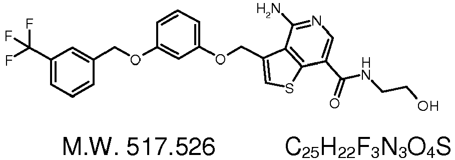

A solution of 4-amino-3-[3-(3-trifluoromethyl-benzyloxy)-phenoxymethyl]-thieno[3,2-c]pyridine-7-carboxylic acid ethyl ester (0.05 g, 0.10 mmol) (from Example 8 *supra)* in dimethylsulfoxide (0.5 mL) was treated with ethanolamine (1.5 mL) (Aldrich) and heated at 130 °C for 2 hours in a microwave reactor. The precipitate formed was filtered and washed with hot ethyl acetate and dried to give 4-amino-3-[3-(3-trifluoromethyl-benzyloxy)-phenoxy-methyl]-thieno[3,2-c]pyridine-7-carboxylic acid (2-hydroxy-ethyl)-amide as a white powder. (Yield 28 mg, 55%).
HRMS (ES⁺) *m*/*z* Calcd for C₂₅H₂₂F₃N₃O₄S + H [(M+H)⁺]: 518.1356. Found: 518.1356.

### Intermediate 18

### 3-Benzylsulfanyl-phenol

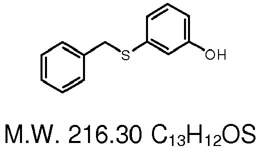

Benzyl bromide (7.61 g, 43.6 mmol) (Fluka) was added to a solution of 3-mercaptophenol (5.0 g, 39.6 mmol) (Aldrich) and sodium hydroxide (1.78 g, 43.6 mmol) in methanol (100 mL). Mixture was stirred at room temperature overnight then diluted with water (200 mL) and acetic acid (10 mL) and concentrated to remove organic solvent. Resulting precipitate was collected and washed with water to give 3-benzylsulfanyl-phenol. (Yield 7.40 g, 86.3%).

### Intermediate 19

### 3-(3-Benzylsulfanyl-phenoxymethyl)-4-chloro-thieno[3,2-c]pyridine-7-carboxylic acid ethyl ester

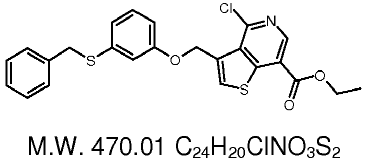

A suspension of potassium carbonate (0.48 g, 3.44 mmol), 3-benzylsulfanyl-phenol (0.69 g, 3.17 mmol) (from Intermediate 18 *supra)* in tetrahydrofuran / *N,N-*dimethylformamide (5:1, 30 mL) was heated at 70 °C for 3 hours. 3-Bromomethyl-4-chloro-thieno[3,2-c]pyridine-7-carboxylic acid ethyl ester (1.0 g, 2.99 mmol) (from Intermediate 8 *supra)* was added. Heating was continued for 1 day. The reaction mixture was partitioned between ethyl acetate and water. The aqueous phase was extracted with ethyl acetate (2X). The combined organic phase was washed with water and brine, dried (magnesium sulfate), filtered and concentrated. The residue was purified by flash chromatography eluting with 10 - 20% ethyl acetate in hexanes to give 3-(3-benzyl-sulfanyl-phenoxymethyl)-4-chloro-thieno[3,2-c]pyridine-7-carboxylic acid ethyl ester. (Yield 0.78 g, 56%).

### Example 10

### 4-Amino-3-(3-benzylsulfanyl-phenoxymethyl)-thieno[3,2-c]pyridine-7-carboxylic acid ethyl ester

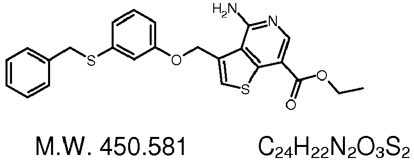

Ammonia gas was bubbled into a solution of 3-(3-benzylsulfanyl-phenoxymethyl)-4-chloro-thieno[3,2-c]pyridine-7-carboxylic acid ethyl ester (0.78 g, 0.25 mmol) (from Intermediate 19 *supra)* in dioxane (20 mL) for 20 minutes. The mixture was heated in a sealed pressure vessel at 160 °C for 1 day. The reaction mixture was then concentrated. The residue was purified by flash chromatography eluting with 5% ethyl acetate in dichloromethane to give 4-amino-3-(3-benzylsulfanyl-phenoxymethyl)-thieno[3,2-c]pyridine-7-carboxylic acid ethyl ester as a white powder. (Yield 0.18 g, 50%).
HRMS (ES⁺) *m*/*z* Calcd for C₂₄H₂₂N₂O₃S₂ + H [(M+H)⁺]: 451.1145. Found: 451.1143.

### Example 11

### 4-Amino-3-(3-benzylsulfanyl-phenoxymethyl]-thieno[3,2-c]pyridine-7-carboxylic acid (2-hydroxy-ethyl)-amide

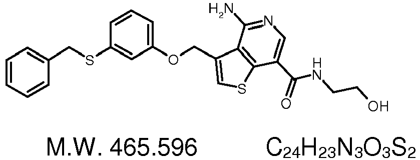

A solution of 4-amino-3-(3-benzylsulfanyl-phenoxymethyl]-thieno[3,2-c]pyridine-7-carboxylic acid ethyl ester (0.05 g, 0.10 mmol) (from Example 10 *supra)* in dimethylsulfoxide (0.5 mL) was treated with ethanolamine (1.5 mL) (Aldrich) and heated at 130 °C for 2 hours in a microwave reactor. The precipitate formed was filtered and washed with hot ethyl acetate and dried to give 4-amino-3-(3-benzylsulfanyl-phenoxymethyl]-thieno[3,2-c]pyridine-7-carboxy-lic acid (2-hydroxy-ethyl)-amide as a white powder. (Yield 23 mg, 37%).
HRMS (ES⁺) *m*/*z* Calcd for C₂₄H₂₃N₃O₃S₂ + H [(M+H)⁺]: 466.1254. Found: 466.1255.

### Example 12

### 4-Amino-3-(3-phenylmethanesulfonyl-phenoxymethyl)-thieno[3,2-c]pyridine-7-carboxylic acid ethyl ester

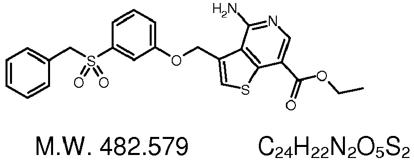

To a solution of 4-amino-3-(3-benzylsulfanyl-phenoxymethyl)-thieno[3,2-c]pyridine-7-carboxylic acid ethyl ester (0.10 g, 0.22 mmol) (from Example 10 *supra)* in dichloromethane (10 mL) at -10 °C was added 3-chloro-peroxybenzoic acid (0.16 g, 0.55 mmol) (Aldrich). The reaction mixture was stirred at 0 °C for 3 hours. The mixture was concentrated. The residue was purified by C18 column chromatography eluting with acetonitrile - water to give 4-amino-3-(3-phenylmethanesulfonyl-phenoxymethyl)-thieno[3,2-c]pyridine-7-carboxylic acid ethyl ester. (Yield 0.05 g, 45%).
HRMS (ES⁺) *m*/*z* Calcd for C₂₄H₂₂N₂O₅S₂ + H [(M+H)⁺]: 483.1043. Found: 483.1047.

### Example 13

### 4-Amino-3-(3 phenylmethanesulfonyl-phenoxymethyl]-thieno[3,2-c]pyridine-7-carboxylic acid (2-hydroxy-ethyl)-amide

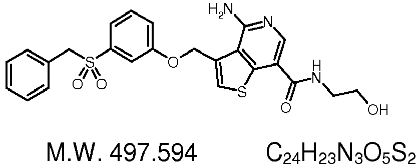

A solution of 4-amino-3-(3-phenylmethanesulfonyl-phenoxymethyl)-thieno[3,2-c]pyridine-7-carboxylic acid ethyl ester (0.04 g, 0.08 mmol) (from Example 12 *supra)* in dimethylsulfoxide (0.5 mL) was treated with ethanolamine (1.5 mL) (Aldrich) and heated at 130 °C for 2 hours in a microwave reactor. The crude material was purified by C18 column chromatography eluting with acetonitrile - water to give 4-amino-3-(3-phenylmethanesulfonyl-phenoxy-methyl]-thieno[3,2-c]pyridine-7-carboxylic acid (2-hydroxy-ethyl)-amide. (Yield 20 mg, 49%).
HRMS (ES⁺) *m*/*z* Calcd for C₂₄H₂₃N₃O₅S₂ + H [(M+H)⁺]: 498.1152. Found: 498.1151.

### Intermediate 20

### 3-(3-Methoxy-benzyloxy)-phenol

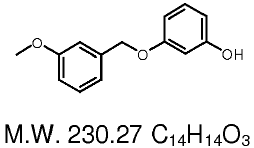

A suspension of potassium carbonate (2.02 g, 14.6 mmol) and resorcinol (16.08 g, 0.146 mol) (Aldrich) in acetonitrile (60 mL) was heated at reflux for 1 hour. A solution of 3-methoxybenzyl bromide (2.94 g, 14.6 mmol) (Aldrich) in acetonitrile (60 mL) was added. The reaction mixture was heated at reflux for 2.5 days. The solution was concentrated. The residue was partitioned between dichloromethane and water. The aqueous phase was extracted with dichloromethane. The combined organic phase was washed with brine, dried (magnesium sulfate), filtered, and concentrated. The residue was purified by flash chromatography eluting with 20% ethyl acetate in hexanes to 3-(3-methoxy-benzyloxy)-phenol. (Yield 2.43 g, 72%).

### Intermediate 21

### 4-Chloro-3-[3-(3-methoxy-benzyloxy)-phenoxymethyl]-thieno[3,2-c]pyridine-7-carboxylic acid ethyl ester

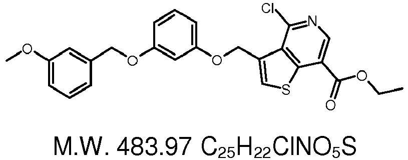

A suspension of potassium carbonate (0.16 g, 1.17 mmol), 3-(3-methoxy-benzyloxy)-phenol (0.25 g, 1.07 mmol) (from Intermediate 20 *supra)* in tetrahydrofuran - *N,N-*dimethylformamide (5:1, 30 mL) was heated at 65 °C for 2 hours. 3-Bromomethyl-4-chloro-thieno[3,2-c]pyridine-7-carboxylic acid ethyl ester (0.34 g, 1.02 mmol) (from Intermediate 8 *supra)* was added. Heating was continued for 10 hours. The reaction mixture was partitioned between ethyl acetate and water. The aqueous phase was extracted with ethyl acetate (2X). The combined organic phase was washed with water and brine, dried (magnesium sulfate), filtered, and concentrated. The residue was purified by flash chromatography eluting with 10 - 20% ethyl acetate in hexanes gradient to give 4-chloro-3-[3-(3-methoxy-benzyloxy)-phenoxymethyl]-thieno[3,2-c]pyridine-7-carboxylic acid ethyl ester. (Yield 0.21 g, 43%).

### Example 14

### 4-Amino-3-[3-(3-methoxy-benzyloxy)-phenoxymethyl]-thieno[3,2-c]pyridine-7-carboxylic acid ethyl ester

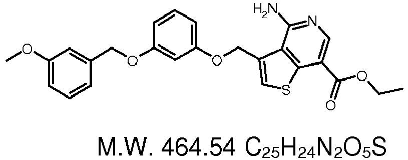

Ammonia gas was bubbled into a solution of 4-chloro-3-[3-(3-methoxy-benzyloxy)-phenoxy-methyl]-thieno[3,2-c]pyridine-7-carboxylic acid ethyl ester (0.21 g, 0.43 mmol) (from Intermediate 21 *supra)* in dioxane (20 mL) for 20 minutes. The mixture was heated in a sealed pressure vessel at 160 °C for 1 day. The reaction mixture was concentrated. The residue was washed with hot methanol to give 4-amino-3-[3-(3-methoxy-benzyl-oxy)-phenoxy-methyl]-thieno[3,2-c]pyridine-7-carboxylic acid ethyl ester as a white powder. (Yield 0.15 g, 75%).
HRMS (ES⁺) *m*/*z* Calcd for C₂₅H₂₄N₂O₅S + H [(M+H)⁺]: 465.1479. Found: 465.1479.

### Example 15

### 4-Amino-3-[3-(3-methoxy-benzyloxy)-phenoxymethyl]-thieno[3,2-c]pyridine-7-carboxylic acid (2-hydroxy-ethyl)-amide

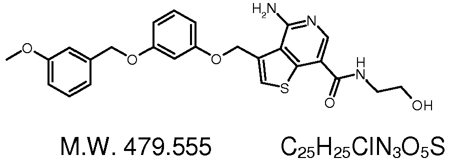

A solution of 4-amino-3-[3-(3-methoxy-benzyloxy)-phenoxymethyl]-thieno[3,2-c]pyridine-7-carboxylic acid ethyl ester (0.05 g, 0.11 mmol) (from Example 14 *supra)* in dimethylsulfoxide (0.5 mL) was treated with ethanolamine (1.5 mL) (Aldrich) and heated at 130 °C for 2 hpur in a microwave reactor. The precipitate was filtered and washed with hot ethyl acetate and dried to give 4-amino-3-[3-(4-methoxy-benzyloxy)-phenoxymethyl]-thieno[3,2-c]pyridine-7-carboxylic acid (2-hydroxy-ethyl)-amide as a white powder. (Yield 34 mg, 65%).
HRMS (ES⁺) *m*/*z* Calcd for C₂₅H₂₅ClN₃O₅S + H [(M+H)⁺]: 480.1588. Found: 480.1587.

### Intermediate 22

### 3-(4-Methoxy-benzyloxy)-phenol

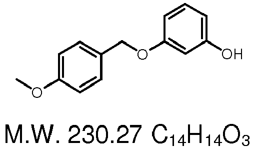

A suspension of potassium carbonate (3.53, 25.5 mmol) and resorcinol (28.12 g, 0.26 mol) (Aldriah) in acetonitrile (60 mL) was heated at reflux for 1 hour. A solution of 4-methoxybenzyl chloride (4.0 g, 25.5 mmol) (Aldrich) in acetonitrile (60 mL) was added. The reaction mixture was heated at reflux for 2.5 days. The solution was concentrated. The residue was partitioned between dichloromethane and water. The aqueous phase was extracted with dichloromethane. The combined organic phase was washed with brine, dried (magnesium sulfate), filtered, and concentrated. The residue was purified by flash chromatography eluting with 20% ethyl acetate in hexanes to give 3-(4-methoxy-benzyloxy)-phenol. (Yield 0.37 g, 6%).

### Intermediate 23

### 4-Chloro-3-[3-(4-methoxy-benzyloxy)-phenoxymethyl]-thieno[3,2-c]pyridine-7-carboxylic acid ethyl ester

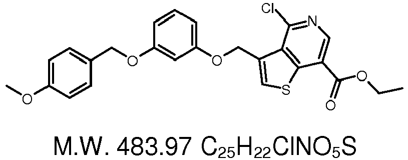

A suspension of potassium carbonate (0.16 g, 1.17 mmol), 3-(4-methoxy-benzyloxy)-phenol (0.25 g, 1.07 mmol) (from Intermediate 22 *supra*) in tetrahydrofuran - *N,N-*dimethylformamide (5:1, 30 mL) was heated at 65 °C for 2 hours. 3-Bromomethyl-4-chloro-thieno[3,2-c]pyridine-7-carboxylic acid ethyl ester (0.34 g, 1.02 mmol) (from Intermediate 8 *supra*) was added. Heating was continued for 10 hours. The reaction mixture was partitioned between ethyl acetate and water. The aqueous phase was extracted with ethyl acetate (2X). The combined organic phase was washed with water and brine, dried (magnesium sulfate), filtered, and concentrated. The residue was purified by flash chromatography eluting with 10 - 20% ethyl acetate in hexanes gradient to give 4-chloro-3-[3-(4-methoxy-benzyloxy)-phenoxymethyl]-thieno[3,2-c]pyridine-7-carboxylic acid ethyl ester. (Yield 0.16 g, 33%).

### Example 16

### 4-Amino-3-[3-(4-methoxy-benzyloxy)-phenoxymethyl]-thieno[3,2-c]pyridine-7-carboxylic acid ethyl ester

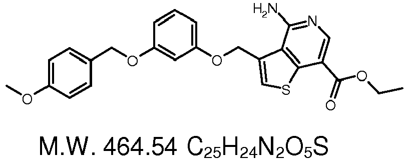

Ammonia gas was bubbled into a solution of 4-chloro-3-[3-(4-methoxy-benzyloxy)-phenoxy-methyl]-thieno[3,2-c]pyridine-7-carboxylic acid ethyl ester (0.16 g, 0.43 mmol) (from Intermediate 23 *supra*) in dioxane (20 mL) for 20 minutes. The mixture was heated in a sealed pressure vessel at 160 °C for 1 day. The reaction mixture was concentrated. The residue was washed with hot methanol to give 4-amino-3-[3-(4-methoxy-benzyloxy)-phenoxy-methyl]-thieno[3,2-c]pyridine-7-carboxylic acid ethyl ester as a white powder. (Yield 0.10 g, 67%).
HRMS (ES⁺) *m*/*z* Calcd for C₂₅H₂₄N₂O₅S + H [(M+H)⁺]: 465.1479. Found: 465.1477.

### Example 17

### 4-Amino-3-[3-(4-methoxy-benzyloxy)-phenoxymethyl]-thieno[3,2-c]pyridine-7-carboxylic acid (2-hydroxy-ethyl)-amide

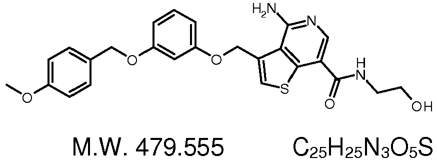

A solution of 4-amino-3-[3-(4-methoxy-benzyloxy)-phenoxymethyl]-thieno[3,2-c]pyridine-7-carboxylic acid ethyl ester (0.05 g, 0.11 mmol) (from Example 16 *supra*) in dimethylsulfoxide (0.5 mL) was treated with ethanolamine (1.5 mL) (Aldrich) and heated at 130 °C for 2 hours in a microwave reactor. The reaction mixture was purified by C18 column chromatography eluting with acetonitrile - water to give 4-amino-3-[3-(4-methoxy-benzyloxy)-phenoxymethyl]-thieno[3,2-c]pyridine-7-carboxylic acid (2-hydroxy-ethyl)-amide as a white powder. (Yield 34 mg, 65%). HRMS (ES⁺) *m*/*z* Calcd for C₂₅H₂₅CIN₃O₅S + H [(M+H)⁺]: 480.1588. Found: 480.1587.

### Intermediate 24

### 3-(4-Methoxy-3-trifluoromethyl-benzyloxy)-phenol

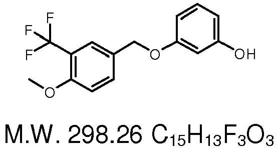

A suspension of potassium carbonate (1.44 g, 10.4 mmol) and resorcinol (11.01 g, 0.10 mol) (Aldrich) in acetonitrile (60 mL) was heated at reflux for 1 hour. A solution of 4-methoxy-3-(trifluoromethyl)-benzyl bromide (2.8 g, 10.4 mmol) (Matrix) in acetonitrile (60 mL) was added. The reaction mixture was heated at reflux for 2.5 days. The solution was concentrated. The residue was partitioned between dichloromethane and water. The aqueous phase was extracted with dichloromethane. The combined organic phase was washed with brine, dried (magnesium sulfate), filtered and concentrated. The residue was purified by flash chromatography eluting with 20% ethyl acetate in hexanes to give 3-(4-methoxy-3-trifluoro-methyl-benzyloxy)-phenol. (Yield 0.99 g, 32%).

### Intermediate 25

### 4-Chloro-3-[3-(4-methoxy-3-trifluoromethyl-benzyloxy)-phenoxymethyl]-thieno[3,2-c]pyridine-7-carboxylic acid ethyl ester

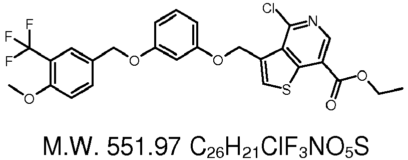

A suspension of potassium carbonate (0.16 g, 1.17 mmol), 3-(4-methoxy-3-trifluoromethyl-benzyloxy)-phenol (0.32 g, 1.07 mmol) (from Intermediate 24 *supra*) and a catalytic amount of 18-crown-6 (Aldrich) in *N,N*-dimethylformamide (15 mL) was heated at 65 °C for 2 hours. 3-Bromomethyl-4-chloro-thieno[3,2-c]pyridine-7-carboxylic acid ethyl ester (0.34 g, 1.02 mmol) (from Intermediate 8 *supra*) was added. Heating was continued for 10 hours. The reaction mixture was partitioned between ethyl acetate and water. The aqueous phase was extracted with ethyl acetate (2X). The combined organic phase was washed with water and brine, dried (magnesium sulfate), filtered and concentrated. The residue was purified by flash chromatography eluting with 10 - 20% ethyl acetate in hexanes to give 4-chloro-3-[3-(4-methoxy-3-trifluoromethyl-benzyloxy)-phenoxymethyl]-thieno[3,2-c]pyridine-7-carboxylic acid ethyl ester. (Yield 0.12 g, 21%).

### Example 18

### 4-Amino-3-[3-(4-methoxy-3-trifluoromethyl-benzyloxy)-phenoxymethyl]-thieno[3,2-c]pyridine-7-carboxylic acid ethyl ester

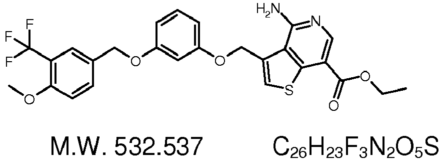

Ammonia gas was bubbled into a solution of 4-chloro-3-[3-(4-methoxy-3-trifluoromethyl-benzyl-oxy)-phenoxymethyl]-thieno[3,2-c]pyridine-7-carboxylic acid ethyl ester (0.12 g, 0.22 mmol) (from intermediate 25 *supra*) in dioxane (20 mL) for 20 minutes. The mixture was heated in a sealed pressure vessel at 160 °C for 1 day. The reaction mixture was concentrated. The residue was purified by flash chromatography eluting with 5% ethyl acetate in dichloromethane to give 4-amino-3-[3-(4-methoxy-3-trifluoromethyl-benzyl-oxy)-phenoxy-methyl]-thieno[3,2-c]pyridine-7-carboxylic acid ethyl ester as a white powder. (Yield 0.065 g, 54%).
HRMS (ES⁺) *m*/*z* Calcd for C₂₆H₂₃F₃N₂O₅S + H [(M+H)⁺]: 533.1353. Found: 533.1352.

### Example 19

### 4-Amino-3-[3-(4-methoxy-3-trifluoromethyl-benzyloxy)-phenoxymethyl]-thieno[3,2-c]pyridine-7-carboxylic acid (2-hydroxy-ethyl)-amide

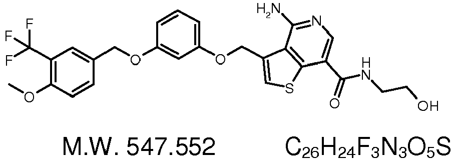

A solution of 4-amino-3-[3-(4-methoxy-3-trifluoromethyl-benzyloxy)-phenoxymethyl]-thieno[3,2-c]pyridine-7-carboxylic acid ethyl ester (0.05 g, 0.10 mmol) (from Example 18 *supra*) in dimethylsulfoxide (0.5 mL) was treated with ethanolamine (1.5 mL) (Aldrich) and heated at 130 °C for 2 hours in a microwave reactor. The crude material was purified by C18 column chromatography eluting with acetonitrile - water to give 4-amino-3-[3-(4-methoxy-3-trifluoro-methyl-benzyloxy)-phenoxymethyl]-thieno[3,2-c]pyridine-7-carboxylic acid (2-hydroxy-ethyl)-amide as a white powder. (Yield 20 mg, 39%).
HRMS (ES⁺) *m*/*z* Calcd for C₂₆H₂₄F₃N₃O₅S + H [(M+H)⁺]: 548.1462. Found: 548.1463.

### Intermediate 26

### 4-Chloro-3-(3-phenoxy-phenoxymethyl)-thieno[3,2-c]pyridine-7-carboxylic acid ethyl ester

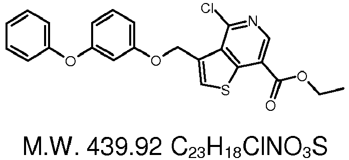

A suspension of potassium carbonate (0.16 g, 1.17 mmol), 3-phenoxyphenol (0.20 g, 1.07 mmol) (Aldrich) in tetrahydrofuran - N,N-dimethylformamide (5:1, 30 mL) was heated at 70 °C for 3 hours. 3-Bromomethyl-4-chloro-thieno[3,2-c]pyridine-7-carboxylic acid ethyl ester (0.34 g, 1.02 mmol) (from Intermediate 8 *supra*) was added. Heating was continued for 1 day. The reaction mixture was partitioned between ethyl acetate and water. The aqueous phase was extracted with ethyl acetate (2X). The combined organic phase was washed with water and brine, dried (magnesium sulfate), filtered and concentrated. The residue was purified by flash chromatography eluting with 10 - 20% ethyl acetate in hexanes to give 4-chloro-3-(3-phenoxy-phenoxymethyl)-thieno[3,2-c]pyridine-7-carboxylic acid ethyl ester. (Yield 0.22 g, 49%).

### Example 20

### 4-Amino-3-(3-phenoxy-phenoxymethyl)-thieno[3,2-c]pyridine-7-carboxylic acid ethyl ester

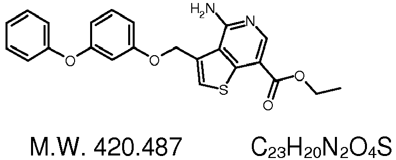

Ammonia gas was bubbled into a solution of 4-chloro-3-(3-phenoxy-phenoxymethyl)-thieno[3,2-c]pyridine-7-carboxylic acid ethyl ester (0.22 g, 0.25 mmol) (from Intermediate 26 *supra*) in dioxane (20 mL) for 20 minutes. The mixture was heated in a sealed pressure vessel at 160 °C for 1 day. The reaction mixture was concentrated. The residue residue was recrystallized from ethyl acetate - hexanes to give 4-amino-3-(3-phenoxy-phenoxymethyl)-thieno[3,2-c]pyridine-7-carboxylic acid ethyl ester as white crystals. (Yield 0.10 g, 48%).
HRMS (ES⁺) *m*/*z* Calcd for C₂₃H₂₀N₂O₄S + H [(M+H)⁺]: 421.1217. Found: 421.1216.

### Example 21

### 4-Amino-3-(3-phenoxy-phenoxymethyl]-thieno[3,2-c]pyridine-7-carboxylic acid (2-hydroxy-ethyl)-amide

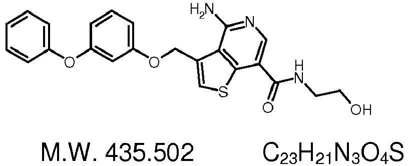

A solution of 4-amino-3-(3-phenoxy-phenoxymethyl]-thieno[3,2-c]pyridine-7-carboxylic acid ethyl ester (0.05 g, 0.12 mmol) (from Example 20 *supra*) in dimethylsulfoxide (0.5 mL) was treated with ethanolamine (1.5 mL) (Aldrich) and heated at 130 °C for 2 hours in a microwave reactor. The precipitate was filtered and washed with hot methanol and dried to give 4-amino-3-(3-phenoxy-phenoxymethyl]-thieno[3,2-c]pyridine-7-carboxylic acid (2-hydroxy-ethyl)-amide as a white powder. (Yield 40 mg, 77%).
HRMS (ES⁺) *m*/*z* Calcd for C₂₃H₂₁N₃O₄S + H [(M+H)⁺]: 436.1326. Found: 436.1326.

### Intermediate 27

### 4-Chloro-3-(3-phenylamino-phenoxymethyl)-thieno[3,2-c]pyridine-7-carboxylic acid ethyl ester

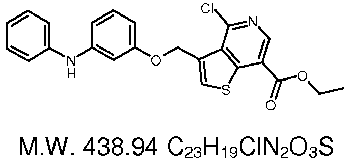

A suspension of potassium carbonate (0.16 g, 1.17 mmol), 3-hydroxydiphenylamine (0.20 g, 1.07 mmol) (Alfa Aesar) in tetrahydrofuran - *N,N*-dimethylformamide (5:1, 30 mL) was heated at 70 °C for 3 hours. 3-Bromomethyl-4-chloro-thieno[3,2-c]pyridine-7-carboxylic acid ethyl ester (0.34 g, 1.02 mmol) (from Intermediate 8 *supra*) was added. Heating was continued for 1 day. The reaction mixture was partitioned between ethyl acetate and water. The aqueous phase was extracted with ethyl acetate (2X). The combined organic phase was washed with water and brine, dried (magnesium sulfate), filtered and concentrated. The residue was purified by flash chromatography eluting with 10 - 20% ethyl acetate in hexanes to give 4-chloro-3-(3-phenylamino-phenoxymethyl)-thieno[3,2-c]pyridine-7-carboxylic acid ethyl ester. (Yield 0.16 g, 36%).

### Example 22

### 4-Amino-3-(3-phenylamino-phenoxymethyl)-thieno[3,2-c]pyridine-7-carboxylic acid ethyl ester

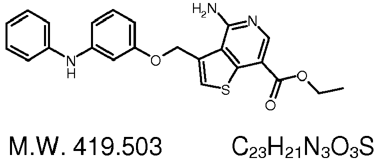

Ammonia gas was bubbled into a solution of 4-chloro-3-(3-phenylamino-phenoxymethyl)-thieno[3,2-c]pyridine-7-carboxylic acid ethyl ester (0.16 g, 0.36 mmol) (from Intermediate 27 *supra*) in dioxane (20 mL) for 20 minutes. The mixture was heated in a sealed pressure vessel at 160 °C for 1 day. The reaction mixture was concentrated. The residue was washed with methanol and dried to give 4-amino-3-(3-phenyiamino-phenoxymethyl)-thieno[3,2-c]pyridine-7-carboxylic acid ethyl ester as a white powder. (Yield 0.10 g, 67%).
HRMS (ES⁺) *m*/*z* Calcd for C₂₃H₂₁N₃O₃S + H [(M+H)⁺]: 420.1377. Found: 420.1375.

### Example 23

### 4-Amino-3-(3-phenylamino-phenoxymethyl]-thieno[3,2-c]pyridine-7-carboxylic acid (2-hydroxy-ethyl)-amide

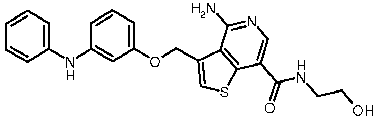

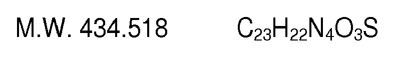

A solution of 4-amino-3-(3-phenylamino-phenoxymethyl]-thieno[3,2-c]pyridine-7-carboxylic acid ethyl ester (0.05 g, 0.12 mmol) (from Example 22 *supra*) in dimethylsulfoxide (0.5 mL) was treated with ethanolamine (1.5 mL) (Aldrich) and heated at 130 °C for 2 hours in a microwave reactor. The crude material was purified by C18 column chromatography eluting with acetonitrile - water to give 4-amino-3-(3-phenylamino-phenoxymethyl]-thieno[3,2-c]pyridine-7-carboxylic acid (2-hydroxy-ethyl)-amide as a white powder. (Yield 30 mg, 58%).
HRMS (ES⁺) *m*/*z* Calcd for C₂₃H₂₂N₄O₃S + H [(M+H)⁺]: 435.1486. Found: 435.1485.

### Intermediate 28

### Benzoic acid 3-benzoylamino-phenyl ester

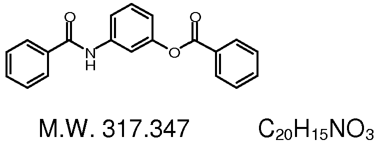

A solution of benzoyl chloride (3.0 g, 21.3 mmol) (J. T. Baker) in tetrahydrofuran (15 mL) was added dropwise at room temperature with magnetic stirring to a solution of m-aminophenol (1.09 g, 10 mmol) (Eastman Organic), 4-dimethyl amino-pyridine (20 mg) (Fluka), and triethylamine (2.54 g, 25 mmol) (Aldrich) in tetrahydrofuran (30 mL). After stirring for 1 hour, precipitate was filtered off and washed with ether. Combined filtrate was comcentrated under reduced pressure and residue recrystallized from dichloromethnae - hexanes to give benzoic acid 3-benzoylamino-phenyl ester as a tan crystalline solid. (Yield 3.08 g, 97.1 %).

### Intermediate 29

### 3-Benzylamino-phenol

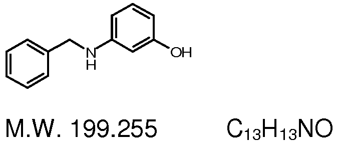

Lithium aluminum hydride solution (19 mL, 1 M in tetrahydrofuran) (Aldrich) was added slowly to a suspension of the benzoic acid 3-benzoylamino-phenyl ester (2.0 g, 6.3 mmol) (from Intermediate 28 *supra*) in anhydrous tetrahydrofuran (30 mL) under an argon atmosphere with magnetic stirring at room temperature (exothermic reaction). When addition was complete, mixture was heated at reflux for 1 hour. Reaction mixture was allowed to cool to room temperature and was quenched by pouring slowly into 15% aqueous ammonium chloride solution (100 mL) and ether (100 mL). After stirring thoroughly, mixture was diluted with ethyl acetate (100 mL) and filtered through Celite^{®}. Filtercake was washed thoroughly with ethyl acetate. Combined filtrate and washing was concentrated under reduced pressure. The resulting oil was purified by flash chromatography (Biotage 40M silica gel, dichloromethane, then 5% ethyl acetate in dichloromethane as solvent) to give crude product as a brown oil containing benzyl alcohol. This material was further purified by flash chromatography (Biotage 40L silica gel, 15% then 30% acetone in hexanes as solvent) to give 3-benzylamino-phenol as a colorless oil. (Yield 0.93 g, 74.1 %).

### Intermediate 30

### 3-(3-Benzylamino-phenoxymethyl)-4-chloro-thieno[3,2-c]pyridine-7-carboxylic acid ethyl ester

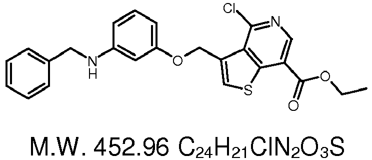

A suspension of potassium carbonate (0.32 g, 2.31 mmol), 3-benzylamino-phenol (0.42 g, 2.11 mmol) (from Intermediate 29 *supra*) in tetrahydrofuran - *N,N-*dimethylformamide (5:1, 30 mL) was heated at 70 °C for 3 hours. 3-Bromomethyl-4-chloro-thieno[3,2-c]pyridine-7-carboxylic acid ethyl ester (0.68 g, 2.03 mmol) (from Intermediate 8 *supra*) was added. Heating was continued for 1 day. The reaction mixture was partitioned between ethyl acetate and water. The aqueous phase was extracted with ethyl acetate (2X). The combined organic phase was washed with water and brine, dried (magnesium sulfate), filtered and concentrated. The residue was purified by flash chromatography eluting with 20% ethyl acetate in hexanes to give 3-(3-benzyl-amino-phenoxymethyl)-4-chloro-thieno[3,2-c]pyridine-7-carboxylic acid ethyl ester. (Yield 0.51 g, 55%).

### Example 24

### 4-Amino-3-(3-benzylamino-phenoxymethyl)-thieno[3,2-c]pyridine-7-carboxylic acid ethyl ester

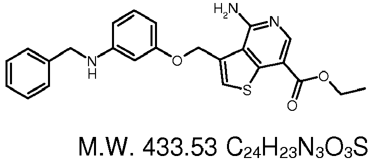

Ammonia gas was bubbled into a solution of 3-(3-benzylamino-phenoxymethyl)-4-chloro-thieno[3,2-c]pyridine-7-carboxylic acid ethyl ester (0.51 g, 0.25 mmol) (from Intermediate 30 *supra*) in dioxane (20 mL) for 20 minutes. The mixture was heated in a sealed pressure vessel at 160 °C for 1 day. The reaction mixture was concentrated. The residue was purified by C18 column chromatography eluting with acetonitrile - water to give 4-amino-3-(3-benzylamino-phenoxymethyl)-thieno[3,2-c]pyridine-7-carboxylic acid ethyl ester as a white powder. (Yield 0.064 g, 13%) and recover starting material.
HRMS (ES⁺) *m*/*z* Calcd for C₂₄H₂₃N₃O₃S + H [(M+H)⁺]: 434.1533. Found: 434.1532.

### Example 25

### 4-Amino-3-(3-benzylamino-phenoxymethyl]-thieno[3,2-c]pyridine-7-carboxylic acid (2-hydroxy-ethyl)-amide

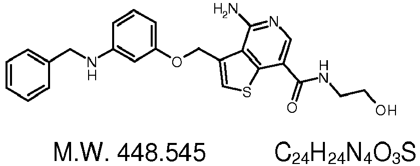

A solution of 4-amino-3-(3-benzylamino-phenoxymethyl]-thieno[3,2-c]pyridine-7-carboxylic acid ethyl ester (0.05 g, 0.10 mmol) (from Example 24 *supra*) in dimethylsulfoxide (0.5 mL) was treated with ethanolamine (2.0 mL) (Aldrich) and heated at 130 °C for 2 hours in a microwave reactor. The reaction mixture was puried by C18 column chromatography eluting with acetonitrile - water to give 4-amino-3-(3-benzylamino-phenoxymethyl]-thieno[3,2-c]pyridine-7-carboxylic acid (2-hydroxy-ethyl)-amide as a white powder. (Yield 22 mg, 42%).
HRMS (ES⁺) *m*/*z* Calcd for C₂₄H₂₄N₄O₃S + H [(M+H)⁺]: 449.1642. Found: 449.1640.

### Intermediate 31

### 4-(3-Hydroxy-phenoxymethyl)-benzonitrile

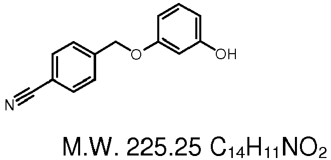

A suspension of potassium carbonate (2.11 g, 15.3 mmol) and resorcinol (16.85 g, 0.153 mol) (Aldrich) in acetonitrile (60 mL) was heated at reflux for 1 hour. A solution of 4-bromomethyl-benzonitrile (3.0 g, 15.3 mmol) (Aldrich) in acetonitrile (60 mL) was added. The reaction mixture was heated at reflux for 2.5 days. The solution was concentrated. The residue was partitioned between dichloromethane and water. The aqueous phase was extracted with dichloromethane (1X). The combined organic phase was washed with brine, dried (magnesium sulfate), filtered and concentrated. The residue was washed with hot methylene chloride. The solid was filtered and dried to give 4-(3-hydroxy-phenoxymethyl)-benzonitrile. (Yield 1.77 g, 51 %).

### Intermediate 32

### 4-Chloro-3-[3-(4-cyano-benzyloxy)-phenoxymethyl]-thieno[3,2-c]pyridine-7-carboxylic acid ethyl ester

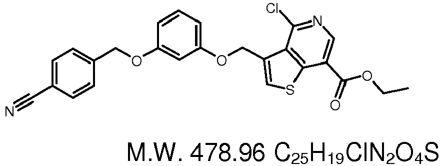

A suspension of potassium carbonate (0.24 g, 1.71 mmol), 4-(3-hydroxy-phenoxymethyl)-benzonitrile (0.35 g, 1.56 mmol) (from Intermediate 31 *supra*) in tetrahydrofuran - *N,N-*dimethylformamide (5:1, 18 mL) was heated at 70 °C for 3 hours. 3-Bromomethyl-4-chloro-thieno[3,2-c]pyridine-7-carboxylic acid ethyl ester (0.50 g, 1.49 mmol) (from Intermediate 8 *supra*) was added. Heating was continued for 18 hours. The reaction mixture was partitioned between ethyl acetate and water. The aqueous phase was extracted with ethyl acetate (1X). The combined organic phase was washed with water and brine, dried (magnesium sulfate), filtered and concentrated. The residue was purified by flash chromatography eluting with 0 - 5% ethyl acetate in dichloromethane to give 4-chloro-3-[3-(4-cyano-benzyloxy)-phenoxymethyl]-thieno[3,2-c]pyri-dine-7-carboxylic acid ethyl ester. (Yield 0.38 g, 54%).

### Example 26

### 4-Amino-3-[3-(4-cyano-benzyloxy)-phenoxymethyl]-thieno[3,2-c]pyridine-7-carboxylic acid ethyl ester

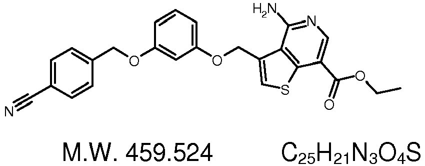

Ammonia gas was bubbled into a solution of 4-chloro-3-[3-(4-cyano-benzyloxy)-phenoxymethyl]-thieno[3,2-c]pyridine-7-carboxylic acid ethyl ester (0.16 g, 0.33 mmol) (from Intermediate 32 *supra*) in dioxane (20 mL) for 20 minutes. The mixture was heated in a sealed pressure vessel at 160 °C for 1 day. The reaction mixture was concentrated. The residue was washed with hot methanol, filtered and dried to give 4-amino-3-[3-(4-cyano-benzyloxy)-phenoxymethyl]-thieno[3,2-c]pyridine-7-carboxylic acid ethyl ester as a white powder. (Yield 0.10 g, 67%).
HRMS (ES⁺) *m*/*z* Calcd for C₂₅H₂₁N₃O₄S + H [(M+H)⁺]: 460.1326. Found: 460.1327.

### Example 27

### 4-Amino-3-[3-(4-cyano-benzyloxy)-phenoxymethyl]-thieno[3,2-c]pyridine-7-carboxylic acid

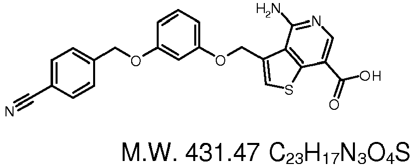

An aqueous solution of sodium hydroxide (1.0 N, 0.63 mL, 0.63 mmol) was added to a solution of 4-amino-3-[3-(4-cyano-benzyloxy)-phenoxymethyl]-thieno[3,2-c]pyridine-7-carboxylic acid ethyl ester (0.17 g, 0.37 mmol) (from Example 26 *supra*) in tetrahydrofuran - methanol (8 mL, 3:1) and the mixture was heated at 40 °C for 1 day. The reaction mixture was concentrated and azeotroped with toluene. The solid residue was triturated with dichloromethane. The solid was then suspended in water and treated with hydrochloric acid (1N, 3 mL). After stirring for 30 minutes, the solid was collected, washed with water and then diethyl ether and dried to give 4-amino-3-[3-(4-cyano-benzyloxy)-phenoxymethyl]-thieno[3,2-c]pyridine-7-carboxylic acid as a white solid which was conteminated with a small amount of 4-amino-3-[3-(4-cyano-benzyloxy)-phenoxymethyl]-thieno[3,2-c]pyridine-7-carboxylic acid methyl ester. (Yield 0.086 g, 54%).

### Example 28

### 4-Amino-3-[3-(4-cyano-benzyloxy)-phenoxymethyl]-thieno[3,2-c]pyridine-7-carboxylic acid (2-hydroxy-ethyl)-amide

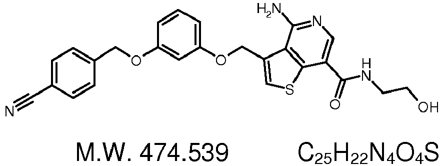

4-Amino-3-[3-(4-cyano-benzyloxy)-phenoxymethyl]-thieno[3,2-c]pyridine-7-carboxylic acid (86 mg, 0.13 mmol) (from Example 27 *Supra*), 1-hydroxybenzotriazole hydrate (43 mg, 0.32 mmol) (Aldrich) and 1,3-diisopropylcarbodiimide (0.044 mL, 0.29 mmol) (Aldrich) were combined in tetrahydrofuran - *N,N*-dimethylformamide (3.6 mL, 5:1) with stirring. After 1 hour, ethanolamine (36 mg, 0.60 mmol) (Aldrich) was added. The mixture was stirred at room temperature for 18 hours and then concentrated. The residue was purified by HPLC eluting with acetonitrile - water to give 4-amino-3-[3-(4-cyano-benzyloxy)-phenoxymethyl]-thieno[3,2-c]pyridine-7-carboxylic acid (2-hydroxy-ethyl)-amide as a white powder. (Yield 65 mg, 68%).
HRMS (ES⁺) *m*/*z* Calcd for C₂₅H₂₂N₄O₄S + Na [(M+Na)⁺]: 497.1254. Found: 497.1254.

### Intermediate 33

### 3-(3-Hydroxy-phenoxymethyl)-benzonitrile

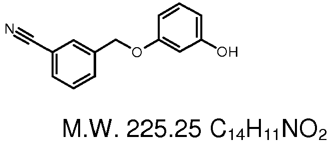

A suspension of potassium carbonate (2.11 g, 15.3 mmol) and resorcinol (16.85 g, 0.153 mol) (Aldrich) in acetonitrile (60 mL) was heated at reflux for 1 hour. A solution of 3-bromomethyl-benzonitrile (3.0 g, 15.3 mmol) (Aldrich) in acetonitrile (60 mL) was added. The reaction mixture was heated at reflux for 2.5 days. The solution was concentrated. The residue was partitioned between dichloromethane and water. The aqueous phase was extracted with dichloromethane (1X). The combined organic phase was washed with brine, dried (magnesium sulfate), filtered and concentrated. The residue was purified by flash chromatography eluting with 10 - 20% ethyl acetate in hexanes to give 3-(3-hydroxy-phenoxymethyl)-benzonitrile. (Yield 2.89 g, 84%).

### Intermediate 34

### 4-Chloro-3-[3-(3-cyano-benzyloxy)-phenoxymethyl]-thieno[3,2-c]pyridine-7-carboxylic acid ethyl ester

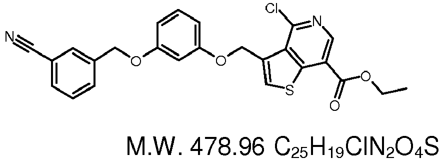

A suspension of potassium carbonate (0.24 g, 1.71 mmol), 3-(3-hydroxy-phenoxymethyl)-benzo-nitrile (0.35 g, 1.56 mmol) (from Intermediate 33 *supra*) in tetrahydrofuran - *N,N-*dimethylformamide (5:1, 18 mL) was heated at 70 °C for 3 hours. 3-Bromomethyl-4-chloro-thieno[3,2-c]pyridine-7-carboxylic acid ethyl ester (0.50 g, 1.49 mmol) (from Intermediate 8 *supra*) was added. Heating was continued for 18 hours. The reaction mixture was partitioned between ethyl acetate and water. The aqueous phase was extracted with ethyl acetate (1X). The combined organic phase was washed with water and brine, dried (magnesium sulfate) filtered and concentrated. The residue was purified by flash chromatography eluting with 0 - 5% ethyl acetate in dichloromethane to give 4-chloro-3-[3-(3-cyano-benzyloxy)-phenoxymethyl]-thieno[3,2-c]pyri-dine-7-carboxylic acid ethyl ester. (Yield 0.43 g, 61%).

### Example 29

### 4-Amino-3-[3-(3-cyano-benzyloxy)-phenoxymethyl]-thieno[3,2-c]pyridine-7-carboxylic acid ethyl ester

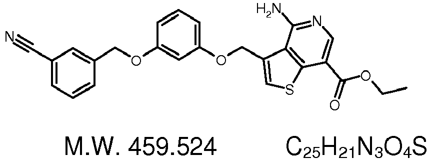

Ammonia gas was bubbled into a solution of 4-chloro-3-[3-(3-cyano-benzyloxy)-phenoxy-methyl]-thieno[3,2-c]pyridine-7-carboxylic acid ethyl ester (0.14 g, 0.29 mmol) (from Intermediate 34 *supra*) in dioxane (20 mL) for 20 minutes. The mixture was heated in a sealed pressure vessel at 160 °C for 1 day. The reaction mixture was concentrated. The residue was washed with hot methanol, filtered and dried to give 4-amino-3-[3-(3-cyano-benzyloxy)-phenoxymethyl]-thieno[3,2-c]pyridine-7-carboxylic acid ethyl ester as a white powder. (Yield 0.10 g, 77%).
HRMS (ES⁺) *m*/*z* Calcd for C₂₅H₂₁N₃O₄S + H [(M+H)⁺]: 460.1326. Found: 460.1325.

### Example 30

### 4-Amino-3-[3-(3-cyano-benzyloxy)-phenoxymethyl]-thieno[3,2-c]pyridine-7-carboxylic acid

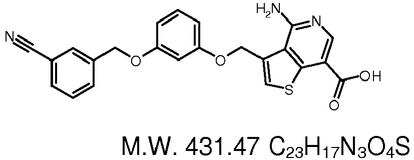

An aqueous solution of sodium hydroxide (1.0 N, 0.63 mL, 0.63 mmol) was added to a solution of 4-amino-3-[3-(3-cyano-benzyloxy)-phenoxymethyl]-thieno[3,2-c]pyridine-7-carboxylic acid ethyl ester (0.17 g, 0.37 mmol) (from Example 29 *supra*) in tetrahydrofuran - methanol (8 mL, 3:1) and the mixture was heated at 40 °C for 1 day. The reaction mixture was concentrated and azeotroped with toluene. The solid residue was triturated with dichloromethane. The solid was then suspended in water and treated with aqueous hydrochloric acid (1 N, 3 mL). After stirring for 30 minutes, the solid was collected, washed with water and then diethyl ether and dried to give 4-amino-3-[3-(4-cyano-benzyloxy)-phenoxymethyl]-thieno[3,2-c]pyridine-7-carboxylic acid as a white powder which was contaminated with a small amount of 4-amino-3-[3-(4-cyano-benzyloxy)-phenoxymethyl]-thieno[3,2-c]pyridine-7-carboxylic acid methyl ester. (Yield 86 mg, 54%).

### Example 31

### 4-Amino-3-[3-(3-cyano-benzyloxy)-phenoxymethyl]-thieno[3,2-c]pyridine-7-carboxylic acid (2-hydroxy-ethyl)-amide trifluoroacetic acid salt

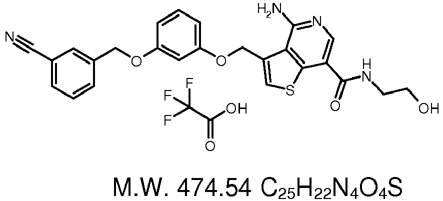

4-amino-3-[3-(3-cyano-benzyloxy)-phenoxymethyl]-thieno[3,2-c]pyridine-7-carboxylic acid (86 mg, 0.13 mmol) (from Example 30 *supra*), 1-hydroxybenzotriazole hydrate (43 mg, 0.32 mmol) (Aldrich) and 1,3-diisopropylcarbodiimide (0.044 mL, 0.29 mmol) (Aldrich) were combined in tetrahydrofuran - *N,N*-dimethylformamide (3.6 mL, 5:1) with stirring. After 1 hour, ethanolamine (0.036 g, 0.60 mmol) (Aldrich) was added. The mixture was stirred at room temperature for 18 hours and then concentrated. The residue was purified by HPLC eluting with acetonitrile - water containing trifluoroacetic acid to give 4-amino-3-[3-(3-cyano-benzyloxy)-phenoxymethyl]-thieno[3,2-c]pyridine-7-carboxylic acid (2-hydroxy-ethyl)-amide trifluoroacetic acid salt as a white powder. (Yield 65 mg, 68%).
HRMS (ES⁺) *m*/*z* Calcd for C₂₅H₂₂N₄O₄S + H [(M+H)⁺]: 475.1435. Found: 475.1434.

### Example 32

### 3-(3-benzyloxy-phenoxymethyl)-4-(2-hydroxy-etnylamino)-thieno[3,2-c]pyridine-7-carboxylic acid (2-hydroxy-ethyl)-amide

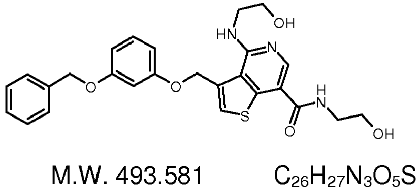

A solution of 3-(3-benzyloxy-phenoxymethyl)-4-chloro-thieno[3,2-c]pyridine-7-carboxylic acid ethyl ester (0.16 g, 0.35 mmol) (from Intermediate 11 *supra*) in dimethylsulfoxide (1.0 mL) was treated with ethanolamine (4.0 mL) (Aldrich) and heated at 130 °C for 2 hours in a microwave reactor. The reaction mixture was diluted with ethyl acetate and washed with water and brine, dried (magnesium sulfate), filtered and concentrated. The residue was recrystallized from ethyl acetate - hexanes to give 3-(3-benzyloxy-phenoxymethyl)-4-(2-hydroxyetnyl-amino)-thieno[3,2-c]pyridine-7-carboxylic acid (2-hydroxy-ethyl)-amide as white crystals. (Yield 0.06 g, 35%).
HRMS (ES⁺) *m*/*z* Calcd for C₂₆H₂₇N₃O₅S + H [(M+H)⁺]: 494.1744. Found: 494.1746

4-amino-3-[3-(3-cyano-benzyloxy)-phenoxymethyl]-thieno[3,2-c]pyridine-7-carboxylic acid (86 mg, 0.13 mmol) (from Example 30 *supra*), 1-hydroxybenzotriazole hydrate (43 mg, 0.32 mmol) (Aldrich) and 1,3-diisopropylcarbodiimide (0.044 mL, 0.29 mmol) (Aldrich) were combined in tetrahydrofuran - *N,N*-dimethylformamide (3.6 mL, 5:1) with stirring. After 1 hour, ethanolamine (0.036 g, 0.60 mmol) (Aldrich) was added. The mixture was stirred at room temperature for 18 hours and then concentrated. The residue was purified by HPLC eluting with acetonitrile - water containing trifluoroacetic acid to give 4-amino-3-[3-(3-cyano-benzyloxy)-phenoxymethyl]-thieno[3,2-c]pyridine-7-carboxylic acid (2-hydroxy-ethyl)-amide trifluoroacetic acid salt as a white powder. (Yield 65 mg, 68%).
HRMS (ES⁺) *m*/*z* Calcd for C₂₅H₂₂N₄O₄S + H [(M+H)⁺]: 475.1435. Found: 475.1434.

### Example 32

### 3-(3-benzyloxy-phenoxymethyl)-4-(2-hydroxy-ethylamino)-thieno[3,2-c]pyridine-7-carboxylic acid (2-hydroxy-ethyl)-amide

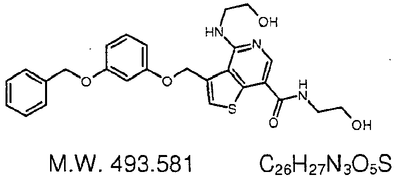

A solution of 3-(3-benzyloxy-phenoxymethyl)-4-chloro-thieno[3,2-c]pyridine-7-carboxylic acid ethyl ester (0.16 g, 0.35 mmol) (from Intermediate 11 *supra*) in dimethylsulfoxide (1.0 mL) was treated with ethanolamine (4.0 mL) (Aldrich) and heated at 130 °C for 2 hours in a microwave reactor. The reaction mixture was diluted with ethyl acetate and washed with water and brine, dried (magnesium sulfate), filtered and concentrated. The residue was recrystallized from ethyl acetate - hexanes to give 3-(3-benzyloxy-phenoxymethyl)-4-(2-hydroxyetnyl-amino)-thieno[3,2-c]pyridine-7-carboxylic acid (2-hydroxy-ethyl)-amide as white crystals. (Yield 0.06 g, 35%).
HRMS (ES⁺) *m*/*z* Calcd for C₂₆H₂₇N₃O₅S + H [(M+H)⁺]: 494.1744. Found: 494.1746 continued for 18 hours. The reaction mixture was partitioned between ethyl acetate and water. The aqueous phase was extracted with ethyl acetate (2X). The combined organic phase was washed with water and brine, dried (magnesium sulfate), filtered and concentrated. The residue was purified by flash chromatography eluting with 10 - 20% ethyl acetate in hexanes to give 3-(3-*tert-*butoxycarbonyl-phenoxymethyl)-4-chloro-thieno[3,2-c]pyridine-7-carboxylic acid ethyl ester. (Yield 2.5 g, 60%).

### Intermediate 37

### 4-Amino-3-(3-tert-butoxycarbonyl-phenoxymethyl)-thieno[3,2-c]pyridine-7-carboxylic acid ethyl ester

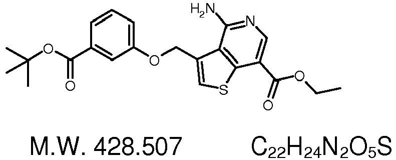

Ammonia gas was bubbled into a solution of 3-(3-*tert*-butoxycarbonyl-phenoxymethyl)-4-chloro-thieno[3,2-c]pyridine-7-carboxylic acid ethyl ester (0.62 g, 1.38 mmol) (from Intermediate 36 *supra*) in dioxane (20 mL) for 30 minutes. The mixture was heated in a sealed pressure vessel at 160 °C for 1 day. The reaction mixture was concentrated. The residue was washed with hot ethyl acetate, the solid was filtered and dried to give 4-amino-3-(3-*tert*-butoxycarbonyl-phenoxymethyl)-thieno[3,2-c]pyridine-7-carboxylic acid ethyl ester (0.46 g). The solution was concentrated. The residue was purified by flash chromatography eluting with 40% ethyl acetate in hexanes to give the product (0.13 g). (Combined yield 0.59 g, 100%).
HRMS (ES⁺) *m*/*z* Calcd for C₂₂H₂₄N₂O₅S + H [(M+H)⁺]: 429.1479. Found: 429.1480.

### Intermediate 38

### 4-Amino-3-(3-carboxy-phenoxymethyl)-thieno[3,2-c]pyridine-7-carboxylic acid ethyl ester

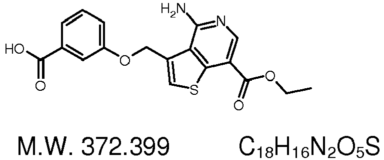

4-Amino-3-(3-*tert*-butoxycarbonyl-phenoxymethyl)-thieno[3,2-c]pyridine-7-carboxylic acid ethyl ester (0.13 g, 0.30 mmol) (from Intermediate 37 *supra*) was dissolved in trifluoroacetic acid - dichloromethane (1:1,4 mL). The mixture was stirred at room temperature for 1 hour then concentrated. The residue was dissolved in saturate aqueous sodium carbonate solution and extracted with dichloromethane. The aqueous phase was acidified with 6 N hydrochloric acid. The solid formed was collected, washed with water and dried to give 4-amino-3-(3-carboxy-phenoxymethyl)-thieno[3,2-c]pyridine-7-carboxylic acid ethyl ester as a white powder. (Yield 0.11 g, 100%).
HRMS (ES⁺) *m*/*z* Calcd for C₁₈H₁₆N₂O₅S + H [(M+H)⁺]: 373.0853. Found: 373.0853.

### Intermediate 39

### 4-Hydroxy-benzoic acid tert-butyl ester

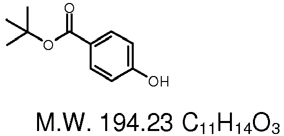

4-Hydroxybenzoic acid (5.29 g, 38.3 mmol) (Aldrich) was suspended in dry benzene (200 mL) and the mixture was heated to reflux. *N,N'*-Dimethylformamide di-*t*-butyl acetal (36.7 mL, 0.15 mol) (Aldrich) was added dropwise and the mixture was heated at reflux for a further 1 hour. The cooled solution was washed with water, saturated aqueous sodium bicarbonate solution and brine. After drying (magnesium sulfate) and filtering, the solvent was evaporated. The residue was purified by flash chromatography eluting with 25% hexanes in dichloromethane and 10% ethyl acetate in dichloromethane to give 4-hydroxy-benzoic acid *tert*-butyl ester. (Yield 1.72 g, 23%).

### Intermediate 40

### 3-(4-tert-Butoxycarbonyl-phenoxymethyl)-4-chloro-thieno[3,2-c]pyridine-7-carboxylic acid ethyl ester

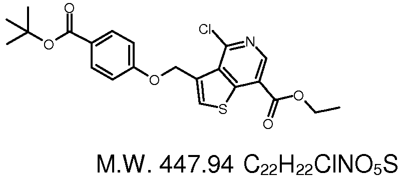

A suspension of potassium carbonate (1.34 g, 9.70 mmol), 4-hydroxy-benzoic acid *tert*-butyl ester (1.72 g, 8.86 mmol) (from Intermediate 39 *supra*) and a catalytic amount of 18-crown-6 (Aldrich) in *N,N*-dimethylformamide (50 mL) was heated at 65 °C for 2 hours. 3-Bromomethyl-4-chloro-thieno[3,2-c]pyridine-7-carboxylic acid ethyl ester (2.82 g, 8.43 mmol) (from Intermediate 8 *supra*) was added. Heating was continued for 18 hours. The reaction mixture was partitioned between ethyl acetate and water. The aqueous phase was extracted with ethyl acetate (2X). The combined organic phase was washed with water and brine, dried (magnesium sulfate) filtered and concentrated. The residue was purified by flash chromatography eluting with 10 - 20% ethyl acetate in hexanes to give 3-(4-*tert*-butoxycarbonyl-phenoxymethyl)-4-chloro-thieno[3,2-c]pyridine-7-carboxylic acid ethyl ester. (Yield 2.65 g, 70%).

### Intermediate 41

### 4-Amino-3-(4-tert-butoxycarbonyl-phenoxymethyl)-thieno[3,2-c]pyridine-7-carboxylic acid ethyl ester

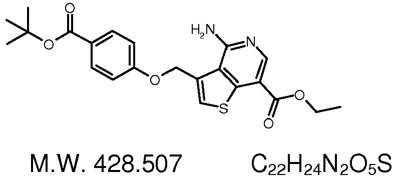

Ammonia gas was bubbled into a solution of 3-(4-*tert*-butoxycarbonyl-phenoxymethyl)-4-chloro-thieno[3,2-c]pyridine-7-carboxylic acid ethyl ester (2.70 g, 6.03 mmol) (from Intermediate 40 *supra*) in dioxane (60 mL) for 30 minutes. The mixture was heated in a sealed pressure vessel at 160 °C for 1 day. The reaction mixture was concentrated. The residue was recrystallized from ethyl acetate, the solid was filtered and dried to give 4-amino-3-(4-*tert*-butoxycarbonyl-phenoxymethyl)-thieno[3,2-c]pyridine-7-carboxylic acid ethyl ester (1.3 g). The mother liquid was concentrated. The residue was purified by flash chromatography eluting with 40% ethyl acetate in hexanes to give the product (0.8 g). (Combined yield 2.1 g, 81 %).
HRMS (ES⁺) *m*/*z* Calcd for C₂₂H₂₄N₂O₅S + H [(M+H)⁺]: 429.1479. Found: 429.1480.

### Intermediate 42

### 4-Amino-3-(4-carboxy-phenoxymethyl)-thieno[3,2-c]pyridine-7-carboxylic acid ethyl ester

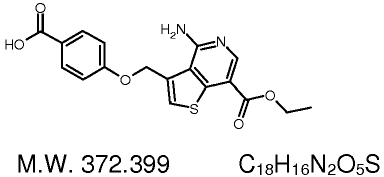

4-Amino-3-(4-*tert*-butoxycarbonyl-phenoxymethyl)-thieno[3,2-c]pyridine-7-carboxylic acid ethyl ester (2.1 g, 4.9 mmol) (from Intermediate 40 *supra*) was dissolved in trifluoroacetic acid - dichloromethane (1:2, 60 mL). The mixture was stirred at room temperature for 3 hour then concentrated. The residue was dissolved in saturated aqueous sodium carbonate solution and extracted with dichloromethane. The aqueous phase was acidified with aqueous 6 N hydrochloric acid. The solid was collected, washed with water and dried to give 4-amino-3-(4-carboxy-phenoxymethyl)-thieno[3,2-c]pyridine-7-carboxylic acid ethyl ester. (Yield 1.83 g, 100%).
HRMS (ES⁺) *m*/*z* Calcd for C₁₈H₁₆N₂O₅S + H [(M+H)⁺]: 373.0853. Found: 373.0853.

### Example 33

### 4-Amino-3-(3-methoxy-phenoxymethyl)-thieno[3,2-c]pyridine-7-carboxylic acid ethyl ester

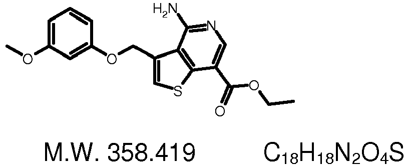

This was prepared following the procedure found in Example 2 *supra.*
HRMS (ES⁺) *m*/*z* Calcd for C₁₈H₁₈N₂O₄S + H [(M+H)⁺]: 359.1060. Found: 359.1058.

### Example 34

### 4-Amino-3-(3-methoxy-phenoxymethyl)-thieno[3,2-c]pyridine-7-carboxylic acid (2-hydroxy-ethyl)-amide

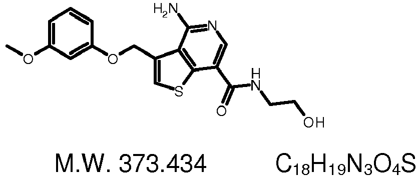

This was prepared following the procedure found in Example 3 *supra.*
HRMS (ES⁺) *m*/*z* Calcd for C₁₈H₁₉N₃O₄S + H [(M+H)⁺]: 374.1169. Found: 374.1169.

### Example 35

### 4-Amino-3-(3-benzoylamino-phenoxymethyl)-thieno[3,2-c]pyridine-7-carboxylic acid ethyl ester

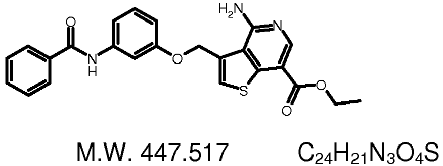

This was prepared following the procedure found in Example 1 *supra.*
HRMS (ES⁺) *m*/*z* Calcd for C₂₄H₂₁N₃O₄S + H [(M+H)⁺]: 448.1326. Found: 448.1325.

### Example 36

### 4-Amino-3-(3-benzoylamino-phenoxymethyl)-thieno[3,2-c]pyridine-7-carboxylic acid (2-hydroxy-ethyl)-amide

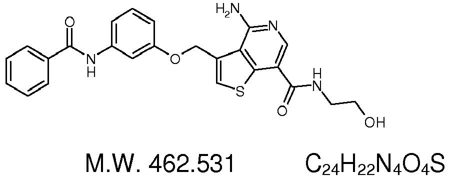

This was prepared following the procedure found in Example 3 *supra.*
HRMS (ES⁺) *m*/*z* Calcd for C₂₄H₂₂N₄O₄S + H [(M+H)⁺]: 463.1435. Found: 463.1436.

### Example 36a

### 4-Amino-3-(3-benzoylamino-phenoxymethyl)-thieno[3,2-c]pyridine-7-carboxylic acid (2-hydroxy-ethyl)-amide hydrochloride

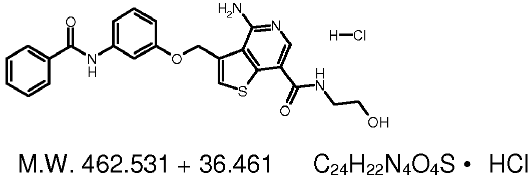

This was prepared by treating 4-amino-3-(3-benzoylamino-phenoxymethyl)-thieno[3,2-c]pyridine-7-carboxylic acid (2-hydroxy-ethyl)-amide with 1 equivalent of hydrochloric acid.
HRMS (ES⁺) *m*/*z* Calcd for C₂₄H₂₂N₄O₄S + H [(M+H)⁺]: 463.1435. Found: 463.1437.
HRMS (ES⁺) *m*/*z* Calcd for C₂₄H₂₂N₄O₄S + Na [(M+Na)⁺]: 485.1254. Found: 485.1256.

### Example 36b

### 4-Amino-3-(3-benzoylamino-phenoxymethyl)-thieno[3,2-c]pyridine-7-carboxylic acid (2-hydroxy-ethyl)-amide toluene-4-sulfonic acid salt

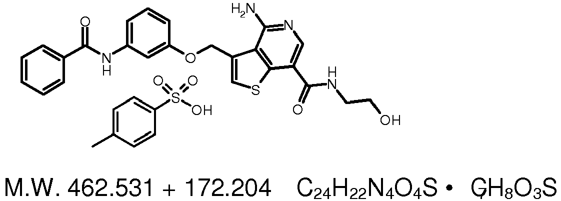

This was prepared by treating 4-amino-3-(3-benzoylamino-phenoxymethyl)-thieno[3,2-c]pyridine-7-carboxylic acid (2-hydroxy-ethyl)-amide with 1 equivalent of toluene-4-sulfonic acid.
HRMS (ES⁺) *m*/*z* Calcd for C₂₄H₂₂N₄O₄S + H [(M+H)⁺]: 463.1435. Found: 463.1437.
HRMS (ES⁺) *m*/*z* Calcd for C₂₄H₂₂N₄O₄S + Na [(M+Na)⁺]: 485.1254. Found: 485.1256.

### Example 37

### 4-Amino-3-[3-(4-chloro-phenylcarbamoyl)-phenoxymethyl]-thieno[3,2-c]pyridine-7-carboxylic acid ethyl ester

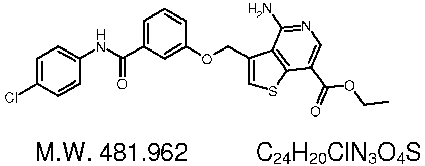

To a suspension of 4-amino-3-(3-carboxy-phenoxymethyl)-thieno[3,2-c]pyridine-7-carboxylic acid ethyl ester (49.2 mg, 0.132 mmol) (from Intermediate 38 *supra*) in DMF (5 mL) were added diisopropylethylamine (68 mg, 0.528 mmol), HOBt (26.7 mg, 0.198 mmol) (Aldrich) and HBTU (75.0 mg, 0.198 mmol) (Advanced ChemTech). After stirring for 30 minutes, 4-chloroaniline (20.0 mg, 0.158 mmol) (Aldrich) in DMF (0.5 mL) was added. The reaction mixture was stirred at room temperature for 2 hours before it was diluted with EtOAc (80 mL), washed with 1 N HCl (10 mL), saturated aqueous Na₂CO₃ solution (10 mL), brine (2x10 mL), dried (Na₂SO₄) and filtered. Removal of the solvent followed by column chromatography (CH₂Cl₂ / MeOH, 98/2) gave 4-amino-3-[3-(4-chloro-phenylcarbamoyl)-phenoxymethyl]-thieno[3,2-c]pyridine-7-carboxylic acid ethyl ester as a white solid. (Yield 34.1 mg, 53.6%).
HRMS (ES⁺) *m*/*z* Calcd for C₂₄H₂₀ClN₃O₄S+ H [(M+H)⁺]: 482.0936. Found: 482.0936.

### Example 38

### 4-Amino-3-[3-(4-chloro-phenylcarbamoyl)-phenoxymethyl]-thieno[3,2-c]pyridine-7-carboxylic acid (2-hydroxy-ethyl)-amide

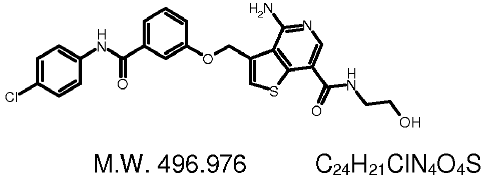

This was prepared following the procedure found in Example 3 *supra.*
HRMS (ES⁺) *m*/*z* Calcd for C₂₄H₂₁ClN₄O₄S + H [(M+H)⁺]: 497.1045. Found: 497.1045.

### Example 39

### 4-Amino-3-[3-(4-fluoro-phenylcarbamoyl)-phenoxymethyl]-thieno[3,2-c]pyridine-7-carboxylic acid ethyl ester

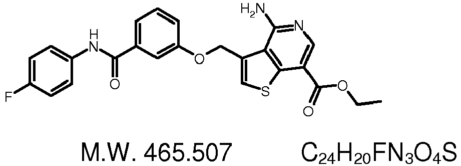

This was prepared following the procedure found in Example 1 *supra.*
HRMS (ES⁺) *m*/*z* Calcd for C₂₄H₂₀FN₃O₄S + H [(M+H)⁺]: 466.1232. Found: 466.1230.

### Example 40

### 4-Amino-3-[3-(4-fluoro-phenylcarbamoyl)-phenoxymethyl]-thieno[3,2-c]pyridine-7-carboxylic acid (2-hydroxy-ethyl)-amide

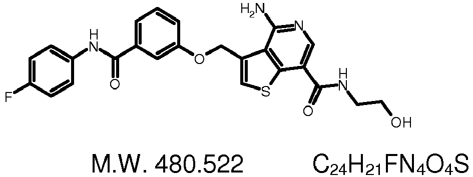

This was prepared following the procedure found in Example 3 *supra.*
HRMS (ES⁺) *m*/*z* Calcd for C₂₄H₂₁FN₄O₄S + Na [(M+Na)⁺]: 503.1160. Found: 503.1158.

### Example 41

### 4-Amino-3-[3-(4-chloro-benzyloxy)-phenoxymethyl]-thieno[3,2-c]pyridine-7-carboxylic acid trifluoro-acetic acid salt

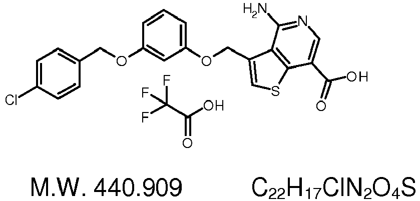

This was prepared by sodium hydroxide hydrolysis of 4-amino-3-[3-(4-chloro-benzyloxy)-phenoxymethyl]-thieno[3,2-c]pyridine-7-carboxylic acid ethyl ester (from Example 3 *supra*) followed by purification with reverse phase chromatography using acetonitrile - water containing 0.1% trifluoroacetic acid as solvent.

### Example 42

### 4-Amino-3-[3-(3-chloro-phenylcarbamoyl)-phenoxymethyl]-thieno[3,2-c]pyridine-7-carboxylic acid ethyl ester

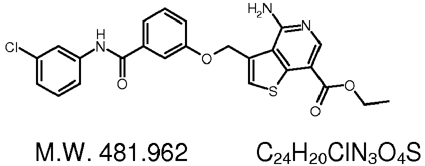

This was prepared following the procedure found in Example 1 *supra.*
HRMS (ES⁺) *m*/*z* Calcd for C₂₄H₂₀ClN₃O₄S + H [(M+H)⁺]: 482.0936. Found: 482.0937.

### Example 43

### 4-Amino-3-[3-(3-chloro-phenylcarbamoyl)-phenoxymethyl]-thieno[3,2-c]pyridine-7-carboxylic acid (2-hydroxy-ethyl)-amide

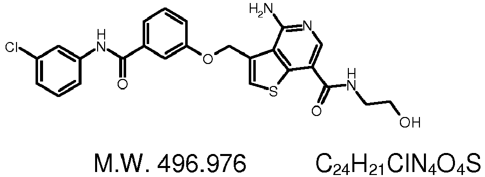

This was prepared following the procedure found in Example 3 *supra.*
HRMS (ES⁺) *m*/*z* Calcd for C₂₄H₂₁ClN₄O₄S + Na [(M+Na)⁺]: 519.064. Found: 519.0864.

### Example 44

### 4-Amino-3-[3-(4-chloro-phenylcarbamoyl)-phenoxymethyl]-thieno[3,2-c]pyridine-7-carboxylic acid (3-hydroxy-propyl)-amide

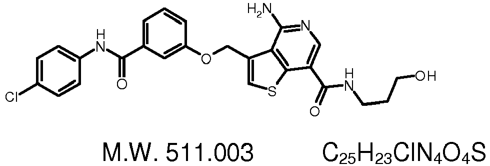

This was prepared by the hydrolysis of the corresponding ethyl ester (from Example 37 *supra*) following the procedure found in Example 27 *supra* and the resulting acid coupled with amino1-propanol.
HRMS (ES⁺) *m*/*z* Calcd for C₂₅H₂₃ClN₄O₄S + Na [(M+Na)⁺]: 533.1021. Found: 533.1022.

### Example 45

### 4-Amino-3-[3-(4-chloro-phenylcarbamoyl)-phenoxymethyl]-thieno[3,2-c]pyridine-7-carboxylic acid propylamide

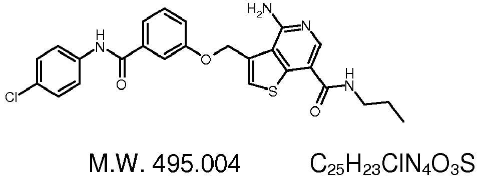

This was prepared by the hydrolysis of the corresponding ethyl ester (from Example 37 *supra*) following the procedure found in Example 27 *supra* and the resulting acid coupled with propylamine.
HRMS (ES⁺) *m*/*z* Calcd for C₂₅H₂₃ClN₄O₃S + Na [(M+Na)⁺]: 517.1071. Found: 517.1072.

### Example 46

### 4-Amino-3-[3-(4-chloro-phenylcarbamoyl)-phenoxymethyl]-thieno[3,2-c]pyridine-7-carboxylic acid ethylamide

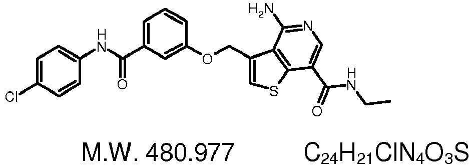

This was prepared by the hydrolysis of the corresponding ethyl ester (from Example 37 *supra*) following the procedure found in Example 27 *supra* and the resulting acid coupled with ethylamine.
HRMS (ES⁺) *m*/*z* Calcd for C₂₄H₂₁ClN₄O₃S + Na [(M+Na)⁺]: 503.0915. Found: 503.0917.

### Example 47

### 4-Amino-3-{3-[(4-chloro-phenyl)-methyl-carbamoyl]-phenoxymethyl}-thieno[3,2-c]pyridine-7-carboxylic acid ethyl ester

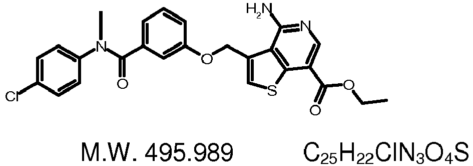

This was prepared following the procedure found in Example 1 *supra.*
HRMS (ES⁺) *m*/*z* Calcd for C₂₅H₂₂ClN₃O₄S + H [(M+H)⁺]: 496.1093. Found: 496.1088.

### Example 48

### 4-Amino-3-[3-(1-methyl-piperidin-4-ylcarbamoyl)-phenoxymethyl]-thieno[3,2-c]pyridine-7-carboxylic acid ethyl ester

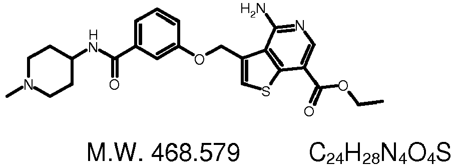

This was prepared following the procedure found in Example 3 *supra.*
HRMS (ES⁺) *m*/*z* Calcd for C₂₄H₂₈N₄O₄S + H [(M+H)⁺]: 469.1904. Found: 469.1900.

### Example 49

### 4-Amino-3-[3-(5-methyl-pyridin-2-ylcarbamoyl)-phenoxymethyl]-thieno[3,2-c]pyridine-7-carboxylic acid ethyl ester

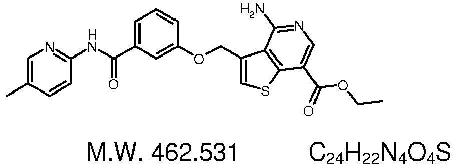

This was prepared following the procedure found in Example 1 *supra.*
HRMS (ES⁺) *m*/*z* Calcd for C₂₄H₂₂N₄O₄S + H [(M+H)⁺]: 463.1435. Found: 463.1430.

### Example 50

### 4-Amino-3-[3-(1-methanesulfonyl-piperidin-4-ylcarbamoyl)-phenoxymethyl]-thieno[3,2-c]pyridine-7-carboxylic acid ethyl ester

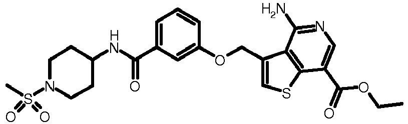

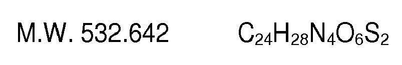

This was prepared following the procedure found in Example 1 *supra.*
HRMS (ES⁺) *m*/*z* Calcd for C₂₄H₂₈N₄O₆S₂ + Na [(M+Na)⁺]: 555.1342. Found: 555.1332.

### Example 51

### 4-Amino-3-{3-[(4-chloro-phenyl)-methyl-carbamoyl]-phenoxymethyl}-thieno[3,2-c]pyridine-7-carboxylic acid (2-hydroxy-ethyl)-amide

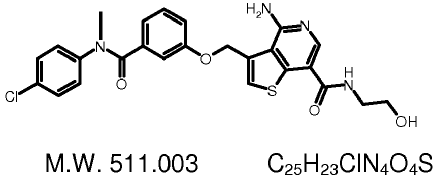

This was prepared following the procedure found in Example 3 *supra.*
HRMS (ES⁺) *m*/*z* Calcd for C₂₅H₂₃ClN₄O₄S + Na [(M+Na)⁺]: 533.1021. Found: 533.1016.

### Example 52

### 4-Amino-3-[5-(3-carbamoyl-phenylcarbamoyl)-2-methyl-phenoxymethyl]-thieno[3,2-c]pyridine-7-carboxylic acid ethyl ester

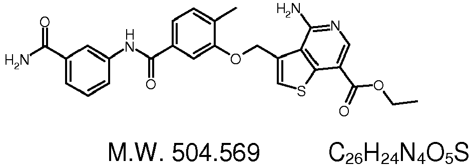

This was prepared following General Procedure 4 *supra.*
LRMS (ES⁺) *m*/*z* Calcd for C₂₆H₂₄N₄O₅S+ H [(M+H)⁺]: 505. Found: 505.

### Example 53

### 4-Amino-3-(2-methyl-5-phenylcarbamoyl-phenoxymethyl)-thieno[3,2-c]pyridine-7-carboxylic acid ethyl ester

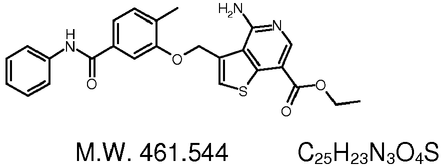

This was prepared following General Procedure 4 *supra.*

### Example 54

### 4-Amino-3-[3-(4-chloro-benzyloxy)-phenoxymethyl]-thieno[3,2-c]pyridine-7-carboxylic acid (3-hydroxy-propyl)-amide trifluoro-acetic acid salt

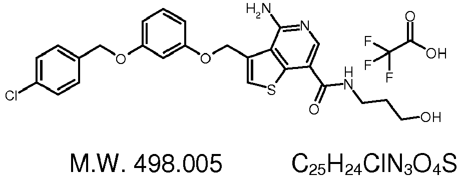

A solution of 4-amino-3-[3-(4-chloro-benzyloxy)-phenoxymethyl]-thieno[3,2-c]pyridine-7-carboxylic acid ethyl ester (0.04 g, 0.09 mmol) (from Example 4 *supra*) in dimethylsulfoxide (0.5 mL) was treated with ethanolamine (1.5 mL) (Aldrich) and heated at 160 °C for 2 hours in a microwave reactor. The mixture was purified by HPLC eluting with acetonitrile/water containing 0.1% trifluoroacetuic acid to give 4-amino-3-[3-(4-chloro-benzyloxy)-phenoxymethyl]-thieno[3,2-c] pyridine-7-carboxylic acid (3-hydroxy-propyl)-amide trifluoro-acetic acid salt. (Yield 9.0 mg, 17%).
HRMS (ES⁺) *m*/*z* Calcd for C₂₅H₂₄ClN₃O₄S + H [(M+H)⁺]: 498.1249. Found: 498.1248.

### Example 55

### 4-Amino-3-[3-(4-chloro-phenoxymethyl)-phenoxymethyl]-thieno[3,2-c]pyridine-7-carboxylic acid ethyl ester

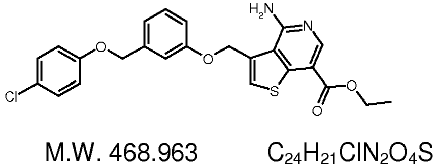

N,N-Diisopropylethylamine (19.2 mL, 110 mmol) (Aldrich) was added to a suspension of 3-hydroxybenzaldehyde (12.21 g, 100 mmol) (Aldrich) in dichloromethane (50 mL) with cooling in an ice-water bath. Chloromethylmethyl ether (15.19 mL, 200 mmol) (Aldrich) was then added dropwise and the mixture stirred at room temperature for 3 hours. The mixture was then washed with mixture of brine (30 mL) and water (30 mL), followed by 0.1 N HCl (3 X 20 mL) and brine (2 X 20 mL). Aqueous layers were back washed with dichloromethane (30 mL). Organic solutions were combined, dried (MgSO₄), filtered and concentrated. Residue was filtered through silica gel (Biotage 40M) eluting with dichloromethane. Fractions were combined and concentrated to give 3-methoxymethoxy-benzaldehyde as pale yellow oil. (Yield 13.94 g, 83.9%).

Sodium borohydride (1.14 g, 30 mmol) (Aldrich) was added in small portions to a solution of 3-methoxymethyloxy-benzaldehyde (4.99 g, 30 mmol) in methanol (90 mL) and water (3 mL). Mixture was stirred for 3 hours at room temperature and then diluted with water (50 mL). Mixture was concentrated under reduced pressure and the resulting aqueous suspension extracted with ethyl acetate (2 X 75 mL). Organic layers were washed with water (50 mL) and brine (50 mL), and then combined, dried (MgSO₄), filtered and concentrated to give crude (3-methoxymethoxy-phenyl)-methanol as a colorless oil. This was used in the next step without further purification. (Yield 5.04 g, 100%).

A neat mixture of (3-methoxymethoxy-phenyl)-methanol (1.68 g, 10 mmol), 4-chlorophenol (1.29 g, 10 mmol) (Aldrich) and N,N'-dicyclohexylcarbodiimide (2.06 g, 10 mmol) (Aldrich) was heated in a sealed tube at 100 °C for 24 hours. Sample was cooled to room temperature then triturated with ether. Dicyclohexylurea was removed by filtration. Filtrate was washed with 1 N aqueous hydrochloric acid and brine. Aqueous layers were back washed with ether. Combined ether layers were dried (MgSO₄), filtered and concentrated. Residue was filtered through a Biotage flash cartridge (40S, eluting with dichloromethane) to give (4-chlorophenoxy-methyl)-3-methyloxymethoxy-benzene as a pale yellow oil. (Yield 2.07 g, 74.3%).

To a solution of (4-chlorophenoxy-methyl)-3-methyloxymethoxy-benzene (2.07 g, 7.43 mmol) in tetrahydrofuran / 2-propanol (1:1, 40 mL) was added 4M HCl in dioxane (18.5 mL) (Aldrich). The reaction mixture was stirred at room temperature for 18 hours. The solution was concentrated. The residue was diluted with ethyl acetate, washed with saturated aqueous sodium bicarbonate solution and brine, dried (MgSO₄) and concentrated. The residue was purified by flash chromatography eluting with 20% ethyl acetate in hexanes to 3-(4-chloro-phenoxymethyl)-phenol. (Yield 1.72 g, 99%).

A suspension of potassium carbonate (0.16 g, 1.17 mmol), 3-(4-chloro-phenoxymethyl)-phenol (0.25 g, 1.07 mmol) in tetrahydrofuran / N,N-dimethylformamide (5:1, 18 mL) was heated at 70 °C for 3 hours. 3-bromomethyl-4-chloro-thieno[3,2-c]pyridine-7-carboxylic acid ethyl ester (0.34 g, 1.02 mmol) (from Intermediate 8 *supra*) was added. Heating was continued for 18 hours. The reaction mixture was partitioned between ethyl acetate and water. The aqueous phase was extracted with ethyl acetate (1 X). The combined organic phase was washed with water and brine, dried (MgSO₄) and concentrated. The residue was purified by flash chromatography eluting with 20% ethyl acetate in hexanes to give 4-chloro-3-[3-(4-chloro-phenoxymethyl)-phenoxymethyl]-thieno[3,2-c]pyridine-7-carboxylic acid ethyl ester. (Yield 0.19 g, 38%).

Ammonia gas was bubbled into a solution of 4-chloro-3-[3-(4-chloro-phenoxymethyl)-phenoxy-methyl]-thieno[3,2-c]pyridine-7-carboxylic acid ethyl ester (0.11 g, 0.23 mmol) in dioxane (20 mL) for 20 minutes. The mixture was heated in a sealed pressure vessel at 160 °C for 1 day. The reaction mixture was concentrated. The residue was washed with hot methanol, filtered and dried to give 4-amino-3-[3-(4-chloro-phenoxymethyl)-phenoxymethyl]-thieno[3,2-c]pyridine-7-carboxylic acid ethyl ester as a white solid. (Yield 0.087g, 79%).
HRMS (ES⁺) *m*/*z* Calcd for C₂₄H₂₁ClN₂O₄S + H [(M+H)⁺]: 469.0984. Found: 469.0984.

### Example 56

### 4-Amino-3-{3-[2-(4-chloro-phenyl)-vinyl]-phenoxymethyl}-thieno[3,2-c]pyridine-7-carboxylic acid ethyl ester

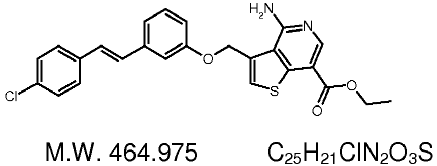

Sodium hydride (60% in oil, 1.0 g, 25 mmol) (Aldrich) was added in small portions to a solution of diethyl 4-chlorobenzylphosphonate (2.63 g, 1.0 mmol) (Alfa Aesar), catalytic amount of 18-Crown-6 (Aldrich) and methanol (0.8 mL, 20 mmol) in dry DMF (15 mL). After stirring for 5 minutes, 3-methoxymethoxy-benzaldehyde (1.66 g, 10 mmol) in dry DMF (5 mL) was added and mixture was stirred at room temperature for 1 hour, then at 120 ºC for 5 hours. After cooling to room temperature, mixture was diluted with water (100 mL) and extracted with ether (2 X 100 mL). Organic layers were washed with water and brine, dried (MgSO₄), filtered and concentrated. Residue was purified by chromatography (Biotage 40M, 20%, then 40% dichloromethane in hexanes as solvent). Product was recrystallized from hexanes to give pure E-[2-(4-chloro-phenyl)-vinyl]-3-methyloxymethoxy-benzene as white crystalline plates. (Yield 2.74 g, 100%).

To a solution of E-[2-(4-chloro-phenyl)-vinyl]-3-methyloxymethoxy-benzene (2.22 g, 8.08 mmol) in tetrahydrofuran / 2-propanol (1:1, 40 mL) was added 4M HCl in dioxane (15 mL) (Aldrich). The reaction mixture was stirred at room temperature for 18 hours. The solution was concentrated. The residue was diluted with ethyl acetate, washed with saturated aqueous sodium bicarbonate solution and brine, dried (MgSO₄) and concentrated. The residue was washed with hot dichloromethane and dried to give 3-[2-(4-chloro-phenyl)-vinyl]-phenol. (Yield 0.81 g, 44%).

A suspension of potassium carbonate (0.16 g, 1.17 mmol), 3-[2-(4-chloro-phenyl)-vinyl]-phenol (0.25 g, 1.07 mmol) in tetrahydrofuran / N,N-dimethylformamide (5:1, 18 mL) was heated at 70 °C for 3 hours. 3-Bromomethyl-4-chloro-thieno[3,2-c]pyridine-7-carboxylic acid ethyl ester (0.34 g, 1.02 mmol) (from Intermediate 8 *supra)* was added. Heating was continued for 18 hours. The reaction mixture was partitioned between ethyl acetate and water. The aqueous phase was extracted with ethyl acetate (1X). The combined organic phase was washed with water and brine, dried (MgSO₄) and concentrated. The residue was purified by flash chromatography eluting with 20% ethyl acetate in hexanes to give 4-chloro-3-{3-[2-(4-chloro-phenyl)-vinyl]-phenoxymethyl}-thieno[3,2-c]pyridine-7-carboxylic acid ethyl ester. (Yield 0.31 g, 63%).

Ammonia gas was bubbled into a solution of 4-chloro-3-{3-[2-(4-chloro-phenyl)-vinyl]-phenoxy-methyl}-thieno[3,2-c]pyridine-7-carboxylic acid ethyl ester (0.11 g, 0.23 mmol) in a mixture of 2-propanol / dioxane (1:1, 20 mL) for 20 minutes. The mixture was heated in a sealed pressure vessel at 160 °C for 1 day. The reaction mixture was concentrated. The residue was washed with hot methanol, filtered and dried to give 4-amino-3-{3-[(E)-2-(4-chloro-phenyl)-vinyl]-phenoxymethyl}-thieno[3,2-c]pyridine-7-carboxylic acid ethyl ester. (Yield 0.093 g, 96%).
HRMS (ES⁺) *m*/*z* Calcd for C₂₅H₂₁ClN₂O₃S + H [(M+H)⁺]: 465.1034. Found: 465.1034.

### Example 57

### 4-Amino-3-{3-[2-(4-chloro-phenyl)-vinyl]-phenoxymethyl}-thieno[3,2-c]pyridine-7-carboxylic acid (2-hydroxy-ethyl)-amide

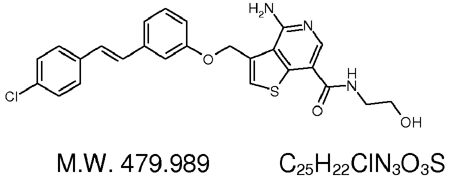

A solution of 4-amino-3-{3-[(E)-2-(4-chloro-phenyl)-vinyl]-phenoxymethyl}-thieno[3,2-c]pyridine-7-carboxylic acid ethyl ester (0.05 g, 0.11 mmol) (from Example 56 *supra*) in dimethylsulfoxide (0.5 mL) was treated with ethanolamine (1.5 mL) (Aldrich) and heated at 160 °C for 2 hours in a microwave reactor. The precipitate formed was filtered and washed with ethyl acetate and dried to give 4-amino-3-{3-[(E)-2-(4-chloro-phenyl)-vinyl]-phenoxy-methyl}-thieno[3,2-c]pyridine-7-carboxylic acid (2-hydroxy-ethyl)-amide as a white powder. (Yield 36.0 mg, 69%).
HRMS (ES⁺) *m*/*z* Calcd for C₂₅H₂₂ClN₃O₃S + H [(M+H)⁺]: 480.1143. Found: 480.1144.

### Example 58

### 4-Amino-3-[3-(4-chloro-phenoxymethyl)-phenoxymethyl]-thieno[3,2-c]pyridine-7-carboxylic acid (2-hydroxy-ethyl)-amide

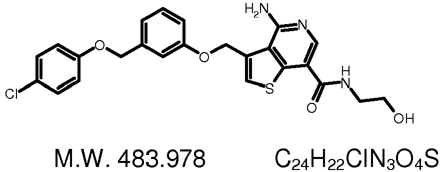

A solution of 4-amino-3-[3-(4-chloro-phenoxymethyl)-phenoxymethyl]-thieno[3,2-c]pyridine-7-carboxylic acid ethyl ester (0.05 g, 0.11 mmol) (from Example 55 *supra*) in dimethylsulfoxide (0.5 mL) was treated with ethanolamine (1.5 mL) (Aldrich) and heated at 160 °C for 2 hours in a microwave reactor. The precipitate formed was filtered and washed with hot methanol and dried to give 4-amino-3-[3-(4-chloro-phenoxymethyl)-phenoxy-methyl]-thieno[3,2-c]pyridine-7-carboxylic acid (2-hydroxy-ethyl)-amide as a white solid. (Yield 25.8 mg, 50%).
HRMS (ES⁺) *m*/*z* Calcd for C₂₄H₂₂ClN₃O₄S + H [(M+H)⁺]: 484.1093. Found: 484.1096.

### Example 59

### 4-Amino-3-[3-(4-chloro-phenylcarbamoyl)-phenoxymethyl]-thieno[3,2-c]pyridine-7-carboxylic acid (2-hydroxy-ethyl)-methyl-amide

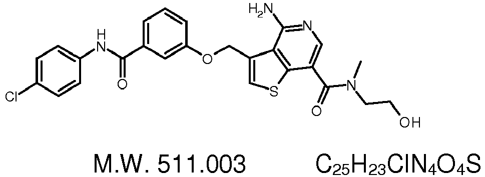

This was prepared by the hydrolysis of the corresponding ethyl ester (from Example 37 *supra*) following the procedure found in Example 27 *supra* and the resulting acid coupled with 2-methylamino-ethanol.
HRMS (ES⁺) *m*/*z* Calcd for C₂₅H₂₃ClN₄O₄S + Na [(M+Na)⁺]: 533.1021. Found: 533.1019.

### Example 60

### 4-Amino-3-[3-(4-chloro-phenylcarbamoyl)-phenoxymethyl]-thieno[3,2-c]pyridine-7-carboxylic acid (2-methoxy-ethyl)-amide

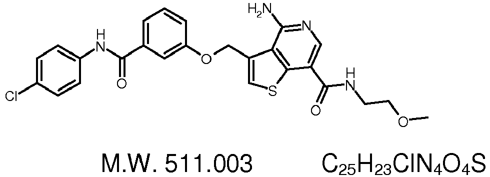

This was prepared by the hydrolysis of the corresponding ethyl ester (from Example 37 *supra*) following the procedure found in Example 27 *supra* and the resulting acid coupled with 2-methoxy-ethylamine.
HRMS (ES⁺) *m*/*z* Calcd for C₂₅H₂₃ClN₄O₄S + Na [(M+Na)⁺]: 533.1021. Found: 533.1020.

### Example 61

### 4-Amino-3-[3-(4-chloro-phenylcarbamoyl)-phenoxymethyl]-thieno[3,2-c]pyridine-7-carboxylic acid methylamide

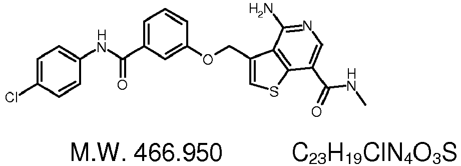

This was prepared by the hydrolysis of the corresponding ethyl ester (from Example 37 *supra*) following the procedure found in Example 27 *supra* and the resulting acid coupled with methylamine.
HRMS (ES⁺) *m*/*z* Calcd for C₂₃H₁₉ClN₄O₃S + H [(M+H)⁺]: 467.0939. Found: 467.0941.

### Example 62

### 3-[4-Amino-7-(morpholine-4-carbonyl)-thieno[3,2-c]pyridin-3-ylmethoxy]-N-(4-chloro-phenyl)-benzamide

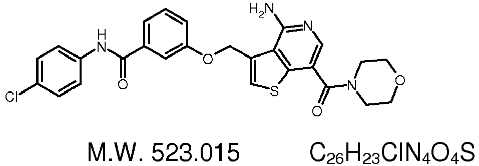

This was prepared by the hydrolysis of the corresponding ethyl ester (from Example 37 *supra*) following the procedure found in Example 27 *supra* and the resulting acid coupled with morpholine.
HRMS (ES⁺) *m*/*z* Calcd for C₂₆H₂₃ClN₄O₄S + H [(M+H)⁺]: 523.1202. Found: 523.1202.
HRMS (ES⁺) *m*/*z* Calcd for C₂₆H₂₃ClN₄O₄S + Na [(M+Na)⁺]: 545.1021. Found: 545.1022.

### Example 63

### 4-Amino-3-(3-cyclohexylcarbamoyl-phenoxymethyl)-thieno[3,2-c]pyridine-7-carboxylic acid ethyl ester

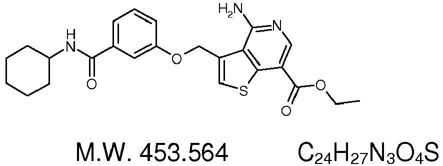

This was prepared following the procedure found in Example 1 *supra.*
HRMS (ES⁺) *m*/*z* Calcd for C₂₄H₂₇N₃O₄S + H [(M+H)⁺]: 454.1795. Found: 454.1797.

### Example 64

### 4-Amino-3-[3-(tetrahydro-pyran-4-ylcarbamoyl)-phenoxymethyl]-thieno[3,2-c]pyridine-7-carboxylic acid ethyl ester

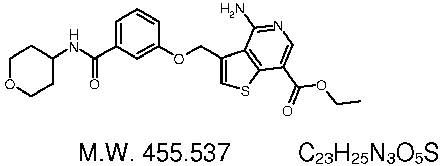

This was prepared following the procedure found in Example 1 *supra.*
HRMS (ES⁺) *m*/*z* Calcd for C₂₃H₂₅N₃O₅S + H [(M+H)⁺]: 456.1588. Found: 456.1590.

### Example 65

### 4-Amino-3-[3-(1-methanesulfonyl-piperidin-4-ylcarbamoyl)-phenoxymethyl]-thieno[3,2-c]pyridine-7-carboxylic acid (2-hydroxy-ethyl)-amide

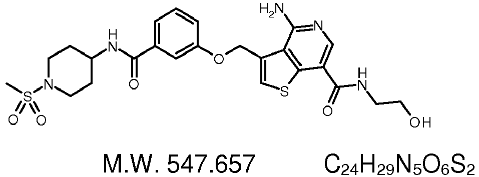

This was prepared following the procedure found in Example 3 *supra.*
HRMS (ES⁺) *m*/*z* Calcd for C₂₄H₂₉N₅O₆S₂ + H [(M+H)⁺]: 548.1632. Found: 548.1636.
HRMS (ES⁺) *m*/*z* Calcd for C₂₄H₂₉N₅O₆S₂ + Na [(M+Na)⁺]: 570.1451. Found: 570.1453.

### Example 66

### 4-Amino-3-[3-(1-methyl-piperidin-4-ylcarbamoyl)-phenoxymethyl]-thieno[3,2-c]pyridine-7-carboxylic acid (2-hydroxy-ethyl)-amide

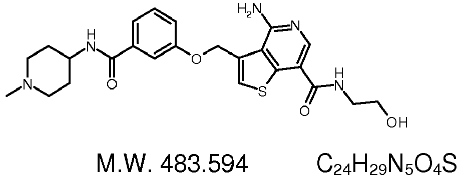

This was prepared following the procedure found in Example 3 *supra.*
HRMS (ES⁺) *m*/*z* Calcd for C₂₄H₂₉N₅O₄S + H [(M+H)⁺]: 484.2013. Found: 484.2012.

### Example 67

### 4-Amino-3-(3-phenylaminomethyl-phenoxymethyl)-thieno[3,2-c]pyridine-7-carboxylic acid (2-hydroxy-ethyl)-amide trifluoro-acetic acid salt

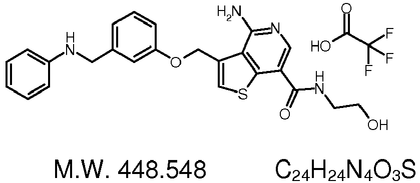

A suspension of 3-acetoxybenzoic acid (1.80 g, 10 mmol) in a mixture of N,N-dimethylformamide (3 drops) and thionyl chloride (3 mL, 40 mmol) (Aldrich) was heated in a 90 °C bath for 2 hours. After cooling, mixture was diluted with toluene (20 mL) and concentrated under reduced pressure to remove excess thionyl chloride. The resulting oil was dissolved in dichloromethane (10 mL) and added dropwise to a solution of aniline (0.93 g, 10 mmol) (Aldrich), 4-dimethylamino-pyridine (0.1 g, 0.08 mmol) (Fluka) and N,N-diisopropylethylamine (1.6 g, 12.4 mmol) (Aldrich) in dichloromethane (10 mL) with magnetic stirring. When addition was complete, mixture was stirred at room temperature for an additional 2 hours. Reaction mixture was then partitioned between water (50 mL) and dichloromethane (2 X 50 mL). Organic layers were combined, and concentrated to dryness. Residue was dissolved in THF (10 mL), methanol (10 mL) and 1 N aqueous sodium hydroxide (10 mL) and stirred at room temperature for 16 hours. The yellow solution was diluted with water (100 mL) and acetic acid (5 mL) and extracted with ethyl acetate (2 X 100 mL). Ethyl acetate layers were washed with brine (100 mL), combined, dried (MgSO₄), and concentrated to give crude 3-hydroxy-N-phenyl-benzamide which was used in the next step without further purification. (Yield 2.20 g, 103%).

Lithium aluminum hydride solution (19 mL, 1 M in tetrahydrofuran) (Aldrich) was added slowly to a suspension of the crude 3-hydroxy-N-phenyl-benzamide (2.0 g, 6.3 mmol) in dry tetrahydrofuran (30 mL) under an argon atmosphere with magnetic stirring with cooling in an ice - water bath (exothermic reaction). When addition was complete, mixture was heated at reflux for 1 hour. Reaction mixture was allowed to cool to room temperature and was quenched by pouring slowly into 15% aqueous ammonium chloride solution (100 mL) and ether (100 mL). After stirring thoroughly, mixture was diluted with ethyl acetate (100 mL) and filtered through Celite. Filtercake was washed thoroughly with ethyl acetate. Combined filtrate and washing was concentrated under reduced pressure. The resulting oil was purified by flash chromatography (Biotage 40M silica gel, 20% ethyl acetate in hexanes as solvent) to give 3-phenylaminomethyl-phenol an a colorless oil. (Yield 1.48 g, 73.5%).

A suspension of potassium carbonate (0.16 g, 1.17 mmol), 3-phenylaminomethyl-phenol (0.21 g, 1.07 mmol) in tetrahydrofuran / N,N-dimethylformamide (5:1, 18 mL) was heated at 70 °C for 3 hours. 3-Bromomethyl-4-chloro-thieno[3,2-c]pyridine-7-carboxylic acid ethyl ester (0.34 g, 1.02 mmol) (from Intermediate 8 *supra*) was added. Heating was continued for 2 days. The reaction mixture was partitioned between ethyl acetate and water. The aqueous phase was extracted with ethyl acetate (1 X). The combined organic phase was washed with water and brine, dried (MgSO₄) and concentrated. The residue was purified by flash chromatography eluting with 20% ethyl acetate in hexanes to give 4-chloro-3-(3-phenylaminomethyl-phenoxymethyl)-thieno[3,2-c]pyridine-7-carboxylic acid ethyl ester. (Yield 0.15g, 33%).

Ammonia gas was bubbled into a solution of 4-chloro-3-(3-phenylaminomethyl-phenoxymethyl)-thieno[3,2-c]pyridine-7-carboxylic acid ethyl ester (0.15 g, 0.33 mmol) in a mixture of 2-propanol / dioxane (1:1, 20 mL) for 20 minutes. The mixture was heated in a sealed pressure vessel at 160 °C for 1 day. The reaction mixture was concentrated. The crude material was purified by HPLC eluting with acetonitrile / water to give to 4-amino-3-(3-phenylaminomethyl-phenoxymethyl)-thieno[3,2-c]pyridine-7-carboxylic acid ethyl ester. (Yield 0.030 g, 21 %).

A solution of 4-amino-3-(3-phenylaminomethyl-phenoxymethyl)-thieno[3,2-c]pyridine-7-carboxylic acid ethyl ester (0.03 g, 0.07 mmol) in dimethylsulfoxide (0.5 mL) was treated with ethanolamine (1.5 mL) (Aldrich) and heated at 160 °C for 2 hours in a microwave reactor. The reaction mixture was purified by HPLC eluting with acetonitrile / water to give to 4-amino-3-(3-phenylaminomethyl-phenoxymethyl)-thieno [3,2-c]pyridine-7-carboxylic acid (2-hydroxy-ethyl)-amide as a white solid. (Yield 13.0 mg, 33%).
HRMS (ES⁺) *m*/*z* Calcd for C₂₄H₂₄N₄O₃S + H [(M+H)⁺]: 449.1642. Found: 449.1645.

### Example 68

### 4-Amino-3-{3-[2-(4-chloro-phenyl)-ethoxy]-phenoxymethyl}-thieno[3,2-c]pyridine-7-carboxylic acid ethyl ester

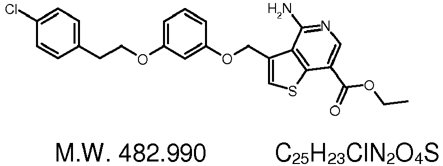

DEAD (2.0 g, 11.5 mmol) (Aldrich) was added to a stirred solution of triphenylphosphine (3.01 g, 11.5 mmol) (Aldrich) in tetrahydrofuran (80 mL) at room temperature. After stirring for 10 minutes, p-chlorophenethyl alcohol (1.40 g, 8.9 mmol) (Aldrich) was added. After stirring for 10 minutes resorcinol (2.92 g, 26.55 mmol) (Aldrich) in tetrahydrofuran (20 mL) was added quickly and mixture stirred for another 18 hours. The reaction mixture was concentrate and the residue was purified by flash chromatography eluting with 20-40 % ethyl acetate in hexanes to give 3-[2-(4-chloro-phenyl)-ethoxy]-phenol. (Yield 1.44 g, 65%).

A suspension of potassium carbonate (0.16 g, 1.17 mmol), 3-[2-(4-chloro-phenyl)-ethoxy]-phenol (0.27 g, 1.07 mmol) in tetrahydrofuran / N,N-dimethylformamide (5:1, 18 mL) was heated at 70 °C for 3 hours. 3-Bromomethyl-4-chloro-thieno[3,2-c]pyridine-7-carboxylic acid ethyl ester (0.34 g, 1.02 mmol) (from Intermediate 8 *supra*) was added. Heating was continued for 18 hours. The reaction mixture was partitioned between ethyl acetate and water. The aqueous phase was extracted with ethyl acetate (1X). The combined organic phase was washed with water and brine, dried (MgSO₄) and concentrated. The residue was purified by flash chromatography eluting with 20% ethyl acetate in hexanes to give 4-chloro-3-{3-[2-(4-chloro-phenyl)-ethoxy]-phenoxymethyl}-thieno[3,2-c]pyridine-7-carboxylic acid ethyl ester. (Yield 0.10 g, 20%).

Ammonia gas was bubbled into a solution of 4-chloro-3-{3-[2-(4-chloro-phenyl)-ethoxy]-phenoxy-methyl}-thieno[3,2-c]pyridine-7-carboxylic acid ethyl ester (0.10 g, 0.20 mmol) in a mixture of 2-propanol / dioxane (1:1, 20 mL) for 20 minutes. The mixture was heated in a sealed pressure vessel at 160 °C for 1 day. The reaction mixture was concentrated. The residue was washed with hot methanol, filtered and dried to give 4-amino-3-{3-[2-(4-chloro-phenyl)-ethoxy]-phenoxymethyl}-thieno[3,2-c]pyridine-7-carboxylic acid ethyl ester as a white powder. (Yield 0.093 g, 96%).
HRMS (ES⁺) *m*/*z* Calcd for C₂₅H₂₃ClN₂O₄S + H [(M+H)⁺]: 483.1140. Found: 483.1143.

### Example 69

### 4-Amino-3-{3-[2-(4-chloro-phenyl)-ethoxy]-phenoxymethyl}-thieno[3,2-c]pyridine-7-carboxylic acid (2-hydroxy-ethyl)-amide

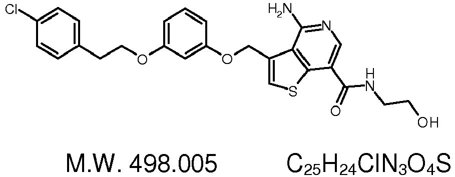

A solution of 4-amino-3-{3-[2-(4-chloro-phenyl)-ethoxy]-phenoxymethyl}-thieno[3,2-c]pyridine-7-carboxylic acid ethyl ester (0.04 g, 0.08 mmol) (from Example 68 *supra*) in dimethylsulfoxide (0.5 mL) was treated with ethanolamine (1.5 mL) (Aldrich) and heated at 160 °C for 2 hours in a microwave reactor. The precipitate formed was filtered and washed with ethyl acetate and methanol, dried to give 4-amino-3-{3-[2-(4-chloro-phenyl)-ethoxy]-phenoxy-methyl}-thieno[3,2-c]pyridine-7-carboxylic acid (2-hydroxy-ethyl)-amide as a white powder. (Yield 28.0 mg, 70%).
HRMS (ES⁺) *m*/*z* Calcd for C₂₅H₂₄ClN₃O₄S + H [(M+H)⁺]: 498.1249. Found: 498.1249.

### Example 70

### 4-Amino-3-[3-(4-chloro-benzoylamino)-phenoxymethyl]-thieno[3,2-c]pyridine-7-carboxylic acid ethyl ester

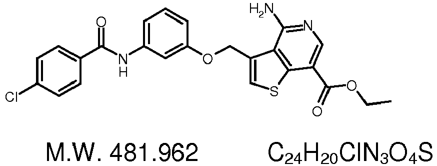

A suspension of potassium carbonate (0.74 g, 5.39 mmol), 4-chloro-N-(3-hydroxyphenyl)-benzamide (1.07 g, 4.30 mmol) (prepared by the reaction of excess 4-chlorobenzoyl chloride with 3-aminophenol and N,N-diisopropylethylamine followed by hydrolysis with 1 N sodium hydroxide) in tetrahydrofuran / N,N-dimethylformamide (5:2, 35 mL) was heated at 70 °C for 3 hours. 3-Bromomethyl-4-chloro-thieno[3,2-c]pyridine-7-carboxylic acid ethyl ester (1.20 g, 3.59 mmol) (from Intermediate 8*supra*) was added. Heating was continued for 2 days. The reaction mixture was partitioned between ethyl acetate and water. The aqueous phase was extracted with ethyl acetate (1X). The combined organic phase was washed with water and brine, dried (MgSO₄) and concentrated. The residue was washed with hot dichloromethane and dried to give 4-chloro-3-[3-(4-chloro-benzoylamino)-phenoxymethyl]-thieno[3,2-c]pyridine-7-carboxylic acid ethyl ester. (Yield 0.99 g, 55%).

Ammonia gas was bubbled into a solution of 4-chloro-3-[3-(4-chloro-benzoylamino)-phenoxy-methyl]-thieno[3,2-c]pyridine-7-carboxylic acid ethyl ester (0.28 g, 0.56 mmol) in a mixture of 2-propanol / dioxane (1:1, 20 mL) for 20 minutes. The mixture was heated in a sealed pressure vessel at 160 °C for 1 day. The reaction mixture was concentrated. The residue was washed with hot methanol, filtered and dried to give 4-amino-3-[3-(4-chloro-benzoylamino)-phenoxymethyl]-thieno[3,2-c]pyridine-7-carboxylic acid ethyl ester as a white powder. (Yield 0.20 g, 74%). HRMS (ES⁺) *m*/*z* Calcd for C₂₄H₂₀ClN₃O₄S + H [(M+H)⁺]: 482.0936. Found: 482.0925.

### Example 71

### 4-Amino-3-[3-(4-chloro-benzoylamino)-phenoxymethyl]-thieno[3,2-c]pyridine-7-carboxylic acid (2-hydroxy-ethyl)-amide

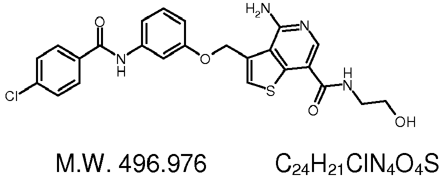

A solution of 4-amino-3-[3-(4-chloro-benzoylamino)-phenoxymethyl]-thieno[3,2-c]pyridine-7-carboxylic acid ethyl ester (0.05 g, 0.10 mmol) (from Example 70 *supra*) in dimethylsulfoxide (0.5 mL) was treated with ethanolamine (1.5 mL) (Aldrich)) and heated at 160 °C for 2 hours in a microwave reactor. The precipitate formed was filtered and washed with ethyl acetate, dried to give 4-amino-3-[3-(4-chloro-benzoylamino)-phenoxymethyl]-thieno[3,2-c]pyridine-7-carboxylic acid (2-hydroxy-ethyl)-amide as a white powder. (Yield 21.0 mg, 40%).
HRMS (ES⁺) *m*/*z* Calcd for C₂₄H₂₁ClN₄O₄S + Na [(M+Na)⁺]: 519.0864. Found: 519.0858.

### Example 72

### 4-Amino-3-[3-(4-chloro-benzoylamino)-phenoxymethyl]-thieno[3,2-c]pyridine-7-carboxylic acid (3-hydroxy-propyl)-amide

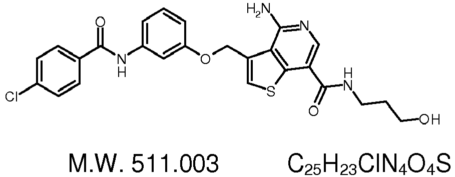

A solution of 4-amino-3-[3-(4-chloro-benzoylamino)-phenoxymethyl]-thieno[3,2-c]pyridine-7-carboxylic acid ethyl ester (0.05 g, 0.10 mmol) (from Example 70 *supra*) in dimethylsulfoxide (0.5 mL) was treated with 3-amino-1-propanol (1.5 mL) (Aldrich) and heated at 160 °C for 2 hours in a microwave reactor. The reaction mixture was purified by C18 column chromatography eluting with acetonitrile / water to give 4-amino-3-[3-(4-chloro-benzoyl-amino)-phenoxymethyl]-thieno[3,2-c]pyridine-7-carboxylic acid (3-hydroxy-propyl)-amide. (Yield 22.0 mg, 43%).
HRMS (ES⁺) *m*/*z* Calcd for C₂₅H₂₃ClN₄O₄S + H [(M+H)⁺]: 511.1202. Found: 511.1195.

### Example 72a

### 4-Amino-3-[3-(4-chloro-benzoylamino)-phenoxymethyl]-thieno[3,2-c]pyridine-7-carboxylic acid (3-hydroxy-propyl)-amide hydrochloride

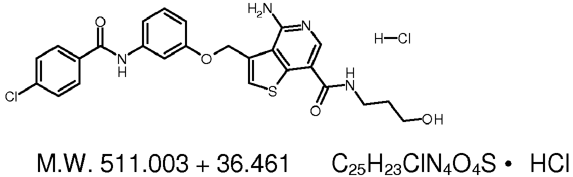

A solution of 4-amino-3-[3-(4-chloro-benzoyl-amino)-phenoxymethyl]-thieno[3,2-c]pyridine-7-carboxylic acid (3-hydroxy-propyl)-amide (0.05 g, 0.10 mmol) (from Example 72 *supra)* in methanol (7.5 mL) was treated with 1 M HCl in diethyl ether (0.15 mL) and heated at 48 °C for 15 minutes. After cooling, diethyl ether (30 mL) was added. The solution was kept in a refrigerator overnight. The solid formed was filtered and dried to give 4-amino-3-[3-(4-chloro-benzoyl-amino)-phenoxymethyl]-thieno[3,2-c]pyridine-7-carboxylic acid (3-hydroxy-propyl)-amide hydrochloride. (Yield 52.0 mg, 96%).
HRMS (ES⁺) *m*/*z* Calcd for C₂₅H₂₃ClN₄O₄S + H [(M+H)⁺]: 511.1202. Found: 511.1203.

### Example 72b

### 4-Amino-3-[3-(4-chloro-benzoylamino)-phenoxymethyl]-thieno[3,2-c]pyridine-7-carboxylic acid (3-hydroxy-propyl)-amide toluene-4-sulfonic acid salt

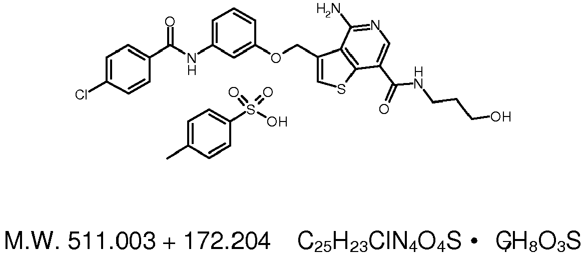

A solution of 4-amino-3-[3-(4-chloro-benzoyl-amino)-phenoxymethyl]-thieno[3,2-c]pyridine-7-carboxylic acid (3-hydroxy-propyl)-amide (0.05 g, 0.10 mmol) (from Example 72 *supra)* in methanol (6 mL) was treated with toluene-4-sulfonic acid hydrate (18.6 mg, 0.10 mmol) (Aldrich) and heated at 40 °C for 30 minutes. The solution was concentrated. The residue was washed with diethyl ether, dissolved in water and lyophilized to give 4-amino-3-[3-(4-chloro-benzoyl-amino)-phenoxymethyl]-thieno[3,2-c]pyridine-7-carboxylic acid (3-hydroxy-propyl)-amide, toluene-4-sulfonic acid salt. (Yield 67.0 mg, 100%).
HRMS (ES⁺) *m*/*z* Calcd for C₂₅H₂₃ClN₄O₄S + H [(M+H)⁺]: 511.1202. Found: 511.1202.

### Example 73

### 4-Amino-3-(2-oxo-1-phenethyl-1,2-dihydro-pyrimidin-4-yloxymethyl)-thieno[3,2-c]pyridine-7-carboxylic acid ethyl ester

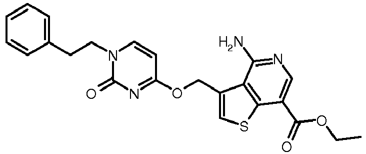

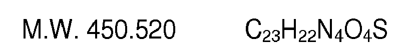

This was prepared following the procedure found in Example 1 *supra.*
HRMS (ES⁺) *m*/*z* Calcd for C₂₃H₂₂N₄O₄S + H [(M+H)⁺]: 451.1435. Found: 451.1434.

### Example 74

### 4-Amino-3-(2,6-dioxo-3-phenethyl-3,6-dihydro-2H-pyrimidin-1-ylmethyl)-thieno[3,2-c]pyridine-7-carboxylic acid ethyl ester

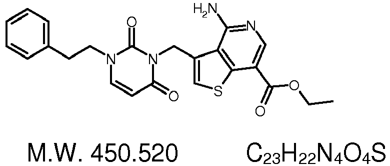

This was prepared following the procedure found in Example 1 *supra.*
HRMS (ES⁺) *m*/*z* Calcd for C₂₃H₂₂N₄O₄S + H [(M+H)⁺]: 451.1435. Found: 451.1434.

### Example 75

### 4-Amino-3-(2,6-dioxo-3-phenethyl-3,6-dihydro-2H-pyrimidin-1-ylmethyl)-thieno[3,2-c]pyridine-7-carboxylic acid (2-hydroxy-ethyl)-amide

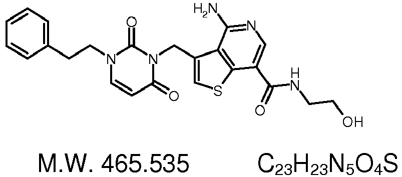

This was prepared following the procedure found in Example 3 *supra.*
HRMS (ES⁺) *m*/*z* Calcd for C₂₃H₂₃N₅O₄S + H [(M+H)⁺]: 466.1544. Found: 466.1548.

### Example 76

### 4-Amino-3-[2-methoxy-5-(3-methoxy-phenylcarbamoyl)-phenoxymethyl]-thieno[3,2-c]pyridine-7-carboxylic acid ethyl ester

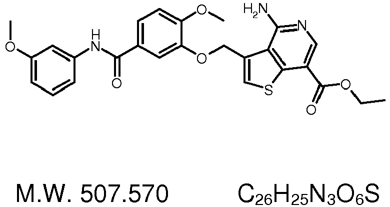

This was prepared following General Procedure 4 *supra.*
LRMS (ES⁺) *m*/*z* Calcd for C₂₆H₂₅N₃O₆S + H [(M+H)⁺]: 508. Found: 508.

### Example 77

### 4-Amino-3-[5-(3,5-dimethoxy-phenylcarbamoyl)-2-methoxy-phenoxymethyl]-thieno[3,2-c]pyridine-7-carboxylic acid ethyl ester

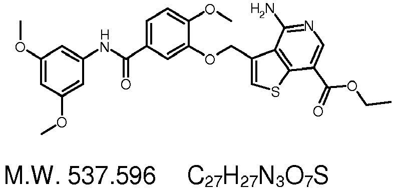

This was prepared following General Procedure 4 *supra.*
LRMS (ES⁺) *m*/*z* Calcd for C₂₇H₂₇N₃O₇S + H [(M+H)⁺]: 538. Found: 538.

### Example 78

### 4-Amino-3-[5-(4-chloro-3-methyl-phenylcarbamoyl)-2-methoxy-phenoxymethyl]-thieno[3,2-c]pyridine-7-carboxylic acid ethyl ester

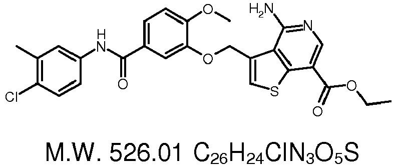

This was prepared following General Procedure 4 *supra.*
LRMS (ES⁺) *m*/*z* Calcd for C₂₇H₂₇N₃O₇S [M⁺]: 526. Found: 526.

### Example 79

### 4-Amino-3-(5-benzylcarbamoyl-2-nitro-phenoxymethyl)-thieno[3,2-c]pyridine-7-carboxylic acid ethyl ester

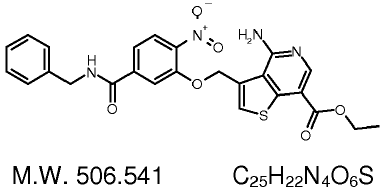

This was prepared following General Procedure 4 *supra.*
LRMS (ES⁺) *m*/*z* Calcd for C₂₅H₂₂N₄O₆S + H [(M+H)⁺]: 507. Found: 507.

### Example 80

### 4-Amino-3-(2-methyl-3-phenylcarbamoyl-phenoxymethyl)-thieno[3,2-c]pyridine-7-carboxylic acid ethyl ester

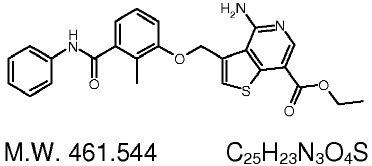

This was prepared following General Procedure 4 *supra.*
LRMS (ES⁺) *m*/*z* Calcd for C₂₅H₂₃N₃O₄S + H [(M+H)⁺]: 462. Found: 462.

### Example 81

### 4-Amino-3-[3-(3,5-dimethoxy-phenylcarbamoyl)-2-methyl-phenoxymethyl]-thieno[3,2-c]pyridine-7-carboxylic acid ethyl ester

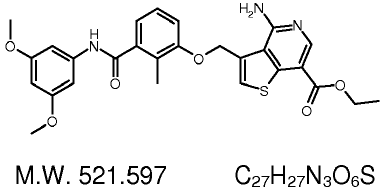

This was prepared following General Procedure 4 *supra.*
LRMS (ES⁺) *m*/*z* Calcd for C₂₅H₂₆N₃O₄S + H [(M+H)⁺]: 522. Found: 522.

### Example 82

### 4-Amino-3-[2-nitro-5-(3-trifluoromethoxy-phenylcarbamoyl)-phenoxymethyl]-thieno[3,2-c]pyridine-7-carboxylic acid ethyl ester

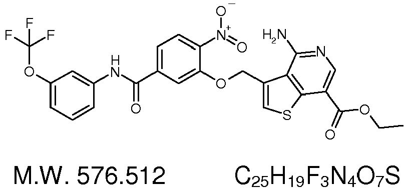

This was prepared following General Procedure 4 *supra.*
LRMS (ES⁺) *m*/*z* Calcd for C₂₅H₁₉F₃N₄O₇S + H [(M+H)⁺]: 577. Found: 577.

### Example 83

### 4-Amino-3-[2-methyl-3-(3-trifluoromethoxy-phenylcarbamoyl)-phenoxymethyl]-thieno[3,2-c]pyridine-7-carboxylic acid ethyl ester

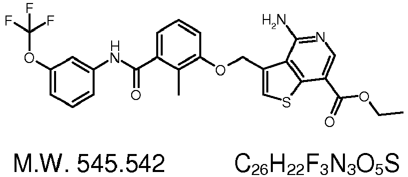

This was prepared following General Procedure 4 *supra.*
LRMS (ES⁺) *m*/*z* Calcd for C₂₆H₂₂F₃N₃O₅S + H [(M+H)⁺]: 546. Found: 546.

### Example 84

### 4-Amino-3-[3-(3-methanesulfonyl-phenylcarbamoyl)-2-methyl-phenoxymethyl]-thieno[3,2-c]pyridine-7-carboxylic acid ethyl ester

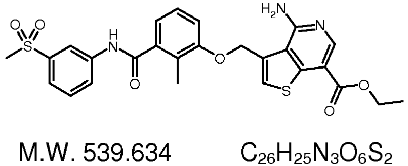

This was prepared following General Procedure 4 *supra.*
LRMS (ES⁺) *m*/*z* Calcd for C₂₆H₂₅N₃O₆S₂ + H [(M+H)⁺]: 540. Found: 540.

### Example 85

### 4-Amino-3-[3-(3-chloro-phenylcarbamoyl)-2-methyl-phenoxymethyl]-thieno[3,2-c]pyridine-7-carboxylic acid ethyl ester

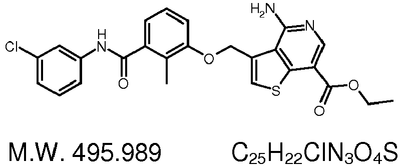

This was prepared following General Procedure 4 *supra.*

### Example 86

### 4-Amino-3-[2-methyl-3-(5-methyl-1 H-pyrazol-3-ylcarbamoyl)-phenoxymethyl]-thieno[3,2-c]pyridine-7-carboxylic acid ethyl ester

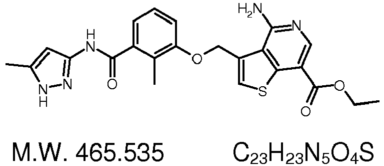

This was prepared following General Procedure 4 *supra.*

### Example 87

### 4-Amino-3-{3-[2-(4-chloro-phenyl)-ethyl]-phenoxymethyl}-thieno[3,2-c]pyridine-7-carboxylic acid ethyl ester

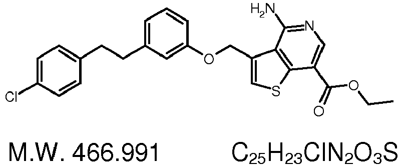

To a solution of 3-[2-(4-chloro-phenyl)-vinyl]-phenol (0.35 g, 1.52 mmol) (from Example 56 *supra)* in methanol (20 mL) was treated with 10% Pd/C (0.04 g) (Aldrich). The reaction mixture was hydrogenated for 6 hours. The reaction mixture was passed through Celite and concentrated. The residue was purified by flash chromatography eluting with 30% ethyl acetate in hexanes to give 3-[2-(4-chlorophenyl)-ethyl]-phenol. (Yield 0.33 g, 94%).

A suspension of potassium carbonate (0.16 g, 1.17 mmol), 3-[2-(4-chlorophenyl)-ethyl]-phenol (0.25 g, 1.07 mmol) in tetrahydrofuran / N,N-dimethylformamide (5:1, 18 mL) was heated at 70 °C for 3 hours. 3-Bromomethyl-4-chloro-thieno[3,2-c]pyridine-7-carboxylic acid ethyl ester (0.34 g, 1.02 mmol) (from Intermediate 8 *supra)* was added. Heating was continued for 18 hours. The reaction mixture was partitioned between ethyl acetate and water. The aqueous phase was extracted with ethyl acetate (1 X). The combined organic phase was washed with water and brine, dried (MgSO₄) and concentrated. The residue was purified by flash chromatography eluting with 20% ethyl acetate in hexanes to give 4-chloro-3-{3-[2-(4-chloro-phenyl)-ethyl]-phenoxymethyl}-thieno[3,2-c]pyridine-7-carboxylic acid ethyl ester. (Yield 0.13 g, 26%).

Ammonia gas was bubbled into a solution of 4-chloro-3-{3-[2-(4-chloro-phenyl)-ethyl]-phenoxy-methyl}-thieno[3,2-c]pyridine-7-carboxylic acid ethyl ester (0.13 g, 0.27 mmol) in a mixture of 2-propanol / dioxane (1:1, 20 mL) for 20 minutes. The mixture was heated in a sealed pressure vessel at 160 °C for 1 day. The reaction mixture was concentrated. The residue was washed with hot methanol, filtered and dried to give 4-Amino-3-{3-[2-(4-chloro-phenyl)-ethyl]-phenoxymethyl}-thieno[3,2-c]pyridine-7-carboxylic acid ethyl ester as a white powder. (Yield 0.08 g, 67%).
HRMS (ES⁺) *m*/*z* Calcd for C₂₅H₂₃ClN₂O₃S + H [(M+H)⁺]: 467.1191. Found: 467.1192.

### Example 88

### 4-Amino-3-{3-[2-(4-chloro-phenyl)-ethyl]-phenoxymethyl}-thieno[3,2-c]pyridine-7-carboxylic acid (2-hydroxy-ethyl)-amide

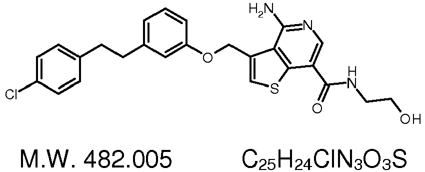

A solution of 4-amino-3-{3-[2-(4-chloro-phenyl)-ethyl]-phenoxymethyl}-thieno[3,2-c]pyridine-7-carboxylic acid ethyl ester (0.05 g, 0.11 mmol) (from Example 87 *supra)* in dimethylsulfoxide (0.5 mL) was treated with ethanolamine (1.5 mL) (Aldrich) and heated at 160 °C for 2 hours in a microwave reactor. The precipitate formed was filtered and washed with hot methanol and dried to give 4-amino-3-{3-[2-(4-chloro-phenyl)-ethyl]-phenoxymethyl}-thieno[3,2-c]pyridine-7-carboxylic acid (2-hydroxy-ethyl)-amide as a white powder. (Yield 31.6 mg, 61 %).
HRMS (ES⁺) *m*/*z* Calcd for C₂₅H₂₄CIN₃O₃S + H [(M+H)⁺]: 482.1300. Found: 482.1302.

### Example 89

### 4-Amino-3-[5-(4-chloro-phenylcarbamoyl)-2-methyl-phenoxymethyl]-thieno[3,2-c]pyridine-7-carboxylic acid ethyl ester

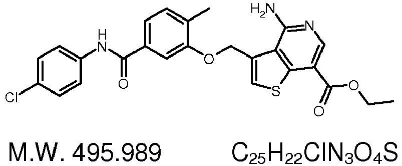

A suspension of 3-hydroxy-4-methylbenzoic acid (10.73 g, 70.5 mmol) (Lancaster) in acetic anhydride (25 mL, 265 mmol) (Aldrich) was heated at reflux for 5 hours. After cooling to toom temperature, mixture was poured into ice - water mixture (600 mL) and stirred overnight. Solid clumps were broken up and collected by filtration. Residue was washed with water and dried in vacuum oven to give 3-acetoxy-4-methyl-benzoic acid as a tan solid. (Yield 11.82 g, 86.3%).

3-Acetoxy-4-methyl-benzoic acid (1.94 g, 10 mmol) was suspended in thionylchloride (3 mL, 40 mmol) (Aldrich) and N,N-dimethyl-formamide (3 drops) and heated at reflux for 2 hours. After cooling to room temperature, mixture was diluted with toluene (30 mL) and concentrated under reduced pressure. Resulting oil was dissolved in dichloromethane (30 mL) and added dropwise to a solution of 4-chloroaniline (1.34 g, 10.5 mmol) (Aldrich) and N,N-diisopropylethylamine (1.6 g, 12.5 mmol) in dichloromethane (50 mL). After stirring for another 2 hours, mixture was diluted with water (50 mL) and stirred for another 30 minutes. Layers were separated. Organic layer was washed with water (50 mL). Aqueous layers were back washed with dichloromethane (50 mL). Organic layers were then combined and concentrated. Residue was dissolved in mixture of tetrahydrofuran (25 mL), methanol (25 mL) and 1 N aqueous sodium hydroxide (10 mL, 10 mmol). After stirring for 16 hours, mixture was diluted with water (100 mL) and acetic acid (5 mL) and concnetrated under reduced pressure to remove most of the organic solvent. Precipitate formed was collected by filtration and washed with water, and dried to give N-(4-chloro-phenyl)-3-hydroxy-4-methyl-benzamide as off-white crystals. (Yield 2.57 g, 98.2%).

A suspension of potassium carbonate (0.21 g, 1.53 mmol), N-(4-chloro-phenyl)-3-hydroxy-4-methyl-benzamide (0.32 g, 1.22 mmol) in tetrahydrofuran / N,N-dimethylformamide (5:2, 21 mL) was heated at 70 °C for 3 hours. 3-Bromomethyl-4-chloro-thieno[3,2-c]pyridine-7-carboxylic acid ethyl ester (0.34 g, 1.02 mmol) (from Intermediate 8 *supra)* was added. Heating was continued for 2 days. The reaction mixture was partitioned between ethyl acetate and water. The aqueous phase was extracted with ethyl acetate (1X). The combined organic phase was washed with water and brine, dried (MgSO₄) and concentrated. The residue was washed with hot dichloromethane and dried to give 4-chloro-3-[5-(4-chloro-phenylcarbamoyl)-2-methyl-phenoxymethyl]-thieno[3,2-c]pyridine-7-carboxylic acid ethyl ester. (Yield 0.23 g, 43%).

Ammonia gas was bubbled into a solution of 4-chloro-3-[5-(4-chloro-phenylcarbamoyl)-2-methyl-phenoxymethyl]-thieno[3,2-c]pyridine-7-carboxylic acid ethyl ester (0.23 g, 0.45 mmol) in a mixture of 2-propanol / dioxane (1:1, 20 mL) for 20 minutes. The mixture was heated in a sealed pressure vessel at 160 °C for 1 day. The reaction mixture was concentrated. The residue was purified by C18 column chromatography eluting with acetonitrile / water to give 4-amino-3-[5-(4-chloro-phenylcarbamoyl)-2-methyl-phenoxy-methyl]-thieno[3,2-c]pyridine-7-carboxylic acid ethyl ester as a white powder. (Yield 0.08 g, 36%).
HRMS (ES⁺) *m*/*z* Calcd for C₂₅H₂₂ClN₃O₄S + H [(M+H)⁺]: 496.1093. Found: 496.1094.

### Example 90

### 4-Amino-3-[5-(4-chloro-phenylcarbamoyl)-2-methyl-phenoxymethyl]-thieno[3,2-c]pyridine-7-carboxylic acid (2-hydroxy-ethyl)-amide

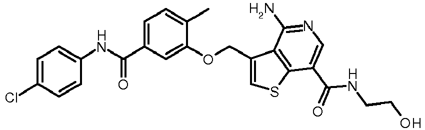

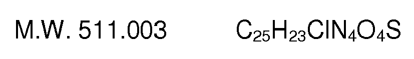

A solution of 4-amino-3-[5-(4-chloro-phenylcarbamoyl)-2-methyl-phenoxymethyl]-thieno[3,2-c] pyridine-7-carboxylic acid ethyl ester (0.05 g, 0.10 mmol) (from Example 89 *supra)* in dimethylsulfoxide (0.5 mL) was treated with ethanolamine (1.5 mL) (Aldrich) and heated at 160 °C for 2 hours in a microwave reactor. The reaction mixture was purified by C18 column chromatography eluting with acetonitrile / water to give 4-amino-3-[5-(4-chloro-phenyl-carbamoyl)-2-methyl-phenoxymethyl]-thieno[3,2-c]pyridine-7-carboxylic acid (2-hydroxy-ethyl)-amide as a white powder. (Yield 25.0 mg, 48%).
HRMS (ES⁺) *m*/*z* Calcd for C₂₅H₂₃ClN₄O₄S + Na [(M+Na)⁺]: 533.1021. Found: 533.1025.

### Example 91

### 4-Amino-3-[5-(4-chloro-benzoylamino)-2-methyl-phenoxymethyl]-thieno[3,2-c]pyridine-7-carboxylic acid ethyl ester

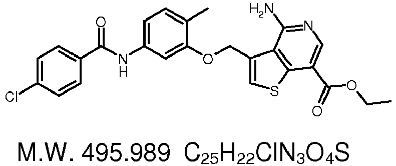

A solution of p-chlorobenzoyl chloride (19.25 g, 110 mmol) (Aldrich) in tetrahydrofuran (50 mL) was added to a solution of 5-amino-o-cresol (6.16 g, 50 mmol) (Aldrich) and triethylamine (17.5 mL, 125 mmol) (Aldrich) and THF (100 mL) dropwise with magnetic stirring and cooling in an ice - water bath. When addition was complete, mixture was allowed to warm to room temperature and stirred for 16 hours. Reaction mixtrue was diluted with water (400 mL). After stirring for another 30 minutes, precipitate was collected to give crude 4-chloro-benzoic acid 5-(4-chloro-benzoylamino)-2-methyl-phenyl ester as an off-white powder. (Yield 21.24 g, 106%).

4-Chloro-benzoic acid 5-(4-chloro-benzoylamino)-2-methyl-phenyl ester (4.04 g, 10 mmol) was dissolved in a mixture of tetrahydrofuran (25 mL, methanol (50 mL) and aqueous 1 N sodium hydroxide (10 mL, 10 mmol). Mixture was stirred at room temperature for 18 hours. Mixture was concentrated under reduced pressure to remove most of the organic solvent. Resulting suspension was diluted with water (100 mL) and saturated aqueous sodium bicarbonate solution (5 mL). After standing for 30 minutes, precipitate was collected and washer with water and dried to give 4-chloro-N-(3-hydroxy-4-methyl-phenyl)-benzamide as a white powder. (Yield 2.46 g, 93.1%).

A suspension of potassium carbonate (0.21 g, 1.53 mmol), 4-chloro-N-(3-hydroxy-4-methyl-phenyl)-benzamide (0.32 g, 1.22 mmol) in tetrahydrofuran / N,N-dimethylformamide (5:2, 21 mL) was heated at 70 °C for 3 hours. 3-Bromomethyl-4-chloro-thieno[3,2-c]pyridine-7-carboxylic acid ethyl ester (0.34 g, 1.02 mmol) (from Intermediate 8 *supra)* was added. Heating was continued for 2 days. The reaction mixture was partitioned between ethyl acetate and water. The aqueous phase was extracted with ethyl acetate (1X). The combined organic phase was washed with water and brine, dried (MgSO₄) and concentrated. The residue was washed with hot dichloromethane and dried to give 4-chloro-3-[5-(4-chloro-benzoylamino)-2-methylphenoxymethyl]-thieno[3,2-c]pyridine-7-carboxylic acid ethyl ester. (Yield 0.10g, 19%).

Ammonia gas was bubbled into a solution of 4-chloro-3-[5-(4-chloro-benzoylamino)-2-methyl-phenoxymethyl]-thieno[3,2-c]pyridine-7-carboxylic acid ethyl ester (0.10 g, 0.19 mmol) in a mixture of 2-propanol / dioxane (1:1, 20 mL) for 20 minutes. The mixture was heated in a sealed pressure vessel at 160 °C for 1 day. The reaction mixture was concentrated. The residue was washed with hot methanol, filtered and dried to give 4-amino-3-[5-(4-chloro-benzoylamino)-2-methyl-phenoxymethyl]-thieno[3,2-c] pyridine-7-carboxylic acid ethyl ester as a white powder. (Yield 95.0 mg, 99%).
HRMS (ES⁺) *m*/*z* Calcd for C₂₅H₂₂ClN₃O₄S + H [(M+H)⁺]: 496.1093. Found: 496.1092.

### Example 92

### 4-Amino-3-[5-(4-chloro-benzoylamino)-2-methyl-phenoxymethyl]-thieno[3,2-c]pyridine-7-carboxylic acid (2-hydroxy-ethyl)-amide

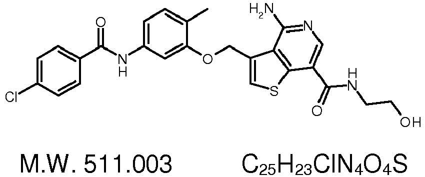

A solution of 4-amino-3-[5-(4-chloro-benzoylamino)-2-methyl-phenoxymethyl]-thieno[3,2-c] pyridine-7-carboxylic acid ethyl ester (0.05 g, 0.10 mmol) (from Example 91 *supra)* in dimethylsulfoxide (0.5 mL) was treated with ethanolamine (1.5 mL) and heated at 160 °C for 2 hours in a microwave reactor. The reaction mixture was purified by C18 column chromatography eluting with acetonitrile / water to give 4-amino-3-[5-(4-chloro-benzoyl-amino)-2-methyl-phenoxymethyl]-thieno[3,2-c]pyridine-7-carboxylic acid (2-hydroxy-ethyl)-amide. (Yield 34.0 mg, 65%).
HRMS (ES⁺) *m*/*z* Calcd for C₂₅H₂₃ClN₄O₄S + H [(M+H)⁺]: 511.1202. Found: 511.1202.

### Example 93

### 4-Amino-3-[3-(4-fluoro-benzoylamino)-phenoxymethyl]-thieno[3,2-c]pyridine-7-carboxylic acid ethyl ester

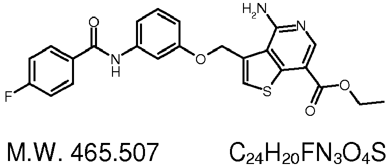

This was prepared following the procedure found in Example 1 *supra.*
HRMS (ES⁺) *m*/*z* Calcd for C₂₄H₂₀FN₃O₄S + H [(M+H)⁺]: 466.12322. Found: 466.1231.

### Example 94

### 4-Amino-3-[5-(4-chloro-2-methyl-phenylcarbamoyl)-2-methoxy-phenoxymethyl]-thieno[3,2-c]pyridine-7-carboxylic acid ethyl ester

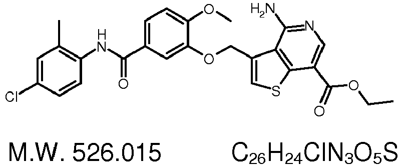

This was prepared following General Procedure 4 *supra.*

### Example 95

### 4-Amino-3-[3-(5-chloro-2-methoxy-phenylcarbamoyl)-2-methyl-phenoxymethyl]-thieno[3,2-c]pyridine-7-carboxylic acid ethyl ester

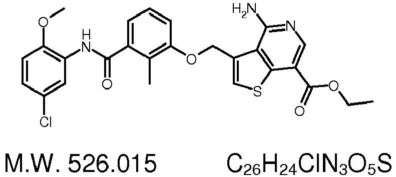

This was prepared following General Procedure 4 *supra.*

### Example 96

### 4-Amino-3-[4-(3-methanesulfonyl-phenylcarbamoyl)-2-methyl-phenoxymethyl]-thieno[3,2-c]pyridine-7-carboxylic acid ethyl ester

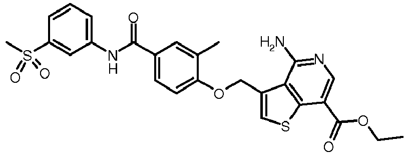

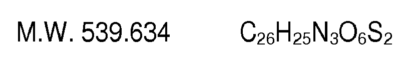

This was prepared following General Procedure 4 *supra.*
LRMS *m*/*z* Calcd for C₂₆H₂₅N₃O₆S₂ [M⁺]: 539. Found: 539.

### Example 97

### 4-Amino-3-(5-phenylcarbamoyl-pyridin-3-yloxymethyl)-thieno[3,2-c]pyridine-7-carboxylic acid ethyl ester

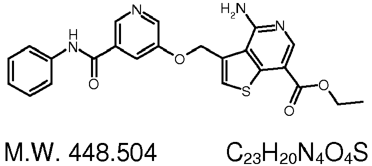

This was prepared following the procedure found in Example 1 *supra.*
HRMS (ES⁺) *m*/*z* Calcd for C₂₃H₂₀N₄O₄S + H [(M+H)⁺]: 449.1278. Found: 449.1278.

### Example 98

### 4-Amino-3-[5-(4-chloro-phenylcarbamoyl)-pyridin-3-yloxymethyl]-thieno[3,2-c]pyridine-7-carboxylic acid ethyl ester

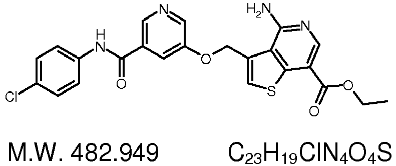

This was prepared following the procedure found in Example 1 *supra.*
HRMS (ES⁺) *m*/*z* Calcd for C₂₃H₁₉ClN₄O₄S + H [(M+H)⁺]: 483.0889. Found: 483.0890.

### Intermediate 43

### 4-Chloro-3-[3-(3-methyl-benzyloxy)-phenoxymethyl]-thieno[3,2-c]pyridine-7-carboxylic acid ethyl ester

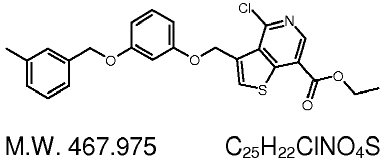

This was prepared following General Procedure 6 *supra.*

### Intermediate 44

### 4-Chloro-3-[3-(4-fluoro-benzyloxy)-phenoxymethyl]-thieno[3,2-c]pyridine-7-carboxylic acid ethyl ester

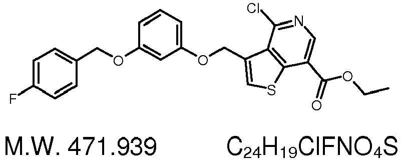

This was prepared following General Procedure 6 *supra.*

### Intermediate 45

### 4-Chloro-3-[3-(4-methyl-benzyloxy)-phenoxymethyl]-thieno[3,2-c]pyridine-7-carboxylic acid ethyl ester

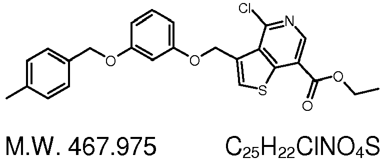

This was prepared following General Procedure 6 *supra.*

### Intermediate 46

### 4-Chloro-3-[3-(2,4-difluoro-benzyloxy)-phenoxymethyl]-thieno[3,2-c]pyridine-7-carboxylic acid ethyl ester

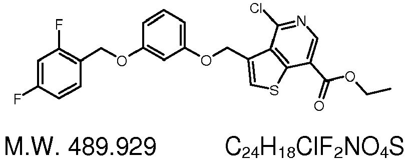

This was prepared following General Procedure 6 *supra.*

### Example 99

### 4-Amino-3-[3-(4-chloro-benzoylamino)-phenoxymethyl]-thieno[3,2-c]pyridine-7-carboxylic acid (2-methoxy-ethyl)-amide

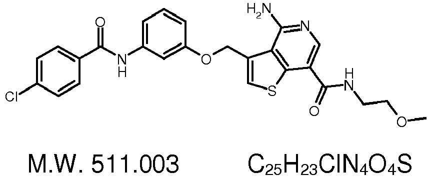

This was prepared following the procedure found in Example 60 *supra.*
HRMS (ES⁺) *m*/*z* Calcd for C₂₅H₂₃ClN₄O₄S + H [(M+H)⁺]: 511.1202. Found: 511.1201.

### Example 100

### 4-Amino-3-[3-(4-chloro-benzoylamino)-phenoxymethyl]-thieno[3,2-c]pyridine-7-carboxylic acid (3-methoxy-propyl)-amide

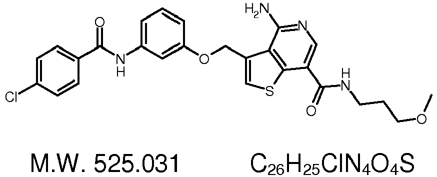

This was prepared following the procedure found in Example 60 *supra.*
HRMS (ES⁺) *m*/*z* Calcd for C₂₆H₂₅ClN₄O₄S + H [(M+H)⁺]: 525.1358. Found: 525.1357.

### Example 101

### 4-Amino-3-[5-(pyridin-4-ylcarbamoyl)-pyridin-3-yloxymethyl]-thieno[3,2-c]pyridine-7-carboxylic acid ethylester

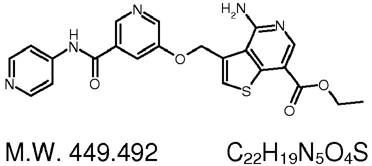

This was prepared following the procedure found in Example 1 *supra.*
HRMS (ES⁺) *m*/*z* Calcd for C₁₈H₁₉N₅O₄S + H [(M+H)⁺]: 450.1231. Found: 450.1229.

### Example 102

### 4-Amino-3-[5-(4-chloro-phenylcarbamoyl)-pyridin-3-yloxymethyl]-thieno[3,2-c]pyridine-7-carboxylic acid (2-hydroxy-ethyl)-amide

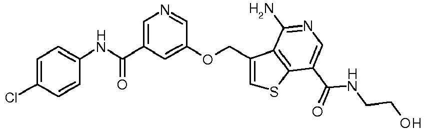

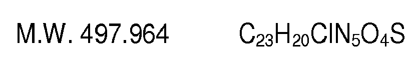

This was prepared following the procedure found in Example 3 *supra.*
HRMS (ES⁺) *m*/*z* Calcd for C₂₃H₂₀ClN₅O₄S + H [(M+H)⁺]: 498.0998. Found: 498.1000.

### Example 103

### 4-Amino-3-(5-phenylcarbamoyl-pyridin-3-yloxymethyl)-thieno[3,2-c]pyridine-7-carboxylic acid (2-hydroxy-ethyl)-amide

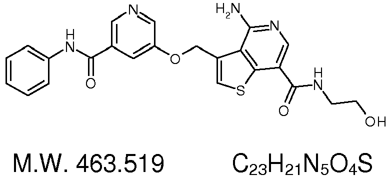

This was prepared following the procedure found in Example 3 *supra.*
HRMS (ES⁺) *m*/*z* Calcd for C₂₃H₂₁N₅O₄S + H [(M+H)⁺]: 464.1387. Found: 464.1390.

### Example 104a

### 4-Amino-3-[3-(4-chloro-benzoylamino)-phenoxymethyl]-thieno[3,2-c]pyridine-7-carboxylic acid (4-pyrrolidin-1-yl-butyl)-amide trifluoro-acetic acid salt

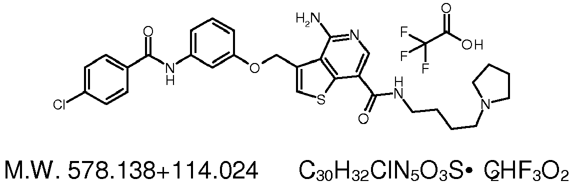

A mixture of 4-amino-3-[3-(4-chloro-benzoylamino)-phenoxymethyl]-thieno[3,2-c]pyridine-7-carboxylic acid (0.15 g, 0.13 mmol) (from Example 151 *infra*), 1-hydroxybenzotriazole hydrate (71 mg, 0.56 mmol) (Aldrich) and 1,3-diisopropylcarbodiimide (0.077 mL, 0.50 mmol) (Aldrich) were combined in tetrahydrofuran : N,N-dimethylformamide (9.0 mL, 5:1) with stirring. After 1 hour, 4-pyrrolidinobutylamine (0.14 g, 1.04 mmol) (Aldrich) was added. The mixture was stirred at room temperature for 3 days and then concentrated. The residue was purified by HPLC eluting with acetonitrile / water containing 0.1% trifluoroacetic acid to give 4-amino-3-[3-(4-chloro-benzoylamino)-phenoxymethyl]-thieno[3,2-c]pyridine-7-carboxylic acid (4-pyrrolidin-1-yl-butyl)-amide, trifluoroacetic acid salt as a white powder. (Yield 0.16 g, 67%).
HRMS (ES⁺) *m*/*z* Calcd for C₃₀H₃₂ClN₅O₃S + H [(M+H)⁺]: 578.1987. Found: 578.1982.

### Example 104b

### 4-Amino-3-[3-(4-chloro-benzoylamino)-phenoxymethyl]-thieno[3,2-c]pyridine-7-carboxylic acid (4-pyrrolidin-1-yl-butyl)-amide toluene-4-sulfonic acid salt

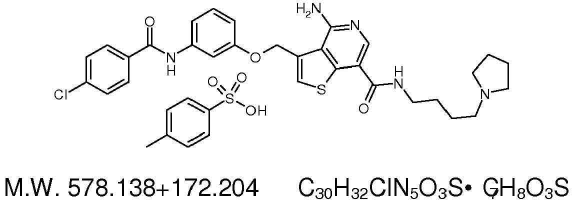

To a solution of 4-amino-3-[3-(4-chloro-benzoylamino)-phenoxymethyl]-thieno[3,2-c]pyridine-7-carboxylic acid (4-pyrrolidin-1-yl-butyl)-amide (0.047 g, 0.08 mmol) (from Example 104a *supra)* in methanol (6 mL) was treated with toluene-4-sulfonic acid hydrate (18.6 mg, 0.10 mmol) (Aldrich) and heated at 40 °C for 30 minutes. The solution was concentrated. The residue was washed with diethyl ether, dissolved in water and lyophilized to give 4-amino-3-[3-(4-chloro-benzoylamino)-phenoxymethyl]-thieno[3,2-c]pyridine-7-carboxylic acid (4-pyrrolidin-1-yl-butyl)-amide toluene-4-sulfonic acid salt as a white powder. (Yield 60.0 mg, 100%).
HRMS (ES⁺) *m*/*z* Calcd for C₃₀H₃₂ClN₅O₃S + H [(M+H)⁺]: 578.1987. Found: 578.1984.

### Example 105

### 4-Amino-3-[5-(4-chloro-benzyloxy)-2-methyl-phenoxymethyl]-thieno[3,2-c]pyridine-7-carboxylic acid ethyl ester

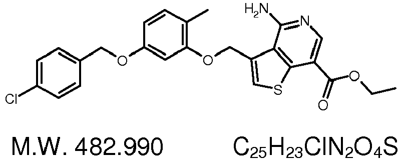

A mixture of 2,4-dihydroxybenzaldehyde (6.62 g, 48 mmol) (Fluka), potassium fluoride (5.57 g, 96 mmol) (Aldrich) and 4-chlorobenzyl chloride (13.50 g, 84 mmol) (Aldrich) in acetonitrile (50 mL) was heated at 90 °C for 24 hours. After cooling, mixture was partitioned between ether (2 X 100 mL) and water (2 X 100 mL). Organic layers were washed with brine (100 mL), combined, dried (MgSO₄), filtered and concentrated. Residue was purified by flash chromatography (Biotage 75S, hexanes, then hexanes - dichloromethane 1:1 as solvent) gave partial separation. Pure fractions of product were combined and concentrated and residue crystallized from hexanes with traces of dichloromethane to give 4-(4-chloro-benzyloxy)-2-hydroxy-benzaldehyde as white crystals. (Yield 4.74 g, 37.6%). Mother liquor and impure fractions were combined and further purified by flash chromatography (Biotage 40L, same solvent as before) and pure fractions combined and concentrated. Residue re-crystallized to give second crop of 4-(4-chloro-benzyloxy)-2-hydroxy-benzaldehyde. (Yield 3.03 g, 24.1 %).

To a solution of 4-(4-chloro-benzyloxy)-2-hydroxybenzaldehyde (1.31 g, 5.0 mmol) and sodium cyanoborohydride (1.0 g, 15.9 mmol) (Aldrich) in tetrahydrofuran (30 mL) was added methyl orange as an indictor, giving the solution a yellow color; 1 N aqueous HCl solution (7.5 mL) was added slowly, keeping the solution orange. The mixture was stirred overnight at room temperature. Water was added, and the mixture was extracted with ethyl acetate three times. The combined organic phase was washed with water and brine, dried (MgSO₄) and concentrated. The residue was purified by flash chromatography eluting with 0 - 40% ethyl acetate in hexanes to give 5-(4-chloro-benzyloxy)-2-methyl-phenol. (Yield 0.43 g, 35%).

A suspension of potassium carbonate (0.33 g, 2.36 mmol), 5-(4-chloro-benzyloxy)-2-methyl-phenol (0.43 g, 1.73 mmol) in tetrahydrofuran / N,N-dimethylformamide (5:2, 17.5 mL) was heated at 70 °C for 3 hours. 3-Bromomethyl-4-chloro-thieno[3,2-c]pyridine-7-carboxylic acid ethyl ester (0.53 g, 1.57 mmol) (from Intermediate 8 *supra)* was added. Heating was continued for 1 day. The reaction mixture was partitioned between ethyl acetate and water. The aqueous phase was extracted with ethyl acetate (2X). The combined organic phase was washed with water and brine, dried (MgS₄) and concentrated. The residue was purified by flash chromatography eluting with 10 - 20% ethyl acetate in hexanes to give 4-chloro-3-[5-(4-chloro-benzyloxy)-2-methyl-phenoxymethyl]-thieno[3,2-c]pyridine-7-carboxylic acid ethyl ester. (Yield 0.37 g, 47%).

Ammonia gas was bubbled into a solution of 4-chloro-3-[5-(4-chloro-benzyloxy)-2-methyl-phenoxymethyl]-thieno[3,2-c]pyridine-7-carboxylic acid ethyl ester (0.21 g, 0.29 mmol) in 2-propanol (15 mL) for 20 minutes. The mixture was heated in a microwave reactor at 140 °C for 2 hours. The reaction mixture was concentrated. The residue was washed with hot methanol, filtered and dried to give 4-amino-3-[5-(4-chloro-benzyloxy)-2-methyl-phenoxymethyl]-thieno[3,2-c]pyridine-7-carboxylic acid ethyl ester as a white powder. (Yield 0.18 g, 90%).
HRMS (ES⁺) *m*/*z* Calcd for C₂₅H₂₃ClN₂O₄S + H [(M+H)⁺]: 483.1140. Found: 483.1143.

### Example 106

### 4-Amino-3-[3-(4-fluoro-benzyloxy)-phenoxymethyl]-thieno[3,2-c]pyridine-7-carboxylic acid ethyl ester

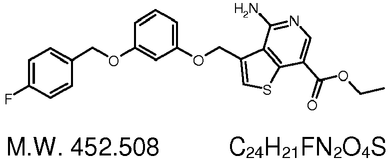

This was prepared following General Procedure 7 *supra.*
LRMS (ES⁺) *m*/*z* Calcd for C₂₄H₂₁FN₂O₄S + H [(M+H)⁺]: 453. Found: 453.

### Example 107

### 4-Amino-3-[3-(4-methyl-benzyloxy)-phenoxymethyl]-thieno[3,2-c]pyridine-7-carboxylic acid ethyl ester

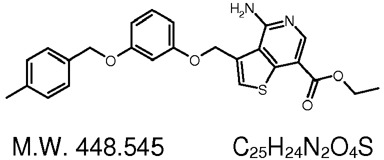

This was prepared following General Procedure 7 *supra.*
LRMS (ES⁺) *m*/*z* Calcd for C₂₅H₂₄N₂O₄ + H [(M+H)⁺]: 449. Found: 449.

### Intermediate 47

### 4-Chloro-3-[3-(3-methoxy-benzyloxy)-phenoxymethyl]-thieno[3,2-c]pyridine-7-carboxylic acid ethyl ester

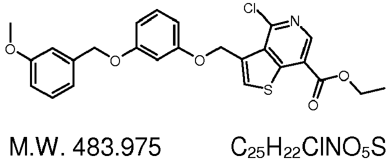

This was prepared following General Procedure 6 *supra.*
LRMS (ES⁺) *m*/*z* Calcd for C₂₅H₂₂ClNO₅S + H [(M+H)⁺]: 484. Found: 484.

### Intermediate 48

### 4-Chloro-3-[3-(3-fluoro-benzyloxy)-phenoxymethyl]-thieno[3,2-c]pyridine-7-carboxylic acid ethyl ester

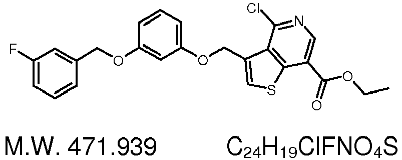

This was prepared following General Procedure 6 *supra.*
LRMS (ES⁺) *m*/*z* Calcd for C₂₄H₁₉ClFNO₄S + H [(M+H)⁺]: 472. Found: 472.

### Intermediate 49

### 4-Chloro-3-[3-(3,5-difluoro-benzyloxy)-phenoxymethyl]-thieno[3,2-c]pyridine-7-carboxylic acid ethyl ester

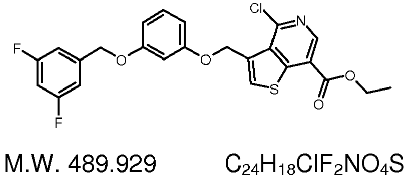

This was prepared following General Procedure 6 *supra.*
LRMS (ES⁺) *m*/*z* Calcd for C₂₄H₁₈ClF₂NO₄S + H [(M+H)⁺]: 490. Found: 490.

### Intermediate 50

### 4-Chloro-3-[3-(2-methyl-benzyloxy)-phenoxymethyl]-thieno[3,2-c]pyridine-7-carboxylic acid ethyl ester

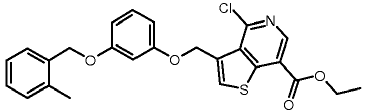

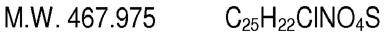

This was prepared following General Procedure 6 *supra.*
LRMS (ES⁺) *m*/*z* Calcd for C₂₅H₂₂ClNO₄S + H [(M+H)⁺]: 468. Found: 468.

### Example 108

### 4-Amino-3-[3-(3-fluoro-benzyloxy)-phenoxymethyl]-thieno[3,2-c]pyridine-7-carboxylic acid ethyl ester

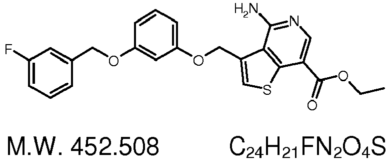

This was prepared following General Procedure 7 *supra.*
LRMS (ES⁺) *m*/*z* Calcd for C₂₄H₂₁FN₂O₄S + H [(M+H)⁺]: 453. Found: 453.

### Example 109

### 4-Amino-3-[3-(3,5-difluoro-benzyloxy)-phenoxymethyl]-thieno[3,2-c]pyridine-7-carboxylic acid ethyl ester

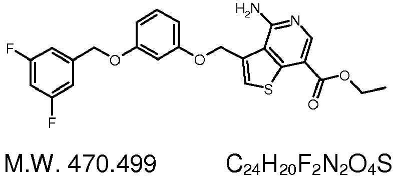

This was prepared following General Procedure 7 *supra.*
LRMS (ES⁺) *m*/*z* Calcd for C₂₄H₂₀F₂N₂O₄S + H [(M+H)⁺]: 471. Found: 471.

### Example 110

### 4-Amino-3-[5-(4-chloro-benzyloxy)-2-methyl-phenoxymethyl]-thieno[3,2-c]pyridine-7-carboxylic acid (2-hydroxy-ethyl)-amide

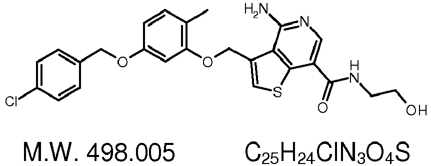

A solution of 4-amino-3-[5-(4-chloro-benzyloxy)-2-methyl-phenoxymethyl]-thieno[3,2-c]pyridine-7-carboxylic acid ethyl ester (0.05 g, 0.10 mmol) (from Example 105 *supra)* in dimethylsulfoxide (0.5 mL) was treated with ethanolamine (1.5 mL) (Aldrich) and heated at 160 °C for 2 hours in a microwave reactor. The reaction mixture was purified by C18 column chromatography eluting with acetonitrile / water to give to 4-amino-3-[5-(4-chloro-benzyloxy)-2-methyl-phenoxymethyl]-thieno[3,2-c]pyridine-7-carboxylic acid (2-hydroxy-ethyl)-amide as a white powder. (Yield 28 mg, 54%).
HRMS (ES⁺) *m*/*z* Calcd for C₂₅H₂₄ClN₃O₄S + H [(M+H)⁺]: 498.1249. Found: 498.1248.

### Example 111

### 4-Amino-3-(5-benzyloxy-2-methyl-phenoxymethyl)-thieno[3,2-c]pyridine-7-carboxylic acid ethyl ester

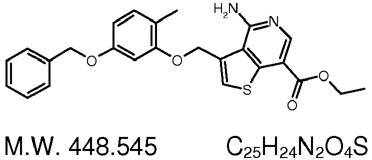

To a solution of 4-benzyloxy-2-hydroxybenzaldehyde (2.28 g, 10.0 mmol) (prepared by the method of Example 105 *supra)* and sodium cyanoborohydride (2.0 g, 15.9 mmol) in tetrahydrofuran (60 mL) was added methyl orange as an indictor, giving the solution a yellow color; 1 N aqueous HCl solution (15 mL) was added slowly, keeping the solution orange. The mixture was stirred overnight at room temperature. Water was added, and the mixture was extracted with ethyl acetate three times. The combined organic phase was washed with water and brine, dried (MgSO₄) and concentrated. The residue was purified by flash chromatography eluting with 0 - 40% ethyl acetate in hexanes to 5-benzyloxy-2-methyl-phenol. (Yield 0.64 g, 30%).

A suspension of potassium carbonate (0.16 g, 1.17 mmol), 5-benzyloxy-2-methylphenol (0.23 g, 1.07 mmol) in tetrahydrofuran / N,N-dimethylformamide (5:2, 14 mL) was heated at 70 °C for 3 hours. 3-Bromomethyl-4-chloro-thieno[3,2-c]pyridine-7-carboxylic acid ethyl ester (0.34g, 1.02 mmol) (from Intermediate 8 *supra)* was added. Heating was continued for 1 day. The reaction mixture was partitioned between ethyl acetate and water. The aqueous phase was extracted with ethyl acetate (2X). The combined organic phase was washed with water and brine, dried (MgSO₄) and concentrated. The residue was purified by flash chromatography eluting with 10 - 20% ethyl acetate in hexanes to give 3-(5-benzyloxy-2-methylphenoxymethyl)-4-chloro-thieno[3,2-c]pyridine-7-carboxylic acid ethyl ester. (Yield 0.17 g, 35%).

Ammonia gas was bubbled into a solution of 3-(5-benzyloxy-2-methylphenoxymethyl)-4-chloro-thieno[3,2-c]pyridine-7-carboxylic acid ethyl ester (0.17 g, 0.36 mmol) in 2-propanol (15 mL) for 20 minutes. The mixture was heated in a microwave reactor at 140 °C for 2 hours. The reaction mixture was concentrated. The residue was washed with hot methanol, filtered and dried to give 4-amino-3-(5-benzyloxy-2-methyl-phenoxymethyl)-thieno[3,2-c]pyridine-7-carboxylic acid ethyl ester as a white powder. (Yield 0.07 g, 44%).
HRMS (ES⁺) *m*/*z* Calcd for C₂₅H₂₄N₂O₄S + H [(M+H)⁺]: 449.1530. Found: 449.1529.

### Example 112

### 4-Amino-3-(5-benzyloxy-2-methyl-phenoxymethyl)-thieno[3,2-c]pyridine-7-carboxylic acid (2-hydroxy-ethyl)-amide

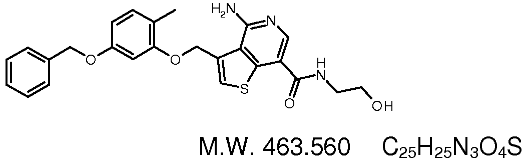

A solution of 4-amino-3-(5-benzyloxy-2-methyl-phenoxymethyl)-thieno[3,2-c]pyridine-7-carboxylic acid ethyl ester (0.05 g, 0.10 mmol) (from Example 111 *supra)* in dimethylsulfoxide (0.5 mL) was treated with ethanolamine (1.5 mL) (Aldrich) and heated at 160 °C for 2 hours in a microwave reactor. The reaction mixture was purified by C18 column chromatography eluting with acetonitile / water to give to 4-amino-3-(5-benzyloxy-2-methyl-phenoxymethyl)-thieno[3,2-c]pyridine-7-carboxylic acid (2-hydroxy-ethyl)-amide as a white powder. (Yield 27 mg, 52%).
HRMS (ES⁺) *m*/*z* Calcd for C₂₅H₂₅N₃O₄S + H [(M+H)⁺]: 464.1639. Found: 464.1642.

### Example 113

### 4-Amino-3-(5-benzoylamino-2-methyl-phenoxymethyl)-thieno[3,2-c]pyridine-7-carboxylic acid ethyl ester

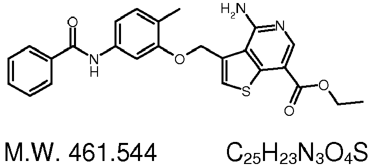

A solution of benzoyl chloride (7.75 g, 55 mmol) (Aldrich) in tetrahydrofuran (25 mL) was added to a solution of 5-amino-o-cresol (3.08 g, 25 mmol) (Aldrich) and triethylamine (8.8 mL, 62.5 mmol) (Aldrich) and THF (50 mL) dropwise with magnetic stirring. When addition was complete, mixture was allowed to warm to room temperature and stirred for 4 hours. Reaction mixtrue was diluted with water (400 mL). After stirring for another 30 minutes, precipitate was collected. This material was dissolved in a mixture of THF (120 mL), methanol (40 mL) and 1 N aqueous sodium hydroxide solution (25 mL, 25 mmol) and stirred at room temperature for 16 hours. Mixture was then diluted with water (100 mL) and concentrated under reduced pressure to remove organic solvent. Precipitate formed was collected and washed with water and dried to give N-(3-hydroxy-4-methyl-phenyl)-benzamide as an off white powder. (Yield 2.14 g, 37.7%). Further concentrating and on standing a second crop of N-(3-hydroxy-4-methyl-phenyl)-benzamide was formed. (Yield 3.66 g, 64.4%).

A suspension of potassium carbonate (0.16 g, 1.17 mmol), N-(3-hydroxy-4-methylphenyl)-benzamide (0.24 g, 1.07 mmol) in tetrahydrofuran / N,N-dimethylformamide (5:2, 14 mLI) was heated at 70 °C for 3 hours. 3-Bromomethyl-4-chloro-thieno[3,2-c]pyridine-7-carboxylic acid ethyl ester (0.34 g, 1.02 mmol) (from Intermediate 8 *supra)* was added. Heating was continued for 2 days. The reaction mixture was partitioned between ethyl acetate and water. The aqueous phase was extracted with ethyl acetate (1 X). The combined organic phase was washed with water and brine, dried (MgSO₄) and concentrated. The residue was purified by flash chromatography eluting with 0 - 20% ethyl acetate in hexanes to give 3-(5-benzoylamino-2-methylphenoxymethyl)-4-chloro-thieno[3,2-c]pyridine-7-carboxylic acid ethyl ester. (Yield 0.12 g, 24%).

Ammonia gas was bubbled into a solution of 3-(5-benzoylamino-2-methylphenoxymethyl)-4-chloro-thieno[3,2-c]pyridine-7-carboxylic acid ethyl ester (0.21 g, 0.44 mmol) in 2-propanol (15 mL) for 20 minutes. The mixture was heated in microwave at 140 °C for 2 hours. The reaction mixture was concentrated. The residue was washed with hot methanol, filtered and dried to give 4-amino-3-(5-benzoylamino-2-methyl-phenoxymethyl)-thieno[3,2-c]pyridine-7-carboxylic acid ethyl ester as a white powder. (Yield 0.12 g, 60%).
HRMS (ES⁺) *m*/*z* Calcd for C₂₅H₂₃N₃O₄S + H [(M+H)⁺]: 462.1482. Found: 462.1483.

### Example 114

### 4-Amino-3-(5-benzoylamino-2-methyl-phenoxymethyl)-thieno[3,2-c]pyridine-7-carboxylic acid (2-hydroxy-ethyl)-amide

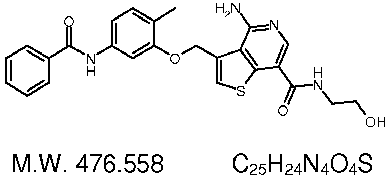

A solution of 4-amino-3-(5-benzoylamino-2-methyl-phenoxymethyl)-thieno[3,2-c]pyridine-7-carboxylic acid ethyl ester (0.05 g, 0.11 mmol) (from Example 113 *supra)* in dimethylsulfoxide (0.5 mL) was treated with ethanolamine (1.5 mL) (Aldrich) and heated at 160 °C for 2 hours in a microwave reactor. The reaction mixture was purified by C18 column chromatography eluting with acetonitrile / water to give 4-amino-3-(5-benzoylamino-2-methyl-phenoxymethyl)-thieno[3,2-c]pyridine-7-carboxylic acid (2-hydroxy-ethyl)-amide as a white powder. (Yield 23.0 mg, 44%).
HRMS (ES⁺) *m*/*z* Calcd for C₂₅H₂₄N₄O₄S + Na [(M+Na)⁺]: 499.1410. Found: 499.1411.

### Intermediate 51

### 4-Chloro-3-[3-(3,5-difluoro-benzyloxy)-5-methyl-phenoxymethyl]-thieno[3,2-c]pyridine-7-carboxylic acid ethyl ester

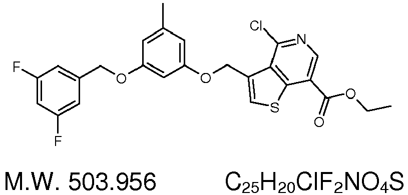

This was prepared following General Procedure 6 *supra.*
LRMS (ES⁺) *m*/*z* Calcd for C₂₅H₂₀ClF₂NO₄S + H [(M+H)⁺]: 504. Found: 504.

### Intermediate 52

### 4-Chloro-3-[3-(3,4-difluoro-benzyloxy)-5-methyl-phenoxymethyl]-thieno[3,2-c]pyridine-7-carboxylic acid ethyl ester

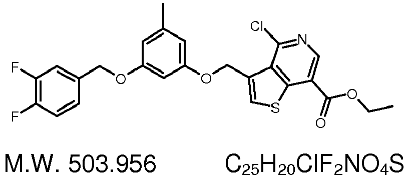

This was prepared following General Procedure 6 *supra.*
LRMS (ES⁺) *m*/*z* Calcd for C₂₅H₂₀ClF₂NO₄S + H [(M+H)⁺]: 504. Found: 504.

### Intermediate 53

### 4-Chloro-3-[3-(2,5-difluoro-benzyloxy)-phenoxymethyl]-thieno[3,2-c]pyridine-7-carboxylic acid ethyl ester

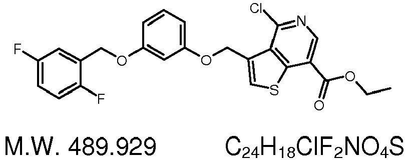

This was prepared following General Procedure 6 *supra.*
LRMS (ES⁺) *m*/*z* Calcd for C₂₄H₁₈ClF₂NO₄S + H [(M+H)⁺]: 490. Found: 490.

### Intermediate 54

### 4-Chloro-3-[3-(2,4-difluoro-benzyloxy)-5-methyl-phenoxymethyl]-thieno[3,2-c]pyridine-7-carboxylic acid ethyl ester

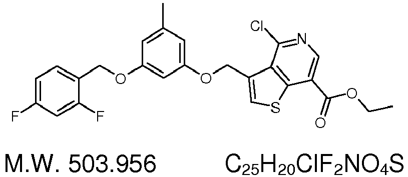

This was prepared following General Procedure 6 *supra.*
LRMS (ES⁺) *m*/*z* Calcd for C₂₅H₂₀ClF₂NO₄S + H [(M+H)⁺]: 504. Found: 504.

### Example 115

### 4-Amino-3-[3-(2-fluoro-benzyloxy)-phenoxymethyl]-thieno[3,2-c]pyridine-7-carboxylic acid ethyl ester

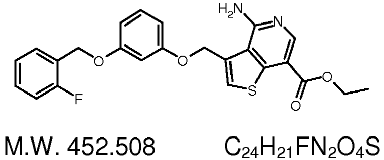

This was prepared following General Procedure 7 *supra.*
LRMS (ES⁺) *m*/*z* Calcd for C₂₄H₂₁FN₂O₄S + H [(M+H)⁺]: 453. Found: 453.

### Intermediate 55

### 4-Chloro-3-[3-(2,3-difluoro-benzyloxy)-phenoxymethyl]-thieno[3,2-c]pyridine-7-carboxylic acid ethyl ester

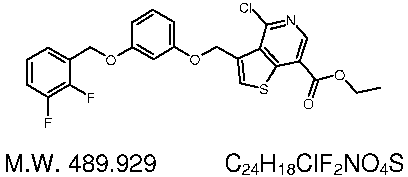

This was prepared following General Procedure 6 *supra.*
LRMS (ES⁺) *m*/*z* Calcd for C₂₄H₁₈ClF₂NO₄S + H [(M+H)⁺]: 490. Found: 490.

### Intermediate 56

### 4-Chloro-3-[3-(4-methoxy-benzyloxy)-phenoxymethyl]-thieno[3,2-c]pyridine-7-carboxylic acid ethyl ester

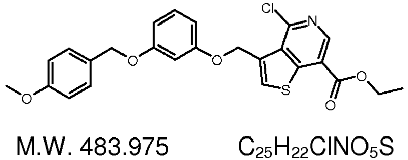

This was prepared following General Procedure 6 *supra.*
LRMS (ES⁺) *m*/*z* Calcd for C₂₅H₂₂ClNO₅S + H [(M+H)⁺]: 484. Found: 484.

### Intermediate 57

### 4-Chloro-3-[3-(5-chloro-thiophen-2-ylmethoxy)-phenoxymethyl]-thieno[3,2-c]pyridine-7-carboxylic acid ethyl ester

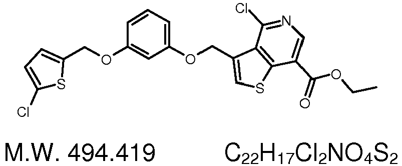

This was prepared following General Procedure 6 *supra.*
LRMS (ES⁺) *m*/*z* Calcd for C₂₂H₁₇Cl₂NO₄S₂ + H [(M+H)⁺]: 494. Found: 494.

### Intermediate 58

### 4-Chloro-3-[3-(4-fluoro-benzyloxy)-5-methyl-phenoxymethyl]-thieno[3,2-c]pyridine-7-carboxylic acid ethyl ester

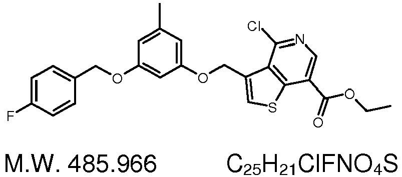

This was prepared following General Procedure 6 *supra.*
LRMS (ES⁺) *m*/*z* Calcd for C₂₅H₂₁ClFNO₄S + H [(M+H)⁺]: 486. Found: 486.

### Intermediate 59

### 4-Chloro-3-[3-(3-fluoro-benzyloxy)-5-methyl-phenoxymethyl]-thieno[3,2-c]pyridine-7-carboxylic acid ethyl ester

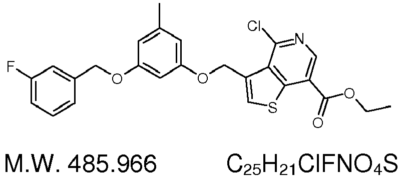

This was prepared following General Procedure 6 *supra.*
LRMS (ES⁺) *m*/*z* Calcd for C₂₅H₂₁ClFNO₄S + H [(M+H)⁺]: 486. Found: 486.

### Intermediate 60

### 4-Chloro-3-[3-(2,5-difluoro-benzyloxy)-5-methyl-phenoxymethyl]-thieno[3,2-c]pyridine-7-carboxylic acid ethyl ester

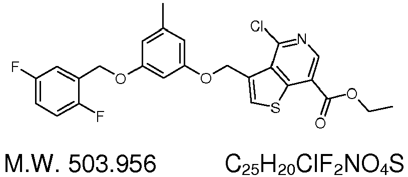

This was prepared following General Procedure 6 *supra.*
LRMS (ES⁺) *m*/*z* Calcd for C₂₅H₂₀ClF₂NO₄S + H [(M+H)⁺]: 504. Found: 504.

### Intermediate 61

### 4-Chloro-3-[3-methyl-5-(4-methyl-benzyloxy)-phenoxymethyl]-thieno[3,2-c]pyridine-7-carboxylic acid ethyl ester

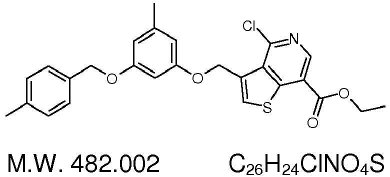

This was prepared following General Procedure 6 *supra.*
LRMS (ES⁺) *m*/*z* Calcd for C₂₆H₂₄ClNO₄S+ H [(M+H)⁺]: 482. Found: 482.

### Intermediate 62

### 4-Chloro-3-[3-methyl-5-(2-methyl-benzyloxy)-phenoxymethyl]-thieno[3,2-c]pyridine-7-carboxylic acid ethyl ester

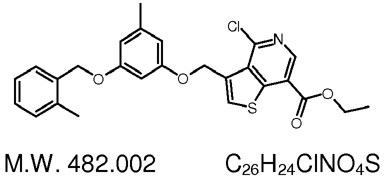

This was prepared following General Procedure 6 *supra.*
LRMS (ES⁺) *m*/*z* Calcd for C₂₆H₂₄ClNO₄S+ H [(M+H)⁺]: 482. Found: 482.

### Intermediate 63

### 4-Chloro-3-[3-(2-fluoro-benzyloxy)-5-methyl-phenoxymethyl]-thieno[3,2-c]pyridine-7-carboxylic acid ethyl ester

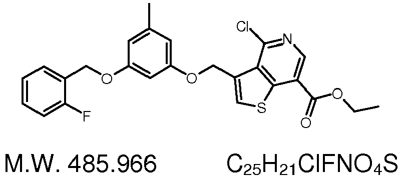

This was prepared following General Procedure 6 *supra.*
LRMS (ES⁺) *m*/*z* Calcd for C₂₅H₂₁ClFNO₄S + H [(M+H)⁺]: 486. Found: 486.

### Intermediate 64

### 4-Chloro-3-[3-(5-chloro-thiophen-2-ylmethoxy)-5-methyl-phenoxymethyl]-thieno[3,2-c]pyridine-7-carboxylic acid ethyl ester

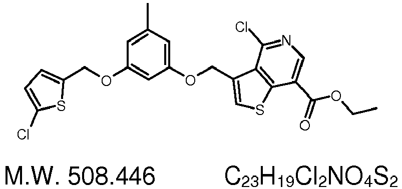

This was prepared following General Procedure 6 *supra.*
LRMS (ES⁺) *m*/*z* Calcd for C₂₃H₁₉Cl₂NO₄S₂ + H [(M+H)⁺]: 508. Found: 508.

### Example 116

### 4-Amino-3-[3-(2,3-difluoro-benzyloxy)-phenoxymethyl]-thieno[3,2-c]pyridine-7-carboxylic acid ethyl ester

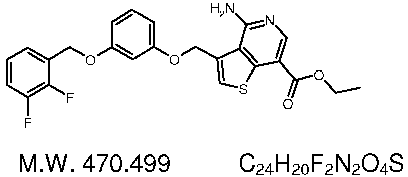

This was prepared following General Procedure 7 *supra.*
LRMS (ES⁺) *m*/*z* Calcd for C₂₄H₂₀F₂N₂O₄S + H [(M+H)⁺]: 471. Found: 471.

### Example 117

### 4-Amino-3-[3-(3-fluoro-benzyloxy)-5-methyl-phenoxymethyl]-thieno[3,2-c]pyridine-7-carboxylic acid ethyl ester

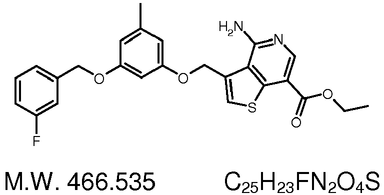

This was prepared following General Procedure 7 *supra.*
LRMS (ES⁺) *m*/*z* Calcd for C₂₅H₂₃FN₂O₄S + H [(M+H)⁺]: 467. Found: 467.

### Example 118

### 4-Amino-3-[3-(3,5-difluoro-benzyloxy)-5-methyl-phenoxymethyl]-thieno[3,2-c]pyridine-7-carboxylic acid ethyl ester

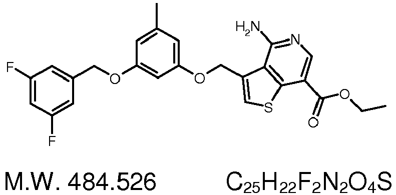

This was prepared following General Procedure 7 *supra.*
LRMS (ES⁺) *m*/*z* Calcd for C₂₅H₂₂F₂N₂O₄S + H [(M+H)⁺]: 485. Found: 485.

### Example 119

### 4-Amino-3-[3-(3,4-difluoro-benzyloxy)-5-methyl-phenoxymethyl]-thieno[3,2-c]pyridine-7-carboxylic acid ethyl ester

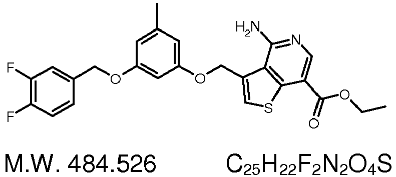

This was prepared following General Procedure 7 *supra.*
LRMS (ES⁺) *m*/*z* Calcd for C₂₅H₂₂F₂N₂O₄S + H [(M+H)⁺]: 485. Found: 485.

### Example 120

### 4-Amino-3-[3-(5-chloro-thiophen-2-ylmethoxy)-5-methyl-phenoxymethyl]-thieno[3,2-c]pyridine-7-carboxylic acid ethyl ester

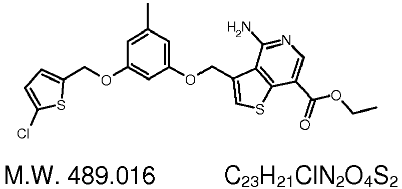

This was prepared following General Procedure 7 *supra.*
LRMS (ES⁺) *m*/*z* Calcd for C₂₃H₂₁ClN₂O₄S₂ + H [(M+H)⁺]: 489. Found: 489.

### Intermediate 65

### 4-Chloro-3-[3-(3-cyano-benzyloxy)-phenoxymethyl]-thieno[3,2-c]pyridine-7-carboxylic acid ethyl ester

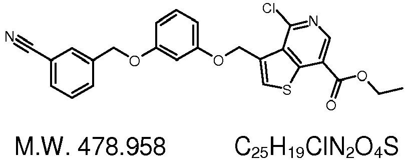

This was prepared following General Procedure 6 *supra.*
LRMS (ES⁺) *m*/*z* Calcd for C₂₅H₁₉ClN₂O₄S+ H [(M+H)⁺]: 479. Found: 479.

### Example 121

### 4-Amino-3-[3-(3-methyl-benzyloxy)-phenoxymethyl]-thieno[3,2-c]pyridine-7-carboxylic acid ethyl ester

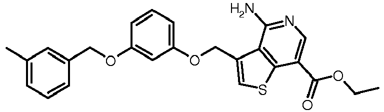

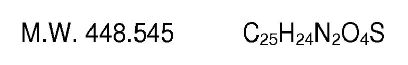

This was prepared following General Procedure 7 *supra.*
LRMS (ES⁺) *m*/*z* Calcd for C₂₅H₂₄N₂O₄S + H [(M+H)⁺]: 449. Found: 449.

### Intermediate 66

### 4-Chloro-3-[3-(3-methoxy-benzyloxy)-5-methyl-phenoxymethyl]-thieno[3,2-c]pyridine-7-carboxylic acid ethyl ester

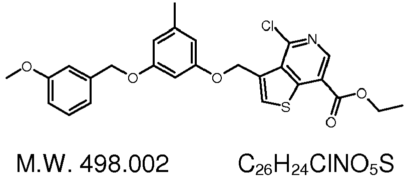

This was prepared following General Procedure 6 *supra.*
LRMS (ES⁺) *m*/*z* Calcd for C₂₆H₂₄ClNO₅S + H [(M+H)⁺]: 498. Found: 498.

### Example 122

### 4-Amino-3-[3-(5-chloro-thiophen-2-ylmethoxy)-phenoxymethyl]-thieno[3,2-c]pyridine-7-carboxylic acid ethyl ester

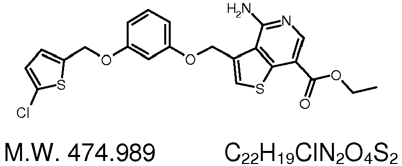

This was prepared following General Procedure 7 *supra.*
LRMS (ES⁺) *m*/*z* Calcd for C₂₂H₁₉ClN₂O₄S₂ + H [(M+H)⁺]: 475. Found: 475.

### Example 123

### 4-Amino-3-[3-(2,5-difluoro-benzyloxy)-phenoxymethyl]-thieno[3,2-c]pyridine-7-carboxylic acid ethyl ester

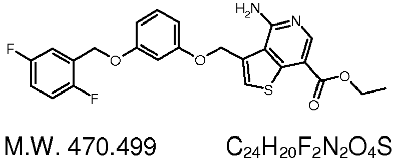

This was prepared following General Procedure 7 *supra.*
LRMS (ES⁺) *m*/*z* Calcd for C₂₄H₂₀F₂N₂O₄S + H [(M+H)⁺]: 471. Found: 471.

### Example 124

### 4-Amino-3-[3-(4-fluoro-benzyloxy)-5-methyl-phenoxymethyl]-thieno[3,2-c]pyridine-7-carboxylic acid ethyl ester

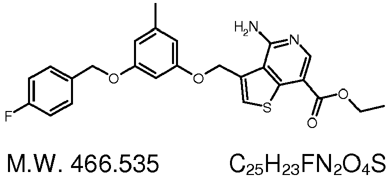

This was prepared following General Procedure 7 *supra.*
LRMS (ES⁺) *m*/*z* Calcd for C₂₅H₂₃FN₂O₄S + H [(M+H)⁺]: 467. Found: 467.

### Example 125

### 4-Amino-3-[3-(2,4-difluoro-benzyloxy)-5-methyl-phenoxymethyl]-thieno[3,2-c]pyridine-7-carboxylic acid ethyl ester

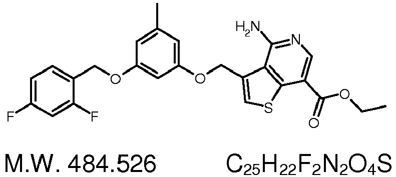

This was prepared following General Procedure 7 *supra.*
LRMS (ES⁺) *m*/*z* Calcd for C₂₅H₂₂F₂N₂O₄S + H [(M+H)⁺]: 485. Found: 485.

### Example 126

### 4-Amino-3-[3-(2,5-difluoro-benzyloxy)-5-methyl-phenoxymethyl]-thieno[3,2-c]pyridine-7-carboxylic acid ethyl ester

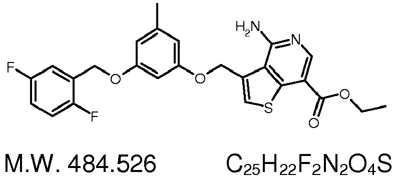

This was prepared following General Procedure 7 *supra.*
LRMS (ES⁺) *m*/*z* Calcd for C₂₅H₂₂F₂N₂O₄S + H [(M+H)⁺]: 485. Found: 485.

### Example 127

### 4-Amino-3-[3-methyl-5-(4-methyl-benzyloxy)-phenoxymethyl]-thieno[3,2-c]pyridine-7-carboxylic acid ethyl ester

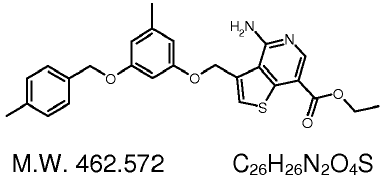

This was prepared following General Procedure 7 *supra.*
LRMS (ES⁺) *m*/*z* Calcd for C₂₆H₂₆N₂O₄S + H [(M+H)⁺]: 463. Found: 463.

### Example 128

### 4-Amino-3-[3-methyl-5-(2-methyl-benzyloxy)-phenoxymethyl]-thieno[3,2-c]pyridine-7-carboxylic acid ethyl ester

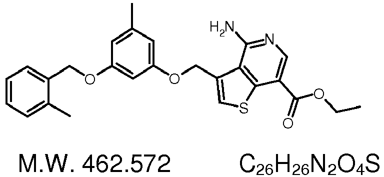

This was prepared following General Procedure 7 *supra.*
LRMS (ES⁺) *m*/*z* Calcd for C₂₆H₂₆N₂O₄S + H [(M+H)⁺]: 463. Found: 463.

### Example 129

### 4-Amino-3-[3-(2-fluoro-benzyloxy)-5-methyl-phenoxymethyl]-thieno[3,2-c]pyridine-7-carboxylic acid ethyl ester

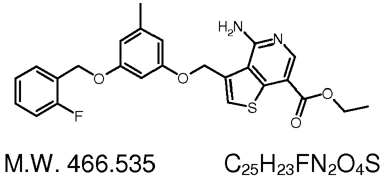

This was prepared following General Procedure 7 *supra.*
LRMS (ES⁺) *m*/*z* Calcd for C₂₅H₂₃FN₂O₄S + H [(M+H)⁺]: 467. Found: 467.

### Example 130

### 4-Amino-3-[3-(2-methoxy-benzyloxy)-phenoxymethyl]-thieno[3,2-c]pyridine-7-carboxylic acid ethyl ester

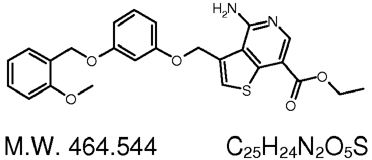

This was prepared following General Procedure 7 *supra.*
LRMS (ES⁺) *m*/*z* Calcd for C₂₅H₂₄N₂O₅S + H [(M+H)⁺]: 465. Found: 465.

### Example 131

### 4-Amino-3-{3-[3-(2-morpholin-4-yl-ethoxy)-benzoylamino]-phenoxymethyl}-thieno[3,2-c]pyridine-7-carboxylic acid (2-hydroxy-ethyl)-amide

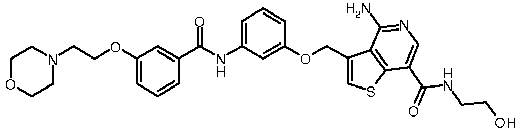

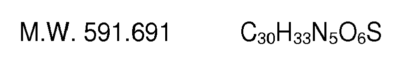

N,N-Diisopropylethylamine (15.51 g, 120 mmol) (Aldrich) was added to a suspension of 3-nitrophenol (13.91 g, 100 mmol) (Aldrich) in dichloromethane (50 mL) with cooling in an ice-water bath. Chloromethylmethyl ether (15.19 mL, 200 mmol) (Aldrich) was then added dropwise and the mixture stirred at room temperature for 3 hours. The mixture was then washed with mixture of brine (30 mL) and water (30 mL), followed by 0.1 N HCl (3 X 20 mL) and brine (2 X 20 mL). Aqueous layers were back washed with dichloromethane (30 mL). Organic solutions were combined, dried (MgSO₄), filtered and concentrated. Residue was filtered through silica gel (Biotage 40M) eluting with dichloromethnae. Fractions were combined and concentrated to give methoxymethoxy-3-nitro-benzene as pale yellow oil. (Yield 17.15 g, 93.6%).

To a solution of methoxymethoxy-3-nitro-benzene (5.06 g, 27.6 mmol) in methanol (50 mLI) was added 10% Pd/C (0.5 g) (Aldrich). The mixture was hydrogenated overnight. The mixture was passed through Celite and concentrated. The residue was purified by flash chromatography eluting with 30% ethyl acetate in hexanes to give 3-methoxymethoxy-phenylamine. (Yield 3.45 g, 15%).

To a solution of methyl-3-hydroxybenzoate (3.04 g, 20.0 mmol) in dimethylformamide (60mL) were added 4-(2-chloroethyl)morpholine (5.58 g, 30.0 mmol) (Aldrich) and potassium carbonate (8.30 g, 60.0 mmol). The mixture was heated at 80 °C for 1 day. The reaction mixture was partitioned between ethyl acetate and water. The aqueous phase was extracted with ethyl acetate (2X). The combined organic phase was washed with water and brine, dried (MgSO₄) and concentrated. The residue was purified by flash chromatography eluting with 10% methanol in dichloromethane to give 3-(2-morpholin-4-yl-ethoxy)-benzoic acid methyl ester. (Yield 5.63 g, 100%).

An aqueous solution of sodium hydroxide (1 N, 32.0 mL, 32 mmol) was added to a solution 3-(2-morpholin-4-yl-ethoxy)-benzoic acid methyl ester (5.31 g, 20.0 mmol) in methanol (20 mL) and the mixture was heated at reflux for 18 hours. The reaction mixture was concentrated and azeotroped with toluene. The solid residue was washed with diethyl ether. The solid was dissolved in water and treated with hydrochloric acid (2.5N) to pH 2. The aqueous solution was concentrated, then dissolved in dichloromethane and filtered. The solution was concentrated to give 3-(2-morpholin-4-yl-ethoxy)-benzoic acid. (Yield 1.05 g, 21 %).

To a mixture of 3-(2-morpholin-4-yl-ethoxy)-benzoic acid (1.05 g, 4.18 mmol), HBTU (1.90 g, 5.01 mmol) (Oakwood) and diisopropylethylamine (1.82 mL, 10.45 mmol) in N,N-dimethylformamide (15.0 mL) was added 3-methoxymethoxy-phenylamine (0.77 g. 5.01 mmol). The mixture was stirred at room temperature for 1 day. The reaction mixture was partitioned between ethyl acetate and water. The aqueous phase was extracted with ethyl acetate (1X). The combined organic phase was washed with water and brine, dried (MgSO₄) and concentrated. The residue was purified by flash chromatography eluting with ethyl acetate to give N-(3-methoxymethoxy-phenyl)-3-(2-morpholin-4-yl-ethoxy)-benzamide. (Yield 0.39 g, 24%).

To a solution of N-(3-methoxymethoxy-phenyl)-3-(2-morpholin-4-yl-ethoxy)-benzamide (0.39 g, 1.0 mmol) in tetrahydrofuran / 2-propanol (1:1, 10 mL) was added 4M HCl in dioxane (2 mL) (Aldrich). The reaction mixture was stirred at room temperature for 18 hours. The solution was concentrated. The residue was diluted with ethyl acetate, washed with saturated aqueous sodium bicarbonate solution and brine, dried (MgSO₄) and concentrated. The residue was purified by C18 column chromatography eluting with acetonitrile / water to give N-(3-hydroxy-phenyl)-3-(2-morpholin-4-yl-ethoxy)-benzamide. (Yield 0.20 g, 57%).

A suspension of cesium carbonate (0.20 g, 0.61 mmol), N-(3-hydroxy-phenyl)-3-(2-morpholin-4-yl-ethoxy)-benzamide (0.14 g, 0.41 mmol) in tetrahydrofuran / N,N-dimethylformamide (5:2, 17.5 mL) was heated at 70 °C for 3 hours. 3-Bromomethyl-4-chloro-thieno[3,2-c]pyridine-7-carboxylic acid ethyl ester (0.14 g, 0.41 mmol) (from Intermediate 8 *supra)* was added. Heating was continued for 2 days. The reaction mixture was partitioned between ethyl acetate and water. The aqueous phase was concentrated. The residue was purified by C18 column chromatography eluting with acetonitrile / water to give 4-chloro-3-{3-[3-(2-morpholin-4-yl-ethoxy)-benzoylamino]-phenoxymethyl}-thieno[3,2-c]pyridine-7-carboxylic acid ethyl ester. (Yield 0.13 g, 54%).

Ammonia gas was bubbled into a solution of 4-chloro-3-{3-[3-(2-morpholin-4-yl-ethoxy)-benzoylamino]-phenoxymethyl}-thieno[3,2-c]pyridine-7-carboxylic acid ethyl ester (0.13 g, 0.44 mmol) in 2-propanol (15 mL) for 20 minutes. The mixture was heated in microwave at 140 °C for 4 hours. The reaction mixture was concentrated. The residue was washed with hot methanol, filtered and dried to give 4-amino-3-{3-[3-(2-morpholin-4-yl-ethoxy)-benzoylamino]-phenoxymethyl}-thieno [3,2-c]pyridine-7-carboxylic acid ethyl ester. (Yield 0.05 g, 38%).

A solution of 4-amino-3-{3-[3-(2-morpholin-4-yl-ethoxy)-benzoylamino]-phenoxymethyl}-thieno [3,2-c]pyridine-7-carboxylic acid ethyl ester (0.05 g, 0.09 mmol) in dimethylsulfoxide (0.5 mL) was treated with ethanolamine (1.5 mL) (Aldrich)) and heated at 160 °C for 2 hours in a microwave reactor. The reaction mixture was purified by C18 column chromatography eluting with acetonitrle / water to give 4-amino-3-{3-[3-(2-morpholin-4-yl-ethoxy)-benzoylamino]-phenoxymethyl}-thieno[3,2-c]pyridine-7-carboxylic acid (2-hydroxy-ethyl)-amide as a white powder. (Yield 20.0 mg, 39%).
HRMS (ES⁺) *m*/*z* Calcd for C₃₀H₃₃N₅O₆S + H [(M+H)⁺]: 592.2225. Found: 592.2220.

### Example 132

### 4-Amino-3-(3-methylcarbamoyl-phenoxymethyl)-thieno[3,2-c]pyridine-7-carboxylic acid ethyl ester

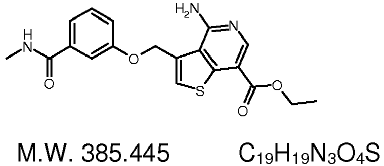

This was prepared following the procedure found in Example 1 *supra.*
HRMS (ES⁺) *m*/*z* Calcd for C₁₉H₁₉N₃O₄S + H [(M+H)⁺]: 386.1169. Found: 386.1170.

### Example 133

### 4-Amino-3-(3-cyclopropylcarbamoyl-phenoxymethyl)-thieno[3,2-c]pyridine-7-carboxylic acid ethyl ester

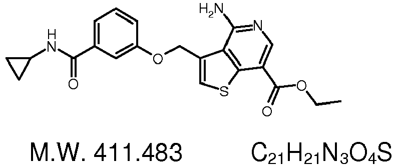

This was prepared following the procedure found in Example 1 *supra.*
HRMS (ES⁺) *m*/*z* Calcd for C₂₁H₂₁N₃O₄S + Na [(M+Na)⁺]: 434.1145. Found: 434.1147.

### Example 134

### 4-Amino-3-(3-cyclopentylcarbamoyl-phenoxymethyl)-thieno[3,2-c]pyridine-7-carboxylic acid ethyl ester

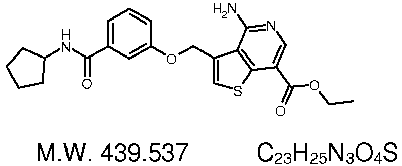

This was prepared following the procedure found in Example 1 *supra.*
HRMS (ES⁺) *m*/*z* Calcd for C₂₃H₂₅N₃O₄S + H [(M+H)⁺]: 440.1639. Found: 440.1637.

### Example 135

### 4-Amino-3-(3-cyclohexylcarbamoyl-phenoxymethyl)-thieno[3,2-c]pyridine-7-carboxylic acid (2-hydroxy-ethyl)-amide

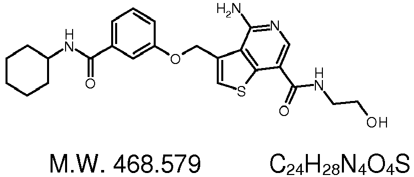

This was prepared following the procedure found in Example 3 *supra.*
HRMS (ES⁺) *m*/*z* Calcd for C₂₄H₂₈N₄O₄S + H [(M+H)⁺]: 469.1904. Found: 469.1905.

### Example 136

### 4-Amino-3-[3-(tetrahydro-pyran-4-ylcarbamoyl)-phenoxymethyl]-thieno[3,2-c]pyridine-7-carboxylic acid (2-hydroxy-ethyl)-amide

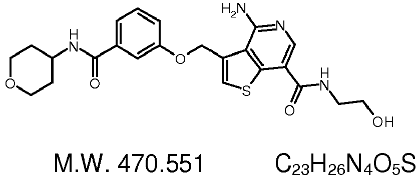

This was prepared following the procedure found in Example 3 *supra.*
HRMS (ES⁺) *m*/*z* Calcd for C₂₃H₂₆N₄O₅S + H [(M+H)⁺]: 471.1697. Found: 471.1699.

### Example 137

### 4-Amino-3-(3-methylcarbamoyl-phenoxymethyl)-thieno[3,2-c]pyridine-7-carboxylic acid (2-hydroxy-ethyl)-amide

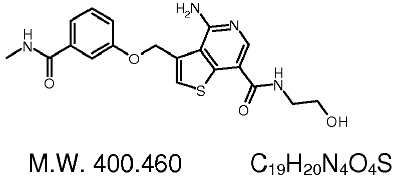

This was prepared following the procedure found in Example 3 *supra.*
HRMS (ES⁺) *m*/*z* Calcd for C₁₉H₂₀N₄O₄S + H [(M+H)⁺]: 401.1278. Found: 401.1281.

### Example 138

### 4-Amino-3-(3-cyclopropylcarbamoyl-phenoxymethyl)-thieno[3,2-c]pyridine-7-carboxylic acid (2-hydroxy-ethyl)-amide

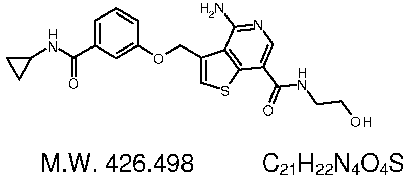

This was prepared following the procedure found in Example 3 *supra.*
HRMS (ES⁺) *m*/*z* Calcd for C₂₁H₂₂N₄O₄S + H [(M+H)⁺]: 427.1435. Found: 427.1437.
HRMS (ES⁺) *m*/*z* Calcd for C₂₁H₂₂N₄O₄S + Na [(M+Na)⁺]: 449.1254. Found: 449.1257.

### Example 139

### 4-Amino-3-(3-cyclopentylcarbamoyl-phenoxymethyl)-thieno[3,2-c]pyridine-7-carboxylic acid (2-hydroxy-ethyl)-amide

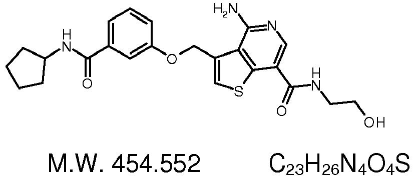

This was prepared following the procedure found in Example 3 *supra.*
HRMS (ES⁺) *m*/*z* Calcd for C₂₃H₂₆N₄O₄S + H [(M+H)⁺]: 455.1748. Found: 455.1752.

### Example 140

### 4-Amino-3-(5-methylcarbamoyl-pyridin-3-yloxymethyl)-thieno[3,2-c]pyridine-7-carboxylic acid ethyl ester

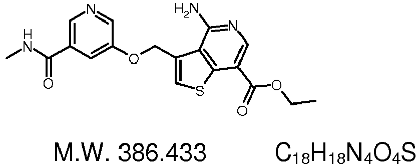

This was prepared following the procedure found in Example 1 *supra.*
HRMS (ES⁺) *m*/*z* Calcd for C₁₈H₁₈N₄O₄S + H [(M+H)⁺]: 387.1122. Found: 387.1122.

### Example 141

### 4-Amino-3-(5-methylcarbamoyl-pyridin-3-yloxymethyl)-thieno[3,2-c]pyridine-7-carboxylic acid (2-hydroxy-ethyl)-amide

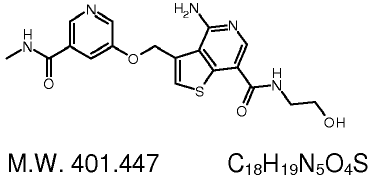

This was prepared following the procedure found in Example 3 *supra.*
HRMS (ES⁺) *m*/*z* Calcd for C₁₈H₁₉N₅O₄S + Na [(M+Na)⁺]: 424.1050. Found: 424.1052.

### Example 142

### 4-Amino-3-[3-(2-fluoro-benzyloxy)-5-methyl-phenoxymethyl]-thieno[3,2-c]pyridine-7-carboxylic acid (2-hydroxy-ethyl)-amide

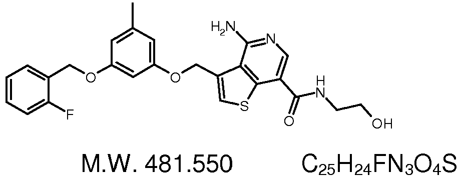

This was prepared following General Procedure 8 *supra.*
LRMS *m*/*z* Calcd for C₂₅H₂₄FN₃O₄S + H [(M+H)⁺]: 482. Found: 482.

### Example 143

### 4-Amino-3-[3-(3,5-difluoro-benzyloxy)-5-methyl-phenoxymethyl]-thieno[3,2-c]pyridine-7-carboxylic acid (2-hydroxy-ethyl)-amide

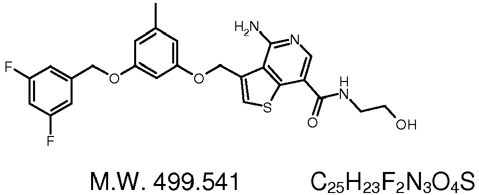

This was prepared following General Procedure 8 *supra.*
LRMS *m*/*z* Calcd for C₂₅H₂₃F₂N₃O₄S + H [(M+H)⁺]: 500. Found: 500.

### Example 144

### 4-Amino-3-[3-methyl-5-(2-methyl-benzyloxy)-phenoxymethyl]-thieno[3,2-c]pyridine-7-carboxylic acid (2-hydroxy-ethyl)-amide

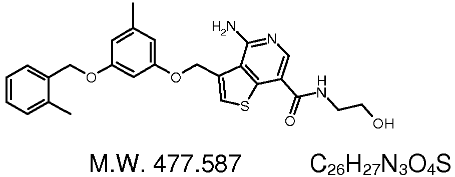

This was prepared following General Procedure 8 *supra.*
LRMS *m*/*z* Calcd for C₂₆H₂₇N₃O₄S + H [(M+H)⁺]: 478. Found: 478.

### Example 145

### 4-Amino-3-[3-(2,5-difluoro-benzyloxy)-phenoxymethyl]-thieno[3,2-c]pyridine-7-carboxylic acid (2-hydroxy-ethyl)-amide

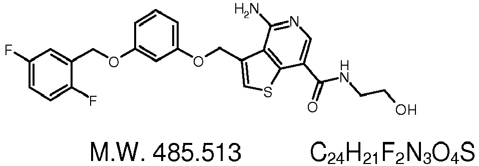

This was prepared following General Procedure 8 *supra.*
LRMS *m*/*z* Calcd for C₂₄H₂₁F₂N₃O₄S + H [(M+H)⁺]: 486. Found: 486.

### Example 146

### 4-Amino-3-[3-(3-fluoro-benzyloxy)-5-methyl-phenoxymethyl]-thieno[3,2-c]pyridine-7-carboxylic acid (2-hydroxy-ethyl)-amide

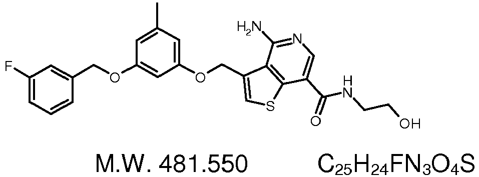

This was prepared following General Procedure 8 *supra.*
LRMS *m*/*z* Calcd for C₂₅H₂₄FN₃O₄S + H [(M+H)⁺]: 482. Found: 482.

### Example 147

### 4-Amino-3-[3-(2,5-difluoro-benzyloxy)-5-methyl-phenoxymethyl]-thieno[3,2-c]pyridine-7-carboxylic acid (2-hydroxy-ethyl)-amide

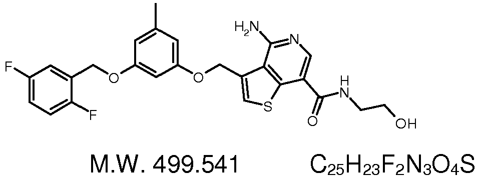

This was prepared following General Procedure 8 *supra.*
LRMS *m*/*z* Calcd for C₂₅H₂₃F₂N₃O₄S + H [(M+H)⁺]: 500. Found: 500.

### Example 148

### 4-Amino-3-[3-methyl-5-(4-methyl-benzyloxy)-phenoxymethyl]-thieno[3,2-c]pyridine-7-carboxylic acid (2-hydroxy-ethyl)-amide

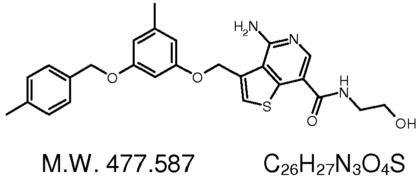

This was prepared following General Procedure 8 *supra.*
LRMS *m*/*z* Calcd for C₂₆H₂₇N₃O₄S + H [(M+H)⁺]: 478. Found: 478.

### Example 149

### 4-Amino-3-[3-(3,4-difluoro-benzyloxy)-5-methyl-phenoxymethyl]-thieno[3,2-c]pyridine-7-carboxylic acid (2-hydroxy-ethyl)-amide

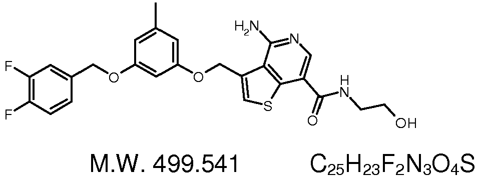

This was prepared following General Procedure 8 *supra.*
LRMS *m*/*z* Calcd for C₂₅H₂₃F₂N₃O₄S + H [(M+H)⁺]: 500. Found: 500.

### Example 150

### 4-Amino-3-[3-(2,3-difluoro-benzyloxy)-phenoxymethyl]-thieno[3,2-c]pyridine-7-carboxylic acid (2-hydroxy-ethyl)-amide

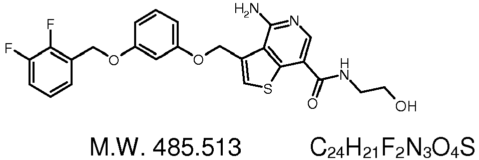

This was prepared following General Procedure 8 *supra.*
LRMS *m*/*z* Calcd for C₂₄H₂₁F₂N₃O₄S + H [(M+H)⁺]: 486. Found: 486.

### Example 151

### 4-Amino-3-[3-(4-chloro-benzoylamino)-phenoxymethyl]-thieno[3,2-c]pyridine-7-carboxylic acid

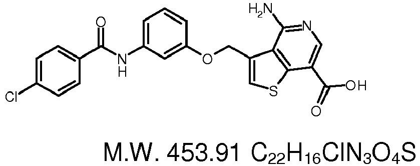

An aqueous solution of sodium hydroxide (1 N, 1.0 mL, 1.0 mmol) was added to a solution of 4-amino-3-[3-(4-chloro-benzoylamino)-phenoxymethyl]-thieno[3,2-c]pyridine-7-carboxylic acid ethyl ester (0.16 g, 0.33 mmol) (from Example 70 *supra)* in tetrahydrofuran - methanol (8 mL, 3:1) and the mixture was heated at 40 °C for 2 days. The reaction mixture was concentrated and azeotroped with toluene. The solid residue was washed with hot ethyl acetate. The solid was then suspended in water and treated with hydrochloric acid (1 N, 2 mL). After stirring 30 minutes, the solid was collected, washed with water and then diethyl ether and dried to give 4-amino-3-[3-(4-chloro-benzoylamino)-phenoxymethyl]-thieno[3,2-c]pyridine-7-carboxylic acid. (Yield 0.15 g, 100%).

### Example 152

### 4-Amino-3-[3-(pyrimidin-5-ylmethoxy)-phenoxymethyl]-thieno[3,2-c]pyridine-7-carboxylic acid ethyl ester

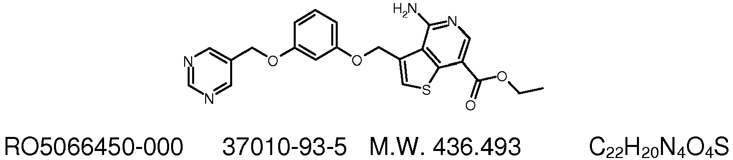

This was prepared following the procedure found in Example 1 *supra.*
HRMS (ES⁺) *m*/*z* Calcd for C₂₂H₂₀N₄O₄S + H [(M+H)⁺]: 437.1278. Found: 437.1278.

### Example 153

### 4-Amino-3-[3-(pyrimidin-5-ylmethoxy)-phenoxymethyl]-thieno[3,2-c]pyridine-7-carboxylic acid (2-hydroxy-ethyl)-amide

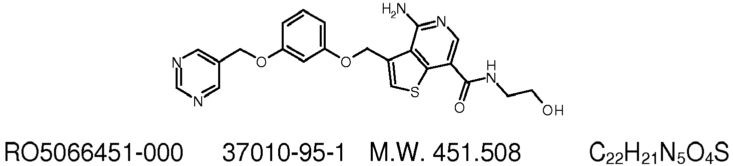

This was prepared following the procedure found in Example 3 *supra.*
HRMS (ES⁺) *m*/*z* Calcd for C₂₂H₂₁N₅O₄S + H [(M+H)⁺]: 452.1387. Found: 452.1385.

### Intermediate 67

### 4-Chloro-3-(3-nitro-phenoxymethyl)-thieno[3,2-c]pyridine-7-carboxylic acid ethyl ester

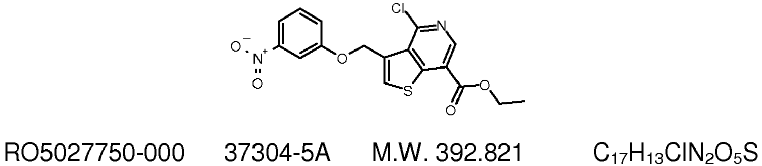

A mixture of 3-nitrophenol (457 mg, 3.29 mmol) (Aldrich) and potassium carbonate powder (498 mg, 3.60 mmol) in dry THF (10 mL) and DMF (5 mL) was stirred at 50 °C for 15 minutes before a solution of 3-bromomethyl-4-chloro-thieno[3,2-c]pyridine-7-carboxylic acid ethyl ester (1.00 g, 2.99 mmol) (from Intermediate 8 *supra)* in THF (10 mL) was added. The reaction was stirred at 50 °C for 3 hours and the resulting mixture was concentrated to remove most of the solvent. The residue was dissolved in EtOAc (200 mL), washed with water (2 x 25 mL) and brine (25 mL), dried (Na₂SO₄), filtered and concentrated. The crude product was purified by column chromatography (CH₂Cl₂ / MeOH, 98/2 to 90/10) to give 4-chloro-3-(3-nitro-phenoxymethyl)-thieno[3,2-c]pyridine-7-carboxylic acid ethyl ester as a white solid. (Yield 911 mg, 78%).
HRMS (ES⁺) *m*/*z* Calcd for C₁₇H₁₃ClN₂O₅S + H [(M+H)⁺]: 393.0307. Found: 393.0308.

### Intermediate 68

### 3-(3-Amino-phenoxymethyl)-4-chloro-thieno[3,2-c]pyridine-7-carboxylic acid ethyl ester

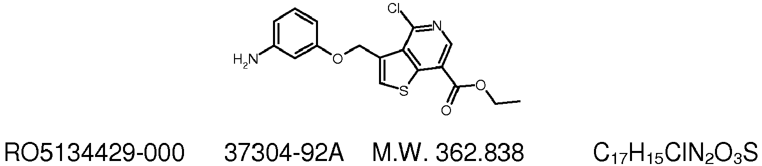

To a solution of 4-chloro-3-(3-nitro-phenoxymethyl)-thieno[3,2-c]pyridine-7-carboxylic acid ethyl ester (503.6 mg, 1.28 mmol) (from Intermediate 67 *supra)* in THF (175 mL) was added 10% Pd/C (253 mg) (Aldrich) and the mixture was stirred in an atmosphere of hydrogen (40 psi) for 4 hours. The Pd/C was filtered off and the filtrate was concentrated to give the crude product which was purified by column chromatography (hexanes / EtOAc, 80/20 to 50/50) to give 3-(3-amino-phenoxymethyl)-4-chloro-thieno[3,2-c]pyridine-7-carboxylic acid ethyl ester as a white solid. (Yield 278 mg, 60%).
HRMS (ES⁺) *m*/*z* Calcd for C₁₇H₁₅ClN₂O₃S + H [(M+H)⁺]: 363.0565. Found: 363.0565.

### Intermediate 69

### 4-Chloro-3-[3-(4-chloro-benzenesulfonylamino)-phenoxymethyl]-thieno[3,2-c]pyridine-7-carboxylic acid ethyl ester

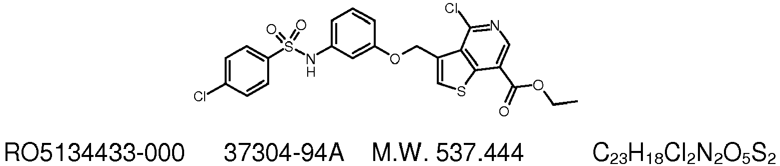

To a solution of 3-(3-amino-phenoxymethyl)-4-chloro-thieno[3,2-c]pyridine-7-carboxylic acid ethyl ester (96.9 mg, 0.267 mmol) (from Intermediate 68 *supra)* in THF (3 mL) were added diisopropylethylamine (78 mg, 0.603 mmol) and then 4-chlorobenzenesulfonyl chloride (64.7 mg, 0.297 mmol) (Aldrich). The reaction was stirred at room temperature for 30 minutes before it was concentrated to remove the solvent. The residue was diluted with EtOAc (50 mL), washed with aqueous 1 N NaOH (10 mL), brine (2 x 10 mL), dried (Na₂SO₄), filtered, and concentrated. The residue was purified by column chromatography (hexanes / EtOAc, 80/20 to 60/40) to give 4-chloro-3-[3-(4-chloro-benzenesulfonylamino)-phenoxymethyl]-thieno[3,2-c]pyridine-7-carboxylic acid ethyl ester as a white solid. (Yield 95.3 mg, 66%).
HRMS (ES⁺) *m*/*z* Calcd for C₂₃H₁₈Cl₂N₂O₅S₂ + H [(M+H)⁺]: 537.0107. Found: 537.1015.

### Example 154

### 4-Amino-3-[3-(4-chloro-benzenesulfonylamino)-phenoxymethyl]-thieno[3,2-c]pyridine-7-carboxylic acid ethyl ester

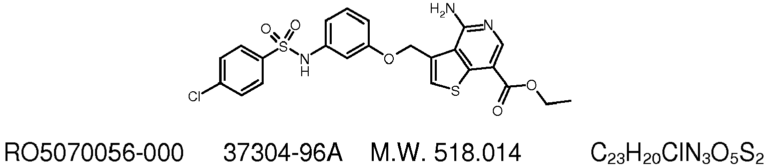

Ammonia gas was bubbled into a solution of 4-chloro-3-[3-(4-chlorobenzenesulfonylamino)-phenoxymethyl]-thieno[3,2-c]pyridine-7-carboxylic acid ethyl ester (89.6 mg, 0.167 mmol) (from Intermediate 69 *supra)* in 2-propanol (15 mL) for 20 minutes. The mixture was heated in a sealed vessel at 130 °C for 1.5 hours in a microwave reactor. The reaction mixture was concentrated and the residue was purifed by column chromatography (CH₂Cl₂ / MeOH, 98/2) to give 4-amino-3-[3-(4-chloro-benzenesulfonylamino)-phenoxymethyl]-thieno[3,2-c]pyridine-7-carboxylic acid ethyl ester as a white solid. (Yield 76.5 mg, 88.6%).
HRMS (ES⁺) *m*/*z* Calcd for C₂₃H₂₀ClN₃O₅S₂ + H [(M+H)⁺]: 518.0606. Found: 518.0602.

### Example 155

### 4-Amino-3-[3-(4-chloro-benzenesulfonylamino)-phenoxymethyl]-thieno[3,2-c]pyridine-7-carboxylic acid (2-hydroxy-ethyl)-amide

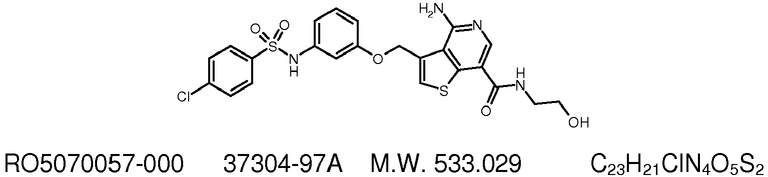

A suspension of 4-amino-3-[3-(4-chloro-benzenesulfonylamino)-phenoxymethyl]-thieno[3,2-c]pyridine-7-carboxylic acid ethyl ester (54.4 mg, 0.105 mmol) (from Example 154 *supra)* in dimethylsulfoxide (1 mL) and ethanolamine (3 mL) (Aldrich) was heated at 135 °C for 2 hours in a microwave reactor. After cooling to room temperature, the precipitate was filtered off, washed with MeOH and dried to give 3-[3-(4-chloro-benzoylamino)-phenoxymethyl]-thieno[3,2-c]pyridine-7-carboxylic acid (2-hydroxy-ethyl)amide as a white solid. (Yield 41.7 mg, 74.5%).
HRMS (ES⁺) *m*/*z* Calcd for C₂₃H₂₁ClN₄O₅S₂ + H [(M+H)⁺]: 533.0715. Found: 533.0710.

### Intermediate 70

### 2-Chloro-5-(4-chloro-benzyloxy)-phenol

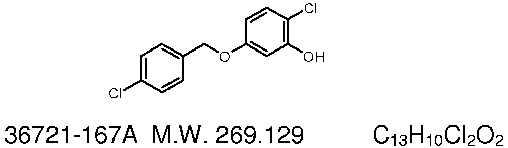

A mixture of 4-chlororesorcinol (2.89 g, 20 mmol) (Aldrich), potassium fluoride (2.32 g, 40 mmol) (Aldrich) and 4-chlorobenzyl chloride (4.83 g, 30 mmol) (Aldrich) in acetonitrile was heated at 90 °C for 24 hours. After cooling to room temperature, mixture was partitioned between ethyl acetate (2 X 100 mL) and water (2 X 100 mL). Organic layers were washed with brine (100 mL), combined, dried (MgSO₄), filtered and concentrated. Residue was purified by flash chromatography (Biotage 40 L, ethyl acetate - hexanes as solvent). No good purification was achieved. Fractions were combined and concentrated. Residue was re-purified by flash chromatography (Biotage 40L, dichloromethane - hexanes 1:1 as solvent) to give product in two crops as white crystals. (Yield 1.93 g, 35.9%).

### Intermediate 71

### 4-Chloro-3-[2-chloro-5-(4-chloro-benzyloxy)-phenoxymethyl]-thieno[3,2-c]pyridine-7-carboxylic acid ethyl ester

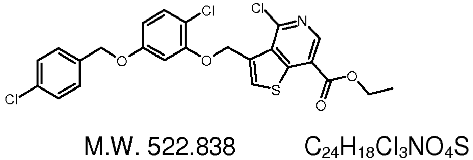

A suspension of cesium carbonate (0.50 g, 1.53 mmol), 2-chloro-5-(4-chlorobenzyloxy)-phenol (0.30 g, 1.12 mmol) (from Intermediate 70 *supra)* in tetrahydrofuran / N,N-dimethylformamide (5:2, 14 mL) was heated at 70 °C for 2 hours. 3-Bromomethyl-4-chloro-thieno[3,2-c]pyridine-7-carboxylic acid ethyl ester (0.34 g, 1.02 mmol) (from Intermediate 8 *supra)* was added. Heating was continued for 1 day. The reaction mixture was partitioned between ethyl acetate and water. The aqueous phase was extracted with ethyl acetate (2X). The combined organic phase was washed with water and brine, dried (magnesium sulfate) and concentrated. The residue was purified by flash chromatography eluting with 20% ethyl acetate in hexanes to give 4-chloro-3-[2-chloro-5-(4-chloro-benzyloxy)-phenoxymethyl]-thieno[3,2-c]pyridine-7-carboxylic acid ethyl ester. (Yield 0.12 g, 23%).

### Example 156

### 4-Amino-3-[2-chloro-5-(4-chloro-benzyloxy)-phenoxymethyl]-thieno[3,2-c]pyridine-7-carboxylic acid ethyl ester

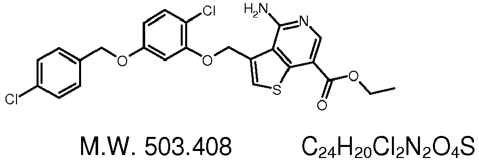

Ammonia gas was bubbled into a solution of 4-chloro-3-[2-chloro-5-(4-chlorobenzyloxy)-phenoxymethyl]-thieno[3,2-c]pyridine-7-carboxylic acid ethyl ester (0.12 g, 0.23 mmol) (from Intermediate 71 *supra)* in 2-propanol (15 mL) for 20 minutes. The mixture was heated in a microwave reactor at 140 °C for 2 hours. After concentrating, the residue was purified by flash chromatography eluting with 2-5% ethyl acetate in dichloromethane to give 4-amino-3-[2-chloro-5-(4-chloro-benzyloxy)-phenoxymethyl]-thieno[3,2-c]pyridine-7-carboxylic acid ethyl ester as a white powder. (Yield 0.10 g, 83%).

### Example 157

### 4-Amino-3-[2-chloro-5-(4-chloro-benzyloxy)-phenoxymethyl]-thieno[3,2-c]pyridine-7-carboxylic acid (2-hydroxy-ethyl)-amide

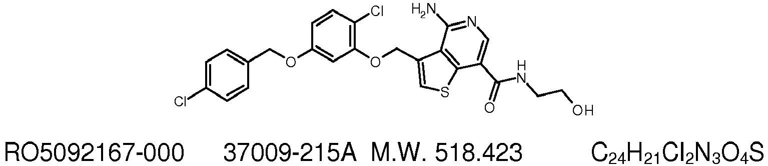

A solution of 4-amino-3-[2-chloro-5-(4-chloro-benzyloxy)-phenoxymethyl]-thieno[3,2-c]pyridine-7-carboxylic acid ethyl ester (0.04 g, 0.08 mmol) (from Example 156 *supra)* in dimethyl-sulfoxide (0.5 mL) was treated with ethanolamine (1.5 mL) and heated at 160 °C for 2 hours in a microwave reactor. The precipitate was filtered, washed with methanol and dried to give 4-amino-3-[2-chloro-5-(4-chloro-benzyloxy)-phenoxymethyl]-thieno[3,2-c]pyridine-7-carboxylic acid (2-hydroxy-ethyl)-amide as a white powder. (Yield 20 mg, 49%).
HRMS (ES⁺) *m*/*z* Calcd for C₂₄H₂₁Cl₂N₃O₄S + H [(M+H)⁺]: 518.0703. Found: 518.0706.

### Intermediate 72

### 4-Chloro-3-{3-[(pyridine-2-carbonyl)-amino]-phenoxymethyl}-thieno[3,2-c]pyridine-7-carboxylic acid ethyl ester

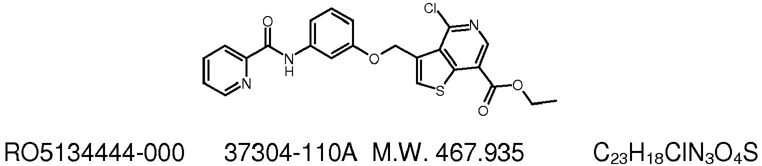

4-Chloro-3-{3-[(pyridine-2-carbonyl)-amino]-phenoxymethyl}-thieno[3,2-c]pyridine-7-carboxylic acid ethyl ester was prepared from 3-(3-amino-phenoxymethyl)-4-chlorothieno[3,2-c]pyridine-7-carboxylic acid ethyl ester (from Intermediate 68 *supra)* and picolinoyl chloride hydrochloride (TCI-US) following a similar procedure as described in Intermediate 69 *supra* as off-white solid.
HRMS (ES⁺) *m*/*z* Calcd for C₂₃H₁₈ClN₃O₄S + H [(M+H)⁺]: 468.0780. Found: 468.0779.

### Example 158

### 4-Amino-3-{3-[(pyridine-2-carbonyl)-amino]-phenoxymethyl}-thieno[3,2-c]pyridine-7-carboxylic acid ethyl ester

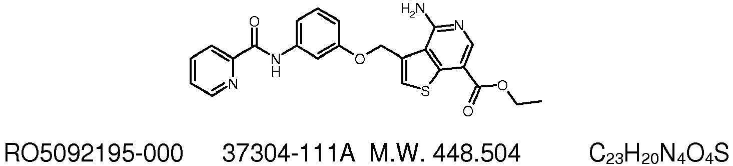

4-Amino-3-{3-[(pyridine-2-carbonyl)-amino]-phenoxymethyl}-thieno[3,2-c]pyridine-7-carboxylic acid ethyl ester was prepared from 4-chloro-3-{3-[(pyridine-2-carbonyl)-amino]-phenoxymethyl}-thieno[3,2-c]pyridine-7-carboxylic acid ethyl ester (from Intermediate 72 *supra)* following a similar procedure as described in Example 154 *supra* as off-white solid.
HRMS (ES⁺) *m*/*z* Calcd for C₂₃H₂₀N₄O₄S + H [(M+H)⁺]: 449.1278. Found: 449.1279.

### Example 159

### 4-Amino-3-{3-[(pyridine-2-carbonyl)-amino]-phenoxymethyl}-thieno[3,2-c]pyridine-7-carboxylic acid (2-hydroxy-ethyl)-amide

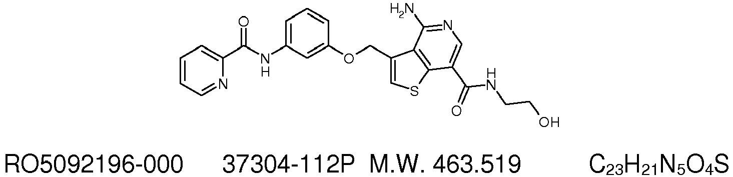

4-Amino-3-{3-[(pyridine-2-carbonyl)-amino]-phenoxymethyl}-thieno[3,2-c]pyridine-7-carboxylic acid (2-hydroxy-ethyl)-amide was prepared from 4-amino-3-{3-[(pyridine-2-carbonyl)-amino]-phenoxymethyl}-thieno[3,2-c]pyridine-7-carboxylic acid ethyl ester (from Example 158 *supra)* following a similar procedure as described in Example 155 *supra* as off-white solid.
HRMS (ES⁺) *m*/*z* Calcd for C₂₃H₂₁N₅O₄S + H [(M+H)⁺]: 464.1387. Found: 464.1388.

### Intermediate 73

### 4-Chloro-3-{3-[(6-methyl-pyridine-3-carbonyl)-amino]-phenoxymethyl}-thieno[3,2c]pyridine-7-carboxylic acid ethyl ester

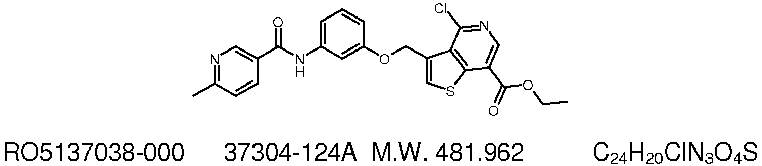

4-Chloro-3-{3-[(6-methyl-pyridine-3-carbonyl)-amino]-phenoxymethyl}-thieno[3,2c]pyridine-7-carboxylic acid ethyl ester was prepared from 3-(3-amino-phenoxymethyl)-4-chloro-thieno[3,2-c]pyridine-7-carboxylic acid ethyl ester (Intermediate 68 *supra)* and 6-methyl-nicotinoyl chloride which was made from 6-methyl-nicotinic acid (from Aldrich) and oxalyl chloride (Aldrich), following a similar procedure as described in Intermediate 69 *supra* as off-white solid.

### Example 160

### 4-Amino-3-{3-[(6-methyl-pyridine-3-carbonyl)-amino]-phenoxymethyl}-thieno[3,2-c]pyridine-7-carboxylic acid ethyl ester

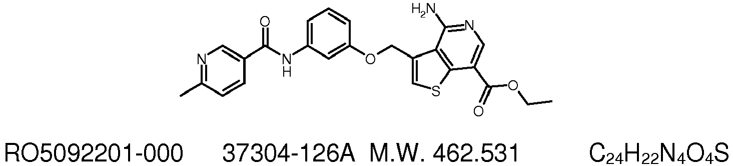

4-Amino-3-{3-[(6-methyl-pyridine-3-carbonyl)-amino]-phenoxymethyl}-thieno[3,2-c]pyridine-7-carboxylic acid ethyl ester was prepared from 4-chloro-3-{3-[(6-methylpyridine-3-carbonyl)-amino]-phenoxymethyl}-thieno[3,2c]pyridine-7-carboxylic acid ethyl ester (from Intermediate 73 *supra)* following a similar procedure as described in Example 154 *supra* as off-white solid.
HRMS (ES⁺) *m*/*z* Calcd for C₂₄H₂₂N₄O₄S + H [(M+H)⁺]: 463.1435. Found: 463.1430.

### Example 161

### 4-Amino-3-{3-[(6-methyl-pyridine-3-carbonyl)-amino]-phenoxymethyl}-thieno[3,2c]pyridine-7-carboxylic acid (2-hydroxy-ethyl)-amide

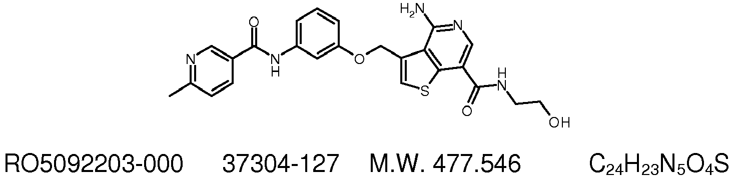

4-Amino-3-{3-[(6-methyl-pyridine-3-carbonyl)-amino]-phenoxymethyl}-thieno[3,2c]pyridine-7-carboxylic acid (2-hydroxy-ethyl)-amide was prepared from 4-amino-3-{3-[(6-methyl-pyridine-3-carbonyl)-amino]-phenoxymethyl}-thieno[3,2-c]pyridine-7-carboxylic acid ethyl ester (from Example 160 *supra)* following a similar procedure as described in Example 155 *supra* as off-white solid.
HRMS (ES⁺) *m*/*z* Calcd for C₂₄H₂₃N₅O₄S + H [(M+H)⁺]: 478.1544. Found: 478.1538.

### Intermediate 74

### 4-Chloro-3-{3-[(pyridine-4-carbonyl)-amino]-phenoxymethyl}-thieno[3,2-c]pyridine-7-carboxylic acid ethyl ester

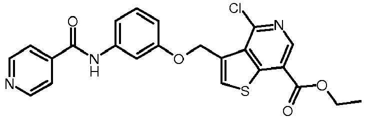

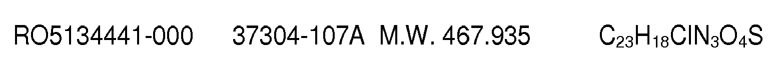

4-Chloro-3-{3-[(pyridine-4-carbonyl)-amino]-phenoxymethyl}-thieno[3,2-c]pyridine-7-carboxylic acid ethyl ester was prepared from 3-(3-amino-phenoxymethyl)-4-chlorothieno[3,2-c]pyridine-7-carboxylic acid ethyl ester (Intermediate 68 *supra)* following a similar procedure as described in Intermediate 69 *supra* as off-white solid.
HRMS (ES⁺) *m*/*z* Calcd for C₂₃H₁₈ClN₃O₄S + H [(M+H)⁺]: 468.0780. Found: 468.0777.

### Example 162

### 4-Amino-3-{3-[(pyridine-4-carbonyl)-amino]-phenoxymethyl}-thieno[3,2-c]pyridine-7-carboxylic acid ethyl ester

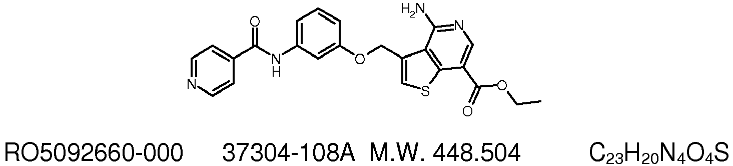

4-Amino-3-{3-[(pyridine-4-carbonyl)-amino]-phenoxymethyl}-thieno[3,2-c]pyridine-7-carboxylic acid ethyl ester was prepared from 4-chloro-3-{3-[(pyridine-4-carbonyl)-amino]-phenoxymethyl}-thieno[3,2-c]pyridine-7-carboxylic acid ethyl ester (from Intermediate 74 *supra)* following a similar procedure as described in Example 154 *supra* as off-white solid.
HRMS (ES⁺) *m*/*z* Calcd for C₂₃H₂₀N₄O₄S + H [(M+H)⁺]: 449.1278. Found: 449.1276.

### Example 163

### 4-Amino-3-{3-[(pyridine-4-carbonyl)-amino]-phenoxymethyl}-thieno[3,2-c]pyridine-7-carboxylic acid (2-hydroxy-ethyl)-amide

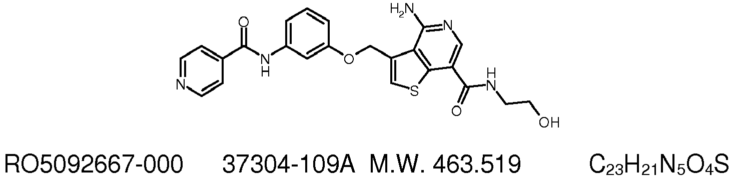

4-Amino-3-{3-[(pyridine-4-carbonyl)-amino]-phenoxymethyl}-thieno[3,2-c]pyridine-7-carboxylic acid (2-hydroxy-ethyl)-amide was prepared from 4-amino-3-{3-[(pyridine-4-carbonyl)-amino]-phenoxymethyl}-thieno[3,2-c]pyridine-7-carboxylic acid ethyl ester (from Example 162 *supra)* following a similar procedure as described in Example 155 *supra* as off-white solid.
HRMS (ES⁺) *m*/*z* Calcd for C₂₃H₂₁N₅O₄S + H [(M+H)⁺]: 464.1387. Found: 464.1388.

### Intermediate 75

### 3-Chloro-4-fluoro-N-(3-hydroxy-4-methyl-phenyl)-benzamide

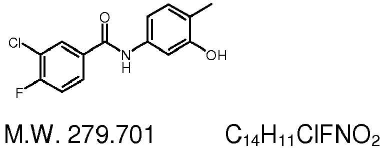

A solution of 3-chloro-4-fluorobenzoyl chloride (21.23 g, 110 mmol) (Avocado) in tetrahydrofuran (50 mL) was added to a solution of 5-amino-o-cresol (6.16 g, 50 mmol) (Aldrich) and triethylamine (17.5 mL, 125 mmol) (Aldrich) and THF (50 mL) dropwise with magnetic stirring and cooling in an ice - water bath. When addition was complete, mixture was allowed to warm to room temperature and stirred for 16 hours. Reaction mixtrue was diluted with water (125 mL) and saturated aqueous sodium bicarbonate solution (125 mL). After stirring for another 30 minutes, precipitate was collected to give crude 3-Chloro-4-fluoro-benzoic acid 5-(3-chloro-4-fluoro-benzoylamino)-2-methyl-phenyl ester as an off-white powder. (Yield 21.83 g, 101%).

3-Chloro-4-fluoro-benzoic acid 5-(3-chloro-4-fluoro-benzoylamino)-2-methyl-phenyl ester (3.93 g, 9 mmol) was dissolved in a mixture of tetrahydrofuran (25 mL), methanol (50 mL) and aqueous 1 N sodium hydroxide (9 mL, 9 mmol). Mixture was stirred at room temperature for 18 hours. Mixture was concentrated under reduced pressure to remove most of the organic solvent. Resulting suspension was diluted with water (45 mL) and saturated aqueous sodium bicarbonate solution (5 mL). After standing for 30 minutes, precipitate was collected and washer with water and dried to give crude 3-chloro-4-fluoro-N-(3-hydroxy-4-methyl-phenyl)-benzamide as a white powder. (Yield 2.59 g, 103%).

### Intermediate 76

### 4-Chloro-3-[5-(3-chloro-4-fluoro-benzoylamino)-2-methyl-phenoxymethyl]-thieno[3,2-c]pyridine-7-carboxylic acid ethyl ester

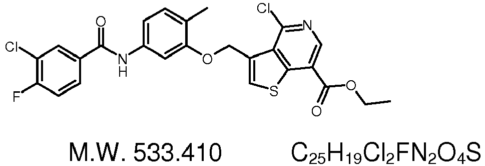

A suspension of cesium carbonate (0.73 g, 2.24 mmol), 3-chloro-4-fluoro-N-(3-hydroxy-4-methyl-phenyl)-benzamide (0.46 g, 1.64 mmol) (from Intermediate 75 *supra)* in tetrahydrofuran / N,N-dimethylformamide (5:2, 21 mL) was heated at 70 °C for 3 hours. 3-Bromomethyl-4-chloro-thieno[3,2-c]pyridine-7-carboxylic acid ethyl ester (0.50 g, 1.49 mmol) (from Intermediate 8 *supra)* was added. Heating was continued for 18 hours. The reaction mixture was partitioned between ethyl acetate and water. The aqueous phase was extracted with ethyl acetate (1X). The combined organic phase was washed with water and brine, dried (magnesium sulfate) and concentrated. The residue was washed with hot dichloromethane / ethyl acetate and dried to give 4-chloro-3-[5-(3-chloro-4-fluoro-benzoylamino)-2-methylphenoxymethyl]-thieno[3,2-c]pyridine-7-carboxylic acid ethyl ester. (Yield 0.14 g, 18%).

### Example 164

### 4-Amino-3-[5-(3-chloro-4-fluoro-benzoylamino)-2-methyl-phenoxymethyl]-thieno[3,2-c]pyridine-7-carboxylic acid ethyl ester

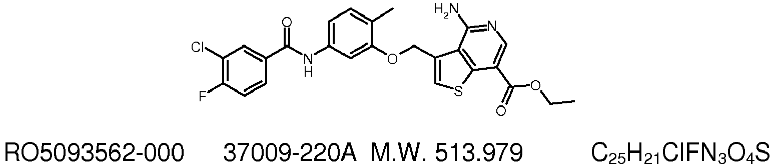

Ammonia gas was bubbled into a solution of 4-chloro-3-[5-(3-chloro-4-fluorobenzoylamino)-2-methyl-phenoxymethyl]-thieno[3,2-c]pyridine-7-carboxylic acid ethyl ester (0.14 g, 0.26 mmol) (from Intermediate 76 *supra)* in 2-propanol (15 mL) for 20 minutes. The mixture was heated in a microwave reactor at 140 °C for 2 hours. The reaction mixture was concentrated. The residue was washed with hot methanol, filtered and dried to give 4-amino-3-[5-(3-chloro-4-fluoro-benzoylamino)-2-methylphenoxymethyl]-thieno[3,2-c]pyridine-7-carboxylic acid ethyl ester as white powder. (Yield 0.09 g, 69%).
HRMS (ES⁺) *m*/*z* Calcd for C₂₅H₂₁ClFN₃O₄S + H [(M+H)⁺]: 514.0998. Found: 514.0995.

### Intermediate 77

### 3-Chloro-N-(3-hydroxy-4-methyl-phenyl)-benzamide

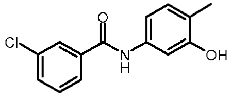

A solution of 3-chlorobenzoyl chloride (32.81 g, 187.5 mmol) (Aldrich) in tetrahydrofuran (50 mL) was added to a solution of 5-amino-o-cresol (9.24 g, 75 mmol) (Aldrich) and triethylamine (31.43 mL, 225 mmol) (Aldrich) and THF (150 mL) dropwise with magnetic stirring and cooling in an ice - water bath. When addition was complete, mixture was allowed to warm to room temperature and stirred for 16 hours. Reaction mixtrue was diluted with water (200 mL) and saturated aqueous sodium bicarbonate solution (200 mL). After stirring for another 30 minutes, precipitate was collected to give crude 3-chloro-benzoic acid 5-(3-chlorobenzoylamino)-2-methyl-phenyl ester as an off-white powder. (Yield 31.74 g, 106%). Crude 3-chloro-benzoic acid 5-(3-chloro-benzoylamino)-2-methyl-phenyl ester (11.42 g, 28.5 mmol) was dissolved in a mixture of tetrahydrofuran (70 mL), methanol (140 mL) and aqueous 1 N sodium hydroxide (28.5 mL, 28.5 mmol). Mixture was stirred at room temperature for 18 hours. Mixture was concentrated under reduced pressure to remove most of the organic solvent. Resulting suspension was diluted with water (90 mL) and saturated aqueous sodium bicarbonate solution (10 mL). After standing for 30 minutes, precipitate was collected and washer with water and dried to give 3-chloro-N-(3-hydroxy-4-methyl-phenyl)-benzamide as an off white powder. (Yield 6.06 g, 81.2%).

### Intermediate 78

### 4-Chloro-3-[5-(3-chloro-benzoylamino)-2-methyl-phenoxymethyl]-thieno[3,2-c]pyridine-7-carboxylic acid ethyl ester

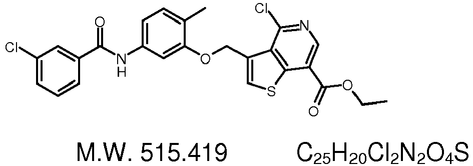

A suspension of cesium carbonate (0.73 g, 2.24 mmol), 3-chloro-N-(3-hydroxy-4-methyl-phenyl)-benzamide (0.43 g, 1.64 mmol) (from Intermediate 77 *supra)* in tetrahydrofuran / N,N-dimethylformamide (5:2, 21 mL) was heated at 70 °C for 3 hours. 3-Bromomethyl-4-chloro-thieno[3,2-c]pyridine-7-carboxylic acid ethyl ester (0.50 g, 1.49 mmol) (from Intermediate 8 *supra)* was added. Heating was continued for 18 hours. The reaction mixture was partitioned between ethyl acetate and water. The aqueous phase was extracted with ethyl acetate (1X). The combined organic phase was washed with water and brine, dried (magnesium sulfate) and concentrated. The residue was washed with hot dichloromethane / ethyl acetate and dried to give 4-chloro-3-[5-(3-chloro-benzoylamino)-2-methyl-phenoxymethyl]-thieno[3,2-c]pyridine-7-carboxylic acid ethyl ester. (Yield 0.35 g, 45%).

### Example 165

### 4-Amino-3-[5-(3-chloro-4-benzoylamino)-2-methyl-phenoxymethyl]-thieno[3,2-c]pyridine-7-carboxylic acid ethyl ester

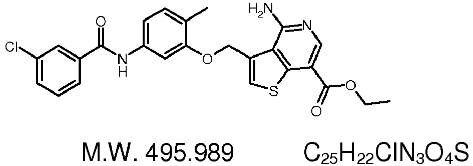

Ammonia gas was bubbled into a solution of 4-chloro-3-[5-(3-chloro-4-benzoylamino)-2-methyl-phenoxymethyl]-thieno[3,2-c]pyridine-7-carboxylic acid ethyl ester (0.10 g, 0.19 mmol) (from Intermediate 78 *supra)* in 2-propanol (15 mL) for 20 minutes. The mixture was heated in a microwave reactor at 140 °C for 2 hours. The reaction mixture was concentrated. The residue was washed with hot methanol, filtered and dried to give 4-amino-3-[5-(3-chloro-benzoylamino)-2-methylphenoxymethyl]-thieno[3,2-c]pyridine-7-carboxylic acid ethyl ester as white powder. (Yield 0.04 g, 43%).

### Example 166

### 4-Amino-3-[5-(3-chloro-benzoylamino)-2-methyl-phenoxymethyl]-thieno[3,2-c]pyridine-7-carboxylic acid (2-hydroxy-ethyl)-amide

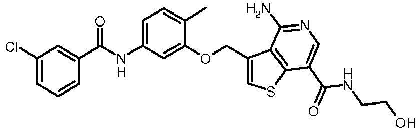

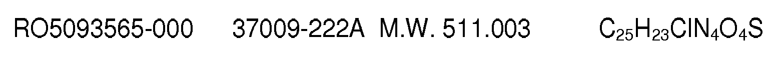

A solution of 4-amino-3-[5-(3-chloro-benzoylamino)-2-methyl-phenoxymethyl]-thieno[3,2-c]pyridine-7-carboxylic acid ethyl ester (0.04 g, 0.08 mmol) (from Example 165 *supra)* in dimethylsulfoxide (0.5 mL) was treated with ethanolamine (1.5 mL) (Aldrich) and heated at 160 °C for 2 hours in a microwave reactor. The reaction mixture was purified by C18 column chromatography eluting with acetonitrile / water to give 4-amino-3-[5-(3-chloro-benzoylamino)-2-methyl-phenoxymethyl]-thieno[3,2-c]pyridine-7-carboxylic acid (2-hydroxy-ethyl)-amide as white powder. (Yield 8.0 mg, 20%).
HRMS (ES⁺) *m*/*z* Calcd for C₂₅H₂₃ClN₄O₄S + H [(M+H)⁺]: 511.1202. Found: 511.1198.

### Example 167

### 4-Amino-3-{5-[3-chloro-4-(2-hydroxy-ethylamino)-benzoylamino)-2-methylphenoxymethyl]-thieno[3,2-c]pyridine-7-carboxylic acid (2-hydroxy-ethyl)-amide

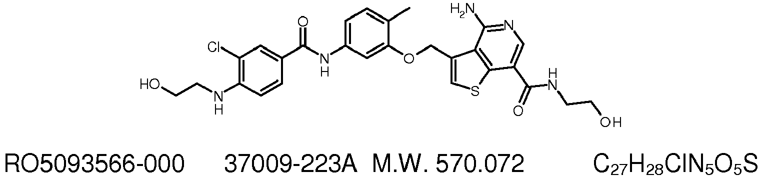

A solution of 4-amino-3-[5-(3-chloro-4-fluoro-benzoylamino)-2-methylphenoxymethyl]-thieno[3,2-c]pyridine-7-carboxylic acid ethyl ester (0.05 g, 0.10 mmol) (from Example 164 *supra)* in dimethylsulfoxide (0.5 mL) was treated with ethanolamine (1.5 mL) (Aldrich) and heated at 160 °C for 2 hours in a microwave reactor. The reaction mixture was purified by C18 column chromatography eluting with acetonitrile / water to give 4-amino-3-{5-[3-chloro-4-(2-hydroxy-ethylamino)-benzoylamino)-2-methyl-phenoxymethyl]-thieno[3,2-c]pyridine-7-carboxylic acid (2-hydroxy-ethyl)-amide as white powder. (Yield 37.0 mg, 67%).
HRMS (ES⁺) *m*/*z* Calcd for C₂₇H₂₈ClN₅O₅S+ H [(M+H)⁺]: 570.1573. Found: 570.1566.

### Example 167a

### 4-Amino-3-{5-[3-chloro-4-(2-hydroxy-ethylamino)-benzoylamino)-2-methylphenoxymethyl]-thieno[3,2-c]pyridine-7-carboxylic acid (2-hydroxy-ethyl)-amide toluene-4-sulfonic acid salt

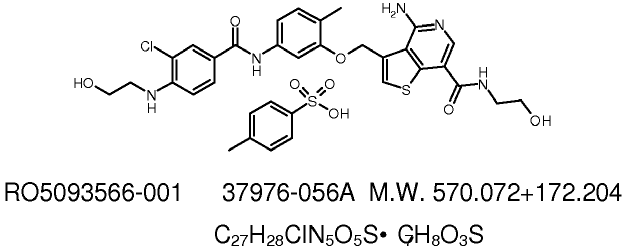

To solution of 4-amino-3-{5-[3-chloro-4-(2-hydroxy-ethylamino)-benzoylamino)-2-methyl-phenoxymethyl]-thieno[3,2-c]pyridine-7-carboxylic acid (2-hydroxy-ethyl)-amide (51.6 mg, 0.09 mmol) (from Example 167 *supra)* in methanol (6 mL) was treated with toluene-4-sulfonic acid hydrate (18.9 mg, 0.10 mmol) (Aldrich) and heated at 40 °C for 30 minutes. The solution was concentrated. The residue was washed with diethyl ether, dissolved in water and lyophilized to give 4-amino-3-{5-[3-chloro-4-(2-hydroxy-ethylamino)-benzoylamino)-2-methyl-phenoxy-methyl]-thieno[3,2-c]pyridine-7-carboxylic acid (2-hydroxy-ethyl)-amide, toluene-4-sulfonic acid salt. (Yield 56.0 mg, 86%).
HRMS (ES⁺) *m*/*z* Calcd for C₂₇H₂₈ClN₅O₅S + H [(M+H)⁺]: 570.1573. Found: 570.1567.

### Intermediate 79

### 3-Chloro-4-(2-hydroxy-ethylamino)-N-(3-hydroxy-4-methyl-phenyl)-benzamide

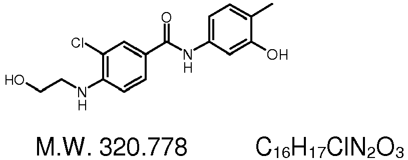

A solution of 3-chloro-4-fluoro-N-(3-hydroxy-4-methyl-phenyl)-benzamide (1.0 g, 3.58 mmol) (from Intermediate 75 *supra)* in dimethylsulfoxide (5.0 mL) was treated with ethanolamine (10.0 mL) (Aldrich) and heated at 140 °C for 50 minutes in a microwave reactor. The reaction mixture was partitioned between ethyl acetate and water. The aqueous phase was acidified with 2N hydrochloric acid. The precipitate was filtered, washed with water and dried. The aqueous phase was extracted with ethyl acetate (2X). The combined organic phase was washed with water and brine, dried (magnesium sulfate) and concentrated. The two solid samples were combined and dried to give 3-chloro-4-(2-hydroxy-ethylamino)-N-(3-hydroxy-4-methyl-phenyl)-benzamide. (Yield 1.13 g, 98%).

### Intermediate 80

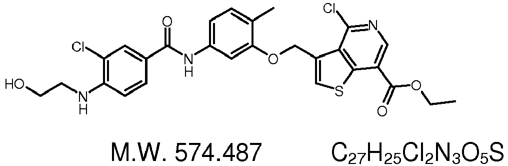

A suspension of potassium carbonate (0.56 g, 4.05 mmol), 3-chloro-4-(2-hydroxy-ethylamino)-N-(3-hydroxy-4-methyl-phenyl)-benzamide (1.13 g, 4.30 mmol) (from Intermediate 79 *supra)* in N,N-dimethylformamide (15 mL) was stirred at room tempreature for 10 minutes. 3-Bromomethyl-4-chloro-thieno[3,2-c]pyridine-7-carboxylic acid ethyl ester (1.44 g, 4.30 mmol) (from Intermediate 8 *supra)* was added. Stirring was continued for 18 hours. The reaction mixture was partitioned between dichloromethane and water. The precipitate was filtered, washed with water and dried. The aqueous phase was extracted with dichloromethane (2X). The combined organic phase was washed with water and brine, dried (magnesium sulfate) and concentrated. The residue was purified by flash chromatography eluting with 40 - 100% ethyl acetate in hexanes to give the second part of product. The combined solid was dried to give 4-chloro-3-{5-[3-chloro-4-(2-hydroxy-ethylamino)-benzoylamino]-2-methyl-phenoxymethyl}-thieno[3,2-c]pyridine-7-carboxylic acid ethyl ester. (Yield 1.68 g, 83%).

### Example 168

### 4-Amino-3-{5-[3-chloro-4-(2-hydroxy-ethylamino)-benzoylamino]-2-methylphenoxymethyl}-thieno[3,2-c]pyridine-7-carboxylic acid ethyl ester

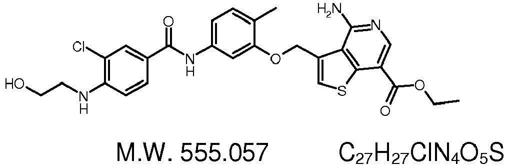

Ammonia gas was bubbled into a solution of 4-chloro-3-{5-[3-chloro-4-(2-hydroxy-ethylamino)-benzoylamino]-2-methyl-phenoxymethyl}-thieno[3,2-c]pyridine-7-carboxylic acid ethyl ester (0.96 g, 1.67 mmol) (from Intermediate 80 *supra)* in 2-propanol (70 mL) for 20 minutes. The mixture was heated in a microwave reactor at 140 °C for 2 hours. The reaction mixture was concentrated. The residue was washed with hot methanol, filtered and dried to give 4-amino-3-{5-[3-chloro-4-(2-hydroxy-ethylamino)-benzoylamino]-2-methyl-phenoxymethyl}-thieno[3,2-c]pyridine-7-carboxylic acid ethyl ester. (Yield 0.83 g, 89%).

### Intermediate 81

### 4-Amino-3-{5-[3-chloro-4-(2-hydroxy-ethylamino)-benzoylamino]-2-methylphenoxymethyl}-thieno[3,2-c]pyridine-7-carboxylic acid

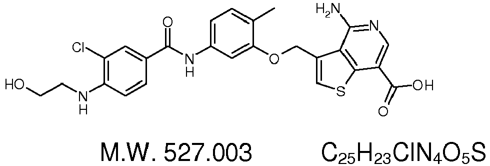

An aqueous solution of sodium hydroxide (2N, 11.0 mL, 5.5 mmol) was added to a solution of 4-amino-3-{5-[3-chloro-4-(2-hydroxy-ethylamino)-benzoylamino]-2-methylphenoxymethyl}-thieno[3,2-c]pyridine-7-carboxylic acid ethyl ester (0.83 g, 1.50 mmol) (from Example 168 *supra)* in tetrahydrofuran / methanol (3:1, 28 mL) and the mixture was heated at 50 °C for 18 hours. The reaction mixture was concentrated and azeotroped with toluene. The solid was then suspended in water and treated with hydrochloric acid (2N). After stirring 30 minutes, the solid was collected, washed with water and dried to give 4-amino-3-{5-[3-chloro-4-(2-hydroxyethylamino)-benzoylamino]-2-methyl-phenoxy-methyl}-thieno[3,2-c]pyridine-7-carboxylic acid as white powder. (Yield 0.90 g, 95%).

### Example 169

### 4-Amino-3-{5-[3-chloro-4-(2-hydroxy-ethylamino)-benzoylamino]-2-methylphenoxymethyl}-thieno[3,2-c]pyridine-7-carboxylic acid amide

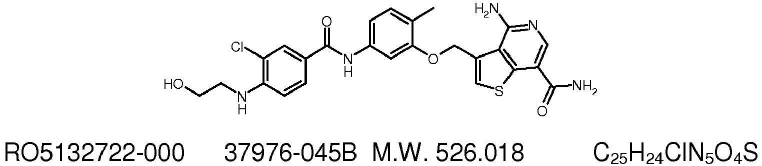

To a mixture of 4-amino-3-{5-[3-chloro-4-(2-hydroxy-ethylamino)-benzoylamino]-2-methyl-phenoxymethyl}-thieno[3,2-c]pyridine-7-carboxylic acid (0.30 g, 0.57 mmol) (from Intermediate 81 *supra),* HBTU (0.27 g, 0.71 mmol) (Advanced ChemTech) and diisopropylethylamine (0.0.24 mL, 1.43 mmol) (Aldrich) in N,N-dimethylformamide (15 mL) was added ammonium chloride powder (39.0 mg, 0.73 mmol). The mixture was stirred at room temperature for 1 day and then concentrated. The residue was purified by C18 colum chromatography eluting with acetonitrile / water to give 4-amino-3-{5-[3-chloro-4-(2-hydroxy-ethylamino)-benzoylamino]-2-methylphenoxymethyl}-thieno[3,2-c]pyridine-7-carboxylic acid amide as a powder. (Yield 0.17 g, 57%).
HRMS (ES⁺) *m*/*z* Calcd for C₂₅H₂₄ClN₅O₄S + H [(M+H)⁺]: 526.1311. Found: 526.1306.

### Example 170

### In vitro Kinase assay

In vitro enzyme activity was determined with c-Raf using a HTRF assay with 6H-MEK as substrate (Dose Response).

### Assay Principle:

The assay utilizes 6H-MEK as the substrate. Upon c-Raf phosphorylation, phosphorylated 6H-MEK is detected with rabbit anti-phospho-MEK1/2, Eu-labeled anti-rabbit, and APC-labeled anti-6H antibodies.

### Reagents and Instruments:

c-Raf enzyme was cloned human c-Raf with EE-tag and was phosphorylated (co-expressed with v-src-FLAG in baculovirus Hi5 cells), 0.2 mg / mL (2.74 µM assuming a molecular weight of 73 kD) and stored at -15 °C. Wild-type full-length 6H-MEK was used as substrate, 4.94 mg / mL (154.4 µM assuming a MW of 32 kD) and stored at -15 °C.

The following antibodies were used in the detection step: Rabbit α-P-(Ser 217/221)-MEK-1/2 Ab (from Cell Signaling, Cat. # 9121S, Lot 4); Eu-α-rabbit IgG (from Wallac, Cat. # AD0083, Lot 107557, labeling date 06/21/02, 8 Eu/protein, 580 µg/mL, 3.63 µM); α-6H-SureLight-APC (from Martek, Cat. #AD0059H, Lot E011AB01, 3.15 µM).

Assays were read with Envision from PerkinElmer, HTRF reading mode with 412 mirrors.

Assays were performed with Costar 384 all-black plates, 120 µL (Cat. # 3710).

Test compounds were dispensed in Weidman 384 polypropylene plates (REMP).

### Assay Procedure:

Prepare Kinase Assay Buffer (KAB): 50 mM HEPES (HyClone) pH7, 10 mM MgCl₂, 1 mM DTT, 0.1 mM Na₃V₂O₄, and 0.2 mg / mL BSA.
Prepare 6H-MEK (150 nM) in KAB. Add 40 µL / well to a Weidman plate.
Prepare ATP (66 µM) in KAB.
Prepare c-Raf (60 nM) in KAB. Add 25 µL / well to a Weidman plate.
Dilute compounds to 2.4 mM in DMSO. Perform 10-point 3x dilution in DMSO.
Withdraw 2.5 ul/well of DMSO solution and add to 27.5 µL / well ATP solution in (3).
Add 12 µL/well 6H-MEK to the assay plate on the Cybiwell, followed by 6 µL / well of solution in (5) and 6 µL / well of solution in (4) for a DMSO concentration of 2.1 % during MEK phosphorylation.
Incubate at 37 °C for 30 minutes.
Prepare rabbit α-P-(Ser 217/221)-MEK-1/2 Ab (1:225 from stock) in AB1: 50 mM HEPES pH7, 0.2 mg / mL BSA, and 53 mM EDTA.
To stop reaction, add 6 µL / well of solution from (8) to the assay plate and incubate at 37 °C for 30 minutes.
Prepare Eu-α-rabbit IgG (9 nM) and α-6H-SureLight-APC (120 nM) in AB2: 50 mM HEPES pH7 and 0.2 mg / mL BSA.
Add 6 µL / well of solution from (10) to the assay plate.

For determining the spectrum cross talk factor, prepare 2 samples following steps (6) to (9). For the blank sample, add 6 µL / well of AB2. For the cross talk factor sample, add 6 µL / well of Eu-anti rabbit IgG (9 nM).
Incubate at 37 °C for 1 hour.
Read HTRF signals at 615 nm and 665 nm on the Envision. Normalize HTRF signals after spectrum cross-talk correction.

The compounds of the present invention in the above assay exhibit C-Raf IC₅₀ values of from 0.001 to 50 µM. Representative IC₅₀ values are, for example,:

| | |
|---|---|
| Example 2 | 0.836 µM |
| Example 7 | 0.022 µM |
| Example 18 | 0.324 µM |
| Example 14 | 2.016 µM |

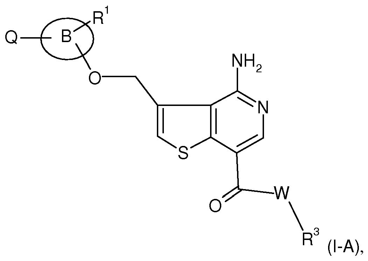

## Claims

1. A compound of the formula (I-A) 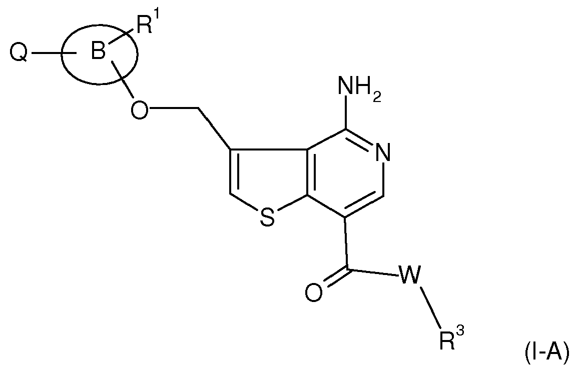 or pharmaceutically acceptable salts thereof
wherein
R¹ is selected from the group consisting of hydrogen, lower alkyl, halogen, cyano, trifluoromethyl, lower alkoxy, -C(O)-NH-lower alkyl, -C(O)-NH-lower alkoxy, oxo and NO₂;
R³ is selected from the group consisting of hydrogen, lower alkyl, lower alkyl substituted by -OR⁴ and -NR⁴R⁵;
R⁴ and R⁵ are selected from hydrogen, lower alkyl, lower alkyl substituted with OH or lower alkoxy; or
R⁴ and R⁵ together with the nitrogen atom to which they are attached form morpholinyl;
Q is a group 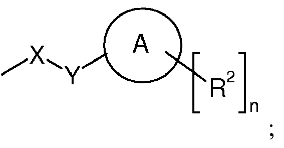
R² may be the same or different lower alkenyl, lower alkynyl, methyl sulfonyl, sulfonamide, hydrogen, lower alkyl, halogen, cyano, trifluoromethyl, lower alkoxy, NO₂, sulfonyl urea, amide, ester, carbamoyl, carbamate, urea,

1. A compound of the formula (I-A) 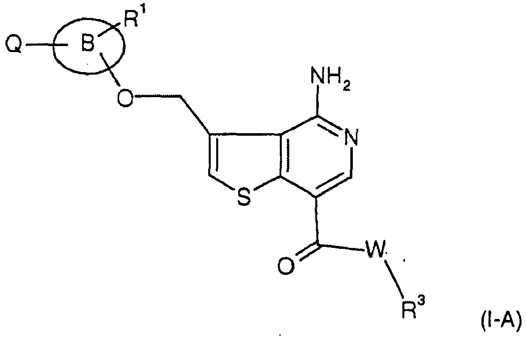 or pharmaceutically acceptable salts thereof
wherein
R¹ is selected from the group consisting of hydrogen, lower alkyl, halogen, cyano, trifluoromethyl, lower alkoxy, -C(O)-NH-lower alkyl, -C(O)-NH-lower alkoxy, oxo and NO₂;
R³ is selected from the group consisting of hydrogen, lower alkyl, lower alkyl substituted by -OR⁴ and -NR⁴R⁵;
R⁴ and R⁵ are selected from hydrogen, lower alkyl, lower alkyl substituted with OH or lower alkoxy; or
R⁴ and R⁵ together with the nitrogen atom to which they are attached form morpholinyl;
Q is a group 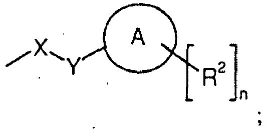
R² may be the same or different lower alkenyl, lower alkynyl, methyl sulfonyl, sulfonamide, hydrogen, lower alkyl, halogen, cyano, trifluoromethyl, lower alkoxy, NO₂, sulfonyl urea, amide, ester, carbamoyl, carbamate, urea,
-NR⁴R⁵; and lower alkyl substituted with -OR⁴ or -NR⁴R⁵;
the group -X-Y- is selected from -OCH₂-, -CH₂O-, -NHCO-, -CONH-, -O-, -OCH₂CH₂-, -CH₂OCH₂, -CH₂CH₂O-, -CF=CH-, -CH=CF-, -NH-, -NHCH₂-, -CH₂NH-, -SCH₂-, -NHSO₂, -SO₂NH-, -CH₂S-, -SOCH₂-, -CH₂SO-, -SO₂CH₂-, -CH₂SO₂-, -S-, -CH=CH- and lower alkylene;
W is -O- or -NH-;
n is 1 or 2;
Ring A is selected from the group consisting of aryl, heteroaryl , cycloalkyl and heterocycle; and
Ring B is selected from the group consisting of aryl, and heteroaryl, or
Q is hydrogen, and
R¹ is -O-CH₃ or -C(O)-NH-CH₃;
R³ is ethyl, which is unsubstituted or once substituted with -OH;
W is -O- or -NH-; and
Ring B is phenyl or pyridinyl
wherein the term "lower alkyl" denotes a straight-chain or branched, saturated aliphatic hydrocarbon having 1 to 6 carbon atoms; the term "lower alkoxy" means a group -O-lower alkyl, wherein "lower alkyl" has the meaning given above; the term "lower alkenyl" refers to a straight-chain or branched hydrocarbon residue comprising an olefinic double bond and up to 7 carbon atoms; the term "lower alkynyl" refers to a straight-chain or branched hydrocarbon residue comprising a triple bond and up to 7 carbon atoms; or
a compound of the following formula : 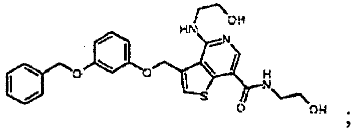 or 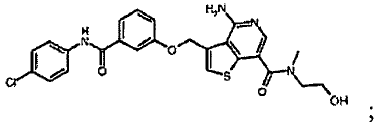 or 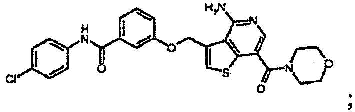 or 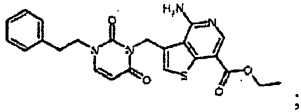 or 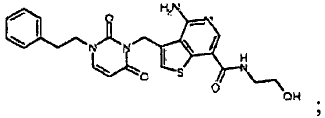 or 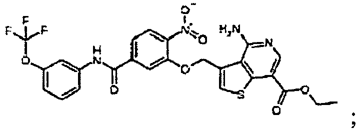 or 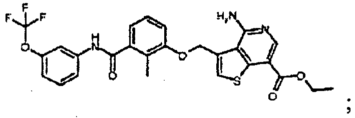 or 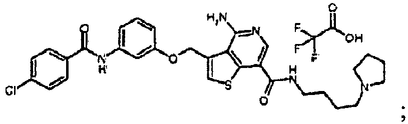 or 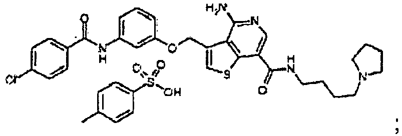 or

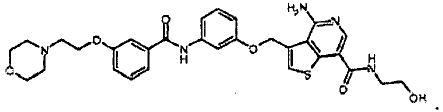

2. A compound of claim 1 having the formula (I) 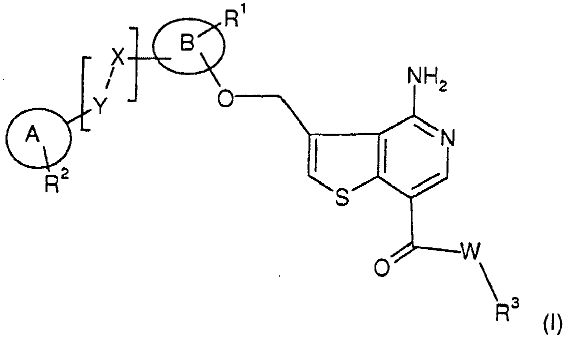 or pharmaceutically acceptable salts thereof
wherein
R¹ is selected from the group consisting of hydrogen, lower alkyl, halogen, cyano, trifluoromethyl, lower alkoxy and NO₂
R² is selected from the group consisting of lower alkenyl, lower alkynyl, methyl sulfonyl, sulfonamide, hydrogen, lower alkyl, halogen, cyano, trifluoromethyl, lower alkoxy, NO₂ sulfonyl urea, amide, ester, carbamoyl, carbamate, urea, and lower alkyl substituted with -OR⁴ or -NR⁴R⁵;
R³ is selected from the group consisting of hydrogen, lower alkyl, lower alkyl substituted by -OR⁴ and -NR⁴R⁵;
R⁴ and R⁵ are selected from hydrogen, lower alkyl, or lower alkyl substituted with -OH or lower alkoxy;
-X-Y- are selected from the group consisting of -OCH₂-, -CH₂O-, -NHCO-, -CONH-, -O-, -OCH₂CH₂-, -CH₂OCH₂, -CH₂CH₂O-, -CF=CH-, -CH=CF-, -NH-, -NHCH₂-, -CH₂NH-, -SCH₂-, -CH₂S-, -SOCH₂-, -CH₂SO-, -SO₂CH₂-, -CH₂SO₂-, -S-, -CH=CH- and lower alkylene;
W is -O- or -NH-;
Ring A is selected from the group consisting of aryl, heteroaryl, cycloalkyl and heterocycle; and
Ring B is selected from the group consisting of aryl, and heteroaryl.

3. The compound according to claim 1, wherein
the ring A is selected from pyrimidinyl, pyridinyl, piperidinyl, cyclopropyl, cyclopentyl, cyclohexyl, tetrahydro-pyranyl, phenyl, pyrazolyl or thiophenyl; and
the ring B is pyrimidinyl, 2-oxo-pyrimidinyl or phenyl.

4. The compound of claim 1, wherein both rings A and B are phenyl.

5. The compound of claims 1 or 2 wherein the group -X-Y- is selected from -OCH₂-, -CH₂O-, -NHCO- and -CONH-.

6. The compound of claim 5 wherein R¹ is selected from the group consisting of -CH₃, -Cl and -F.

7. The compound of claim 6 wherein R² is selected from the group consisting of -Cl, -F, -CF₃, -CONH₂, alkoxy, NR⁴R⁵, and lower alkyl.

8. A compound of claim 1 or 2 selected from the group consisting of
4-Amino-3-[3-(4-chloro-3-trifluoromethyl-phenylcarbamoyl)-phenoxymethyl]-thieno[3,2-c]pyridine-7-carboxylic acid ethyl ester;
4-Amino-3-(3-benzyloxy-phenoxymethyl)-thieno[3,2-c]pyridine-7-carboxylic acid ethyl ester;
4-Amino-3-(3-benzyloxy-phenoxymethyl)-thieno[3,2-c]pyridine-7-carboxylic acid (2-hydroxy-ethyl)-amide;
4-Amino-3-[3-(4-chloro-benzyloxy)-phenoxymethyl]-thieno[3,2-c]pyridine-7-carboxylic acid ethyl ester;
4-Amino-3-[3-(4-chloro-benzyloxy)-phenoxymethylJ-thieno[3,2-c]pyridine-7-carboxylic acid (2-hydroxy-ethyl)-amide;
4-Amino-3-[3-(3-chloro-benzyloxy)-phenoxymethyl]-thieno[3,2-c]pyridine-7-carboxylic acid ethyl ester;
4-Amino-3-[3-(3-chloro-benzyloxy)-phenoxymethyl]-thieno[3,2-c]pyridine-7-carboxylic acid (2-hydroxy-ethyl)-amide;
4-Amino-3-[3-(3-trifluoromethyl-benzyloxy)-phenoxymethyl]-thieno[3,2-c]pyridine-7-carboxylic acid ethyl ester;
4-Amino-3-[3-(3-trifluoromethyl-benzyloxy)-phenoxymethyl]-thieno[3,2-c]pyridine-7-carboxylic acid (2-hydroxy-ethyl)-amide; and
4-Amino-3-(3-benzylsulfanyl-phenoxymethyl)-thieno[3,2-c]pyridine-7-carboxylic acid ethyl ester.

9. A compound of claim 1 or 2 selected from the group consisting of
4-Amino-3-(3-benzylsulfanyl-phenoxymethyl)-thieno[3,2-c]pyridine-7-carboxylic acid (2-hydroxy-ethyl)-amide;
4-Amino-3-(3-phenylmethanesulfonyl-phenoxymethyl)-thieno[3,2-c]pyridine-7-carboxylic acid ethyl ester;
4-Amino-3-(3-phenylmethanesulfonyl-phenoxymethyl]-thieno[3,2-c]pyridine-7-carboxylic acid (2-hydroxy-ethyl)-amide;
4-Amino-3-[3-(3-methoxy-benzyloxy)-phenoxymethyl]-thieno[3,2-c]pyridine-7-carboxylic acid ethyl ester;
4-Amino-3-[3-(3-methoxy-benzyloxy)-phenoxymethyl]-thieno[3,2-c]pyridine-7-carboxylic acid (2-hydroxy-ethyl)-amide;
4-Amino-3-[3-(4-methoxy-benzyloxy)-phenoxymethyl]-thieno[3,2-c]pyridine-7-carboxylic acid ethyl ester;
4-Amino-3-[3-(4-methoxy-benzyloxy)-phenoxymethyl]-thieno[3,2-c]pyridine-7-carboxylic acid (2-hydroxy-ethyl)-amide;
4-Amino-3-[3-(4-methoxy-3-trifluoromethyl-benzyloxy)-phenoxymethyl]-thieno[3,2-c]pyridine-7-carboxylic acid ethyl ester;
4-Amino-3-[3-(4-methoxy-3-trifluoromethyl-benzyloxy)-phenoxymethyl]-thieno[3,2-c]pyridine-7-carboxylic acid (2-hydroxy-ethyl)-amide; and
4-Amino-3-(3-phenoxy-phenoxymethyl)-thieno[3,2-c]pyridine-7-carboxylic acid ethyl ester.

10. A compound of claim 1 or 2 selected from the group consisting of
4-Amino-3-(3-phenylamino-phenoxymethyl]-thieno[3,2-c]pyridine-7-carboxylic acid (2-hydroxy-ethyl)-amide;
4-Amino-3-(3-benzylamino-phenoxymethyl)-thieno[3,2-c]pyridine-7-carboxylic acid ethyl ester;
4-Amino-3-(3-benzylamino-phenoxymethyl]-thieno[3,2-c]pyridine-7-carboxylic acid (2-hydroxy-ethyl)-amide;
4-Amino-3-[3-(4-cyano-benzyloxy)-phenoxymethyl]-thieno[3,2-c]pyridine-7-carboxylic acid ethyl ester;
4-Amino-3-[3-(4-cyano-benzyloxy)-phenoxymethyl]-thieno[3,2-c]pyridine-7-carboxylic acid;
4-Amino-3-[3-(4-cyano-benzyloxy)-phenoxymethyl]-thieno[3,2-c]pyridine-7-carboxylic acid (2-hydroxy-ethyl)-amide;
4-Amino-3-[3-(3-cyano-benzyloxy)-phenoxymethyl]-thieno[3,2-c]pyridine-7-carboxylic acid ethyl ester;
4-Amino-3-[3-(3-cyano-benzyloxy)-phenoxymethyl]-thieno[3,2-c]pyridine-7-carboxylic acid;
4-Amino-3-[3-(3-cyano-benzyloxy)-phenoxymethyl]-thieno[3,2-c]pyridine-7-carboxylic acid (2-hydroxy-ethyl)-amide trifluoroacetic acid salt;
3-(3-benzyloxy-phenoxymethyl)-4-(2-hydroxy-ethylamino)-thieno[3,2-c]pyridine-7-carboxylic acid (2-hydroxy-ethyl)-amide; and
4-Amino-3-(3-methoxy-phenoxymethyl)-thieno[3,2-c]pyridine-7-carboxylic acid ethyl ester.

11. A compound of claim 1 or 2 selected from the group consisting of
4-Amino-3-(3-methoxy-phenoxymethyl)-thieno[3,2-c]pyridine-7-carboxylic acid (2-hydroxy-ethyl)-amide;
4-Amino-3-(3-benzoylamino-phenoxymethyl)-thieno[3,2-c]pyridine-7-carboxylic acid ethyl ester;
4-Amino-3-(3-benzoylamino-phenoxymethyl)-thieno[3,2-c]pyridine-7-carboxylic acid (2-hydroxy-ethyl)-amide;
4-Amino-3-(3-benzoylamino-phenoxymethyl)-thieno[3,2-c]pyridine-7-carboxylic acid (2-hydroxy-ethyl)-amide hydrochloride;
4-Amino-3-(3-benzoylamino-phenoxymethyl)-thieno[3,2-c]pyridine-7-carboxylic acid (2-hydroxy-ethyl)-amide toluene-4-sulfonic acid salt;
4-Amino-3-[3-(4-chloro-phenylcarbamoyl)-phenoxymethyl]-thieno[3,2-c]pyridine-7-carboxylic acid ethyl ester;
4-Amino-3-[3-(4-chloro-phenylcarbamoyl)-phenoxymethyl]-thieno[3,2-c]pyridine-7-carboxylic acid (2-hydroxy-ethyl)-amide;
4-Amino-3-[3-(4-fluoro-phenylcarbamoyl)-phenoxymethyl]-thieno[3,2-c]pyridine-7-carboxylic acid ethyl ester;
4-Amino-3-[3-(4-fluoro-phenylcarbamoyl)-phenoxymethyl]-thieno[3,2-c]pyridine-7-carboxylic acid (2-hydroxy-ethyl)-amide;
4-Amino-3-[3-(4-chloro-benzyloxy)-phenoxymethyl]-thieno[3,2-c]pyridine-7-carboxylic acid trifluoro-acetic acid salt; and
4-Amino-3-[3-(3-chloro-phenylcarbamoyl)-phenoxymethyl]-thieno[3,2-c]pyridine-7-carboxylic acid ethyl ester.

12. A compound of claim 1 or 2 selected from the group consisting of
4-Amino-3-[3-(3-chloro-phenylcarbamoyl)-phenoxymethyl]-thieno[3,2-c]pyridine-7-carboxylic acid (2-hydroxy-ethyl)-amide;
4-Amino-3-[3-(4-chloro-phenylcarbamoyl)-phenoxymethyl]-thieno[3,2-c]pyridine-7-carboxylic acid (3-hydroxy-propyl)-amide;
4-Amino-3-[3-(4-chloro-phenylcarbamoyl)-phenoxymethyl]-thieno[3,2-c]pyridine-7-carboxylic acid propylamide;
4-Amino-3-[3-(4-chloro-phenylcarbamoyl)-phenoxymethyl]-thieno[3,2-c]pyridine-7-carboxylic acid ethylamide;
4-Amino-3-{3-[(4-chloro-phenyl)-methyl-carbamoyl]-phenoxymethyl}-thieno[3,2-c]pyridine-7-carboxylic acid ethyl ester;
4-Amino-3-[3-(1-methyl-piperidin-4-ylcarbamoyl)-phenoxymethyl]-thieno[3,2-c]pyridine-7-carboxylic acid ethyl ester;
4-Amino-3-[3-(5-methyl-pyridin-2-ylcarbamoyl)-phenoxymethyl]-thieno[3,2-c]pyridine-7-carboxylic acid ethyl ester;
4-Amino-3-[3-(1-methanesulfonyl-piperidin-4-ylcarbamoyl)-phenoxymethyl]-thieno[3,2-c]pyridine-7-carboxylic acid ethyl ester;
4-Amino-3-{3-[(4-chloro-phenyl)-methyl-carbamoyl]-phenoxymethyl}-thieno[3,2-c]pyridine-7-carboxylic acid (2-hydroxy-ethyl)-amide;
4-Amino-3-[5-(3-carbamoyl-phenylcarbamoyl)-2-methyl-phenoxymethyl]-thieno[3,2-c]pyridine-7-carboxylic acid ethyl ester;
4-Amino-3-(2-methyl-5-phenylcarbamoyl-phenoxymethyl)-thieno[3,2-c]pyridine-7-carboxylic acid ethyl ester; and
4-Amino-3-[3-(4-chloro-benzyloxy)-phenoxymethyl]-thieno[3,2-c]pyridine-7-carboxylic acid (3-hydroxy-propyl)-amide trifluoro-acetic acid salt.

13. A compound of claim 1 or 2 selected from the group consisting of
4-Amino-3-[3-(4-chloro-phenoxymethyl)-phenoxymethyl]-thieno[3,2-c]pyridine-7-carboxylic acid ethyl ester;
4-Amino-3-{3-[2-(4-chloro-phenyl)-vinyl]-phenoxymethyl}-thieno[3,2-c]pyridine-7-carboxylic acid ethyl ester;
4-Amino-3-{3-[2-(4-chloro-phenyl)-vinyl]-phenoxymethyl}-thieno[3,2-c]pyridine-7-carboxylic acid (2-hydroxy-ethyl)-amide;
4-Amino-3-[3-(4-chloro-phenoxymethyl)-phenoxymethyl]-thieno[3,2-c]pyridine-7-carboxylic acid (2-hydroxy-ethyl)-amide;
4-Amino-3-[3-(4-chloro-phenylcarbamoyl)-phenoxymethyl]-thieno[3,2-c]pyridine-7-carboxylic acid (2-hydroxy-ethyl)-methyl-amide;
4-Am i no-3-[3-(4-chloro-phenylcarbamoyl)-phenoxymeth yl]-th ieno[3,2-c]pyridi ne-7-carboxylic acid (2-methoxy-ethyl)-amide;
4-Amino-3-[3-(4-chloro-phenylcarbamoyl)-phenoxymethyl]-thieno[3,2-c]pyridine-7-carboxylic acid methylamide;
3-[4-Amino-7-(morpholine-4-carbonyl)-thieno[3,2-c]pyridin-3-ylmethoxy]-N-(4-chlorophenyl)-benzamide;
4-Amino-3-(3-cyclohexylcarbamoyl-phenoxymethyl)-thieno[3,2-c]pyridine-7-carboxylic acid ethyl ester;
4-Amino-3-[3-(tetrahydro-pyran-4-ylcarbamoyl)-phenoxymethyl]-thieno[3,2-c]pyridine-7-carboxylic acid ethyl ester; and
4-Amino-3-[3-(1-methanesulfonyl-piperidin-4-ylcarbamoyl)-phenoxymethyl]-thieno[3,2-c]pyridine-7-carboxylic acid (2-hydroxy-ethyl)-amide.

14. A compound of claim 1 or 2 selected from the group consisting of
4-Amino-3-[3-(1-methyl-piperidin-4-ylcarbamoyl)-phenoxymethyl]-thieno[3,2-c]pyridine-7-carboxylic acid (2-hydroxy-ethyl)-amide;
4-Amino-3-(3-phenylaminomethyl-phenoxymethyl)-thieno[3,2-c]pyridine-7-carboxylic acid (2-hydroxy-ethyl)-amide trifluoro-acetic acid salt;
4-Amino-3-{3-[2-(4-chloro-phenyl)-ethoxy]-phenoxymethyl}-thieno[3,2-c]pyridine-7-carboxylic acid ethyl ester;
4-Amino-3-{3-[2-(4-chloro-phenyl)-ethoxy]-phenoxymethyl}-thieno[3,2-c]pyridine-7-carboxylic acid (2-hydroxy-ethyl)-amide;
4-Amino-3-[3-(4-chloro-benzoylamino)-phenoxymethyl]-thieno[3,2-c]pyridine-7-carboxylic acid ethyl ester;
4-Amino-3-[3-(4-chloro-benzoylamino)-phenoxymethyl]-thieno[3,2-c]pyridine-7-carboxylic acid (2-hydroxy-ethyl)-amide;
4-Amino-3-[3-(4-chloro-benzoylamino)-phenoxymethyl]-thieno[3,2-c]pyridine-7-carboxylic acid (3-hydroxy-propyl)-amide;
4-Amino-3-[3-(4-chloro-benzoylamino)-phenoxymethyl]-thieno[3,2-c]pyridine-7-carboxylic acid (3-hydroxy-propyl)-amide hydrochloride;
4-Amino-3-[3-(4-chloro-benzoylamino)-phenoxymethyl]-thieno[3,2-c]pyridine-7-carboxylic acid (3-hydroxy-propyl)-amide toluene-4-sulfonic acid salt;
4-Amino-3-(2-oxo-1-phenethyl-1,2-dihydro-pyrimidin-4-yloxymethyl)-thieno[3,2-c]pyridine-7-carboxylic acid ethyl ester; and
4-Amino-3-(2,6-dioxo-3-phenethyl-3,6-dihydro-2H-pyrimidin-1-ylmethyl)-thieno[3,2-c]pyridine-7-carboxylic acid ethyl ester.

15. A compound of claim 1 or 2 selected from the group consisting of
4-Amino-3-(2,6-dioxo-3-phenethyl-3,6-dihydro-2H-pyrimidin-1-ylmethyl)-thieno[3,2-c]pyridine-7-carboxylic acid (2-hydroxy-ethyl)-amide;
4-Amino-3-[2-methoxy-5-(3-methoxy-phenylcarbamoyl)-phenoxymethyl]-thieno[3,2-c]pyridine-7-carboxylic acid ethyl ester;
4-Amino-3-[5-(3,5-dimethoxy-phenylcarbamoyl)-2-methoxy-phenoxymethyl]-thieno[3,2-c]pyridine-7-carboxylic acid ethyl ester;
4-Amino-3-[5-(4-chloro-3-methyl-phenylcarbamoyl)-2-methoxy-phenoxymethyl]-thieno[3,2-c]pyridine-7-carboxylic acid ethyl ester;
4-Amino-3-(5-benzylcarbamoyl-2-nitro-phenoxymethyl)-thieno[3,2-c]pyridine-7-carboxylic acid ethyl ester;
4-Amino-3-(2-methyl-3-phenylcarbamoyl-phenoxymethyl)-thieno[3,2-c]pyridine-7-carboxylic acid ethyl ester;
4-Amino-3-[3-(3,5-dimethoxy-phenylcarbamoyl)-2-methyl-phenoxymethyl]-thieno[3,2-c]pyridine-7-carboxylic acid ethyl ester;
4-Amino-3-[2-nitro-5-(3-trifluoromethoxy-phenylcarbamoyl)-phenoxymethyl]-thieno[3,2-c]pyridine-7-carboxylic acid ethyl ester;
4-Amino-3-[2-methyl-3-(3-trifluoromethoxy-phenylcarbamoyl)-phenoxymethyl]-thieno[3,2-c]pyridine-7-carboxylic acid ethyl ester;
4-Amino-3-[3-(3-methanesulfonyl-phenylcarbamoyl)-2-methyl-phenoxymethyl]-thieno[3,2-c]pyridine-7-carboxylic acid ethyl ester;
4-Amino-3-[3-(3-chloro-phenylcarbamoyl)-2-methyl-phenoxymethyl]-thieno[3,2-c]pyridine-7-carboxylic acid ethyl ester; and
4-Amino-3-[2-methyl-3-(5-methyl-1H-pyrazol-3-ylcarbamoyl)-phenoxymethyl]-thieno[3,2-c]pyridine-7-carboxylic acid ethyl ester.

16. A compound of claim 1 or 2 selected from the group consisting of
4-Amino-3-[3-(3,5-difluoro-benzyloxy)-5-methyl-phenoxymethyl]-thieno[3,2-c]pyridine-7-carboxylic acid ethyl ester;
4-Amino-3-[3-(3,4-difluoro-benzyloxy)-5-methyl-phenoxymethyl]-thieno[3,2-c]pyridine-7-carboxylic acid ethyl ester;
4-Amino-3-[3-(5-chloro-thiophen-2-ylmethoxy)-5-methyl-phenoxymethyl]-thieno[3,2-c]pyridine-7-carboxylic acid ethyl ester;
4-Amino-3-[3-(3-methyl-benzyloxy)-phenoxymethyl]-thieno[3,2-c]pyridine-7-carboxylic acid ethyl ester;
4-Amino-3-[3-(5-chloro-thiophen-2-ylmethoxy)-phenoxymethyl]-thieno[3,2-c]pyridine-7-carboxylic acid ethyl ester;
4-Amino-3-[3-(2,5-difluoro-benzyloxy)-phenoxymethyl]-thieno[3,2-c]pyridine-7-carboxylic acid ethyl ester;
4-Amino-3-[3-(4-fluoro-benzyloxy)-5-methyl-phenoxymethyl]-thieno[3,2-c]pyridine-7-carboxylic acid ethyl ester; and
4-Amino-3-[3-(2,4-difluoro-benzyloxy)-5-methyl-phenoxymethyl]-thieno[3,2-c]pyridine-7-carboxylic acid ethyl ester.

17. A compound of claim 1 or 2 selected from the group consisting of
4-Amino-3-[3-(2,5-difluoro-benzyloxy)-5-methyl-phenoxymethyl]-thieno[3,2-c]pyridine-7-carboxylic acid ethyl ester;
4-Amino-3-[3-methyl-5-(4-methyl-benzyloxy)-phenoxymethyl]-thieno[3,2-c]pyridine-7-carboxylic acid ethyl ester;
4-Amino-3-[3-methyl-5-(2-methyl-benzyloxy)-phenoxymethyl]-thieno[3,2-c]pyridine-7-carboxylic acid ethyl ester;
4-Amino-3-[3-(2-fluoro-benzyloxy)-5-methyl-phenoxymethyl]-thieno[3,2-c]pyridine-7-carboxylic acid ethyl ester;
4-Amino-3-[3-(2-methoxy-benzyloxy)-phenoxymethyl]-thieno[3,2-c]pyridine-7-carboxylic acid ethyl ester;
4-Amino-3-{3-[3-(2-morpholin-4-yl-ethoxy)-benzoylamino]-phenoxymethyl}-thieno[3,2-c]pyridine-7-carboxylic acid (2-hydroxy-ethyl)-amide;
4-Amino-3-(3-methylcarbamoyl-phenoxymethyl)-thieno[3,2-c]pyridine-7-carboxylic acid ethyl ester;
4-Amino-3-(3-cyclopropylcarbamoyl-phenoxymethyl)-thieno[3,2-c]pyridine-7-carboxylic acid ethyl ester;
4-Amino-3-(3-cyclopentylcarbamoyl-phenoxymethyl)-thieno[3,2-c]pyridine-7-carboxylic acid ethyl ester; and
4-Amino-3-(3-cyclohexylcarbamoyl-phenoxymethyl}-thieno[3,2-c]pyridine-7-carboxylic acid (2-hydroxy-ethyl)-amide.

18. A compound of claim 1 or 2 selected from the group consisting of
4-Amino-3-[3-(tetrahydro-pyran-4-ylcarbamoyl)-phenoxymethyl]-thieno[3,2-c]pyridine-7-carboxylic acid (2-hydroxy-ethyl)-amide;
4-Amino-3-(3-methylcarbamoyl-phenoxymethyl)-thieno[3,2-c]pyridine-7-carboxylic acid (2-hydroxy-ethyl)-amide;
4-Amino-3-(3-cyclopropylcarbamoyl-phenoxymethyl)-thieno[3,2-c]pyridine-7-carboxylic acid (2-hydroxy-ethyl)-amide;
4-Amino-3-(3-cyclopentylcarbamoyl-phenoxymethyl)-thieno[3,2-c]pyridine-7-carboxylic acid (2-hydroxy-ethyl)-amide;
4-Amino-3-(5-methylcarbamoyl-pyridin-3-yloxymethyl)-thieno[3,2-c]pyridine-7-carboxylic acid ethyl ester; and
4-Amino-3-(5-methylcarbamoyl-pyridin-3-yloxymethyl)-thieno[3,2-c]pyridine-7-carboxylic acid (2-hydroxy-ethyl)-amide.

19. A compound of claim 1 or 2 selected from the group consisting of
4-Amino-3-[3-(4-chloro-benzoylamino)-phenoxymethyl]-thieno[3,2-c]pyridine-7-carboxylic acid;
4-Amino-3-[3-(pyrimidin-5-ylmethoxy)-phenoxymethyl]-thieno[3,2-c]pyridine-7-carboxylic acid ethyl ester;
4-Amino-3-[3-(pyrimidin-5-ylmethoxy)-phenoxymethyl]-thieno[3,2-c]pyridine-7-carboxylic acid (2-hydroxy-ethyl)-amide;
4-Amino-3-[3-(4-chloro-benzenesulfonylamino)-phenoxymethyl]-thieno[3,2-c]pyridine-7-carboxylic acid ethyl ester;
4-Amino-3-[3-(4-chloro-benzenesulfonylamino)-phenoxymethyl]-thieno[3,2-c]pyridine-7-carboxylic acid (2-hydroxy-ethyl)-amide;
4-Amino-3-[2-chloro-5-(4-chloro-benzyloxy)-phenoxymethyl]-thieno[3,2-c]pyridine-7-carboxylic acid ethyl ester;
4-Amino-3-[2-chloro-5-(4-chloro-benzyloxy)-phenoxymethyl]-thieno[3,2-c]pyridine-7-carboxylic acid (2-hydroxy-ethyl)-amide;
4-Amino-3-{3-[(pyridine-2-carbonyl)-amino]-phenoxymethyl}-thieno[3,2-c]pyridine-7-carboxylic acid ethyl ester;
4-Amino-3-{3-[(pyridine-2-carbonyl)-amino]-phenoxymethyl}-thieno[3,2-c]pyridine-7-carboxylic acid (2-hydroxy-ethyl)-amide; and
4-Amino-3-{3-[(6-methyl-pyridine-3-carbonyl)-amino]-phenoxymethyl}-thieno[3,2-c]pyridine-7-carboxylic acid ethyl ester.

20. A compound of claim 1 or 2 selected from the group consisting of
4-Amino-3-{3-[(6-methyl-pyridine-3-carbonyl)-amino]-phenoxymethyl}-thieno[3,2c]pyridine-7-carboxylic acid (2-hydroxy-ethyl)-amide;
4-Amino-3-{3-[(pyridine-4-carbonyl)-amino]-phenoxymethyl}-thieno[3,2-c]pyridine-7-carboxylic acid ethyl ester;
4-Amino-3-{3-[(pyridine-4-carbonyl)-amino]-phenoxymethyl}-thieno[3,2-c]pyridine-7-carboxylic acid (2-hydroxy-ethyl)-amide;
4-Amino-3-[5-(3-chloro-4-fluoro-benzoylamino)-2-methyl-phenoxymethyl]-thieno[3,2-c]pyridine-7-carboxylic acid ethyl ester;
4-Amino-3-[5-(3-chloro-4-benzoylamino)-2-methyl-phenoxymethyl]-thieno[3,2-c]pyridine-7-carboxylic acid ethyl ester;
4-Amino-3-[5-(3-chloro-benzoylamino)-2-methyl-phenoxymethyl]-thieno[3,2-c]pyridine-7-carboxylic acid (2-hydroxy-ethyl)-amide;
4-Amino-3-{5-[3-chloro-4-(2-hydroxy-ethylamino)-benzoylamino)-2-methyl-phenoxymethyl]-thieno[3,2-c]pyridine-7-carboxylic acid (2-hydroxy-ethyl)-amide;
4-Amino-3-{5-[3-chloro-4-(2-hydroxy-ethylamino)-benzoylamino)-2-methylphenoxymethyl]-thieno[3,2-c]pyridine-7-carboxylic acid (2-hydroxy-ethyl)-amide toluene-4-sulfonic acid salt;
4-Amino-3-{5-[3-chloro-4-(2-hydroxy-ethylamino)-benzoylamino]-2-methylphenoxymethyl}-thieno[3,2-c]pyridine-7-carboxylic acid ethyl ester; and
4-Amino-3-{5-[3-chloro-4-(2-hydroxy-ethylamino)-benzoylamino]-2-methylphenoxymethyl}-thieno[3,2-c]pyridine-7-carboxylic acid amide.

21. A compound of claim 1 or 2 selected from the group consisting of
4-Amino-3-[3-(2-fluoro-benzyloxy)-5-methyl-phenoxymethyl]-thieno[3,2-c]pyridine-7-carboxylic acid (2-hydroxy-ethyl)-amide;
4-Amino-3-[3-(3,5-difluoro-benzyloxy)-5-methyl-phenoxymethyl]-thieno[3,2-c]pyridine-7-carboxylic acid (2-hydroxy-ethyl)-amide;
4-Amino-3-[3-methyl-5-(2-methyl-benzyloxy)-phenoxymethyl]-thieno[3,2-c]pyridine-7-carboxylic acid (2-hydroxy-ethyl)-amide;
4-Amino-3-[3-(2,5-difluoro-benzyloxy)-phenoxymethyl]-thieno[3,2-c]pyridine-7-carboxylic acid (2-hydroxy-ethyl)-amide;
4-Amino-3-[3-(3-fluoro-benzyloxy)-5-methyl-phenoxymethyl]-thieno[3,2-c]pyridine-7-carboxylic acid (2-hydroxy-ethyl)-amide;
4-Amino-3-[3-(2,5-difluoro-benzyloxy)-5-methyl-phenoxymethyl]-thieno[3,2-c]pyridine-7-carboxylic acid (2-hydroxy-ethyl)-amide;
4-Amino-3-[3-methyl-5-(4-methyl-benzyloxy)-phenoxymethyl]-thieno[3,2-c]pyridine-7-carboxylic acid (2-hydroxy-ethyl)-amide;
4-Amino-3-[3-(3,4-difluoro-benzyloxy)-5-methyl-phenoxymethy]-thieno[3,2-c]pyridine-7-carboxylic acid (2-hydroxy-ethyl)-amide;
4-Amino-3-[3-(2,3-difluoro-benzyloxy)-phenoxymethyl]-thieno[3,2-c]pyridine-7-carboxylic acid (2-hydroxy-ethyl)-amide;
4-Amino-3-[3-(4-chloro-benzoylamino)-phenoxymethyl]-thieno[3,2-c]pyridine-7-carboxylic acid (4-pyrrolidin-1-yl-butyl)-amide trifluoro-acetic acid salt; and
4-Amino-3-[3-(4-chloro-benzoylamino)-phenoxymethyl]-thieno[3,2-c]pyridine-7-carboxylic acid (4-pyrrolidin-1 -yl-butyl)-amide toluene-4-sulfonic acid salt.

22. A compound according to any one of claims 1 to 21 for use in the treatment of cancer, in particular colorectal, breast, lung and prostate cancers.

23. A pharmaceutical formulation comprising a compound according to any one of claims 1 to 21 together with pharmaceutically acceptable carriers or excipients.

24. The pharmaceutical formulation according to claim 23 for use in the treatment of cancer, in particular colorectal, breast, lung and prostate cancers.

25. The use of a compound according to anyone of claims 1 to 21 for the manufacture of a medicament for the treatment of cancer, in particular colorectal, breast, lung and prostate cancers.

26. A compound of the formula (I-A) according to claim 1 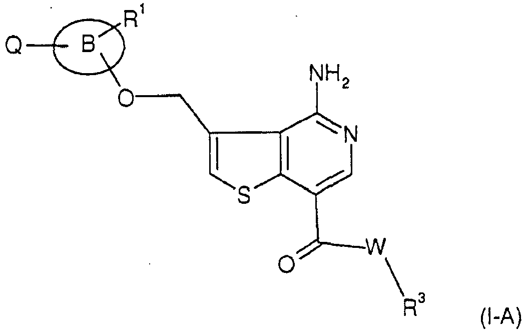 or pharmaceutically acceptable salts thereof
for use in the treatment of pancreatic, gastric, bladder, ovarian, melanoma, neuroblastoma, cervical, kidney or renal cancers, leukemias or lymphomas.

27. A pharmaceutical formulation comprising a compound as defined in claim 26 together with pharmaceutically acceptable carriers or excipients for use in the treatment of pancreatic, gastric, bladder, ovarian, melanoma, neuroblastoma, cervical, kidney or renal cancers, leukemias or lymphomas.

28. The use of a compound as defined in claim 26 for the manufacture of a medicament for the treatment of pancreatic, gastric, bladder, ovarian, melanoma, neuroblastoma, cervical, kidney or renal cancers, leukemias or lymphomas.

## Patentansprüche

1. Eine Verbindung der Formel (I-A) 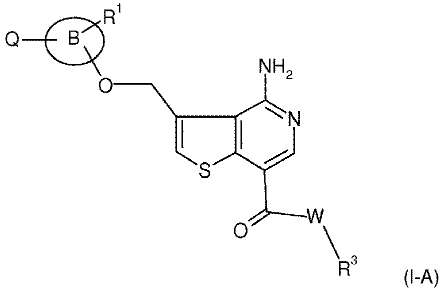 oder pharmazeutisch verträgliche Salze davon,
wobei
R¹ ausgewählt ist aus der Gruppe bestehend aus Wasserstoff, Niederalkyl, Halogen, Cyano, Trifluormethyl, Niederalkoxy, -C(O)-NH-Niederalkyl, -C(O)-NH-Niederalkoxy, Oxo und NO₂;
R³ ausgewählt ist aus der Gruppe bestehend aus Wasserstoff, Niederalkyl, Niederalkyl substituiert mit -OR⁴ und -NR⁴R⁵;
R⁴ und R⁵ ausgewählt sind aus Wasserstoff, Niederalkyl, Niederalkyl substituiert mit OH oder Niederalkoxy; oder
R⁴ und R⁵ zusammen mit dem Stickstoffatom, an das sie gebunden sind, Morpholinyl bilden;
Q eine Gruppe 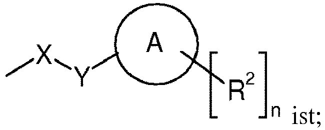
R² gleich oder verschieden Niederalkenyl, Niederalkinyl, Methylsulfonyl, Sulfonamid, Wasserstoff, Niederalkyl, Halogen, Cyano, Trifluormethyl, Niederalkoxy, NO₂, Sulfonylharnstoff, Amid, Ester, Carbamoyl, Carbamat, Harnstoff, -NR⁴R⁵; und Niederalkyl substituiert mit -OR⁴ oder -NR⁴R⁵ sein können;
die Gruppe -X-Y- ausgewählt ist aus -OCH₂-, -CH₂O-, -NHCO-, -CONH-, -O-, -OCH₂CH₂-, -CH₂OCH₂-, -CH₂CH₂O-, -CF=CH-, -CH=CF-, -NH-, -NHCH₂-, -CH₂NH-, -SCH₂-, -NHSO₂, -SO₂NH-, -CH₂S-, -SOCH₂-, -CH₂SO-, -SO₂CH₂-, -CH₂SO₂-, -S-, -CH=CH- und Niederalkylen;
W gleich -O- oder -NH- ist;
n gleich 1 oder 2 ist;
Ring A ausgewählt ist aus der Gruppe bestehend aus Aryl, Heteroaryl, Cycloalkyl und Heterocyclus; und
Ring B ausgewählt ist aus der Gruppe bestehend aus Aryl und Heteroaryl oder
Q gleich Wasserstoff ist, und
R¹ gleich -O-CH₃ oder -C(O)-NH-CH₃ ist;
R³ Ethyl ist, das unsubstituiert oder einmal mit -OH substituiert ist;
W gleich -O- oder -NH- ist; und
Ring B Phenyl oder Pyridinyl ist,
wobei der Begriff "Niederalkyl" einen geradkettigen oder verzweigten, gesättigten aliphatischen Kohlenwasserstoff mit 1 bis 6 Kohlenstoffatomen darstellt; der Begriff "Niederalkoxy" eine Gruppe -O-Niederalkyl bedeutet, wobei "Niederalkyl" die vorstehende Bedeutung aufweist; sich der Begriff "Niederalkenyl" auf einen geradkettigen oder verzweigten Kohlenwasserstoffrest bezieht, der eine olefinische Doppelbindung und bis zu 7 Kohlenstoffatome umfasst; sich der Begriff "Niederalkinyl" auf einen geradkettigen oder verzweigten Kohlenwasserstoffrest bezieht, der eine Dreifachbindung und bis zu 7 Kohlenstoffatome umfasst; oder
eine Verbindung der nachstehenden Formel: 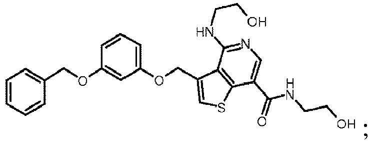 oder 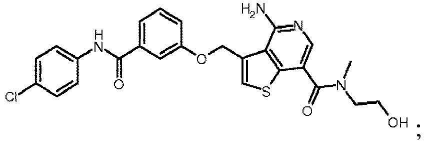 oder 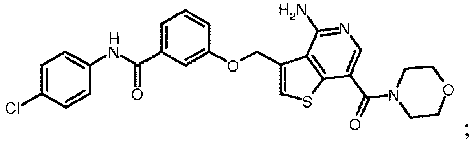 oder 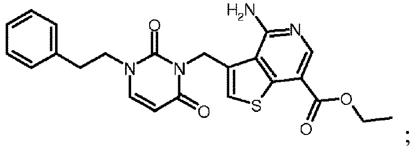 oder 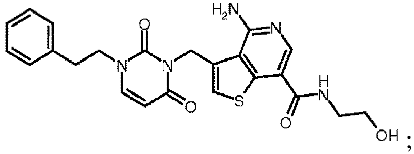 oder 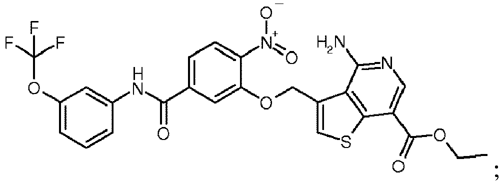 oder 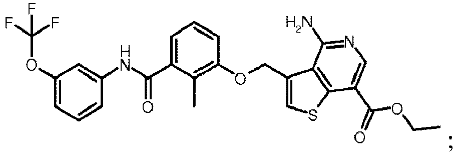 oder 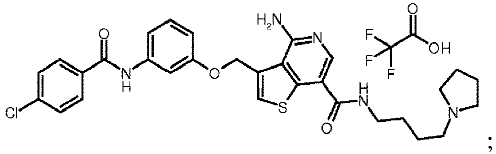 oder 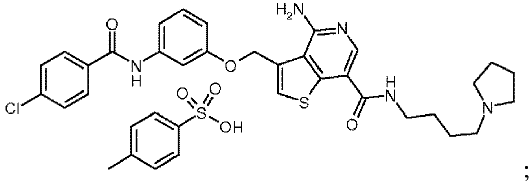 oder

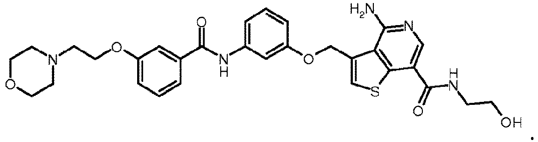

2. Eine Verbindung gemäß Anspruch 1, die die Formel (I) aufweist 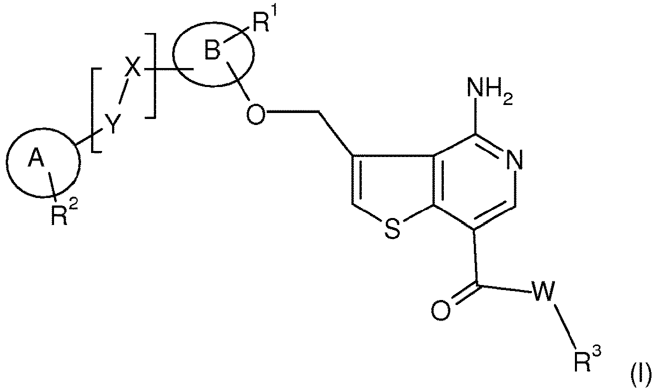 oder pharmazeutisch verträgliche Salze davon
wobei
R¹ ausgewählt ist aus der Gruppe bestehend aus Wasserstoff, Niederalkyl, Halogen, Cyano, Trifluormethyl, Niederalkoxy und NO₂;
R² ausgewählt ist aus der Gruppe bestehend aus Niederalkenyl, Niederalkinyl, Methylsulfonyl, Sulfonamid, Wasserstoff, Niederalkyl, Halogen, Cyano, Trifluormethyl, Niederalkoxy, NO₂ Sulfonylharnstoff, Amid, Ester, Carbamoyl, Carbamat, Harnstoff und Niederalkyl substituiert mit -OR⁴ oder - NR⁴R⁵;
R³ ausgewählt ist aus Gruppe bestehend aus Wasserstoff, Niederalkyl, Niederalkyl substituiert mit -OR⁴ und -NR⁴R⁵;
R⁴ und R⁵ ausgewählt sind aus Wasserstoff, Niederalkyl oder Niederalkyl substituiert mit -OH oder Niederalkoxy;
-X-Y- ausgewählt sind aus der Gruppe bestehend aus -OCH₂-, -CH₂O-, -NHCO-, -CONH-, -O-, -OCH₂CH₂-, -CH₂OCH₂-, -CH₂CH₂O-, -CF=CH-, -CH=CF-, -NH-, -NHCH₂-, -CH₂NH-, -SCH₂-, -CH₂S-, -SOCH₂-, -CH₂SO-, -SO₂CH₂-, -CH₂SO₂-, -S-, -CH=CH- und Niederalkylen;
W gleich -O- oder -NH-;
Ring A ausgewählt ist aus der Gruppe bestehend aus Aryl, Heteroaryl, Cycloalkyl und Hetercyclus; und
Ring B ausgewählt ist aus der Gruppe bestehend aus Aryl und Heteroaryl.

3. Die Verbindung gemäß Anspruch 1, wobei
der Ring A ausgewählt ist aus Pyrimidinyl, Pyridinyl, Piperidinyl, Cyclopropyl, Cyclopentyl, Cyclohexyl, Tetrahydro-pyranyl, Phenyl, Pyrazolyl oder Thiophenyl; und
der Ring B Pyrimidinyl, 2-Oxo-pyrimidinyl oder Phenyl ist.

4. Die Verbindung gemäß Anspruch 1, wobei beide Ringe A und B Phenyl sind.

5. Die Verbindung gemäß Anspruch 1 oder 2, wobei die Gruppe -X-Y- ausgewählt ist aus -OCH₂-, -CH₂O-, -NHCO- und -CONH-.

6. Die Verbindung gemäß Anspruch 5, wobei R¹ ausgewählt ist aus der Gruppe bestehend aus -CH₃, -Cl und -F.

7. Die Verbindung gemäß Anspruch 6, wobei R² ausgewählt ist aus der Gruppe bestehend aus -Cl, -F, -CF₃, -CONH₂, Alkoxy, NR⁴R⁵ und Niederalkyl.

8. Eine Verbindung gemäß Anspruch 1 oder 2, ausgewählt aus der Gruppe bestehend aus
4-Amino-3-[3-(4-chlor-3-trifluormethyl-phenylcarbamoyl)-phenoxymethyl]-thieno[3,2-c]pyridin-7-carbonsäure-ethylester;
4-Amino-3-(3-benzyloxy-phenoxymethyl)-thieno[3,2-c]pyridin-7-carbonsäure-ethylester;
4-Amino-3-(3-benzyloxy-phenoxymethyl)-thieno[3,2-c]pyridin-7-carbonsäure-(2-hydroxy-ethyl)-amid;
4-Amino-3-[3-(4-chlor-benzyloxy)-phenoxymethyl]-thieno[3,2-c]pyridin-7-carbonsäure-ethylester;
4-Amino-3-[3-(4-chlor-benzyloxy)-phenoxymethyl]-thieno[3,2-c]pyridin-7-carbonsäure-(2-hydroxy-ethyl)-amid;
4-Amino-3-[3-(3-chlor-benzyloxy)-phenoxymethyl]-thieno[3,2-c]pyridin-7-carbonsäure-ethylester;
4-Amino-3-[3-(3-chlor-benzyloxy)-phenoxymethyl]-thieno[3,2-c]pyridin-7-carbonsäure-(2-hydroxy-ethyl)-amid;
4-Amino-3-[3-(3-trifluormethyl-benzyloxy)-phenoxymethyl]-thieno[3,2-c]pyridin-7-carbonsäure-ethylester;
4-Amino-3-[3-(3-trifluormethyl-benzyloxy)-phenoxymethyl]-thieno[3,2-c]pyridin-7-carbonsäure-(2-hydroxy-ethyl)-amid; und
4-Amino-3-(3-benzylsulfanyl-phenoxymethyl)-thieno[3,2-c]pyridin-7-carbonsäure-ethylester.

9. Eine Verbindung gemäß Anspruch 1 oder 2, ausgewählt aus der Gruppe bestehend aus
4-Amino-3-(3-benzylsulfanyl-phenoxymethyl)-thieno[3,2-c]pyridin-7-carbonsäure-(2-hydroxy-ethyl)-amid;
4-Amino-3-(3-phenylmethansulfonyl-phenoxymethyl)-thieno[3,2-c]pyridin-7-carbonsäure-ethylester;
4-Amino-3-(3-phenylmethansulfonyl-phenoxymethyl)-thieno[3,2-c]pyridin-7-carbonsäure-(2-hydroxy-ethyl)-amid;
4-Amino-3-[3-(3-methoxy-benzyloxy)-phenoxymethyl]-thieno[3,2-c]pyridin-7-carbonsäure-ethylester;
4-Amino-3-[3-(3-methoxy-benzyloxy)-phenoxymethyl]-thieno[3,2-c]pyridin-7-carbonsäure-(2-hydroxy-ethyl)-amid;
4-Amino-3-[3-(4-methoxy-benzyloxy)-phenoxymethyl]-thieno[3,2-c]pyridin-7-carbonsäure-ethylester;
4-Amino-3-[3-(4-methoxy-benzyloxy)-phenoxymethyl]-thieno[3,2-c]pyridin-7-carbonsäure-(2-hydroxy-ethyl)-amid;
4-Amino-3-[3-(4-methoxy-3-trifluormethyl-benzyloxy)-phenoxymethyl]-thieno[3,2-c]pyridin-7-carbonsäure-ethylester;
4-Amino-3-[3-(4-methoxy-3-trifluormethyl-benzyloxy)-phenoxymethyl]-thieno[3,2-c]pyridin-7-carbonsäure-(2-hydroxy-ethyl)-amid; und
4-Amino-3-(3-phenoxy-phenoxymethyl)-thieno[3,2-c]pyridin-7-carbonsäure-ethylester.

10. Eine Verbindung gemäß Anspruch 1 oder 2, ausgewählt aus der Gruppe bestehend aus
4-Amino-3-(3-phenylamino-phenoxymethyl)-thieno[3,2-c]pyridin-7-carbonsäure-(2-hydroxy-ethyl)-amid;
4-Amino-3-(3-benzylamino-phenoxymethyl)-thieno[3,2-c]pyridin-7-carbonsäure-ethylester;
4-Amino-3-(3-benzylamino-phenoxymethyl)-thieno[3,2-c]pyridin-7-carbonsäure-(2-hydroxy-ethyl)-amid;
4-Amino-3-[3-(4-cyano-benzyloxy)-phenoxymethyl]-thieno[3,2-c]pyridin-7-carbonsäure-ethylester;
4-Amino-3-[3-(4-cyano-benzyloxy)-phenoxymethyl]-thieno[3,2-c]pyridin-7-carbonsäure;
4-Amino-3-[3-(4-cyano-benzyloxy)-phenoxymethyl]-thieno[3,2-c]pyridin-7-carbonsäure-(2-hydroxy-ethyl)-amid;
4-Amino-3-[3-(3-cyano-benzyloxy)-phenoxymethyl]-thieno[3,2-c]pyridin-7-carbonsäure-ethylester;
4-Amino-3-[3-(3-cyano-benzyloxy)-phenoxymethyl]-thieno[3,2-c]pyridin-7-carbonsäure;
4-Amino-3-[3-(3-cyano-benzyloxy)-phenoxymethyl]-thieno[3,2-c]pyridin-7-carbonsäure-(2-hydroxy-ethyl)-amid-trifluor-essigsäuresalz;
3-(3-Benzyloxy-phenoxymethyl)-4-(2-hydroxy-ethylamino)-thieno[3,2-c]pyridin-7-carbonsäure-(2-hydroxy-ethyl)-amid; und
4-Amino-3-(3-methoxy-phenoxymethyl)-thieno[3,2-c]pyridin-7-carbonsäureethylester.

11. Eine Verbindung gemäß Anspruch 1 oder 2, ausgewählt aus der Gruppe bestehend aus
4-Amino-3-(3-methoxy-phenoxymethyl)-thieno[3,2-c]pyridin-7-carbonsäure-(2-hydroxy-ethyl)-amid;
4-Amino-3-(3-benzoylamino-phenoxymethyl)-thieno[3,2-c]pyridin-7-carbonsäure-ethylester;
4-Amino-3-(3-benzoylamino-phenoxymethyl)-thieno[3,2-c]pyridin-7-carbonsäure-(2-hydroxy-ethyl)-amid;
4-Amino-3-(3-benzoylamino-phenoxymethyl)-thieno[3,2-c]pyridin-7-carbonsäure-(2-hydroxy-ethyl)-amid-hydrochlorid;
4-Amino-3-(3-benzoylamino-phenoxymethyl)-thieno[3,2-c]pyridin-7-carbonsäure-(2-hydroxy-ethyl)-amid-toluol-4-sulfonsäuresalz;
4-Amino-3-[3-(4-chlor-phenylcarbamoyl)-phenoxymethyl]-thieno[3,2-c]pyridin-7-carbonsäure-ethylester;
4-Amino-3-[3-(4-chlor-phenylcarbamoyl)-phenoxymethyl]-thieno[3,2-c]pyridin-7-carbonsäure-(2-hydroxy-ethyl)-amid;
4-Amino-3-[3-(4-fluor-phenylcarbamoyl)-phenoxymethyl]-thieno[3,2-c]pyridin-7-carbonsäure-ethylester;
4-Amino-3-[3-(4-fluor-phenylcarbamoyl)-phenoxymethyl]-thieno[3,2-c]pyridin-7-carbonsäure-(2-hydroxy-ethyl)-amid;
4-Amino-3-[3-(4-chlor-benzyloxy)-phenoxymethyl]-thieno[3,2-c]pyridin-7-carbonsäure-trifluoressigsäuresalz; und
4-Amino-3-[3-(3-chlor-phenylcarbamoyl)-phenoxymethyl]-thieno[3,2-c]pyridin-7-carbonsäure-ethylester.

12. Eine Verbindung gemäß Anspruch 1 oder 2, ausgewählt aus der Gruppe bestehend aus
4-Amino-3-[3-(3-chlor-phenylcarbamoyl)-phenoxymethyl]-thieno[3,2-c]pyridin-7-carbonsäure-(2-hydroxy-ethyl)-amid;
4-Amino-3-[3-(4-chlor-phenylcarbamoyl)-phenoxymethyl]-thieno[3,2-c]pyridin-7-carbonsäure-(3-hydroxy-propyl)-amid;
4-Amino-3-[3-(4-chlor-phenylcarbamoyl)-phenoxymethyl]-thieno[3,2-c]pyridin-7-carbonsäure-propylamid;
4-Amino-3-[3-(4-chlor-phenylcarbamoyl)-phenoxymethyl]-thieno[3,2-c]pyridin-7-carbonsäure-ethylamid;
4-Amino-3-{3-[(4-chlor-phenyl)-methyl-carbamoyl]-phenoxymethyl}-thieno[3,2-c]pyridin-7-carbonsäure-ethylester;
4-Amino-3-[3-(1-methyl-piperidin-4-ylcarbamoyl)-phenoxymethyl]-thieno[3,2-c]pyridin-7-carbonsäure-ethylester;
4-Amino-3-[3-(5-methyl-pyridin-2-ylcarbamoyl)-phenoxymethyl]-thieno[3,2-c]pyridin-7-carbonsäure-ethylester;
4-Amino-3-[3-(1-methansulfonyl-piperidin-4-ylcarbamoyl)-phenoxymethyl]-thieno[3,2-c]pyridin-7-carbonsäure-ethylester;
4-Amino-3-{3-[(4-chlor-phenyl)-methyl-carbamoyl]-phenoxymethyl}-thieno[3,2-c]-pyridin-7-carbonsäure-(2-hydroxy-ethyl)-amid;
4-Amino-3-[5-(3-carbamoyl-phenylcarbamoyl)-2-methyl-phenoxymethyl]-thieno[3,2-c]pyridin-7-carbonsäure-ethylester;
4-Amino-3-(2-methyl-5-phenylcarbamoyl-phenoxymethyl)-thieno[3,2-c]pyridin-7-carbonsäure-ethylester; und
4-Amino-3-[3-(4-chlor-benzyloxy)-phenoxymethyl]-thieno[3,2-c]pyridin-7-carbonsäure-(3-hydroxy-propyl)-amid-trifluor-essigsäuresalz.

13. Eine Verbindung gemäß Anspruch 1 oder 2, ausgewählt aus der Gruppe bestehend aus
4-Amino-3-[3-(4-chlor-phenoxymethyl)-phenoxymethyl]-thieno[3,2-c]pyridin-7-carbonsäure-ethylester;
4-Amino-3-{3-[2-(4-chlor-phenyl)-vinyl]-phenoxymethyl}-thieno[3,2-c]pyndin-7-carbonsäure-ethylester;
4-Amino-3-{3-[2-(4-chlor-phenyl)-vinyl]-phenoxymethyl}-thieno[3,2-c]pyndin-7-carbonsäure (2-hydroxy-ethyl)-amid;
4-Amino-3-[3-(4-chlor-phenoxymethyl)-phenoxymethyl]-thieno[3,2-c]pyridin-7-carbonsäure-(2-hydroxy-ethyl)-amid;
4-Amino-3-[3-(4-chlor-phenylcarbamoyl)-phenoxymethyl]-thieno[3,2-c]pyridin-7-carbonsäure (2-hydroxy-ethyl)-methyl-amid;
4-Amino-3-[3-(4-chlor-phenylcarbamoyl)-phenoxymethyl]-thieno[3,2-c]pyridin-7-carbonsäure-(2-methoxy-ethyl)-amid;
4-Amino-3-[3-(4-chlor-phenylcarbamoyl)-phenoxymethyl]-thieno[3,2-c]pyridin-7-carbonsäure-methylamide;
3-[4-Amino-7-(morpholin-4-carbonyl)-thieno[3,2-c]pyridin-3-ylmethoxy]-N-(4-chlor-phenyl)-benzamid;
4-Amino-3-(3-cyclohexylcarbamoyl-phenoxymethyl)-thieno[3,2-c]pyridin-7-carbonsäure-ethylester;
4-Amino-3-[3-(tetrahydro-pyran-4-ylcarbamoyl)-phenoxymethyl]-thieno[3,2-c]pyridin-7-carbonsäure-ethylester; und
4-Amino-3-[3-(1-methansulfonyl-piperidin-4-ylcarbamoyl)-phenoxymethyl]-thieno[3,2-c]pyridin-7-carbonsäure-(2-hydroxy-ethyl)-amid.

14. Eine Verbindung gemäß Anspruch 1 oder 2, ausgewählt aus der Gruppe bestehend aus
4-Amino-3-[3-(1-methyl-piperidin-4-ylcarbamoyl)-phenoxymethyl]-thieno[3,2-c]pyridin-7-carbonsäure-(2-hydroxy-ethyl)-amid;
4-Amino-3-(3-phenylaminomethyl-phenoxymethyl)-thieno[3,2-c]pyridin-7-carbonsäure-(2-hydroxy-ethyl)-amid-trifluoressigsäuresalz;
4-Amino-3-{3-[2-(4-chlor-phenyl)-ethoxy]-phenoxymethyl}-thieno[3,2-c]pyridin-7-carbonsäure-ethylester;
4-Amino-3-{3-[2-(4-chlor-phenyl)-ethoxy]-phenoxymethyl}-thieno[3,2-c]pyndin-7-carbonsäure-(2-hydroxy-ethyl)-amid;
4-Amino-3-[3-(4-chlor-benzoylamino)-phenoxymethyl]-thieno[3,2-c]pyridin-7-carbonsäure-ethylester;
4-Amino-3-[3-(4-chlor-benzoylamino)-phenoxymethyl]-thieno[3,2-c]pyridin-7-carbonsäure-(2-hydroxy-ethyl)-amid;
4-Amino-3-[3-(4-chlor-benzoylamino)-phenoxymethyl]-thieno[3,2-c]pyridin-7-carbonsäure-(3-hydroxy-propyl)-amid;
4-Amino-3-[3-(4-chlor-benzoylamino)-phenoxymethyl]-thieno[3,2-c]pyridin-7-carbonsäure-(3-hydroxy-propyl)-amid-hydrochlorid;
4-Amino-3-[3-(4-chlor-benzoylamino)-phenoxymethyl]-thieno[3,2-c]pyridin-7-carbonsäure-(3-hydroxy-propyl)-amid-toluol-4-sulfonsäuresalz;
4-Amino-3-(2-oxo-1-phenethyl-1,2-dihydro-pyrimidin-4-yloxymethyl)-thieno[3,2-c]pyridin-7-carbonsäure-ethylester; und
4-Amino-3-(2,6-dioxo-3-phenethyl-3,6-dihydro-2H-pyrimidin-1-ylmethyl)-thieno[3,2-c]pyridin-7-carbonsäure-ethylester.

15. Eine Verbindung gemäß Anspruch 1 oder 2, ausgewählt aus der Gruppe bestehend aus
4-Amino-3-(2,6-dioxo-3-phenethyl-3,6-dihydro-2H-pyrimidin-1-ylmethyl)-thieno[3,2-c]-pyridin-7-carbonsäure-(2-hydroxy-ethyl)-amid;
4-Amino-3-[2-methoxy-5-(3-methoxy-phenylcarbamoyl)-phenoxymethyl]-thieno[3,2-c]-pyridin-7-carbonsäure-ethylester;
4-Amino-3-[5-(3,5-dimethoxy-phenylcarbamoyl)-2-methoxy-phenoxymethyl]-thieno[3,2-c]pyridin-7-carbonsäure-ethylester;
4-Amino-3-[5-(4-chlor-3-methyl-phenylcarbamoyl)-2-methoxy-phenoxymethyl]-thieno[3,2-c]pyridin-7-carbonsäure-ethylester;
4-Amino-3-(5-benzylcarbamoyl-2-nitro-phenoxymethyl)-thieno[3,2-c]pyridin-7-carbonsäure-ethylester;
4-Amino-3-(2-methyl-3-phenylcarbamoyl-phenoxymethyl)-thieno[3,2-c]pyridin-7-carbonsäure-ethylester;
4-Amino-3-[3-(3,5-dimethoxy-phenylcarbamoyl)-2-methyl-phenoxymethyl]-thieno-[3,2-c]pyridin-7-carbonsäure-ethylester;
4-Amino-3-[2-nitro-5-(3-trifluormethoxy-phenylcarbamoyl)-phenoxymethyl]-thieno[3,2-c]pyridin-7-carbonsäure-ethylester;
4-Amino-3-[2-methyl-3-(3-trifluormethoxy-phenylcarbamoyl)-phenoxymethyl]-thieno[3,2-c]pyridin-7-carbonsäure-ethylester;
4-Amino-3-[3-(3-methansulfonyl-phenylcarbamoyl)-2-methyl-phenoxymethyl]-thieno[3,2-c]pyridin-7-carbonsäure-ethylester;
4-Amino-3-[3-(3-chlor-phenylcarbamoyl)-2-methyl-phenoxymethyl]-thieno[3,2-c]pyridin-7-carbonsäure-ethylester; und
4-Amino-3-[2-methyl-3-(5-methyl-1H-pyrazol-3-ylcarbamoyl)-phenoxymethyl]-thieno[3,2-c]pyridin-7-carbonsäure-ethylester.

16. Eine Verbindung gemäß Anspruch 1 oder 2, ausgewählt aus der Gruppe bestehend aus
4-Amino-3-[3-(3,5-difluor-benzyloxy)-5-methyl-phenoxymethyl]-thieno[3,2-c]pyridin-7-carbonsäure-ethylester;
4-Amino-3-[3-(3,4-difluor-benzyloxy)-5-methyl-phenoxymethyl]-thieno[3,2-c]pyridin-7-carbonsäure-ethylester;
4-Amino-3-[3-(5-chlor-thiophen-2-ylmethoxy)-5-methyl-phenoxymethyl]-thieno[3,2-c]-pyridin-7-carbonsäure-ethylester;
4-Amino-3-[3-(3-methyl-benzyloxy)-phenoxymethyl]-thieno[3,2-c]pyridin-7-carbonsäure-ethylester;
4-Amino-3-[3-(5-chlor-thiophen-2-ylmethoxy)-phenoxymethyl]-thieno[3,2-c]pyridin-7-carbonsäure-ethylester;
4-Amino-3-[3-(2,5-difluor-benzyloxy)-phenoxymethyl]-thieno[3,2-c]pyridin-7-carbonsäure-ethylester;
4-Amino-3-[3-(4-fluor-benzyloxy)-5-methyl-phenoxymethyl]-thieno[3,2-c]pyridin-7-carbonsäure-ethylester; und
4-Amino-3-[3-(2,4-difluor-benzyloxy)-5-methyl-phenoxymethyl]-thieno[3,2-c]pyridin-7-carbonsäure-ethylester.

17. Eine Verbindung gemäß Anspruch 1 oder 2, ausgewählt aus der Gruppe bestehend aus
4-Amino-3-[3-(2,5-difluor-benzyloxy)-5-methyl-phenoxymethyl]-thieno[3,2-c]pyridin-7-carbonsäure-ethylester;
4-Amino-3-[3-methyl-5-(4-methyl-benzyloxy)-phenoxymethyl]-thieno[3,2-c]pyridin-7-carbonsäure-ethylester;
4-Amino-3-[3-methyl-5-(2-methyl-benzyloxy)-phenoxymethyl]-thieno[3,2-c]pyridin-7-carbonsäure-ethylester;
4-Amino-3-[3-(2-fluor-benzyloxy)-5-methyl-phenoxymethyl]-thieno[3,2-c]pyridin-7-carbonsäure-ethylester;
4-Amino-3-[3-(2-methoxy-benzyloxy)-phenoxymethyl]-thieno[3,2-c]pyridin-7-carbonsäure-ethylester;
4-Amino-3-{3-[3-(2-morpholin-4-yl-ethoxy)-benzoylamino]-phenoxymethyl}-thieno[3,2-c]pyridin-7-carbonsäure-(2-hydroxy-ethyl)-amid;
4-Amino-3-(3-methylcarbamoyl-phenoxymethyl)-thieno[3,2-c]pyridin-7-carbonsäure-ethylester;
4-Amino-3-(3-cyclopropylcarbamoyl-phenoxymethyl)-thieno[3,2-c]pyridin-7-carbonsäure-ethylester;
4-Amino-3-(3-cyclopentylcarbamoyl-phenoxymethyl)-thieno[3,2-c]pyridin-7-carbonsäure-ethylester; und
4-Amino-3-(3-cyclohexylcarbamoyl-phenoxymethyl)-thieno[3,2-c]pyridin-7-carbonsäure-(2-hydroxy-ethyl)-amid.

18. Eine Verbindung gemäß Anspruch 1 oder 2, ausgewählt aus der Gruppe bestehend aus
4-Amino-3-[3-(tetrahydro-pyran-4-ylcarbamoyl)-phenoxymethyl]-thieno[3,2-c]pyridin-7-carbonsäure-(2-hydroxy-ethyl)-amid;
4-Amino-3-(3-methylcarbamoyl-phenoxymethyl)-thieno[3,2-c]pyridin-7-carbonsäure-(2-hydroxy-ethyl)-amid;
4-Amino-3-(3-cyclopropylcarbamoyl-phenoxymethyl)-thieno[3,2-c]pyridin-7-carbonsäure-(2-hydroxy-ethyl)-amid;
4-Amino-3-(3-cyclopentylcarbamoyl-phenoxymethyl)-thieno[3,2-c]pyridin-7-carbonsäure-(2-hydroxy-ethyl)-amid;
4-Amino-3-(5-methylcarbamoyl-pyridin-3-yloxymethyl)-thieno[3,2-c]pyridin-7-carbonsäure-ethylester; und
4-Amino-3-(5-methylcarbamoyl-pyridin-3-yloxymethyl)-thieno[3,2-c]pyridin-7-carbonsäure-(2-hydroxy-ethyl)-amid.

19. Eine Verbindung gemäß Anspruch 1 oder 2, ausgewählt aus der Gruppe bestehend aus
4-Amino-3-[3-(4-chlor-benzoylamino)-phenoxymethyl]-thieno[3,2-c]pyridin-7-carbonsäure;
4-Amino-3-[3-(pyrimidin-5-ylmethoxy)-phenoxymethyl]-thieno[3,2-c]pyridin-7-carbonsäure-ethylester;
4-Amino-3-[3-(pyrimidin-5-ylmethoxy)-phenoxymethyl]-thieno[3,2-c]pyridin-7-carbonsäure-(2-hydroxy-ethyl)-amid;
4-Amino-3-[3-(4-chlor-benzolsulfonylamino)-phenoxymethyl]-thieno[3,2-c]pyridin-7-carbonsäure-ethylester,
4-Amino-3-[3-(4-chlor-benzolsulfonylamino)-phenoxymethyl]-thieno[3,2-c]pyridin-7-carbonsäure-(2-hydroxy-ethyl)-amid;
4-Amino-3-[2-chlor-5-(4-chlor-benzyloxy)-phenoxymethyl]-thieno[3,2-c]pyridin-7-carbonsäure-ethylester,
4-Amino-3-[2-chlor-5-(4-chlor-benzyloxy)-phenoxymethyl]-thieno[3,2-c]pyridin-7-carbonsäure-(2-hydroxy-ethyl)-amid;
4-Amino-3-{3-[(pyridin-2-carbonyl)-amino]-phenoxymethyl}-thieno[3,2-c]pyridin-7-carbonsäure-ethylester;
4-Amino-3-{3-[(pyridin-2-carbonyl)-amino]-phenoxymethyl}-thieno[3,2-c]pyridin-7-carbonsäure-(2-hydroxy-ethyl)-amid; und
4-Amino-3-{3-[(6-methyl-pyridin-3-carbonyl)-amino]-phenoxymethyl}-thieno[3,2-c]-pyridin-7-carbonsäure-ethylester.

20. Eine Verbindung gemäß Anspruch 1 oder 2, ausgewählt aus der Gruppe bestehend aus
4-Amino-3-{3-[(6-methyl-pyridin-3-carbonyl)-amino]-phenoxymethyl}-thieno[3,2-c]-pyridin-7-carbonsäure-(2-hydroxy-ethyl)-amid;
4-Amino-3-{3-[(pyridin-4-carbonyl)-amino]-phenoxymethyl}-thieno[3,2-c]pyridin-7-carbonsäure-ethylester;
4-Amino-3-{3-[(pyridin-4-carbonyl)-amino]-phenoxymethyl}-thieno[3,2-c]pyridin-7-carbonsäure-(2-hydroxy-ethyl)-amid;
4-Amino-3-[5-(3-chlor-4-fluor-benzoylamino)-2-methyl-phenoxymethyl]-thieno[3,2-c]pyridin-7-carbonsäure-ethylester;
4-Amino-3-[5-(3-chlor-4-benzoylamino)-2-methyl-phenoxymethyl]-thieno[3,2-c]pyridin-7-carbonsäure-ethylester;
4-Amino-3-[5-(3-chlor-benzoylamino)-2-methyl-phenoxymethyl]-thieno[3,2-c]pyridin-7-carbonsäure-(2-hydroxy-ethyl)-amid;
4-Amino-3-{5-[3-chlor-4-(2-hydroxy-ethylamino)-benzoylamino)-2-methyl-phenoxymethyl]-thieno[3,2-c]pyridin-7-carbonsäure-(2-hydroxy-ethyl)-amid;
4-Amino-3-{5-[3-chlor-4-(2-hydroxy-ethylamino)-benzoylamino)-2-methyl-phenoxymethyl]-thieno[3,2-c]pyridin-7-carbonsäure-(2-hydroxy-ethyl)-amid-toluol-4-sulfonsäuresalz;
4-Amino-3-{5-[3-chlor-4-(2-hydroxy-ethylamino)-benzoylamino]-2-methyl-phenoxymethyl}-thieno[3,2-c]pyridin-7-carbonsäure-ethylester; und
4-Amino-3-{5-[3-chlor-4-(2-hydroxy-ethylamino)-benzoylamino]-2-methyl-phenoxymethyl}-thieno[3,2-c]pyridin-7-carbonsäureamid.

21. Eine Verbindung gemäß Anspruch 1 oder 2, ausgewählt aus der Gruppe bestehend aus
4-Amino-3-[3-(2-fluor-benzyloxy)-5-methyl-phenoxymethyl]-thieno[3,2-c]pyridin-7-carbonsäure-(2-hydroxy-ethyl)-amid;
4-Amino-3-[3-(3,5-difluor-benzyloxy)-5-methyl-phenoxymethyl]-thieno[3,2-c]pyridin-7-carbonsäure-(2-hydroxy-ethyl)-amid;
4-Amino-3-[3-methyl-5-(2-methyl-benzyloxy)-phenoxymethyl]-thieno[3,2-c]pyridin-7-carbonsäure-(2-hydroxy-ethyl)-amid;
4-Amino-3-[3-(2,5-difluor-benzyloxy)-phenoxymethyl]-thieno[3,2-c]pyridin-7-carbonsäure-(2-hydroxy-ethyl)-amid;
4-Amino-3-[3-(3-fluor-benzyloxy)-5-methyl-phenoxymethyl]-thieno[3,2-c]pyridin-7-carbonsäure-(2-hydroxy-ethyl)-amid;
4-Amino-3-[3-(2,5-difluor-benzyloxy)-5-methyl-phenoxymethyl]-thieno[3,2-c]pyridin-7-carbonsäure-(2-hydroxy-ethyl)-amid;
4-Amino-3-[3-methyl-5-(4-methyl-benzyloxy)-phenoxymethyl]-thieno[3,2-c]pyridin-7-carbonsäure-(2-hydroxy-ethyl)-amid;
4-Amino-3-[3-(3,4-difluor-benzyloxy)-5-methyl-phenoxymethyl]-thieno[3,2-c]pyridin-7-carbonsäure-(2-hydroxy-ethyl)-amid;
4-Amino-3-[3-(2,3-difluor-benzyloxy)-phenoxymethyl]-thieno[3,2-c]pyridin-7-carbonsäure-(2-hydroxy-ethyl)-amid;
4-Amino-3-[3-(4-chlor-benzoylamino)-phenoxymethyl-]thieno[3,2-c]pyridin-7-carbonsäure-(4-pyrrolidin-1-yl-butyl)-amid-trifluor-essigsäuresalz; und
4-Amino-3-[3-(4-chlor-benzoylamino)-phenoxymethyl]-thieno[3,2-c]pyridin-7-carbonsäure-(4-pyrrolidin-1-yl-butyl)-amid-toluol-4-sulfonsäuresalz.

22. Eine Verbindung gemäß einem der Ansprüche 1 bis 21 zur Verwendung bei der Behandlung von Krebs, insbesondere Kolorektal-, Brust-, Lungen- und Prostatakrebserkrankungen.

23. Eine pharmazeutische Formulierung, umfassend eine Verbindung gemäß einem der Ansprüche 1 bis 21 zusammen mit pharmazeutisch verträglichen Trägern oder Excipienten.

24. Die pharmazeutische Formulierung gemäß Anspruch 23 zur Verwendung bei der Behandlung von Krebs, insbesondere Kolorektal-, Brust-, Lungen- und Prostatakrebserkrankungen.

25. Verwendung einer Verbindung gemäß einem der Ansprüche 1 bis 21 zur Herstellung eines Medikaments zur Behandlung von Krebs, insbesondere Kolorektal-, Brust-, Lungen- und Prostatakrebserkrankungen.

26. Eine Verbindung der Formel (I-A) gemäß Anspruch 1 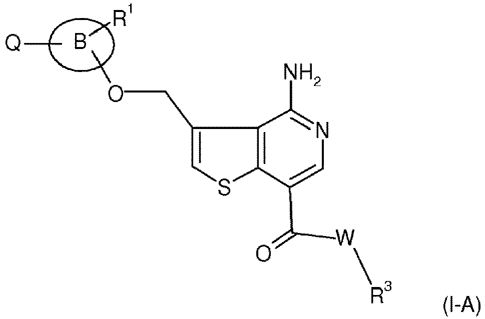 oder pharmazeutisch verträgliche Salze davon
zur Verwendung bei der Behandlung von Bauchspeicheldrüsen-, Magen-, Blasen-, Eierstock-, Melanom-, Neuroblastom-, Gebärmutterhals-, Nieren-, oder renalen Krebserkrankungen, Leukämien oder Lymphomen.

27. Eine pharmazeutische Formulierung, umfassend eine Verbindung, wie in Anspruch 26 definiert, zusammen mit pharmazeutisch verträglichen Trägern oder Excipienten zur Verwendung bei der Behandlung von Bauchspeicheldrüsen-, Magen-, Blasen-, Eierstock-, Melanom-, Neuroblastom-, Gebärmutterhals-, Nieren-, oder renalen Krebserkrankungen, Leukämien oder Lymphomen.

28. Verwendung einer Verbindung, wie in Anspruch 26 definiert, zur Herstellung eines Medikaments zur Behandlung von Bauchspeicheldrüsen-, Magen-, Blasen-, Eierstock-, Melanom-, Neuroblastom-, Gebärmutterhals-, Nieren-, oder renalen Krebserkrankungen, Leukämien oder Lymphomen.

## Revendications

1. Composé de formule (I-A) 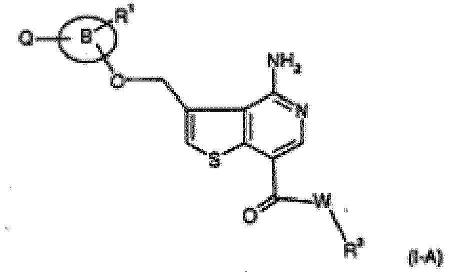 ou un de ses sels pharmaceutiquement acceptables
dans laquelle :
R¹ est choisi dans le groupe constitué par un atome d'hydrogène, des groupes alkyle inférieur, halogéno, cyano, trifluorométhyle, alkoxy inférieur, -C(O)NH-alkyle inférieur, -C(O)-NH-alkoxy inférieur, oxo et NO₂ ;
R³ est choisi dans le groupe constitué par un atome d'hydrogène, des groupes alkyle inférieur, alkyle inférieur substitué par des groupes -OR⁴ et -NR⁴R⁵ ;
R⁴ et R⁵ sont choisis parmi un atome d'hydrogène, des groupes alkyle inférieur, alkyle inférieur substitué avec un groupe OH ou alkoxy inférieur ; ou
R⁴ et R⁵ avec l'atome d'azote auquel ils sont attachés forment un groupe morpholinyle ;
Q est un groupe 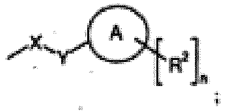
R² peut être identique ou différent parmi des groupes alcényle inférieur, alcynyle inférieur, méthylsulfonyle, sulfonamide, hydrogéno, alkyle inférieur, halogéno, cyano, trifluorométhyle, alkoxy inférieur, NO₂, sulfonylurée, amide, ester, carbamoyle, carbamate, urée, -NR⁴R⁵ et alkyle inférieur substitué avec -OR⁴ ou -NR⁴R⁵ ;
le groupe -X-Y- est choisi parmi les groupes -OCH₂, -CH₂O-, -NHCO-, -CONH-, -O-, -OCH₂CH₂-, -CH₂OCH₂, -CH₂CH₂O-, -CF=CH-, -CH=CF-, -NH-, -NHCH₂-, -CH₂NH-, -SCH₂-, -NHSO₂, -SO₂NH-, -CH₂S-, -SOCH₂-, -CH₂SO-, -SO₂CH₂-, -CH₂SO₂-, -S-, -CH=CH- et alkylène inférieur ;
W est -0- ou un groupe -NH- ;
n vaut 1 ou 2 ;
le noyau A est choisi dans le groupe constitué par des groupes aryle, hétéroaryle, cycloalkyle et hétérocycle ; et
le noyau B est choisi dans le groupe constitué par des groupes aryle et hétéroaryle, ou
Q est un atome d'hydrogène, et
R¹ est un groupe -O-CH₃ ou -C(O)-NH-CH₃ ;
R³ est un groupe éthyle, qui est non substitué ou substitué une fois avec -OH ;
W est -0- ou un groupe -NH- ; et
le noyau B est un groupe phényle ou pyridinyle,
où le terme « alkyle inférieur » désigne un groupe hydrocarboné aliphatique saturé à chaîne linéaire ou ramifiée ayant 1 à 6 atomes de carbone ; le terme « alkoxy inférieur » signifie un groupe -O-alkyle inférieur, où « alkyle inférieur » a la signification donnée ci-dessus ; le terme « alcényle inférieur » désigne un résidu hydrocarboné à chaîne linéaire ou ramifiée comprenant une double liaison oléfinique et jusqu'à 7 atomes de carbone ; le terme « alcynyle inférieur » désigne un résidu hydrocarboné à chaîne linéaire ou ramifiée comprenant une triple liaison et jusqu'à 7 atomes de carbone; ou
un composé de la formule suivante : 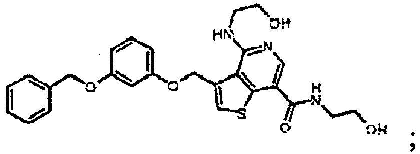 ou 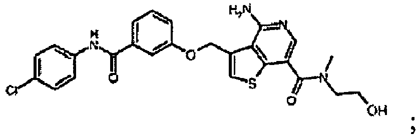 ou 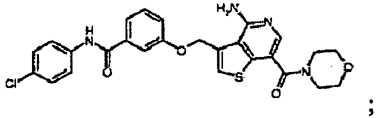 ou 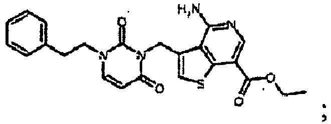 ou 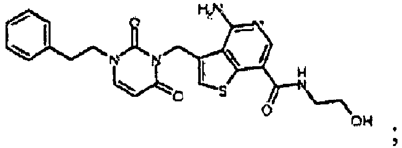 ou 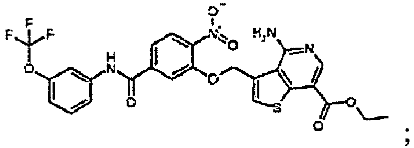 ou 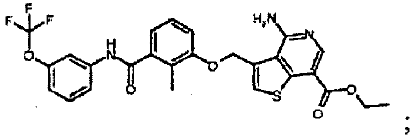 ou 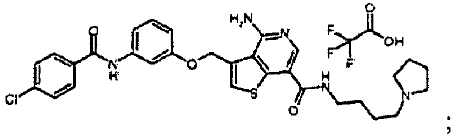 ou 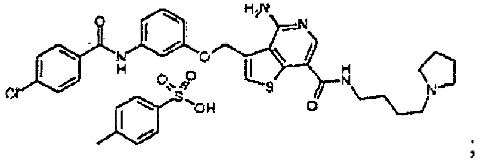 ou

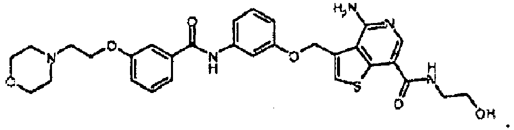

2. Composé selon la revendication 1 ayant la formule (I) 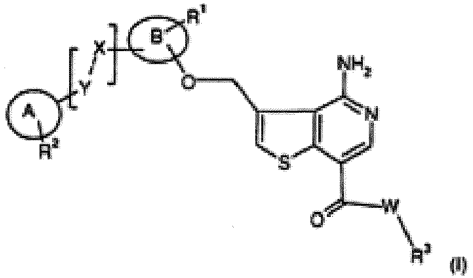 ou un de ses sels pharmaceutiquement acceptables
dans laquelle :
R¹ est choisi dans le groupe constitué par un atome d'hydrogène, des groupes alkyle inférieur, halogéno, cyano, trifluorométhyle, alkoxy inférieur et NO₂ ;
R² est choisi dans le groupe constitué par des groupes alcényle inférieur, alcynyle inférieur, méthylsulfonyle, sulfonamide, hydrogène, alkyle inférieur, halogéno, cyano, trifluorométhyle, alkoxy inférieur, NO₂, sulfonylurée, amide, ester, carbamoyle, carbamate, urée, et alkyle inférieur substitué avec -OR⁴ ou -NR⁴R⁵ ;
R³ est choisi dans le groupe constitué par un atome d'hydrogène, des groupes alkyle inférieur, alkyle inférieur substitué par des groupes -OR⁴ et -NR⁴R⁵ ;
R⁴ et R⁵ sont choisis parmi un atome d'hydrogène, des groupes alkyle inférieur, alkyle inférieur substitué avec un groupe OH ou alkoxy inférieur ;
-X-Y- sont choisis dans le groupe constitué par des groupes -OCH₂, -CH₂O-, -NHCO-, -CONH-, -0-, -OCH₂CH₂-, -CH₂OCH₂, -CH₂CH₂O-, -CF=CH-, -CH=CF-, -NH-, -NHCH₂-, -CH₂NH-, -SCH₂-, -CH₂S-, -SOCH₂-, -CH₂SO-, -SO₂CH₂-, -CH₂SO₂-, -S-, -CH=CH- et alkylène inférieur ;
W est -0- ou un groupe -NH- ;
le noyau A est choisi dans le groupe constitué par des groupes aryle, hétéroaryle, cycloalkyle et hétérocycle ; et
le noyau B est choisi dans le groupe constitué par des groupes aryle et hétéroaryle.

3. Composé selon la revendication 1, dans lequel :
le noyau A est choisi parmi les groupes pyrimidinyle, pyridinyle, pipéridinyle, cyclopropyle, cyclopentyle, cyclohexyle, tétrahydro-pyranyle, phényle, pyrazolyle ou thiophényle ;
et
le noyau B est un groupe pyrimidinyle, 2-oxo-pyrimidinyle ou phényle.

4. Composé selon la revendication 1, dans lequel les deux noyaux A et B sont un groupe phényle.

5. Composé selon la revendication 1 ou 2, dans lequel le groupe -X-Y- est choisi parmi les groupes -OCH₂, -CH₂O-, -NHCO- et -CONH-.

6. Composé selon la revendication 5, dans lequel R¹ est choisi dans le groupe constitué par les groupes -CH₃, -Cl et -F.

7. Composé selon la revendication 6, dans lequel R² est choisi dans le groupe constitué par les groupes -Cl, -F, -CF₃, -CONH₂, alkoxy, NR⁴R⁵ et alkyle inférieur.

8. Composé selon la revendication 1 ou 2, choisi dans le groupe constitué par :
l'ester éthylique d'acide 4-amino-3-[3-(4-chloro-3-trifluorométhyl-phénylcarbamoyl)-phénoxyméthyl]-thiéno[3,2-c]pyridine-7-carboxylique ;
l'ester éthylique d'acide 4-amino-3-(3-benzyloxy-phénoxyméthyl)-thiéno[3,2-c]pyridine-7-carboxylique ;
le (2-hydroxy-éthyl)-amide d'acide 4-amino-3-(3-benzyloxy-phénoxyméthyl)-thiéno[3,2-c]pyridine-7-carboxylique ;
l'ester éthylique d'acide 4-amino-3-[3-(4-chloro-benzyloxy)-phénoxyméthyl]-thiéno[3,2-c]pyridine-7-carboxylique ;
le (2-hydroxy-éthyl)-amide d'acide 4-amino-3-[3-(4-chloro-benzyloxy)-phénoxyméthyl]-thiéno[3,2-c]pyridine-7-carboxylique ;
l'ester éthylique d'acide 4-amino-3-[3-(3-chloro-benzyloxy)-phénoxyméthyl]-thiéno[3,2-c]pyridine-7-carboxylique ;
le (2-hydroxy-éthyl)-amide d'acide 4-amino-3-[3-(3-chloro-benzyloxy)-phénoxyméthyl]-thiéno[3,2-c]pyridine-7-carboxylique ;
l'ester éthylique d'acide 4-amino-3-[3-(3-trifluoro-méthyl-benzyloxy)-phénoxyméthyl]-thiéno[3,2-c]pyridine-7-carboxylique ;
le (2-hydroxy-éthyl)-amide d'acide 4-amino-3-[3-(3-trifluorométhyl-benzyloxy)-phénoxyméthyl]-thiéno[3,2-c]-pyridine-7-carboxylique ; et
l'ester éthylique d'acide 4-amino-3-(3-benzylsulfanyl-phénoxyméthyl)-thiéno[3,2-c]pyridine-7-carboxylique.

9. Composé selon la revendication 1 ou 2, choisi dans le groupe constitué par :
le (2-hydroxy-éthyl)-amide d'acide 4-amino-3-(3-benzylsulfanyl-phénoxyméthyl)-thiéno[3,2-c]pyridine-7-carboxylique ;
l'ester éthylique d'acide 4-amino-3-(3-phénylméthane-sulfonyl-phénoxyméthyl)-thiéno[3,2-c]pyridine-7-carboxylique ;
le (2-hydroxy-éthyl)-amide d'acide 4-amino-3-(3-phénylméthanesulfonyl-phénoxyméthyl)-thiéno[3,2-c]pyridine-7-carboxylique ;
l'ester éthylique d'acide 4-amino-3-[3-(3-méthoxy-benzyloxy)-phénoxyméthyl]-thiéno[3,2-c]pyridine-7-carboxylique ;
le (2-hydroxy-éthyl)-amide d'acide 4-amino-3-[3-(3-méthoxy-benzyloxy)-phénoxyméthyl]-thiéno[3,2-c]pyridine-7-carboxylique
l'ester éthylique d'acide 4-amino-3-[3-(4-méthoxy-benzyloxy)-phénoxyméthyl]-thiéno[3,2-c]pyridine-7-carboxylique ;
le (2-hydroxy-éthyl)-amide d'acide 4-amino-3-[3-(4-méthoxy-benzyloxy)-phénoxyméthyl]-thiéno[3,2-c]pyridine-7-carboxylique
l'ester éthylique d'acide 4-amino-3-[3-(4-méthoxy-3-trifluorométhyl-benzyloxy)-phénoxyméthyl]-thiéno[3,2-c]-pyridine-7-carboxylique ;
le (2-hydroxy-éthyl)-amide d'acide 4-amino-3-[3-(4-méthoxy-3-trifluorométhyl-benzyloxy)-phénoxyméthyl]-thiéno-[3,2-c]pyridine-7-carboxylique ; et
l'ester éthylique d'acide 4-amino-3-(3-phénoxy-phénoxyméthyl)-thiéno[3,2-c]pyridine-7-carboxylique.

10. Composé selon la revendication 1 ou 2, choisi dans le groupe constitué par :
le (2-hydroxy-éthyl)-amide d'acide 4-amino-3-(3-phényl-amino-phénoxyméthyl)-thiéno[3,2-c]pyridine-7-carboxylique ;
l'ester éthylique d'acide 4-amino-3-(3-benzylamino-phénoxyméthyl)-thiéno[3,2-c]pyridine-7-carboxylique ;
le (2-hydroxy-éthyl)-amide d'acide 4-amino-3-(3-benzyl-amino-phénoxyméthyl)-thiéno[3,2-c]pyridine-7-carboxylique ;
l'ester éthylique d'acide 4-amino-3-[3-(4-cyano-benzyl-oxy)-phénoxyméthyl]-thiéno[3,2-c]pyridine-7-carboxylique ;
l'acide 4-amino-3-[3-(4-cyano-benzyloxy)-phénoxyméthyl]-thiéno[3,2-c]pyridine-7-carboxylique ;
le (2-hydroxy-éthyl)-amide d'acide 4-amino-3-[3-(4-cyano-benzyloxy)-phénoxyméthyl]-thiéno[3,2-c]pyridine-7-carboxylique ;
l'ester éthylique d'acide 4-amino-3-[3-(3-cyano-benzyl-oxy)-phénoxyméthyl]-thiéno[3,2-c]pyridine-7-carboxylique ;
l'acide 4-amino-3-[3-(3-cyano-benzyloxy)-phénoxyméthyl]-thiéno[3,2-c]pyridine-7-carboxylique ;
le sel d'acide trifluoroacétique de (2-hydroxy-éthyl)-amide d'acide 4-amino-3-[3-(3-cyano-benzyloxy)-phénoxyméthyl]-thiéno[3,2-c]pyridine-7-carboxylique ;
le (2-hydroxy-éthyl)-amide d'acide 3-(3-benzyloxy-phénoxyméthyl)-4-(2-hydroxy-éthylamino) -thiéno [3,2-c]pyridine-7-carboxylique ; et
l'ester éthylique d'acide 4-amino-3-(3-méthoxy-phénoxyméthyl)-thiéno[3,2-c]pyridine-7-carboxylique.

11. Composé selon la revendication 1 ou 2, choisi dans le groupe constitué par :
le (2-hydroxy-éthyl)-amide d'acide 4-amino-3-(3-méthoxy-phénoxyméthyl)-thiéno[3,2-c]pyridine-7-carboxylique ;
l'ester éthylique d'acide 4-amino-3-(3-benzoylamino-phénoxyméthyl)-thiéno[3,2-c]pyridine-7-carboxylique ;
le (2-hydroxy-éthyl)-amide d'acide 4-amino-3-(3-benzoyl-amino-phénoxyméthyl)-thiéno[3,2-c]pyridine-7-carboxylique ;
le chlorhydrate de (2-hydroxy-éthyl)-amide d'acide 4-amino-3-(3-benzoylamino-phénoxyméthyl)-thiéno[3,2-c]-pyridine-7-carboxylique ;
le sel d'acide toluène-4-sulfonique de (2-hydroxy-éthyl)-amide d'acide 4-amino-3-(3-benzoylamino-phénoxyméthyl)-thiéno[3,2-c]pyridine-7-carboxylique ;
l'ester éthylique d'acide 4-amino-3-[3-(4-chloro-phénylcarbamoyl)-phénoxyméthyl]-thiéno[3,2-c]pyridine-7-carboxylique ;
le (2-hydroxy-éthyl)-amide d'acide 4-amino-3-[3-(4-chloro-phénylcarbamoyl)-phénoxyméthyl]-thiéno[3,2-c]-pyridine-7-carboxylique ;
l'ester éthylique d'acide 4-amino-3-[3-(4-fluoro-phénylcarbamoyl)-phénoxyméthyl]-thiéno[3,2-c]pyridine-7-carboxylique ;
le (2-hydroxy-éthyl)-amide d'acide 4-amino-3-[3-(4-fluoro-phénylcarbamoyl)-phénoxyméthyl]-thiéno[3,2-c]pyridine-7-carboxylique ;
le sel d'acide trifluoro-acétique d'acide 4-amino-3-[3-(4-chloro-benzyloxy)-phénoxyméthyl]-thiéno [3,2-c]pyridine-7-carboxylique ; et
l'ester éthylique d'acide 4-amino-3-[3-(3-chloro-phénylcarbamoyl)-phénoxyméthyl]-thiéno[3,2-c]pyridine-7-carboxylique.

12. Composé selon la revendication 1 ou 2, choisi dans le groupe constitué par :
le (2-hydroxy-éthyl)-amide d'acide 4-amino-3-[3-(3-chloro-phénylcarbamoyl)-phénoxyméthyl]-thiéno[3,2-c]pyridine-7-carboxylique ;
le (3-hydroxy-propyl)-amide d'acide 4-amino-3-[3-(4-chloro-phénylcarbamoyl)-phénoxyméthyl]-thiéno[3,2-c]pyridine-7-carboxylique ;
le propylamide d'acide 4-amino-3-[3-(4-chloro-phénylcarbamoyl)-phénoxyméthyl]-thiéno[3,2-c]pyridine-7-carboxylique ;
l'éthylamide d'acide 4-amino-3-[3-(4-chloro-phényl-carbamoyl)-phénoxyméthyl]-thiéno[3,2-c]pyridine-7-carboxylique ;
l'ester éthylique d'acide 4-amino-3-{3-[(4-chloro-phényl)-méthyl-carbamoyl]-phénoxyméthyl}-thiéno[3,2-c]pyridine-7-carboxylique ;
l'ester éthylique d'acide 4-amino-3-[3-(1-méthyl-pipéridine-4-ylcarbamoyl)-phénoxyméthyl]-thiéno[3,2-c]pyridine-7-carboxylique ;
l'ester éthylique d'acide 4-amino-3-[3-(5-méthyl-pyridine-2-ylcarbamoyl)-phénoxyméthyl]-thiéno[3,2-c]pyridine-7-carboxylique ;
l'ester éthylique d'acide 4-amino-3-[3-(1-méthanesulfonyl-pipéridine-4-ylcarbamoyl)-phénoxyméthyl]-thiéno[3,2-c]pyridine-7-carboxylique ;
le (2-hydroxy-éthyl)-amide d'acide 4-amino-3-{3-[(4-chlorophényl)-méthyl-carbamoyl]-phénoxyméthyl}-thiéno[3,2-c]-pyridine-7-carboxylique ;
l'ester éthylique d'acide 4-amino-3-[5-(3-carbamoyl-phénylcarbamoyl)-2-méthyl-phénoxyméthyl]-thiéno-[3,2-c]-pyridine-7-carboxylique ;
l'ester éthylique d'acide 4-amino-3-(2-méthyl-5-phénylcarbamoyl-phénoxyméthyl)-thiéno[3,2-c]pyridine-7-carboxylique ; et
le sel d'acide trifluoro-acétique de (3-hydroy-propyl)-amide d'acide 4-amino-3-[3-(4-chloro-benzyloxy)-phénoxyméthyl]-thiéno[3,2-c]pyridine-7-carboxylique.

13. Composé selon la revendication 1 ou 2, choisi dans le groupe constitué par :
l'ester éthylique d'acide 4-amino-3-[3-(4-chloro-phénoxyméthyl)-phénoxyméthyl]-thiéno[3,2-c]pyridine-7-carboxylique ;
l'ester éthylique d'acide 4-amino-3-{3-[2-(4-chloro-phényl)-vinyl]-phénoxyméthyl}-thiéno[3,2-c]pyridine-7-carboxylique ;
le (2-hydroxy-éthyl)-amide d'acide 4-amino-3-{3-[2-(4-chloro-phényl)-vinyl]-phénoxyméthyl}-thiéno[3,2-c]pyridine-7-carboxylique ;
le (2-hydroxy-éthyl)-amide d'acide 4-amino-3-[3-(4-chloro-phénoxyméthyl)-phénoxyméthyl]-thiéno[3,2-c]pyridine-7-carboxylique ;
le (2-hydroxy-éthyl)-amide d'acide 4-amino-3-[3-(4-chloro-phénylcarbamoyl)-phénoxyméthyl]-thiéno[3,2-c]pyridine-7-carboxylique ;
le (2-méthoxy-éthyl)-amide d'acide 4-amino-3-[3-(4-chloro-phénylcarbamoyl)-phénoxyméthyl]-thiéno[3,2-c]pyridine-7-carboxylique ;
le méthylamide d'acide 4-amino-3-[3-(4-chloro-phénylcarbamoyl)-phénoxyméthyl]-thiéno[3,2-c]pyridine-7-carboxylique ;
le 3-[4-amino-7-(morpholine-4-carbonyl)-thiéno[3,2-c]-pyridine-3-ylméthoxy]-N-(4-chlorophényl)-benzamide ;
l'ester éthylique d'acide 4-amino-3-(3-cyclohexyl-carbamoyl-phénoxyméthyl)-thiéno[3,2-c]pyridine-7-carboxylique ;
l'ester éthylique d'acide 4-amino-3-[3-(tétrahydro-pyrane-4-ylcarbamoyl)-phénoxyméthyl]-thiéno[3,2-c]pyridine-7-carboxylique ; et
le (2-hydroxy-éthyl)-amide d'acide 4-amino-3-[3-(1-méthanesulfonyl-pipéridine-4-ylcarbamoyl)-phénoxyméthyl]-thiéno[3,2-c]pyridine-7-carboxylique.

14. Composé selon la revendication 1 ou 2, choisi dans le groupe constitué par :
le (2-hydroxy-éthyl)-amide d'acide 4-amino-3-[3-(1-méthyl-pipéridine-4-ylcarbamoyl)-phénoxyméthyl]-thiéno[3,2-c]pyridine-7-carboxylique ;
le sel d'acide trifluoro-acétique de (2-hydroxy-éthyl)-amide d'acide 4-amino-3-(3-phénylaminométhyl-phénoxyméthyl)-thiéno[3,2-c]pyridine-7-carboxylique ;
l'ester éthylique d'acide 4-amino-3-{3-[2-(4-chloro-phényl)-éthoxy]-phénoxyméthyl}-thiéno[3,2-c]pyridine-7-carboxylique ;
le (2-hydroxy-éthyl)-amide d'acide 4-amino-3-{3-[2-(4-chloro-phényl)-éthoxy]-phénoxyméthyl}-thiéno[3,2-c]pyridine-7-carboxylique ;
l'ester éthylique d'acide 4-amino-3-[3-(4-chloro-benzoylamino)-phénoxyméthyl]-thiéno[3,2-c]pyridine-7-carboxylique ;
le (2-hydroxy-éthyl)-amide d'acide 4-amino-3-[3-(4-chloro-benzoylamino)-phénoxyméthyl]-thiéno[3,2-c]pyridine-7-carboxylique ;
le (3-hydroxy-propyl)-amide d'acide 4-amino-3-[3-(4-chloro-benzoylamino)-phénoxyméthyl]-thiéno[3,2-c]pyridine-7-carboxylique ;
le chlorhydrate de (3-hydroxy-propyl)-amide d'acide 4-amino-3-[3-(4-chloro-benzoylamino)-phénoxyméthyl]-thiéno-[3,2-c]pyridine-7-carboxylique ;
le sel d'acide toluène-4-sulfonique de (3-hydroxy-propyl)-amide d'acide 4-amino-3-[3-(4-chloro-benzoylamino)-phénoxyméthyl]-thiéno[3,2-c]pyridine-7-carboxylique ;
l'ester éthylique d'acide 4-amino-3-(2-oxo-1-phénéthyl-1,2-dihydro-pyrimidine-4-yloxyméthyl)-thiéno[3,2-c]pyridine-7-carboxylique ; et
l'ester éthylique d'acide 4-amino-3-(2,6-dioxo-3-phénéthyl-3,6-dihydro-2H-pyrimidine-1-ylméthyl)-thiéno[3,2-c]pyridine-7-carboxylique.

15. Composé selon la revendication 1 ou 2, choisi dans le groupe constitué par :
le (2-hydroxy-éthyl)-amide d'acide 4-amino-3-(2,6-dioxo-3-phénéthyl-3,6-dihydro-2H-pyrimidine-1-ylméthyl)-thiéno[3,2-c]pyridine-7-carboxylique ;
l'ester éthylique d'acide 4-amino-3-[2-méthoxy-5-(3-méthoxy-phénylcarbamoyl)-phénoxyméthyl]-thiéno[3,2-c]pyridine-7-carboxylique ;
l'ester éthylique d'acide 4-amino-3-[5-(3,5-diméthoxy-phénylcarbamoyl)-2-méthoxy-phénoxyméthyl]-thiéno[3,2-c]-pyridine-7-carboxylique ;
l'ester éthylique d'acide 4-amino-3-[5-(4-chloro-3-méthyl-phénylcarbamoyl)-2-méthoxy-phénoxyméthyl]-thiéno-[3,2-c]pyridine-7-carboxylique ;
l'ester éthylique d'acide 4-amino-3-(5-benzyl-carbamoyl-2-nitro-phénoxyméthyl)-thiéno[3,2-c]pyridine-7-carboxylique ;
l'ester éthylique d'acide 4-amino-3-(2-méthyl-3-phénylcarbamoyl-phénoxyméthyl]-thiéno[3,2-c]pyridine-7-carboxylique ;
l'ester éthylique d'acide 4-amino-3-[3-(3,5-diméthoxy-phénylcarbamoyl)-2-méthyl-phénoxyméthyl]-thiéno[3,2-c]-pyridine-7-carboxylique ;
l'ester éthylique d'acide 4-amino-3-[2-nitro-5-(3-trifluorométhoxy-phénylcarbamoyl)-phénoxyméthyl]-thiéno-[3,2-c]pyridine-7-carboxylique ;
l'ester éthylique d'acide 4-amino-3-[2-méthyl-3-(3-trifluorométhoxy-phénylcarbamoyl)-phénoxyméthyl]-thiéno-[3,2-c]pyridine-7-carboxylique ;
l'ester éthylique d'acide 4-amino-3-[3-(3-méthane-sulfonyl-phénylcarbamoyl)-2-méthyl-phénoxyméthyl]-thiéno-[3,2-c]pyridine-7-carboxylique ;
l'ester éthylique d'acide 4-amino-3-[3-(3-chloro-phénylcarbamoyl)-2-méthyl-phénoxyméthyl]-thiéno[3,2-c]-pyridine-7-carboxylique ; et
l'ester éthylique d'acide 4-amino-3-[2-méthyl-3-(5-méthyl-1H-pyrazole-3-ylcarbamoyl)-phénoxyméthyl]-thiéno-[3,2-c]pyridine-7-carboxylique.

16. Composé selon la revendication 1 ou 2, choisi dans le groupe constitué par :
l'ester éthylique d'acide 4-amino-3-[3-(3,5-difluoro-benzyloxy)-5-méthyl-phénoxyméthyl]-thiéno[3,2-c]pyridine-7-carboxylique ;
l'ester éthylique d'acide 4-amino-3-[3-(3,4-difluoro-benzyloxy)-5-méthyl-phénoxyméthyl]-thiéno[3,2-c]pyridine-7-carboxylique ;
l'ester éthylique d'acide 4-amino-3-[3-(5-chloro-thiophène-2-ylméthoxy)-5-méthyl-phénoxyméthyl]-thiéno[3,2-c]pyridine-7-carboxylique ;
l'ester éthylique d'acide 4-amino-3-[3-(3-méthyl-benzyloxy)-phénoxyméthyl]-thiéno[3,2-c]pyridine-7-carboxylique ;
l'ester éthylique d'acide 4-amino-3-[3-(5-chloro-thiophène-2-ylméthoxy)-phénoxyméthyl]-thiéno[3,2-c]pyridine-7-carboxylique ;
l'ester éthylique d'acide 4-amino-3-[3-(2,5-difluoro-benzyloxy)-phénoxyméthyl]-thiéno[3,2-c]pyridine-7-carboxylique ;
l'ester éthylique d'acide 4-amino-3-[3-(4-fluoro-benzyloxy)-5-méthyl-phénoxyméthyl]-thiéno[3,2-c]pyridine-7-carboxylique ; et
l'ester éthylique d'acide 4-amino-3-[3-(2,4-difluoro-benzyloxy)-5-méthyl-phénoxyméthyl]-thiéno[3,2-c]pyridine-7-carboxylique.

17. Composé selon la revendication 1 ou 2, choisi dans le groupe constitué par :
l'ester éthylique d'acide 4-amino-3-[3-(2,5-difluoro-benzyloxy)-5-méthyl-phénoxyméthyl]-thiéno[3,2-c]pyridine-7-carboxylique ;
l'ester éthylique d'acide 4-amino-3-[3-méthyl-5-(4-méthyl-benzyloxy)-phénoxyméthyl]-thiéno[3,2-c]pyridine-7-carboxylique ;
l'ester éthylique d'acide 4-amino-3-[3-méthyl-5-(2-méthyl-benzyloxy)-phénoxyméthyl]-thiéno[3,2-c]pyridine-7-carboxylique ;
l'ester éthylique d'acide 4-amino-3-[3-(2-fluoro-benzyloxy)-5-méthyl-phénoxyméthyl]-thiéno[3,2-c]pyridine-7-carboxylique ;
l'ester éthylique d'acide 4-amino-3-[3-(2-méthoxy-benzyloxy)-phénoxyméthyl]-thiéno[3,2-c]pyridine-7-carboxylique ;
le (2-hydroxy-éthyl)-amide d'acide 4-amino-3-{3-[3-(2-morpholine-4yl-éthoxy)-benzoylamino]-phénoxyméthyl}thiéno-[3,2-c]pyridine-7-carboxylique ;
l'ester éthylique d'acide 4-amino-3-(3-méthyl-carbamoyl-phénoxyméthyl)-thiéno[3,2-c]pyridine-7-carboxylique ;
l'ester éthylique d'acide 4-amino-3-(3-cyclopropyl-carbamoyl-phénoxyméthyl)-thiéno[3,2-c]pyridine-7-carboxylique ;
l'ester éthylique d'acide 4-amino-3-(3-cyclopentyl-carbamoyl-phénoxyméthyl)-thiéno[3,2-c]pyridine-7-carboxylique ; et
le (2-hydroxy-éthyl)-amide d'acide 4-amino-3-(3-cyclohexylcarbamoyl-phénoxyméthyl)-thiéno[3,2-c]pyridine-7-carboxylique.

18. Composé selon la revendication 1 ou 2, choisi dans le groupe constitué par :
le (2-hydroxy-éthyl)-amide d'acide 4-amino-3-[3-(tétrahydro-pyrane-4-ylcarbamoyl)-phénoxyméthyl]-thiéno-[3,2-c]pyridine-7-carboxylique ;
le (2-hydroxy-éthyl)-amide d'acide 4-amino-3-(3-méthylcarbamoyl-phénoxyméthyl)-thiéno[3,2-c]pyridine-7-carboxylique ;
le (2-hydroxy-éthyl)-amide d'acide 4-amino-3-(3-cyclopropylcarbamoyl-phénoxyméthyl)-thiéno[3,2-c]pyridine-7-carboxylique ;
le (2-hydroxy-éthyl)-amide d'acide 4-amino-3-(3-cyclopentylcarbamoyl-phénoxyméthyl)-thiéno[3,2-clpyridine-7-carboxylique ;
l'ester éthylique d'acide 4-amino-3-(5-méthyl-carbamoyl-pyridine-3-yloxyméthyl)-thiéno[3,2-c]pyridine-7-carboxylique ; et
le (2-hydroxy-éthyl)-amide d'acide 4-amino-3-(5-méthylcarbamoyl-pyridine-3-yloxyméthyl)-thiéno[3,2-c]pyridine-7-carboxylique.

19. Composé selon la revendication 1 ou 2, choisi dans le groupe constitué par :
l'acide 4-amino-3-[3-(4-chloro-benzoylamino)-phénoxyméthyl]-thiéno[3,2-c]pyridine-7-carboxylique ;
l'ester éthylique d'acide 4-amino-3-[3-(pyrimidine-5-ylméthoxy)-phénoxyméthyl]-thiéno[3,2-c]pyridine-7-carboxylique ;
le (2-hydroxy-éthyl)-amide d'acide 4-amino-3-[3-(pyrimidine-5-ylméthoxy)-phénoxyméthyl]-thiéno[3,2-c]pyridine-7-carboxylique ;
l'ester éthylique d'acide 4-amino-3-[3-(4-chloro-benzènesulfonylamino)-phénoxyméthyl]-thiéno[3,2-c]pyridine-7-carboxylique ;
le (2-hydroxy-éthyl)-amide d'acide 4-amino-3-[3-(4-chloro-benzènesulfonylamino)-phénoxyméthyl]-thiéno[3,2-c]-pyridine-7-carboxylique ;
l'ester éthylique d'acide 4-amino-3-[2-chloro-5-(4-chloro-benzyloxy)-phénoxyméthyl]-thiéno[3,2-c]pyridine-7-carboxylique ;
le (2-hydroxy-éthyl)-amide d'acide 4-amino-3-[2-chloro-5-(4-chloro-benzyloxy)-phénoxyméhyl]-thiéno[3,2-c]-pyridine-7-carboxylique ;
l'ester éthylique d'acide 4-amino-3-{3-[(pyridine-2-carbonyl)-amino]-phénoxyméthyl}-thiéno[3,2-c]pyridine-7-carboxylique ;
le (2-hydroxy-éthyl)-amide d'acide 4-amino-3-{3-[(pyridine-2-carbonyl)-amino]-phénoxyméthyl}-thiéno[3,2-c]-pyridine-7-carboxylique ; et
l'ester éthylique d'acide 4-amino-3-{3-[(6-méthyl-pyridine-3-carbonyl)-amino]-phénoxyméthyl}-thiéno[3,2-c]-pyridine-7-carboxylique.

20. Composé selon la revendication 1 ou 2, choisi dans le groupe constitué par :
le (2-hydroxy-éthyl)-amide d'acide 4-amino-3-{3-[(6-méthyl-pyridine-3-carbonyl)-amino]-phénoxyméthyl}-thiéno-[3,2-c]pyridine-7-carboxylique ;
l'ester éthylique d'acide 4-amino-3-{3-[(pyridine-4-carbonyl)-amino]-phénoxyméthyl}-thiéno[3,2-c]pyridine-7-carboxylique ;
le (2-hydroxy-éthyl)-amide d'acide 4-amino-3-{3-[(pyridine-4-carbonyl)-amino]-phénoxyméthyl}-thiéno[3,2-c]-pyridine-7-carboxylique ;
l'ester éthylique d'acide 4-amino-3-[5-(3-chloro-4-fluoro-benzoylamino)-2-méthyl-phénoxyméthyl]-thiéno[3,2-c]-pyridine-7-carboxylique ;
l'ester éthylique d'acide 4-amino-3-[5-(3-chloro-4-benzoylamino)-2-méthyl-phénoxyméthyl]-thiéno[3,2-c]pyridine-7-carboxylique ;
le (2-hydroxy-éthyl)-amide d'acide 4-amino-3-[5-(3-chloro-benzoylamino)-2-méthyl-phénoxyméthyl]-thiéno[3,2-c]-pyridine-7-carboxylique ;
le (2-hydroxy-éthyl)-amide d'acide 4-amino-3-{5-[3-chloro-4-(2-hydroxy-éthylamino)-benzoylamino]-2-méthyl-phénoxyméthyl}-thiéno[3,2-c]pyridine-7-carboxylique ;
le sel d'acide toluène-4-sulfonique de (2-hydroxy-éthyl)-amide d'acide 4-amino-3-{5-[3-chloro-4-(2-hydroxy-éthylamino)-benzoylamino]-2-méthyl-phénoxyméthyl}-thiéno-[3,2-c]pyridine-7-carboxylique ;
l'ester éthylique d'acide 4-amino-3-{5-[3-chloro-4-(2-hydroxy-éthylamino)-benzoylamino]-2-méthyl-phénoxyméthyl}-thiéno[3,2-c]pyridine-7-carboxylique ; et
l'amide d'acide 4-amino-3-{5-[3-chloro-4-(2-hydroxy-éthylamino)-benzoylamino]-2-méthyl-phénoxyméthyl}-thiéno-[3,2-c]pyridine-7-carboxylique.

21. Composé selon la revendication 1 ou 2, choisi dans le groupe constitué par :
le (2-hydroxy-éthyl)-amide d'acide 4-amino-3-[3-(2-fluoro-benzyloxy)-5-méthyl-phénoxyméthyl]-thiéno[3,2-c]-pyridine-7-carboxylique ;
le (2-hydroxy-éthyl)-amide d'acide 4-amino-3-[3-(3,5-difluoro-benzyloxy)-5-méthyl-phénoxyméthyl]-thiéno[3,2-c]-pyridine-7-carboxylique ;
le (2-hydroxy-éthyl)-amide d'acide 4-amino-3-[3-méthyl-5-(2-méthyl-benzyloxy)-phénoxyméthyl]-thiéno[3,2-c]pyridine-7-carboxylique ;
le (2-hydroxy-éthyl)-amide d'acide 4-amino-3-[3-(2,5-difluoro-benzyloxy)-phénoxyméthyl]-thiéno[3,2-c]-pyridine-7-carboxylique ;
le (2-hydroxy-éthyl)-amide d'acide 4-amino-3-[3-(3-fluoro-benzyloxy)-5-méthyl-phénoxyméthyl]-thiéno[3,2-c]-pyridine-7-carboxylique ;
le (2-hydroxy-éthyl)-amide d'acide 4-amino-3-[3-(2,5-difluoro-benzyloxy)-5-méthyl-phénoxyméthyl]-thiéno[3,2-c]-pyridine-7-carboxylique ;
le (2-hydroxy-éthyl)-amide d'acide 4-amino-3-[3-méthyl-5-(4-méthyl-benzyloxy)-phénoxyméthyl]-thiéno[3,2-c]-pyridine-7-carboxylique ;
le (2-hydroxy-éthyl)-amide d'acide 4-amino-3-[3-(3,4-difluoro-benzyloxy)-5-méthyl-phénoxyméthyl]-thiéno[3,2-c]-pyridine-7-carboxylique ;
le (2-hydroxy-éthyl)-amide d'acide 4-amino-3-[3-(2,3-difluoro-benzyloxy)-phénoxyméthyl]-thiéno[3,2-c]pyridine-7-carboxylique ;
le sel d'acide trifluoro-acétique de (4-pyrrolidine-1-yl-butyl)-amide d'acide 4-amino-3-[3-(4-chloro-benzoyl-amino)-phénoxyméthyl]-thiéno[3,2-c]pyridine-7-carboxylique ; et
le sel d'acide toluène-4-sulfonique de (4-pyrrolidine-1-yl-butyl)-amide d'acide 4-amino-3-[3-(4-chloro-benzoyl-amino)-phénoxyméthyl]-thiéno[3,2-c]pyridine-7-carboxylique.

22. Composé selon l'une quelconque des revendications 1 à 21, pour une utilisation dans le traitement du cancer, en particulier des cancers colorectal, du sein, du poumon et de la prostate.

23. Formulation pharmaceutique comprenant un composé selon l'une quelconque des revendications 1 à 21, conjointement avec des véhicules ou excipients pharmaceutiquement acceptables.

24. Formulation pharmaceutique selon la revendication 23, pour une utilisation dans le traitement du cancer, en particulier des cancers colorectal, du sein, du poumon et de la prostate.

25. Utilisation d'un composé selon l'une quelconque des revendications 1 à 21, pour la fabrication d'un médicament pour le traitement du cancer, en particulier des cancers colorectal, du sein, du poumon et de la prostate.

26. Composé de formule (I-A) selon la revendication 1 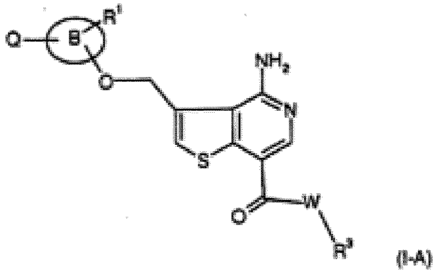 ou un de ses sels pharmaceutiquement acceptables
pour une utilisation dans le traitement de cancers pancréatique, gastrique, de la vessie, des ovaires, d'un mélanome, d'un neuroblastome, du col de l'utérus, du rein ou rénal, de leucémies ou de lymphomes.

27. Composition pharmaceutique comprenant un composé selon la revendication 26, conjointement avec des véhicules ou excipients pharmaceutiquement acceptables pour une utilisation dans le traitement de cancers pancréatique, gastrique, de la vessie, des ovaires, d'un mélanome, d'un neuroblastome, du col de l'utérus, du rein ou rénal, de leucémies ou de lymphomes.

28. Utilisation d'un composé selon la revendication 26, pour la fabrication d'un médicament pour le traitement de cancers pancréatique, gastrique, de la vessie, des ovaires, d'un mélanome, d'un neuroblastome, du col de l'utérus, du rein ou rénal, de leucémies ou de lymphomes.
